# EUROPEAN PATENT SPECIFICATION

(11) **EP 3 313 999 B1**
(45) Date of publication and mention of the grant of the patent: **28.04.2021**
(21) Application number: 16735600.5
(22) Date of filing: 24.06.2016
(51) Int. Cl.: C12P 7/22, C12P 7/66, C12N 9/02, C02F 3/00, C11D 3/386, D06M 16/00, D21H 17/00

(54) **POLYPEPTIDES HAVING DEMETHYLATING ACTIVITY**
POLYPEPTIDE MIT DEMETHYLIERENDER AKTIVITÄT
POLYPEPTIDES AYANT UNE ACTIVITÉ DE DÉMÉTHYLATION

(30) Priority: 24.06.2015 NL 2015020
(43) Date of publication of application: 02.05.2018
(73) Proprietor: Genencor International B.V., 2333 CN Leiden (NL)
(72) Inventor: KOETSIER, Martijn Johannes, 6708 MP Wageningen (NL); VISSER, Jacob, 6703 CK Wageningen (NL); IANCU, Svetlana Laura, 6708 TN Wageningen (NL); KABEL, Mirjam Anna, 6703 AS Wageningen (NL); FROMMHAGEN, Matthias, Rhenen 3911 Mg (NL); GRUPPEN, Harm, 6708 NA Wageningen (NL); LANGE, Heiko, 00141 Roma (IT); CRESTINI, Claudia, 00177 Roma (IT); BENJELLOUN-MLAYAH, Bouchra, 31450 Pompertuzat (FR)
(74) Representative: Mewburn Ellis LLP
(86) International application number: PCT/EP2016/064666
(87) International publication number: WO 2016/207351

(56) References cited:
- WO-A1-2008/134259
- WO-A1-2014/081700
- WO-A1-2014/186567
- WO-A1-2015/035029
- WO-A2-2012/068236
- RAHOUTI M ET AL: "METABOLISM OF FERULIC ACID BY PAECILOMYCES-VARIOTI AND PESTALOTIA-PALMARUM", APPLIED AND ENVIRONMENTAL MICROBIOLOGY, vol. 55, no. 9, 1989, pages 2391-2398, XP002755557, ISSN: 0099-2240

## Description

### CROSS-REFERENCE TO RELATED APPLICATIONS

This application claims the benefit of priority to Netherlands Patent Application No. NL2015020, filed on 24 June 2015.

### FIELD OF THE INVENTION

The present disclosure relates to the area of fungal enzymes, more in particular of polyphenoloxidases (PPOs). The disclosure is based on a newly discovered class of PPOs, based on their enzymatic activity, i.e. de-methylation of R-substituted mono- or di-methoxyphenols such as present in lignin, lignin derived compounds and/or in lignocellulosic biomass. This newly discovered enzymatic activity renders these enzymes highly suitable for a plethora of new applications in industry.

### BACKGROUND OF THE INVENTION

Lignin is a natural biopolymer, which can be extracted from biomass e.g. wood. Lignin is obtainable as a by-product of the paper and pulp industry and biorefinery industries and from a variety of low-value agricultural commodities such as straws, corn stover and bagasse. In biorefineries lignocellulosic biomass is pretreated using e.g. dilute sulfuric or other acids, hot water (hydrothermal), high pH (alkaline) by adding for example calcium-, sodium- or potassium hydroxide, or ammonia to breakdown the lignocellulose and producing a lignin fraction as a coproduct. For example lignin is coproduced as sulfite-, sulfonate-, kraft-, soda- organosolv (*e.g*. Pan et al., Biotechnol. Bioeng. 2005 May 20;90(4):473-81.), or biolignin (WO2009/092749).

Potential high-value products from isolated lignin include low-cost carbon fibre, plastics and thermoplastic elastomers and fuels or chemicals currently derived from petroleum. For example lignin is considered to be a possible substitute for phenol for example in phenol-formaldehyde (PF) resin synthesis.

The low reactivity of lignin due to its chemical structure is preventing large scale commercial utilization of lignin. Demethylation increases the reactivity of lignin by forming catechol moieties in the lignin macromolecule (Hu et al. BIORESOURCES, Vol.6 (3), 3515-3525: 2011).

Lignin is an aromatic heteropolymer consisting of the three monolignols (or hydroxycinnamyl alcohols) coniferyl (guaiacyl or "G" unit when incorporated in lignin polymer), sinapyl (syringyl or "S" unit when incorporated in lignin polymer) and p-coumaryl alcohol (p-hydroxyphenyl H unit when incorporated in lignin polymer), which are methoxylated in various degrees (Vanholme et al, Plant Physiology, July 2010, Vol. 153, pp. 895-905, 2012).

The lignin structure is depended on the type of plant source and the lignin isolation procedure. For example the ratio of syringyl (S), guaiacyl (G) and hydroxyphenyl (H) groups in the lignin is different in trees, grasses and straws. Especially the reactivity of lignins that are rich in S-groups and to a lesser extent G-groups (mono- and di-orthophenols) will be enhanced by demethylation.

It is known that lignin or lignin derived materials may be treated to cleave the methoxy groups (referred is to US 2,816,832, US 2,840,614, US 2,908,716, US 3,326,980 and US 4,250,088). Each of these methods demethylate lignin to some extent, however these methods operate at high temperature and/or pressure, making them energy intensive and may result in modification of the lignin structure. It is therefore desirable to identify milder demethylation methods.

An alternative for a thermochemical demethylation is a biological demethylation. Several biological systems have been described to demethylate lignin and/or lignin-derived compounds. These biological systems rely for example on the activity of laccases, tetrahydrofolate-dependent transferases, or a dioxygenases to catalyze lignin demethylation. A laccase catalyzed demethylation has the drawback that this is not very specific, whereas the tetrahydrofolate-dependent transferase, and dioxygenase described for *Sphingomonas paucimobilis* SYK-6 strain are intracellular enzymes making isolation of the enzymes laborious and expensive. It would be desirable to obtain new methods for the demethylation. WO 2014/081700 describes recombinant fungal polypeptides. WO 2012/068236 describes novel fungal oxidoreductases. WO 2015/035029 describes processes for increasing enzymatic hydrolysis of cellulosic material. WO 2014/186567 describes enhancing enzymatic hydrolysis by enzymatic preconditioning. Finally, WO 2008/134259 describes detoxifying pre-treated lignocellulose-containing materials.

### BRIEF SUMMARY OF TH E INVENTION

The invention is defined by the claims. Provided herein are polyphenoloxidases (PPOs) capable of demethylating an R-substituted mono- or di-methoxyphenol represented by 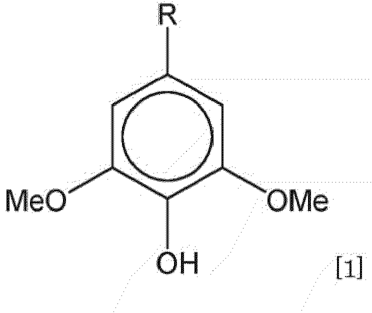 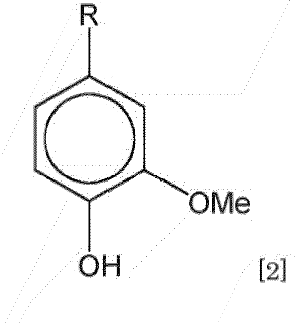 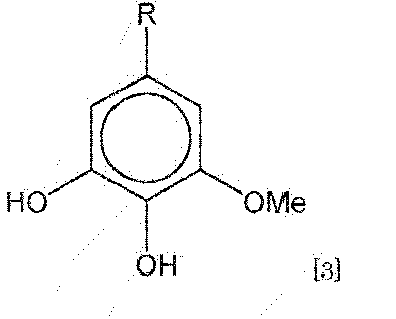 the above formula [1], [2] or [3] wherein R can be any single atom or chemical moiety. R may be an organic moiety. Preferably these PPOs comprise or consist of an amino acid sequence that is at least 70% identical to the amino acid sequence of at least one of SEQ ID NO: 37, 41 or 45. Preferably, the PPO is an enzyme that comprises a central tyrosinase domain. The PPO may be obtainable from a fungus, preferably a *Myceliophthora.* The PPO may be obtainable from *Myceliophthora thermophila* C1. Preferably, the PPO is an enzyme that releases methanol from an R-substituted di-methoxyphenol represented by 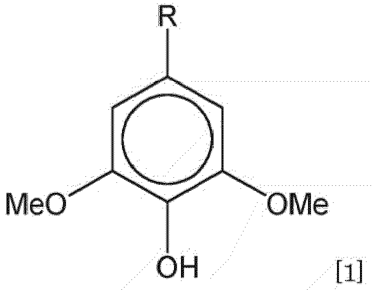 , wherein R is an organic moiety. Preferably, the R-substituted mono- or di-methoxyphenol is an R-substituted di-methoxyphenol represented by formula [1].

Provided herein are methods of demethylation of an R-substituted mono- or di-methoxyphenol represented by formula [1], [2] or [3] wherein R can be any single atom or chemical moiety, comprising contacting a substrate comprising said R-substituted mono- or di-methoxyphenol with the a polyphenoloxidase (PPO) capable of demethylating said R-substituted mono- or di-methoxyphenol as provided herein. The R-substituted mono- or di-methoxyphenol-comprising substrate may further comprise or be contacted with a copper salt. The copper salt may be selected from the group consisting of CuSO4, CuCl₂, CuBr₂, CuF₂, Cu(OH)₂ and Cu(NO₃)₂. In some disclosures, the concentration of copper salt is 1 - 1000µM. In some disclosures, the R-substituted mono- or di-methoxyplienol-comprising substrate has a pH between 5.0 and 8.0. In some disclosures, the method is performed at a temperature between 20-60°C. In some disclosures, the R-substituted mono- or di-methoxyphenol is syringic acid, sinapic acid, syringol, and/or a combination thereof. Lignin may be the source of the R-substituted mono- or di-methoxyphenol in the demethylation method provided herein.

Provided herein are processes to increase the reactivity of lignin or lignin-comprising biomass, wherein said process comprises a method of demethylation provided herein. Also provided herein are processes for the conversion of lignin or lignin comprising biomass to products such as biofuel, macromolecules and/or aromatic chemicals, wherein said process comprises a method of demethylation provided herein.

Provided herein are processes for degrading and/or modifying cellulose in a hemicellulose-comprising substrate, wherein said process comprises the step of contacting said substrate with an accessory enzyme and a PPO capable of demethylating an R-substituted mono- or di-methoxyphenol represented by formula [1], [2], or [3] wherein R can be any single atom or chemical moiety. In embodiments, the process comprises the step of admixing an R-substituted mono- or di-methoxyphenol represented by formula [1], [2], or [3] wherein R can be any single atom or chemical moiety. The accessory enzyme is an lytic polysaccharide monoxoygenases (LPMO) that comprises or consists of an amino acid sequence that is at least 70% identical to the amino acid sequence of SEQ ID NO: 1 or SEQ ID NO: 93. In embodiments, the process is a process for saccharification, for degrading DDG, for baking, for preparing dough, for clarifying fruit, vegetable or cereal juice, for macerating vegetables or food and/or for increasing digestibility and/or nutritional properties of animal feedstocks or animal food.

Provided herein are expression vectors encoding a PPO capable of demethylating a compound of formula [1], [2], or [3] wherein R can be any single atom or chemical moiety. In some disclosures, the PPO comprises or consists of an amino acid sequence that is at least 70% identical to the amino acid sequence of SEQ ID NO: 37, 41 or 45. In some disclosures, expression vectors may further encode an LPMO that is at least 70% identical to the amino acid sequence of SEQ ID NO: 1 or SEQ ID NO: 93.

Also provided herein are host cells transformed with expression vectors provided herein.

Provided herein are liquid, paste or solid formulations for use in methods of demethylation provided herein, wherein said formulation comprises a PPO provided, and preferably further comprising one or more additional enzymes. In some disclosures, the formulation further comprises an LPMO that is at least 70% identical to the amino acid sequence of SEQ ID NO: 1 or SEQ ID NO: 93

Provided herein are compositions comprising an R-substituted mono- or di-methoxyphenol-comprising substrate, wherein said composition further comprises a PPO disclosed herein, an expression vector disclosed herein, a host cell disclosed herein, a liquid, paste or solid formulation disclosed herein and/or a combination thereof. In some disclosures, the composition is a biomass saccharification mixture, a baking composition or dough, animal feed or a feed premix, lignocellulosic material, paper and/or pulp.

Provided herein are uses of a PPO a PPO disclosed herein, an expression vector disclosed herein, a host cell disclosed herein, a liquid, paste or solid formulation disclosed herein, and/or a composition provided herein, in a method or process.

### BRIEF DESCRIPTION OF THE DRAWINGS

**Figure 1****:** Proposed PPO7 catalyzed reaction with syringic acid. The enzyme of the present invention catalyzes the demethylation of dimethoxy substituted phenol compounds (ortho substituents). The enzyme also catalyzes the subsequent oxidation of the catechol to the quinone.
**Figure 2****:** Difference absorbance spectra (syringic acid blank subtracted) of the reaction of syringic acid with purified PPO7 and PPO2 after 24h.
**Figure 3****:** Visual color change upon incubation of syringic acid with purified PPO7, PP02 and blank as described in Example.
**Figure 4****:** UV-VIS data of the reactions of crude PPOs with synaptic acid (Figure 4a), syringic acid (Figure 4b), and syringol (Figure 4c). Enzyme blanks were subtracted.
**Figure 5****:** UHPLC (Figure 5a), MS (Figure 5b) and MS² (Figure 5c) data for the PPO reaction with syringic acid. Data confirm the formation of 3,4-dihydroxy-5-methoxybenzoic acid.

### DETAILED DESCRIPTION

In order to further define this invention, the following terms and definitions are herein provided.

Described herein is the discovery of PPO catalyzed demethylation of lignin, lignin derived compounds and other R-substituted mono- or di-methoxyphenols as defined herein. To our knowledge a (tyrosinase-like) PPO has not previously been found to catalyze this activity. As used herein, the terms "lignin", "lignen", "saccharification", "fermentable sugars", "biomass", "lignocellulosic material", "agricultural biomass", "energy crops", "cellulose" and "hemicellulose" are to be understood herein as defined in WO2013/159005 A2. As used herein, "oxidoreductase", "oxidase", "monooxygenases", "hydroxylases" "dehydrogenases", "cellobiose dehydrogenase", "cellobiose dehydrogenases", "cellobiose oxidases", "carbohydrase", "glycoside hydrolase", "glycosyl hydrolase", "glycosidase", "endoglucanase", "cellobiohydrolase" and "hemicellulase", "xylanase", "β-mannanase", "endo-1,4-β-mannosidase", "mannan endo-1,6-α-mannosidase", "β-mannosidase", "galactanase", "endo-β-1,6-galactanse", "arabinogalactan endo-1,4-β-galactosidase", "glucoamylase" "β-hexosaminidase", "β-N-acetylglucosaminidase", "α-L-arabinofuranosidase", "α-N-arabinofuranosidase", "α-arabinofuranosidase", "arabinosidase", "arabinofuranosidase", "endo-arabinase", "exo-arabinase", "β-xylosidase", "chitosanase", "exo-polygalacturonase", "acetyl xylan esterase", "acetyl mannan esterase", "ferulic esterase", "ferulic acid esterase", "coumaric acid esterase", "pectate lyase", "pectin lyases", "endo-1,3-β-giucanase", "laminarinase", "lichenase", "glycosidases" and "ligninase" are to be understood herein as defined in WO 2013/159005 A2.

As used herein, a "biomass" is understood herein as defined in WO2013/159005 A2, i.e. including materials containing cellulose and/or hemicellulose. Generally, these materials also contain pectin, lignin, protein, carbohydrates (such as starch and sugar) and ash. Lignocellulose is generally found, for example, in the stems, leaves, hulls, husks, and cobs of plants or leaves, branches, and wood of trees. Biomass can include virgin biomass and/or non-virgin biomass such as agricultural biomass, commercial organics, construction and demolition debris, municipal solid waste, waste paper and yard waste. Common forms of biomass include trees, shrubs and grasses, wheat, wheat straw, sugar cane bagasse, sugar beet, soybean, corn, corn husks, corn kernel including fiber from kernels, products and by-products from milling of grains such as corn, tobacco, wheat and barley (including wet milling and dry milling) as well as municipal solid waste, waste paper and yard waste. The biomass can also be, but is not limited to, herbaceous material, agricultural residues, forestry residues, municipal solid wastes, waste paper, and pulp and paper mill residues. "Agricultural biomass" includes branches, bushes, canes, corn and corn husks, energy crops, algae, fruits, flowers, grains, grasses, herbaceous crops, leaves, bark, needles, logs, roots, saplings, short rotation woody crops, shrubs, switch grasses, trees, vegetables, fruit peels, vines, sugar beet pulp, wheat midlings, oat hulls, peat moss, mushroom compost and hard and soft woods (not including woods with deleterious materials). In addition, agricultural biomass includes organic waste materials generated from agricultural processes including farming and forestry activities, specifically including forestry wood waste. Agricultural biomass may be any of the aforestated singularly or in any combination or mixture thereof "Agricultural biomass" includes branches, bushes, canes, corn and corn husks, energy crops, algae, fruits, flowers, grains, grasses, herbaceous crops, leaves, bark, needles, logs, roots, saplings, short rotation woody crops, shrubs, switch grasses, trees, vegetables, fruit peels, vines, sugar beet pulp, wheat midlings, oat hulls, peat moss, mushroom compost and hard and soft woods (not including woods with deleterious materials). In addition, agricultural biomass includes organic waste materials generated from agricultural processes including forming and forestry activities, specifically including forestry wood waste. Agricultural biomass may be any of the aforestated singularly or in any combination or mixture thereof.

As used herein, an "organic moiety" is an H or O atom, an -OH group or a branched, straight or cyclic alkyl which is optionally substituted and which can vary from a single atom to a large, optionally branched, structure.

As used herein, reference to an "enzyme" includes full-length naturally occurring or wild-type enzymes or proteins (the terms enzyme and protein are used herein interchangeably) and their glycosylated or otherwise modified forms, fusion proteins, or any fragment or homologue or variant of such an enzyme or protein. Preferably, an enzyme is an isolated enzyme or protein. An isolated enzyme or protein, is to be understood herein as an enzyme or protein (including a polypeptide or peptide) that has been removed from its natural milieu (i.e., that has been subject to human manipulation) and can include purified proteins, partially purified proteins, recombinantly produced proteins, synthetically produced proteins, proteins complexed with lipids, soluble proteins, and isolated proteins associated with other proteins, for example. As such, "isolated" does not reflect the extent to which the protein has been purified. Preferably, an isolated protein is produced recombinantly. In addition, and by way of example, a "*M*. *thermophila* protein" or "*M. thermophila* enzyme" refers to a protein (generally including a homologue or variant of a naturally occurring protein) from *Myceliophthora thermophila* or to a protein that has been otherwise produced from the knowledge of the structure (e.g., sequence) and preferably the function of a naturally occurring protein from *Myceliophthora thermophila.* In other words, a *M. thermophila* protein includes any protein that has substantially similar structure and function of a naturally occurring *M*. *thermophila* protein or that is a biologically active (i.e., has biological activity) homologue or variant of a naturally occurring protein from *M. thermophila* as described in detail herein. As such, a *M*. *thermophila* protein can include purified, partially purified, recombinant, mutated/modified and synthetic proteins.

As used herein, the phrase "biological activity" of a protein refers to any function(s) exhibited or performed by the protein that is ascribed to the naturally occurring form of the protein as measured or observed *in vitro* or *in vivo.* A protein fragment preferably comprises a domain of a protein that has the catalytic activity of the full-length enzyme. A protein fragment includes, but is not limited to, a fragment comprising a catalytic domain (CD) and/or a carbohydrate binding module (CBM). For example, a protein fragment may comprise a CD of a protein but not a CBM of the protein or a CBM of a protein but not a CD. Similarly, domains from different proteins may be combined. Protein fragments comprising a CD, CBM or combinations thereof for each protein disclosed herein can be readily produced using standard techniques known in the art.

An enzyme or protein, including a biologically active homologue, variant, or fragment thereof, has at least one characteristic of biological activity of a wild-type, or naturally occurring, protein. As discussed above, in general, the biological activity or biological action of a protein refers to any function(s) exhibited or performed by the protein that is ascribed to the naturally occurring form of the protein as measured or observed *in vivo* (i.e., in the natural physiological environment of the protein) or *in vitro* (i.e., under laboratory conditions). The biological activity of a protein as disclosed herein can include an enzyme activity (catalytic activity and/or substrate binding activity), such as oxidases, oxygenases, monoxygenases, Baeyer-Villiger monooxygenases, dioxygenases, peroxidases, dehydrogenases, reductases that catalyze an oxidation-reduction reaction or any other activity disclosed herein. Specific biological activities of the proteins disclosed herein are described in detail herein and in the Examples. Methods of detecting and measuring the biological activity of a protein as disclosed herein include, but are not limited to, the assays described in the Examples section below. Such assays include, but are not limited to, measurement of enzyme activity (e.g., catalytic activity), measurement of substrate binding, and the like. Additional assays and methods for examining the activity of the enzymes are found in U.S. Patent Applications 60/806,876, 60/970,876, 11/487,547, 11/775,777, 11/833,133, and 12/205,694 and U.S. Patent Application Publications US20080076159A1, US20090099079A1, US20070238155A1, US20090280105A1.

It is noted that an enzyme or protein (including homologues or variants) is not required to have a biological activity such as catalytic activity. A protein can be a truncated, mutated or inactive protein, or lack at least one activity of the wild-type enzyme, for example. Inactive proteins may be useful in some screening assays, Methods to measure protein expression levels of a protein include, but are not limited to: western blotting, immunocytochemistry, flow cytometry or other immunologic-based assays; assays based on a property of the protein including but not limited to, ligand binding or interaction with other protein partners.

Modifications of a protein, such as in a homologue or variant, may result in proteins having the same biological activity as the naturally occurring protein, or in proteins having decreased or increased biological activity as compared to the naturally occurring protein. Modifications which result in a decrease in protein expression or a decrease in the activity of the protein, can be referred to as inactivation (complete or partial), down-regulation, or decreased action of a protein. Similarly, modifications that result in an increase in protein expression or an increase in the activity of the protein, can be referred to as amplification, overproduction, activation, enhancement, up-regulation or increased action of a protein.

As used herein, the terms "modification," "mutation," and "variant" can be used interchangeably with regard to the modifications/mutations to the amino acid sequence of a *M*. *thermophila* protein (or nucleotide sequences) described herein.

The term "modification" can also be used to describe post-translational modifications to a protein or peptide including, but not limited to, methylation, farnesylation, carboxymethylation, geranyl geranylation, glycosylation, phosphorylation, acetylation, myristoylation, prenylation, palmitation, and/or amidation. Modification can also include the cleavage of a signal peptide, or methionine, or other portions of the peptide that require cleavage to generate the mature peptide.

As used herein, the terms "homologue" or "variants" are used to refer to a protein or peptide which differs from a naturally occurring protein or peptide (i.e., the "prototype" or "wild-type" protein) by minor modifications to the naturally occurring protein or peptide, but which maintains the basic protein and side chain structure of the naturally occurring form. Such changes include, but are not limited to: changes in one or a few amino acid side chains; changes one or a few amino acids, including deletions (e.g., a truncated version of the protein or peptide), insertions and/or substitutions; changes in stereochemistry of one or a few atoms; and/or minor derivatizations, including but not limited to for example: methylation, glycosylation and phosphorylation. A homologue or variant can have either enhanced, decreased, or substantially similar properties as compared to the naturally occurring protein or peptide. A homologue or variant can include an agonist of a protein or an antagonist of a protein.

Homologues or variants can be the result of natural allelic variation or natural mutation. A naturally occurring allelic variant of a nucleic acid encoding a protein is a gene that occurs at essentially the same locus (or loci) in the genome as the gene which encodes such protein, but which, due to natural variations caused by, for example, mutation or recombination, has a similar but not identical sequence. Homologous can also be the result of a gene duplication and rearrangement, resulting in a different location. Allelic variants typically encode proteins having similar activity to that of the protein encoded by the gene to which they are being compared. One class of allelic variants can encode the same protein but have different nucleotide sequences due to the degeneracy of the genetic code. Allelic variants can also comprise alterations in the 5' or 3' untranslated regions of the gene (e.g., in regulatory control regions). Allelic variants are well known to those skilled in the art.

Homologues or variants can be produced using techniques known in the art for the production of proteins including, but not limited to, direct modifications to the naturally occurring protein, direct protein synthesis, or modifications to the nucleotide sequence encoding the protein using, for example, classic or recombinant DNA techniques to effect random or targeted mutagenesis.

A modified protein also includes a fusion protein that includes a domain of a protein as disclosed herein (including a homologue or variant) attached to one or more fusion segments, which are typically heterologous in sequence to the protein sequence *(i.e.,* different than protein sequence). Suitable fusion segments in a modified protein include, but are not limited to, segments that can: enhance a protein's stability; provide other desirable biological activity; and/or assist with the purification of the protein (e.g., by affinity chromatography). A suitable fusion segment can be a domain of any size that has the desired function *(e.g.,* imparts increased stability, solubility, action or biological activity; and/or simplifies purification of a protein). Fusion segments can be joined to amino and/or carboxyl termini of the domain of a protein as disclosed herein and can be susceptible to cleavage in order to enable straight-forward recovery of the protein. Fusion proteins are preferably produced by culturing a recombinant cell transfected with a fusion nucleic acid molecule that encodes a protein including the fusion segment attached to either the carboxyl and/or amino terminal end of a domain of a protein as disclosed herein. Accordingly, proteins as disclosed herein also include expression products of gene fusions (for example, used to overexpress soluble, active forms of the recombinant protein), of mutagenized genes (such as genes having codon modifications to enhance gene transcription and translation), and of truncated genes (such as genes having membrane binding modules removed to generate soluble forms of a membrane protein, or genes having signal sequences removed which are poorly tolerated in a particular recombinant host).

A modified protein also includes a protein as disclosed that has been modified by conservative amino acid substitution. "Conservative amino acid substitutions" refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg; Gln or Glu; Met to Leu or Ile; Phe to Met, Leu or Tyr; Ser to Thr; Thr to Ser; Trp to Tyr; Tyr to Trp or Phe; and, Val to Ile or Leu.

A protein or polypeptide defined as "consisting essentially of" a specified amino acid sequence, is an amino acid sequences described herein produced from at least one, and up to about 20, additional heterologous amino acids flanking each of the C- and/or N-terminal ends of the specified amino acid sequence. Heterologous amino acids are a sequence of amino acids that are not naturally found (i.e., not found in nature, *in vivo*) flanking the specified amino acid sequence, or that are not related to the function of the specified amino acid sequence, or that would not be encoded by the nucleotides that flank the naturally occurring nucleotide sequence encoding the specified amino acid sequence as it occurs in the gene, if such nucleotides in the naturally occurring sequence were translated using standard codon usage for the organism from which the given amino acid sequence is derived. In addition, and solely by way of example, a protein referenced as being derived from is derived from a particular organism, such as a fungus as defined herein above.

Many of the enzymes and proteins disclosed herein may be desirable targets for modification and use in the processes described herein. These proteins have been described in terms of function and amino acid sequence (and nucleotide sequence encoding the same) of representative wild-type proteins. Homologues or variants of a protein encompassed preferably comprise, consist essentially of, or consist of, an amino acid sequence that is at least 35%, 45%, 55%, 65%, 70%, 75%, 80%, 90%, identical, and more preferably at least 95% identical, 96%, 97%, identical, and most preferably at least about 99% identical, or any percent identity between 35% and 99%, in whole integers (i.e., 36%, 37%, etc.), to an amino acid sequence disclosed herein that represents the amino acid sequence of an enzyme or protein disclosed herein (including a biologically active domain of a full-length protein). Preferably, the amino acid sequence of the homologue or variant has a biological activity of the wild-type or reference protein or of a biologically active domain thereof (e.g., a catalytic domain). When denoting mutation positions, the amino acid position of the wild-type is typically used. The wild-type can also be referred to as the "parent". Additionally, any generation before the variant at issue can be a parent.

The minimum size of a protein and/or homologue, variant or fragment is a size sufficient to have biological activity or, when the protein is not required to have such activity, sufficient to be useful for another purpose associated with a protein as disclosed herein, such as for the production of antibodies that bind to a naturally occurring protein. Preferably, the protein disclosed herein is at least 20, 25, 30, 40, 50, 60, 70, 80, 90, 100, 125, 150, 175, 200, 250 amino acids in length, and so on up to a full length of each protein, and including any size in between in increments of one whole integer (one amino acid). There is no limit, other than a practical limit, on the maximum size of such a protein in that the protein can include a portion of a protein or a full-length protein, plus additional sequence (e.g., a fusion protein sequence), if desired.

As used herein, unless otherwise specified, reference to a percent (%) identity refers to an evaluation of homology which is performed using: 1) a BLAST 2.0 Basic BLAST homology search using blastp for amino acid searches and blastn for nucleic acid searches with standard default parameters, wherein the query sequence is filtered for low complexity regions by default (described in Altschul, S.F., Madden, T.L., Schääffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402); 2) a BLAST 2 alignment (using the parameters described below); 3) PSI-BLAST with the standard default parameters (Position-Specific Iterated BLAST; and/or 4) CAZy homology determined using standard default parameters from the Carbohydrate Active EnZymes database (Coutinho, P.M. & Henrissat, B. (1999) Carbohydrate-active enzymes: an integrated database approach. In "Recent Advances in Carbohydrate Bioengineering", H.J. Gilbert, G. Davies, B. Henrissat and B. Svensson eds., The Royal Society of Chemistry, Cambridge, pp. 3-12) and/or applying a similar strategy using databases such as the Foly database (website: foly.esil.univ-mrs.fr) and the PeroxiBase (website: peroxibase.isb-sib.ch).

It is noted that due to some differences in the standard parameters between BLAST 2.0 Basic BLAST and BLAST 2, two specific sequences might be recognized as having significant homology using the BLAST 2 program, whereas a search performed in BLAST 2.0 Basic BLAST using one of the sequences as the query sequence may not identify the second sequence in the top matches. In addition, PSI-BLAST provides an automated, easy-to-use version of a "profile" search, which is a sensitive way to look for sequence homologues or variants. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using any one of these programs.

Two specific sequences can be aligned to one another using BLAST 2 sequence as described in Tatusova and Madden, (1999), "Blast 2 sequences - a new tool for comparing protein and nucleotide sequences", FEMS Microbiol Lett. 174:247-250. BLAST 2 sequence alignment is performed in blastp or blastn using the BLAST 2.0 algorithm to perform a Gapped BLAST search (BLAST 2.0) between the two sequences allowing for the introduction of gaps (deletions and insertions) in the resulting alignment. For purposes of clarity herein, a BLAST 2 sequence alignment is performed using the standard default parameters as follows.

For blastn, using 0 BLOSUM62 matrix:
Reward for match = 1
Penalty for mismatch = -2
Open gap (5) and extension gap (2) penalties
gap x_dropoff (50) expect (10) word size (11) filter (on)
For blastp, using 0 BLOSUM62 matrix:
   Open gap (11) and extension gap (1) penalties
   gap x_dropoff (50) expect (10) word size (3) filter (on).
   Unless otherwise indicated herein, identity with a given SEQ ID NO means identity based on the full length of said sequence (*i.e*. over its whole length or as a whole).

As used herein, the term "contiguous" or "consecutive", with regard to nucleic acid or amino acid sequences described herein, means to be connected in an unbroken sequence. For example, for a first sequence to comprise 30 contiguous (or consecutive) amino acids of a second sequence, means that the first sequence includes an unbroken sequence of 30 amino acid residues that is 100% identical to an unbroken sequence of 30 amino acid residues in the second sequence. Similarly, for a first sequence to have "100% identity" or being "100% identical" with a second sequence means that the first sequence exactly matches the second sequence with no gaps between nucleotides or amino acids. A protein as disclosed herein, including a homologue or variant, preferably includes a protein having an amino acid sequence that is sufficiently similar to a natural amino acid sequence that a nucleotide sequence encoding the homologue or variant is capable of hybridizing under moderate, high or very high stringency conditions (described below) to *(i.e.,* with) a nucleic acid molecule encoding the natural protein *(i.e.,* to the complement of the nucleic acid strand encoding the natural amino acid sequence). Preferably, a homologue or variant of a protein is encoded by a nucleic acid molecule comprising a nucleotide sequence that hybridizes under low, moderate, or high stringency conditions to the complement of a nucleotide sequence that encodes a protein comprising, consisting essentially of, or consisting of, an amino acid sequence represented by any one of SEQ ID NOs 1-6, 10, 13, 14, 17, 18, 21, 22, 25, 26, 29, 30, 33, 34, 37, 38, 41, 42, 45 and 46. Such hybridization conditions are described in detail below.

A nucleotide sequence complement of nucleotide sequence encoding a protein as disclosed herein refers to the nucleotide sequence of the nucleic acid strand that is complementary to the strand, which encodes the protein. It will be appreciated that a double stranded DNA which encodes a given amino acid sequence comprises a single strand DNA and its complementary strand having a sequence that is a complement to the single strand DNA. As such, nucleic acid molecules can be either double-stranded or single-stranded, and include those nucleic acid molecules that form stable hybrids under stringent hybridization conditions with a nucleotide sequence that encodes an amino acid sequence such as the amino acid sequences of SEQ ID NOs 1-6, 10, 13, 14, 17, 18, 21, 22, 25, 26, 29, 30, 33, 34, 37, 38, 41, 42, 45 or 46 or a nucleotide sequence of SEQ ID NOs 7-9, 11, 12, 15, 16, 19, 20, 23, 24, 27, 28, 31, 32, 35, 36, 39, 40, 43, 44, 47 or 48. Methods to deduce a complementary sequence are known to those skilled in the art. It should be noted that since nucleic acid sequencing technologies are not entirely error-free, the sequences presented herein, at best, represent apparent sequences of the proteins disclosed herein.

As used herein, reference to hybridization conditions refers to standard hybridization conditions under which nucleic acid molecules are used to identify similar nucleic acid molecules. Such standard conditions are disclosed, for example, in Sambrook et al., Molecular Cloning: A Laboratory Manual, Cold Spring Harbor Labs Press, 1989. Sambrook et al., *ibid.,* (see specifically, pages 9.31-9.62). In addition, formulae to calculate the appropriate hybridization and wash conditions to achieve hybridization permitting varying degrees of mismatch of nucleotides are disclosed, for example, in Meinkoth et al., 1984, Anal. Biochem. 138, 267-284; Meinkoth et al., *ibid.*

More particularly, moderate stringency hybridization and washing conditions, as referred to herein, refer to conditions which permit isolation of nucleic acid molecules having at least about 70% nucleotide sequence identity with the nucleic acid molecule being used to probe in the hybridization reaction (i.e., conditions permitting about 30% or less mismatch of nucleotides). High stringency hybridization and washing conditions, as referred to herein, refer to conditions which permit isolation of nucleic acid molecules having at least about 80% nucleotide sequence identity with the nucleic acid molecule being used to probe in the hybridization reaction (i.e., conditions permitting about 20% or less mismatch of nucleotides). Very high stringency hybridization and washing conditions, as referred to herein, refer to conditions which permit isolation of nucleic acid molecules having at least about 90% nucleotide sequence identity with the nucleic acid molecule being used to probe in the hybridization reaction (i.e., conditions permitting about 10% or less mismatch of nucleotides). As discussed above, one of skill in the art can use the formulae in Meinkoth et al., *ibid.* to calculate the appropriate hybridization and wash conditions to achieve these particular levels of nucleotide mismatch. Such conditions will vary, depending on whether DNA:RNA or DNA:DNA hybrids are being formed. Calculated melting temperatures for DNA:DNA hybrids are 10°C less than for DNA:RNA hybrids. Preferably, stringent hybridization conditions for DNA:DNA hybrids include hybridization at an ionic strength of 6X SSC (0.9 M Na⁺) at a temperature of between about 20°C and about 35°C (lower stringency), more preferably, between about 28°C and about 40°C (more stringent), and even more preferably, between about 35°C and about 45°e (even more stringent), with appropriate wash conditions. Preferably, stringent hybridization conditions for DNA:RNA hybrids include hybridization at an ionic strength of 6X SSC (0.9 M Na⁺) at a temperature of between about 30°C and about 45°C, more preferably, between about 38°C and about 50°C, and even more preferably, between about 45°C and about 55°C, with similarly stringent wash conditions. These values are based on calculations of a melting temperature (Tm) for molecules larger than about 100 nucleotides, 0% formamide and a G + C content of about 40%. Alternatively, Tm can be calculated empirically as set forth in Sambrook et al., *supra,* pages 9.31 to 9.62. In general, the wash conditions should be as stringent as possible, and should be appropriate for the chosen hybridization conditions. For example, hybridization conditions can include a combination of salt and temperature conditions that are approximately 20-25°C below the calculated Tm of a particular hybrid, and wash conditions typically include a combination of salt and temperature conditions that are approximately 12-20°C below the calculated Tm of the particular hybrid. One example of hybridization conditions suitable for use with DNA:DNA hybrids includes a 2-24 hour hybridization in 6X SSC (50% formamide) at about 42°C, followed by washing steps that include one or more washes at room temperature in about 2X SSC, followed by additional washes at higher temperatures and lower ionic strength (e.g., at least one wash as about 37°C in about 0.1X-0.5X SSC, followed by at least one wash at about 68°C in about 0.1X-0.5X SSC).

A nucleic acid molecule includes a nucleic acid molecule comprising, consisting essentially of, or consisting of, a nucleotide sequence encoding any of the enzymes or proteins disclosed herein, including a fragment or a homologue or variant of such proteins, described above. Nucleic acid molecules can include a nucleotide sequence that encodes a fragment of a protein that does not have biological activity, and can also include portions of a gene or polynucleotide encoding the protein that are not part of the coding region for the protein *(e.g.,* introns or regulatory regions of a gene encoding the protein). Nucleic acid molecules can include a nucleotide sequence that is useful as a probe or primer (oligonucleotide sequences). Preferably, a nucleic acid molecule is an isolated nucleic acid molecule. As used herein, an isolated nucleic acid molecule is a nucleic acid molecule (polynucleotide) that has been removed from its natural milieu *(i.e.,* that has been subject to human manipulation) and can include DNA, RNA, or derivatives of either DNA or RNA, including cDNA. As such, "isolated" does not reflect the extent to which the nucleic acid molecule has been purified. Although the phrase "nucleic acid molecule" primarily refers to the physical nucleic acid molecule, and the phrase "nucleotide sequence" primarily refers to the sequence of nucleotides on the nucleic acid molecule, the two phrases can be used interchangeably, especially with respect to a nucleic acid molecule, or a nucleotide sequence, being capable of encoding a protein. An isolated nucleic acid molecule can be isolated from its natural source or produced using recombinant DNA technology *(e.g.,* polymerase chain reaction (PCR) amplification, cloning) or chemical synthesis. Nucleic acid molecules or isolated nucleic acid molecules can include, for example, genes, natural allelic variants of genes, coding regions or portions thereof, and coding and/or regulatory regions modified by nucleotide insertions, deletions, substitutions, and/or inversions in a manner such that the modifications do not substantially interfere with the nucleic acid molecule's ability to encode a protein or to form stable hybrids under stringent conditions with natural gene isolates. A nucleic acid molecule can include degeneracies. As used herein, nucleotide degeneracy refers to the phenomenon that one amino acid can be encoded by different nucleotide codons. Thus, the nucleotide sequence of a nucleic acid molecule that encodes a protein disclosed herein can vary due to degeneracies. It is noted that a nucleic acid molecule disclosed herein is not required to encode a protein having protein activity. A nucleic acid molecule can encode a truncated, mutated or inactive protein, for example. In addition, nucleic acid molecules disclosed herein are useful as probes and primers for the identification, isolation and/or purification of other nucleic acid molecules. If the nucleic acid molecule is an oligonucleotide, such as a probe or primer, the oligonucleotide preferably ranges from about 5 to about 50 or about 500 nucleotides, more preferably from about 10 to about 40 nucleotides, and most preferably from about 15 to about 40 nucleotides in length.

Reference to a gene includes all nucleotide sequences related to a natural *(i.e.* wild-type) gene, such as regulatory regions that control production of the protein encoded by that gene (such as, but not limited to, transcription, translation or post-translation control regions) as well as the coding region itself. A gene may be a naturally occurring allelic variant that includes a similar but not identical sequence to the nucleotide sequence encoding a given protein. Allelic variants have been previously described above. Genes can include or exclude one or more introns or any portions thereof or any other sequences or which are not included in the cDNA for that protein. The phrases "nucleic acid molecule" and "gene" can be used interchangeably when the nucleic acid molecule comprises a gene as described above.

Modified genes include natural genes modified by substitution, insertion, and/or deletion of single or multiple nucleotide sequences, which can occur within the coding sequence including exons of regions encoding a polypeptide, or in flanking regions, such as regulatory regions typically upstream (e.g., promoters, enhancers, and related sequences), downstream (e.g., transcriptional termination, and poly(A) signals), or internal regions (e.g., introns) that affect the transcription, translation, and/or activation of a polypeptide or regulatory molecule of interest. Activation of a polypeptide, for example, may require removal of one or more N-terminal, C-terminal, or internal polypeptide regions, and/or post-translational modification of specific amino acid residues, such as by glycosylation, amidation, etc., that may alter the targeting, degradation, catalytic activity, of an enzyme.

Preferably, a nucleic acid molecule as disclosed herein is produced using recombinant DNA technology *(e.g.,* polymerase chain reaction (PCR) amplification, cloning, etc.) or chemical synthesis. Nucleic acid molecules include any nucleic acid molecules and homologues or variants thereof that are part of a gene described herein and/or that encode a protein described herein, including, but not limited to, natural allelic variants and modified nucleic acid molecules (homologues or variants) in which nucleotides have been inserted, deleted, substituted, and/or inverted in such a manner that such modifications provide the desired effect on protein biological activity or on the activity of the nucleic acid molecule. Allelic variants and protein homologues or variants (e.g., proteins encoded by nucleic acid homologues or variants) have been discussed in detail above.

A nucleic acid molecule homologue or variant (i.e., encoding a homologue or variant of a protein disclosed herein) can be produced using a number of methods known to those skilled in the art (see, for example, Sambrook *et al.).* For example, nucleic acid molecules can be modified using a variety of techniques including, but not limited to, by classic mutagenesis and recombinant DNA techniques (e.g., site-directed mutagenesis, chemical treatment, restriction enzyme cleavage, ligation of nucleic acid fragments and/or PCR amplification), or synthesis of oligonucleotide mixtures and ligation of mixture groups to "build" a mixture of nucleic acid molecules and combinations thereof. Another method for modifying a recombinant nucleic acid molecule encoding a protein is gene shuffling (i.e., molecular breeding) (See, for example, U.S. Patent No. 5,605,793 to Stemmer; Minshull and Stemmer; 1999, Curr. Opin. Chem. Biol. 3:284-290; Stemmer, 1994, P.N.A.S. USA 91:10747-10751). This technique can be used to efficiently introduce multiple simultaneous changes in the protein. Nucleic acid molecule homologues or variants can be selected by hybridization with a gene or polynucleotide, or by screening for the function of a protein encoded by a nucleic acid molecule *(i.e.,* biological activity).

The minimum size of a nucleic acid molecule as disclosed herein is a size sufficient to encode a protein (including a fragment, homologue, or variant of a full-length protein) having biological activity, sufficient to encode a protein comprising at least one epitope which binds to an antibody, or sufficient to form a probe or oligonucleotide primer that is capable of forming a stable hybrid with the complementary sequence of a nucleic acid molecule encoding a natural protein (e.g., under moderate, high, or high stringency conditions). As such, the size of the nucleic acid molecule encoding such a protein can be dependent on nucleic acid composition and percent homology or identity between the nucleic acid molecule and complementary sequence as well as upon hybridization conditions per se (e.g., temperature, salt concentration, and formamide concentration). The minimal size of a nucleic acid molecule that is used as an oligonucleotide primer or as a probe is typically at least about 12 to about 15 nucleotides in length if the nucleic acid molecules are GC-rich and at least about 15 to about 18 bases in length if they are AT-rich. There is no limit, other than a practical limit, on the maximal size of a nucleic acid molecule as disclosed herein, in that the nucleic acid molecule can include a portion of a protein encoding sequence, a nucleotide sequence encoding a full-length protein (including a gene), including any length fragment between about 20 nucleotides and the number of nucleotides that make up the full length cDNA encoding a protein, in whole integers (e.g., 20, 21, 22, 23, 24, 25 nucleotides), or multiple genes, or portions thereof. The phrase "consisting essentially of", when used with reference to a nucleotide sequence herein, refers to a nucleotide sequence encoding a specified amino acid sequence that can be flanked by from at least one, and up to as many as about 60, additional heterologous nucleotides at each of the 5 and/or the 3' end of the nucleotide sequence encoding the specified amino acid sequence. The heterologous nucleotides are not naturally found *(i.e.,* not found in nature, *in vivo)* flanking the nucleotide sequence encoding the specified amino acid sequence as it occurs in the natural gene or do not encode a protein that imparts any additional function to the protein or changes the function of the protein having the specified amino acid sequence.

A nucleic acid molecule as disclosed herein may be a recombinant nucleic acid molecule which comprises the nucleic acid molecule described above which is operatively linked to at least one expression control sequence. More particularly, a recombinant nucleic acid molecule typically comprises a recombinant vector and any one or more of the nucleic acid molecules as described herein. As used herein, a recombinant vector is an engineered *(i.e.,* artificially produced) nucleic acid molecule that is used as a tool for manipulating a nucleotide sequence of choice and/or for introducing such a nucleotide sequence into a host cell. The recombinant vector is therefore suitable for use in cloning, sequencing, and/or otherwise manipulating the nucleotide sequence of choice, such as by expressing and/or delivering the nucleotide sequence of choice into a host cell to form a recombinant cell. Such a vector typically contains nucleotide sequences that are not naturally found adjacent to nucleotide sequence to be cloned or delivered, although the vector can also contain regulatory nucleotide sequences (e.g., promoters, untranslated regions) which are naturally found adjacent to nucleotide sequences disclosed herein or which are useful for expression of the nucleic acid molecules disclosed herein (discussed in detail below). The vector can be either RNA or DNA, either prokaryotic or eukaryotic, and typically is a plasmid. The vector can be maintained as an extrachromosomal element (e.g., a plasmid) or it can be integrated into the chromosome of a recombinant host cell, although it is preferred if the vector remains separate from the genome. The entire vector can remain in place within a host cell, or under certain conditions, the plasmid DNA can be deleted, leaving behind the nucleic acid molecule disclosed herein. An integrated nucleic acid molecule can be under chromosomal promoter control, under native or plasmid promoter control, or under a combination of several promoter controls. Single or multiple copies of the nucleic acid molecule can be integrated into the chromosome. A recombinant vector disclosed herein can contain at least one selectable marker.

A recombinant vector used in a recombinant nucleic acid molecule disclosed herein may be an expression vector. As used herein, the phrase "expression vector" is used to refer to a vector that is suitable for production of an encoded product *(e.g.,* a protein of interest, such as an enzyme as disclosed herein). A nucleotide sequence encoding the product to be produced *(e.g.,* the protein or homologue or variant thereof) may be inserted into a recombinant vector to produce a recombinant nucleic acid molecule. The nucleotide sequence encoding the protein to be produced is inserted into the vector in a manner that operatively links the nucleotide sequence to regulatory sequences in the vector, which enable the transcription and translation of the nucleotide sequence within the recombinant host cell. Typically, a recombinant nucleic acid molecule includes at least one nucleic acid molecule as disclosed herein operatively linked to one or more expression control sequences *(e.g.,* transcription control sequences or translation control sequences). As used herein, the phrase "recombinant molecule" or "recombinant nucleic acid molecule" primarily refers to a nucleic acid molecule or nucleotide sequence operatively linked to a transcription control sequence, but can be used interchangeably with the phrase "nucleic acid molecule", when such nucleic acid molecule is a recombinant molecule as discussed herein. As used herein, the phrase "operatively linked" refers to linking a nucleic acid molecule to an expression control sequence in a manner such that the molecule can be expressed when transfected (i.e., transformed, transduced, transfected, conjugated or conduced) into a host cell. Transcription control sequences are sequences that control the initiation, elongation, or termination of transcription. Particularly important transcription control sequences are those that control transcription initiation, such as promoter, enhancer, operator and repressor sequences. Suitable transcription control sequences include any transcription control sequence that can function in a host cell or organism into which the recombinant nucleic acid molecule is to be introduced. Transcription control sequences may also include any combination of one or more of any of the foregoing.

Recombinant nucleic acid molecules can also contain additional regulatory sequences, such as translation regulatory sequences, origins of replication, and other regulatory sequences that are compatible with the recombinant cell. A recombinant molecule, including those that are integrated into the host cell chromosome, may also contain secretory signals (i.e., signal segment nucleotide sequences) to enable an expressed protein to be secreted from the cell that produces the protein. Suitable signal segments include a signal segment that is naturally associated with the protein to be expressed or any heterologous signal segment capable of directing the secretion of the protein as disclosed herein. A recombinant molecule may comprise a leader sequence to enable an expressed protein to be delivered to and inserted into the membrane of a host cell. Suitable leader sequences include a leader sequence that is naturally associated with the protein, or any heterologous leader sequence capable of directing the delivery and insertion of the protein to the membrane of a cell.

The term "transfection" is generally used to refer to any method by which an exogenous nucleic acid molecule (i.e., a recombinant nucleic acid molecule) can be inserted into a cell. The term "transformation" can be used interchangeably with the term "transfection" when such term is used to refer to the introduction of nucleic acid molecules into microbial cells or plants and describes an inherited change due to the acquisition of exogenous nucleic acids by the microorganism that is essentially synonymous with the term "transfection." Transfection techniques include, but are not limited to, transformation, particle bombardment, electroporation, microinjection, lipofection, adsorption, infection and protoplast fusion.

A "xylo-oligomer" is defined herein as an oligomer that is produced after cleavage of the β-1,4 bond in the xylan backbone. Preferably, a xylo-oligmer is an, optionally substituted, β-(1→4)-linked xylan consisting of 2 to 50, or 2 to 30, or of 2 to 15, or 2 to 10, or of 2 to 7 xylan residues. A substituted xylo-oligomer may be, but is not limited to, an arabino-, an acetyl-, a glucorono- and/or a methyglucurono-substituted xylo-oligomer. An "oxidised xylo-oligomer" is a xylo-oligomer that is oxidised on the C1- and/or C4-carbon atom of the xylo-oligomer.

A "gluco-oligomer" is defined herein as an oligomer that is produced after cleavage of the β-1,4 bond in the cellulose backbone. Preferably, a gluco-oligmer is a β-(1→4)-linked glucan consisting of 2 to 50, or 2 to 30, or preferably of 2 to 15, or 2 to 10, or most preferably of 2 to 6 glucan residues. An "oxidised gluco-oligomer" is a gluco-oligomer that is oxidised on the C1- and/or C4-carbon atom of the gluco-oligomer.

The word "approximately" or "about" when used in association with a numerical value (approximately 10, about 10) preferably means that the value may be the given value of more or less 10% of the value.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The present invention relates to a newly discovered enzyme activity, *i.e*. to the demethylation of an R-substituted mono- or di-methoxyphenol by polyphenoloxidase (PPO) enzymes, wherein said R-substituted mono- or di-methoxyphenol is represented by the formula [1], [2] or [3], preferably by formula [1] or [2], most preferably by formula [1]. Within each of the formulas [1], [2] and [3], R can be any single atom or chemical moiety, but preferably is an organic moiety as defined herein, preferably comprising or consisting of 1-1000 atoms, wherein preferably each atom is selected from the group consisting of C, N, H, O and S. R preferably is -H, -OH, -COOH, - CH=CHCOOH, -CH₂=CH₂CH₃OH or lignin mono-, di- or polymer. The R-substituted mono- or di-methoxyphenol represented by the formula [1], [2] or [3] is, for example, any one selected from the group consisting of coniferyl, sinapyl, syringic acid, sinapic acid, ferulic acid, vanillic acid, 3,4 dihydroxy-5-methoxybenzoic acid, 3,4-dihydroxy-5-methoxycinnamic acid, 2-methoxyphenol (guaiacol) and syringol. It will be appreciated that, combinations of such R-substituted mono- or di-methoxyphenol may be employed. R-substituted mono- or di-methoxyphenol is, for example, 3-hydroxy-4-methoxycinnamic acid, 4-hydroxy-3-methoxyphenylactone, coniferyl aldehyde, ferulic acid, guaiacol, hesperidin, homovanillic acid, vanillic acid, 4-allyl-2,4-methoxyphenol, 3,4-dihydroxybenzoic acid, 3-methylcatechol, caffeic acid, 3,4-dihydroxy-5-methoxybenzoic acid or 3,4-dihydroxy-5-methoxycinnamic acid. R-substituted mono-methoxyphenol may, for example, be 3-hydroxy-4-methoxycinnamic acid, 4-hydroxy-3-methoxyphenylactone, coniferyl aldehyde, ferulic acid, guaiacol, homovanillic acid, vanillic acid, or caffeic acid. R-substituted di-methoxyphenol may, for example, be 4-allyl-2,4-methoxyphenol. R-substituted di-methoxyphenol may, for example, be 3,4-dihydroxy-5-methoxybenzoic acid or 3,4-dihydroxy-5-methoxycinnamic acid. R-substituted mono- or di-methoxyphenol represented by formulas [1], [2] or [3], wherein R is defined as indicated above, is indicated herein further as an "R-substituted mono- or di-methoxyphenol".

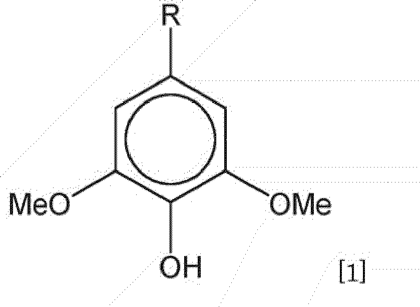

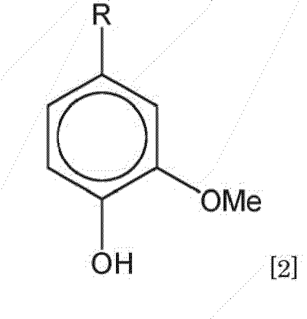

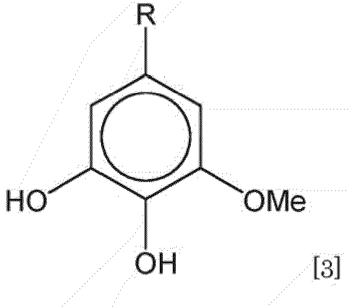

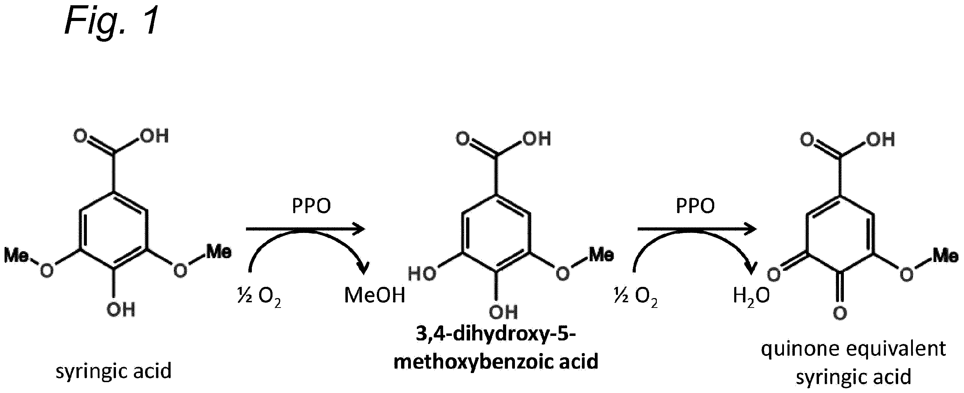

R-substituted mono- or di-methoxyphenols are present in lignin or lignin derived materials. The inventors discovered that the PPOs demethylate R-substituted di-methoxyphenols such as 4-hydroxy-3,5-dimethoxybenzoic acid (syringic acid) with the formation of 3,4-dihydroxy-5-methoxybenzoic acid and simultaneous release of methanol (see Figure 1 and Examples). The mode of action of the PPO is important for improving the reactivity of lignin or lignin derived materials and enable valorization.

Disclosed herein is an enzyme that belongs to a class of enzymes, *i.e* fungal tyrosinas-like polyphenoloxidases or PPOs, for which demethylation of R-substituted mono- or di-methoxyphenol compounds, such as dimethoxy phenol (syringol), 4-hydroxy-3,5-dimethoxybenzoic acid (syringic acid) and 3-(4-hydroxy-3,5-dimethoxyphenyl)prop-2-enoic acid (sinapic acid) has been established for the first time. Preferably, the enzyme or consists of a common central tyrosinase domain, preferably of the family with NCBI accession number pfam00264 (Volbeda and Hol, J. Mol. Biol. Volume 209(2): pages 249-279, 1989). Preferably, the enzyme is an enzyme that comprises or consists of an amino acid sequence that is at least 50%, 60%, 70%, 80%, more preferably at least 90%, 91%, 92%, 93%, 94%, even more preferably at least 95%, 96%, 97%, 98%, 99% or at least 100% identical to any one of SEQ ID NO: 37 (PPO4), SEQID NO: 41 (PPO6) and SEQ ID NO: 45 (PPO7). SEQ ID NO: 37, 41 and 45 represent the mature form of PPO4, PPO6, and PPO7, respectively. SEQ ID NO: 38, 42 and 46 represent PPO4, PP06 and PPO7 including the signal sequence, respectively. Preferably, the enzyme is an enzyme that comprises or consists of an amino acid sequence that is at least 50%, 60%, 70%, 80%, more preferably at least 90%, 91%, 92%, 93%, 94%, even more preferably at least 95%, 96%, 97%, 98%, 99% or at least 100% identical to any one of SEQ ID NO: 37 (PPO4), SEQ ID NO: 41 (PPO6) or SEQ ID NO: 45 (PPO7) and comprises a common central tyrosinase domain, preferably of the family with NCBI accession number pfam00264. Preferably said PPO is obtainable from a fungus, preferably of the genus *Myceliophthora.* Being "obtainable from" is to be understood herein as that the enzyme originates from the indicated organism, *i.e.* is naturally produced by the indicated organism as a native enzyme. More preferably, the PPO originates from *M. thermophila* C1 fungus or derivatives thereof, such as a *M. thermophila* C1 fungus selected from Garg 27K, (Accession No. VKM F- 3500D), UV13-6 (Accession No. VKM F-3632 D); NG7C-19 (Accession No. VKM F-3633 D); UV18-25 (Accession No. VKM F-3631 D); strain W1L (Accession No. CBS122189) or W1L#100L (Accession No. CBS122190), preferably a *M. thermophila* C1 (Accession No. VKM F- 3500D).

Preferably, the PPO of the disclosure, preferably for use in a method or process of the disclosure, has at least 50%, 60%, 70%, 80%, more preferably at least 90%, 91%, 92%, 93%, 94%, even more preferably at least 95%, 96%, 97%, 98%, 99% or at least 100% identical to any one of SEQ ID NO: 37 (PPO4), SEQ ID NO: 41 (PPO6) or SEQ ID NO: 45 (PPO7), is capable of demethylating an R-substituted mono- or di-methoxyphenol as defined herein. Being capable of demethylating an R-substituted mono- or di-methoxyphenol as defined herein, is to be understood herein as having at least 10%, 20%, 30%, 40%, 50%, 60%, 70%, 80%, 90%, 91%, 92%, 93%, 94%, 95%, 96%, 97%, 98%, 99% or 100%, preferably at least 40% of the demethylation activity of PPO7 as assessed by a method known in the art for assessing demethylation activity, preferably by using an assay as exemplified herein and when using a R-substituted mono- or di-methoxyphcnol as substrate, preferably said R-substituted mono- or di-methoxyphenol being syringic acid. In brief, in said method, the coding sequence of the enzyme of the disclosure is cloned in a pChi vector which is subsequently transformed in the *Myceliophthora thermophila* C1 strain W1L#100.1Δpyr5ΔAlp1 using the pyr5 marker as examplified herein (Example 1). Fermentations are preformed and subsequently, the broth is harvested, filtrated to remove fungal biomass, concentrated and dialyzed against 20 mM KPi pH 7.0 plus 150 mM KCl using a 5 kDa PES membrane (Vivacell® 70, Sartorius) as exemplified herein (Example 2). Optionally, samples are subsequently freeze-dried for storage and/or purified as exemplified herein (Example 5). Demethylating activity of the crude or purified enzyme can be determined by measuring an increased negative absorbance between 230 nm and 300 nm of syringic acid solution incubated with the enzyme (at RT for 24H as compared to 0H), preferably as exemplified herein (Example 6). An enzyme of the disclosure is understood herein to have at least 10% (or higher) of the demethylation activity of PPO7 if the enzyme shows an increase in negative absorbance that is at least this percentage (i.e. 10% or higher) of the increase found when expressing, producing and purifying PPO7 under the exact same conditions (*i.e*. using the coding sequence of PPO7, SEQ ID NO: 48).

Demethylating activity of the crude or purified enzyme can also be determined by measuring methanol production of syringic acid solution incubated with the enzyme (at RT for 90H) as exemplified herein (Example 8). An enzyme of the disclosure is understood to have at least 10% (or higher) of the demethylation activity of PPO7 if the enzyme shows an increase in methanol production that is at least this percentage (i.e. 10% or higher) of the increase found when expressing, producing and purifying PPO7 under the exact same conditions (*i.e*. using the coding sequence of PPO7, SEQ ID NO: 48).

Preferably, the enzyme of the disclosure has an optimal demethylation activity in the presence of a copper salt at a concentration of between about 1 and about 1000µM, preferably between about 20 and about 1000µM, wherein said copper salt is preferably selected from the group consisting of CuSO4, CuCl₂, CuBr₂, CuF₂, Cu(OH)₂ and Cu(NO₃)₂. Preferably the enzyme of the disclosure has an optimal demethylation activity at a pH between about 5.0 and about 8.0. Preferably the enzyme of the disclosure has an optimal demethylation activity at a temperature between about 20 and about 60°C. Preferably, the enzyme of the disclosure has demethylation activity in the presence of a copper salt at a concentration of between about 1 and about 1000µM, preferably between about 20 and about 1000µM, wherein said copper salt is preferably selected from the group consisting of CuSO4, CuCl₂, CuBr₂, CuF₂, Cu(OH)₂ and Cu(NO₃)₂, at a pH between about 5.0 and about 8.0 and at a temperature between about 20 and about 60°C.

Preferably, the enzyme of the disclosure is capable of demethylating the R-substituted mono- or di-methoxyphenol syringic acid. Preferably, the enzyme is capable of demethylating a substrate comprising R-substituted di-methoxyphenol (represented by formula I, wherein R is an organic moiety). More preferably, the enzyme of the disclosure is capable of demethylating the R-substituted mono- or di-methoxyphenol syringic acid, sinapic acid and syringol, preferably as assessed in a method as exemplified herein (Example 7). Preferably, the enzyme of the disclosure is capable of demethylating lignin and lignin-derived compounds from substrates such as wheat straw, corn stover, lowest molecular weight from lignin fractionation and carboxymodified lignin (wheat or corn), preferably as assessed as exemplified herein (Example 9). Preferably, the enzyme of the disclosure is not capable of demethylating 3-dimethoxybenzene and 3,5-dimethoxybenzoic acid, for example as assessed as exemplified herein (Example 9).

Disclosed herein is a nucleic acid molecule comprising, consisting essentially of, or consisting of, a nucleotide sequence encoding any of the enzyme of the first aspect. As further detailed in the Definitions Section, the nucleic acid molecule of the invention may include portions of a gene or polynucleotide encoding the protein that are not part of the coding region for the protein (e.g., introns or regulatory regions of a gene encoding the protein) and may be double stranded, single stranded and can include DNA, RNA, or derivatives of either DNA or RNA, including cDNA, probes and primers. Preferably, said encoding nucleic acid sequence has at least 50%, 60%, 70%, 80%, or more preferably at least 90%, 91%, 92%, 93%, 94%, 95%, 96%, 97%, 98%, 99% or 100% identity with SEQ ID NO: 39 (cDNA encoding PPO4), 40 (genomic DNA "gDNA" encoding PPO4), SEQ ID NO: 43 (cDNA encoding PPO6), SEQ ID NO: 44 (gDNA encoding PPO6), SEQ ID NO: 47 (cDNA encoding PPO7) or SEQ ID NO: 48 (gDNA encoding PPO7). Preferably, said encoding nucleic acid sequence has at least 50%, 60%, 70%, 80%, or more preferably at least 90%, 91%, 92%, 93%, 94%, 95%, 96%, 97%, 98%, 99% or 100% identity with SEQ ID NO: 40 (gDNA encoding PPO4), 44 (gDNA encoding PPO6), or 48 (gDNA encoding PPO7). Preferably, the nucleic acid molecule is an isolated nucleic acid molecule. Preferably, within the nucleic acid molecule, the nucleic acid sequence encoding any of the enzyme disclosed is operably linked to at least one expression control sequence and is preferably part of a (recombinant) expression vector as further detailed in the Definitions Section and/or as specifically exemplified herein (Example 1).

Optionally, the nucleic acid molecule encodes for one or more further enzymes. The nucleic acid molecule may be an expression vector (chimeric vector) encoding at least two enzymes of any one of the multi-enzyme complexes as disclosed herein, preferably said at least two enzymes or the PPO enzyme of the disclosure and the AA9 LPMO or LPMO9B as defined herein and as defined in EP15158572 or WO2010/107303A2, respectively. Also encompassed is an expression vector (chimeric vector) that expresses at the PPO enzyme, the LPMO, and an enzyme encoding a cellulase, for example an endoglucanase. Preferably, within said chimeric vector, the sequence encoding said AA9 LPMO encodes for an LPMO enzyme that has at least 50%, 60%, 70%, 80%, more preferably at least 90%, 91%, 92%, 93%, 94%, even more preferably at least 95%, 96%, 97%, 98%, or at least 99% identical to SEQ ID NO: 1. Preferably, said nucleic acid molecule comprises or consists of a nucleotide sequence that is at least 50%, 60%, 70%, 80%, more preferably at least 90%, 91%, 92%, 93%, 94%, even more preferably at least 95%, 96%, 97%, 98%, 99% or 100% identical to SEQ ID NO: 7, wherein SEQ ID NO: 7 represents the cDNA encoding *Mt*LPMO9A. Preferably, said nucleic acid molecule comprises or consists of a nucleotide sequence that is at least 50%, 60%, 70%, 80%, more preferably at least 90%, 91%, 92%, 93%, 94%, even more preferably at least 95%, 96%, 97%, 98%, 99% or 100% identical to SEQ ID NO: 8, wherein SEQ ID NO: 8 represents the gDNA encoding *Mt*LPMO9A, wherein the gDNA is to be understood herein as encompassing intron sequences. Preferably, said nucleic acid molecule comprises or consists of a nucleotide sequence that is at least 50%, 60%, 70%, 80%, more preferably at least 90%, 91%, 92%, 93%, 94%, even more preferably at least 95%, 96%, 97%, 98%, 99% or 100% identical to SEQ ID NO: 91, wherein SEQ ID NO: 91 represents the gDNA encoding *Mt*PMO9B. Optionally, within said chimeric vector, the sequence encoding said endoglucanase encodes for an endoglucanase enzyme that has at least 50%, 60%, 70%, 80%, more preferably at least 90%, 91%, 92%, 93%, 94%, even more preferably at least 95%, 96%, 97%, 98%, 99% or at least 100% identical to SEQ ID NO: 3, SEQ ID NO: 10, SEQ ID NO: 14, SEQ ID NO: 18, SEQ ID NO: 22, SEQ ID NO: 26 or SEQ ID NO: 30. Preferably, said endoglucanase encoding sequence is at least 50%, 60%, 70%, 80%, more preferably at least 90%, 91%, 92%, 93%, 94%, even more preferably at least 95%, 96%, 97%, 98%, 99% or 100% identical to SEQ ID NO: 9, wherein SEQ ID NO: 9 represents the cDNA encoding *Tv*EG I as defined herein. Preferably, said endoglucanase encoding sequence is at least 50%, 60%, 70%, 80%, more preferably at least 90%, 91%, 92%, 93%, 94%, even more preferably at least 95%, 96%, 97%, 98%, 99%, or 100% identical to SEQ ID NO: 9 (cDNA encoding *Tv*EG I), SEQ ID NO: 11 (cDNA encoding *Mt*EG VIII), SEQ ID NO: 15 (cDNA encoding *Mt*EG II), SEQ ID NO: 19 (cDNA encoding *M*tEG I), SEQ ID NO: 23 (cDNA encoding *Mt*EG III), SEQ ID NO: 27 (cDNA encoding *Mt*EG V), SEQ ID NO: 31 (cDNA encoding *Mt*EG VI). Preferably, said endoglucanase encoding nucleotide sequence is at least 50%, 60%, 700%, 80%, more preferably at least 90%, 91%, 92%, 93%, 94%, even more preferably at least 95%, 96%, 97%, 98%, 99% or 100% identical to SEQ ID NO: 12 (gDNA encoding *Mt*EG VIII), SEQ ID NO: 16 (gDNA encoding *Mt*EG II), SEQ ID NO: 20 (gDNA encoding *Mt*EG I), SEQ ID NO: 24 (gDNA encoding *Mt*EG III), SEQ ID NO: 28 (gDNA encoding *Mt*EG V), SEQ ID NO: 32 (gDNA encoding *Mt*EG VI).

It will be appreciated by one skilled in the art that use of recombinant DNA technologies can improve control of expression of transfected nucleic acid molecules by manipulating, for example, the number of copies of the nucleic acid molecules within the host cell, the efficiency with which those nucleic acid molecules are transcribed, the efficiency with which the resultant transcripts are translated, and the efficiency of posttranslational modifications. Additionally, the promoter sequence might be genetically engineered to improve the level of expression as compared to the native promoter. Recombinant techniques useful for controlling the expression of nucleic acid molecules include, but are not limited to, integration of the nucleic acid molecules into one or more host cell chromosomes, addition of vector stability sequences to plasmids, substitutions or modifications of transcription control signals (e.g., promoters, operators, enhancers), substitutions or modifications of translational control signals (e.g., ribosome binding sites), modification of nucleic acid molecules to correspond to the codon usage of the host cell, and deletion of sequences that destabilize transcripts.

Disclosed herein is a host cell comprising a nucleic acid molecule of the disclosure. Preferably, said host cell is transfected with an expression vector disclosed herein and said host cell is capable of expressing the enzyme disclosed herein encoded by said expression vector. The host cell of the disclosure may be, but is not limited to, any bacterial, fungal (*e.g*., filamentous fungi or yeast or mushrooms), algae, plant, insect, or animal cell that can be transfected. Preferably the host cell is a cultured cell. The host cell (e.g., a host cell or production organism) may include any microorganism (e.g., a bacterium, a protist, an alga, a fungus, or other microbe), and is preferably a bacterium, a yeast or a filamentous fungus. Suitable bacterial genera include, but are not limited to, *Escherichia. Bacillus, Lactobacillus, Pseudomonas* and *Streptomyces.* Suitable bacterial species include, but are not limited to, *Escherichia coli, Bacillus subtilis, Bacillus licheniformis, Bacillus stearothermophilus. Lactobacillus brevis, Pseudomonas aeruginosa* and *Streptomyces lividans.* Suitable genera of yeast include, but are not limited to, *Saccharomyces, Schizosaccharomyces, Candida, Hansenula, Pichia, Kluyveromyces,* and *Phaffia.* Suitable yeast species include, but are not limited to, *Saccharomyces cerevisiae, Schizosaccharomyces pombe, Candida albicans, Hansenula polymorpha, Pichia pastoris, Pichia canadensis, Kluyveromyces marxianus* and *Phaffia rhodozyma.*

Suitable fungal genera include, but are not limited to, *Chrysosporium, Thielavia. Neurospora, Aureobasidium, Filibasidium, Piromyces, Corynascus, Cryptococcus, Acremonium, Tolypocladium, Scytalidium, Schizophyllum, Sporotrichum, Penicillium, Gibberella, Myceliophthora, Mucor, Aspergillus, Fusarium, Humicola,* and *Trichoderma, Talaromyces, Rasamsonia* and anamorphs and teleomorphs thereof. Suitable fungal species include, but are not limited to, *Aspergillus niger, Aspergillus oryzae, Aspergillus nidulans, Aspergillus japonicus, Absidia coerulea, Rhizopus oryzae, Myceliophthora thermophila, Neurospora crassa, Neurospora intermedia, Trichoderma reesei, Trichoderma longibrachiatum, Penicillium canescens, Penicillium solitum, Penicillium funiculosum, Myceliophthora thermophila, Acremonium alabamense, Thielavia terrestris, Sporotrichum thermophile, Sporotrichum cellulophilum. Chaetomium globosum, Corynascus heterothallicus, Talaromyces emersonii, Rasamsonia elnersonii* and *Talaromyces flavus.*

The host cell of the disclosure may be a genetically modified cell or microorganism. Preferably, the host cell is modified/optimized for the production of the enzyme of the disclosure. Preferably, the host cell is modified in order to produce reduced amounts or lacks the production of enzymes that interfere/compete with the expression and or secretion of the enzyme disclosed herein and/or with the enzymatic activity of the method as disclosed herein. Such host cell may be modified in order to downregulate the interfering enzyme activity. The downregulation may be achieved, for example, by introduction of inhibitors (chemical or biological) of the enzyme activity, by manipulating the efficiency with which those nucleic acid molecules are transcribed, the efficiency with which the resultant transcripts are translated, and the efficiency of post-translational modifications, or by gene mutation, disruption or deletion, i.e. "knocking out" the endogenous copy of the gene. A "knock out" of a gene refers to a molecular biological technique by which the gene in the organism is made inoperative, so that the expression of the gene is substantially reduced or eliminated. Alternatively, the activity of the enzyme may be upregulated. Also contemplated herein is downregulating activity of one or more enzymes while simultaneously upregulating activity of one or more enzymes to achieve the desired outcome.

Preferably, a low cellulose strain is used. Preferably, the host cell is a fungal cell of strain Cl (VKM F-3500 D) or a mutant strain derived therefrom (e.g., UV13-6 (Accession No. VKM F-3632 D); NG7C-19 (Accession No. VKM F-3633 D); UV18-25 (VKM F-3631D), W1L (CBS122189), or W1L#100L (CBS122190)). Preferably, the host strain is a strain with reduced expression of protease and (hemi-)cellulase, more preferably free of protease and (hemi-)cellulase expression. Most preferred, the host strain is W1L#100.1Δpyr5ΔA1p1, also denominated as the LC strain (Visser H, et al., Ind. Biotechnol. 7:214-222, 2011 and WO2010/107303. As described in U.S. Patent No. 6,015,707 or U.S. Patent No. 6,573,086 a strain called Cl (Accession No. VKM F-3500 D), was isolated from samples of forest alkaline soil from Sola Lake, Far East of the Russian Federation. This strain was deposited at the All-Russian Collection of Microorganisms of Russian Academy of Sciences (VKM), (www.vkm.ru; Bakhurhina St. 8, Moscow, Russia, 113184; Prospekt Nauki No. 5, Pushchino, Moscow Region, 142290, Russia), under the terms of the Budapest Treaty on the International Regulation of the Deposit of Microorganisms for the Purposes of Patent Procedure on August 29, 1996, (by A.P. Sinitsyn, O.N. Okunev, I.V. Solov'eva, V.M. Chernoglasov, M.A. Emalfarb, A. Ben-Bassat; "FermTech" LTD Acad. Kapitsky str. 32-2, Moscow, 117647, Russia)as *Chrysosporium lucknowense* Garg 27K, VKM F- 3500 D. Various mutant strains of Cl have been produced and these strains have also been deposited at the All-Russian Collection of Microorganisms of Russian Academy of Sciences (VKM), (Bakhurhina St. 8, Moscow, Russia, 113184, Prospekt Nauki No. 5, Pushchino, Moscow Region, 142290, Russia), under the terms of the Budapest Treaty on the International Regulation of the Deposit of Microorganisms for the Purposes of Patent Procedure on September 2, 1998 (by O.N. Okunev, A.P. Sinitsyn, V.M. Chernoglasov, and M.A. Emalfarb; "FermTech" LTD, Acad. Kapitsy str., 32-2, Moscow, 117647, Russia) or at the Centraal Bureau voor Schimmelcultures (CBS), Uppsalalaan 8, 3584 CT Utrecht, The Netherlands for the purposes of Patent Procedure on December 5, 2007 (by Dyadic Nederland B.V., Nieuwe Kanaal 7s, 6709 PA Wageningen, Nederland). For example, Strain Cl was mutagenised by subjecting it to ultraviolet light to generate strain UV13-6 (Accession No. VKM F-3632 D; deposited with VKM on Sept. 2, 1998)). This strain was subsequently further mutated with N-methyl-N'-nitro-N-nitrosoguanidine to generate strain NG7C-19 (Accession No. VKM F-3633 D; deposited with VKM on Sept. 2, 1998). This latter strain in turn was subjected to mutation by ultraviolet light, resulting in strain UV18-25 (Accession No. VKM F-3631 D; deposited with VKM on Sept. 2, 1998). This strain in turn was again subjected to mutation by ultraviolet light, resulting in strain W1L (Accession No. CBS122189; deposited with CBS Dec. 5, 2007), which was subsequently subjected to mutation by ultraviolet light, resulting in strain W1L#100L (Accession No. CBS122190 ; deposited with CBS Dec. 5, 2007). Strain Cl was initially classified as a *Chrysosporium lucknowense* based on morphological and growth characteristics of the microorganism, as discussed in detail in U.S. Patent No. 6,015,707, U.S. Patent No. 6,573,086 and patent PCT/NL2010/000045. The Cl strain was subsequently reclassified as *Myceliophthora thermophila* based on genetic tests. *C*. *luknowense* has also appeared in the literature as *Sporotrichum thermophile.*

Also preferred is a genetically modified cell or microorganism, which is modified by having a reduction or elimination of enzymatic activities causing the formation of cellobionolactone/cellobionic acid and/or gluconolactone/ gluconic acid. Enzymes causing the formation of cellobionolactone/cellobionic acid and/or gluconolactone/ gluconic acid include. Preferably, such genetically modified cell or microorganism is a fungal strain lacking functional genes encoding enzymes causing the formation of cellobionolactone/cellobionic acid and/or gluconolactone/ gluconic acid e.g., may be generated by gene deletion, gene disruption, gene silencing or mutation; or by deletion, disruption or mutation of gene expression regulatory sequences such as promoter sequences, terminator sequences, promoter activating sequences and sequences encoding transcription factors; or by random or site-directed mutation of the genes encoding the enzymes causing the formation of cellobionolactone/cellobionic acid and/or gluconolactone/ gluconic acid. Preferably the modified fungus is a modified fungus wherein the one or more genes encoding enzymes responsible for the production of one or more products selected from cellobionolactone, cellobionic acid, gluconolactone, and gluconic acid encode a cellobiose dehydrogenase (CDH) is deleted, disrupted or mutated. Preferably, the one or more genes encode an enzyme having an amino acid sequence of the cellobiose dehydrogenase (CDH) selected from a group of polypeptides having at least 90%, 95%, or 99% homology with any of the polypeptides of SEQ ID NOS: 4-6. The modified fungus may be a modified fungus wherein the gene encoding the cellobiose dehydrogenase (CDH) CDH1 (SEQ ID NO: 4), CDH2 (SEQ ID NO: 5), or CDH3 (SEQ ID NO: 6) is deleted, disrupted or mutated. Preferably, the modified fungus is a fungus wherein CDH activity is reduced from about 50% to about 100%, or at least 75%, 90%, or 95%, when measured by a ferricyanide reduction assay. Preferably the gene encoding CDH1 (SEQ ID NO: 4) or encoding CDH2 (SEQ ID NO: 5) in *Myceliophthora thermophila* Cl is knocked out. More preferably, the genes encoding CDH 1 and CDH2 in *Myceliophthora thermophila* Cl are both knocked out (double knock out), preferably as described in WO2013/159005A2 and WO2012/061432A1.

Further suitable host cells include insect cells (most particularly *Drosophila melanogaster cells, Spodoptera frugiperda* Sf9 and Sf21 cells and *Trichoplusia* High-Five cells), nematode cells (particularly C. *elegans* cells), avian cells, amphibian cells (particularly *Xenopus laevis* cells), reptilian cells, and mammalian cells (most particularly human, simian, canine, rodent, bovine, or sheep cells, e.g. NIH3T3, CHO (Chinese hamster ovary cell), COS, VERO, BHK, HEK, and other rodent or human cells).

The present disclosure also contemplates genetically modified organisms such as algae, bacterial, and plants transformed with one or more nucleic acid molecules of the disclosure. The plants may be used for production of the enzymes, and/or as the lignin, lignocellulosic, cellulosic and/or hemicellulosic material used as a substrate in a method of the disclosure. Methods to generate recombinant plants are known in the art. For instance, numerous methods for plant transformation have been developed, including biological and physical transformation protocols. See, for example, Miki et al., "Procedures for Introducing Foreign DNA into Plants" in Methods in Plant Molecular Biology and Biotechnology, Glick, B.R. and Thompson, J.E. Eds. (CRC Press, Inc., Boca Raton, 1993) pp. 67-88. In addition, vectors and *in vitro* culture methods for plant cell or tissue transformation and regeneration of plants are available. See, for example, Gruber et al., "Vectors for Plant Transformation" in Methods in Plant Molecular Biology and Biotechnology, Glick, B.R. and Thompson, J.E. Eds, (CRC Press, Inc., Boca Raton, 1993) pp. 89-119.

The most widely utilized method for introducing an expression vector into plants is based on the natural transformation system of *Agrobacterium.* See, for example, Horsch et al., Science 227:1229 (1985). Descriptions of *Agrobacterium* vector systems and methods for *Agrobacterium-mediated* gene transfer are provided by numerous references, including Gruber et al., supra, Miki et al., supra, Moloney et al., Plant Cell Reports 8:238 (1989), and U.S. Patents Nos. 4,940,838 and 5,464,763.

Another generally applicable method of plant transformation is microprojectile-mediated transformation, see e.g., Sanford et al., Part. Sci. Technol. 5:27 (1987), Sanford, J.C., Trends Biotech. 6:299 (1988), Sanford, J.C., Physiol. Plant 79:206 (1990), Klein et al., Biotechnology 10:268 (1992). Another method for physical delivery of DNA to plants is sonication of target cells. Zhang et al., Bio/Technology 9:996 (1991). Alternatively, liposome or spheroplast fusion have been used to introduce expression vectors into plants. Deshayes et al., EMBO J., 4:2731 (1985), Christou et al., Proc Natl. Acad. Sci. USA 84:3962 (1987). Direct uptake of DNA into protoplasts using CaCl₂ precipitation, polyvinyl alcohol or poly-L-ornithine have also been reported. Hain et al., Mol. Gen. Genet. 199:161 (1985) and Draper et al., Plant Cell Physiol. 23:451 (1982). Electroporation of protoplasts and whole cells and tissues have also been described. Donn et al., In Abstracts of VIIth International Congress on Plant Cell and Tissue Culture IAPTC, A2-38, p. 53 (1990); D'Halluin et al., Plant Cell 4:1495-1505 (1992) and Spencer et al., Plant Mol. Biol, 24:51-61 (1994).

Disclosed herein is an enzyme composition or formulation comprising at least the enzyme of the disclosure.

The enzyme composition may be a crude fermentation product, preferably arising from recombinant microorganisms encoding the enzyme of the disclosure, or a preparation originating from a crude fermentation product that has been purified or partially purified using protein purification procedures known in the art. Alternatively, the enzyme of the enzyme composition is a protein or peptide that is produced synthetically (e.g., chemically, such as by peptide synthesis) or recombinantly in a substantial pure form.

The enzyme comprising formulation may be a cell-free composition or a composition comprising a host cell which comprises a nucleic acid molecule encoding the enzyme as defined herein, which is preferably capable of expressing the enzyme as defined herein.

The enzyme composition may be a multi-enzyme composition, which is understood to comprise the enzyme of the disclosure and at least one further (different) enzyme. A further enzyme is understood herein as an enzyme that is different in nature than the PPO enzyme of the disclosure and preferably has a different enzymatic activity. Specific multi-enzyme compositions as defined herein comprise the enzyme of the disclosure and at least one further enzyme that is particular suitable for use in a method as detailed herein below. The additional enzyme may also arise from an (optionally purified) crude fermentation product and/or be produced synthetically or recombinantly in a substantial pure form.

Preferably the enzyme or multi-enzyme composition is suitable for use in a method or process disclosed herein.

The enzyme or multi-enzyme composition of the disclosure may be a liquid, paste or solid formulation comprising the enzyme of the disclosure. Preferably, the solid formulation is a solid formulation that has the physical appearance of a granulate or a powder. A preferred formulation is a formulation comprising or consisting of coated or uncoated granules or micro-granules. The liquid, paste or solid formulation may be formulated in accordance with methods known in the art, preferably using methods known in the art of formulating enzymes and/or multi-enzyme compositions, feedstocks, chemical feedstocks, feed premixes, food supplements, bread improvers, flour treatment agents and/or pharmaceutical products. Preferably, the liquid, past or solid formulation according to this aspect is an enzyme or multi-enzyme composition, feedstock, chemical feedstock, animal feedstock, feed premix, food supplement, bread improver, flour treatment agent and/or pharmaceutical product, wherein preferably said composition comprises one or more further compounds known in the art to be suitable for use in such composition. Preferably the composition comprises stabilizers known in the art for enzyme stabilization, e.g. an antioxidant, a reducing agent, a polymer such as PVP, PVA, PEG or other suitable polymer known to be beneficial to the stability of polypeptides in solid or liquid compositions.

Preferably, granulates or agglomerated powder has a narrow particle size distribution with more than 95 % (by weight) of the particles in the range from 10 to 2000 µm, preferably from 25 to 500µm. Granulates and agglomerated powders may be prepared by conventional methods, *e*.*g*. by spraying the enzyme of the disclosure and optionally further enzymes onto a carrier in a fluid-bed granulator. The carrier may consist of particulate cores having a suitable particle size. The carrier may be soluble or insoluble, e.g. a salt (such as NaCl or sodium sulfate), a sugar (such as sucrose or lactose), a sugar alcohol (such as sorbitol), starch, rice, corn grits, or soy. Hence the disclosure also provides a granule comprising a composition comprising the enzyme of the disclosure, and optionally further enzymes, as defined herein.

It is to be understood that any of the enzymes described specifically herein can be combined with any one or more of the enzymes described herein or with any other available and suitable enzymes, to produce a multi-enzyme composition. The disclosure is not restricted or limited to the specific exemplary combinations listed below. One or more components of a multi-enzyme composition (other than the enzyme of the present disclosure) can be obtained from or derived from a microbial, plant, or other source or combination thereof.

The disclosure provides for a multi-enzyme composition, comprising at least the enzyme of the disclosure, that is for use in a method for conversion of lignin or a lignin-containing substrate to a value-added product, e.g. the production of biofuel, macromolecules and/or aromatic chemicals. Such multi-enzyme composition, preferably for use in a process to produce of macromolecules such as dispersants, emulsifiers, binders, sequestrants, carbon fibres, polymer modifiers, resins and adhesives, preferably further comprises one or more enzymes that specifically catalyze any one of sulfonation, introduction of ethyoxy, epoxy, vinyl and/or carbonyl moieties, depolymerization, intermolecular cross-linking, carbonylation, carboxylation, amination, epoxidation, de-etherification, oxidation and bioconjugation.

A further multi-enzyme composition, preferably for use in a process to produce aromatic monomers such as benzene, toluene, xylene, phenol, terephthalic acid monomeric lignin molecules, preferably further comprises enzymes that speficially catalyze dehyroxylation, dealkylation, hydrogenolysis, (selective) depolymerization, polymerization, sulfonation.

The multi-enzyme composition comprising at least the enzyme of the disclosure, may further comprise enzymes for degrading a lignocellulosic and/or hemicellulosic material or a biologically active fragment of an enzyme for degrading a lignocellulosic and/or hemicellulosic material. Such multi-enzyme composition is useful in particular for saccharification of (hemi-)cellulose in order to obtain fermentable product such as sugars. Apart from the enzyme of the disclosure, the multi-enzyme composition may further comprise at least one cello bio hydro lase, at least one xylanase, at least one endoglucanase, at least one β-glucosidase, at least one β-xylosidase, and at least one accessory enzyme. Preferably, the multi enzyme composition is free of enzymes which lead to the formation of cellobionolactone/cellobionic acid and/or gluconolactone/gluconic acid. A xylanase may be selected from the group consisting of: endoxylanases, exoxylanases, and β-xylosidases. Accessory enzymes include an enzyme selected from the group consisting of: cellulase, glucosidase, lytic polysaccharide monooxygenase (also referred to in the art as, for example, GH61 or polypeptide having cellulolytic enhancing activity), xylanase, xylosidase, ligninase, glucuronidase, arabinofuranosidase, arabinase, arabinogalactanase, ferulic acid esterase, lipase, pectinase, glucomannase, amylase, laminarinase, xyloglucanase, galactanase, galactosidase, glucoamylase, pectate lyase, chitosanase, exo-β-D-glucosaminidase, cellobiose dehydrogenase, and acetylxylan esterase. A preferred accessory enzyme is an LPMO of family AA9 (AA9 LPMO), preferably obtainable from Myceliophthora, preferably as defined in EP 151585 72 which is, more preferably obtainable from *Myceliophthora thermophila* C1. Preferably, the LPMO is an enzyme that comprises or consists of an amino acid sequence that is at least at least 50%, 60%, 70%, 80%, more preferably at least 90%, 91%, 92%, 93%, 94%, even more preferably at least 95%, 96%, 97%, 98%, 99% or at least 100% identical to SEQ ID NO: 1. The LPMO represented by SEQ ID NO: 1 is an AA9 family LPMO, denominated MLPMO9A, originating from *Myceliophthora thermophila* C1 (Accession No. VKM F- 3500D). The amino acid sequence of MLPM09A including the signal sequence is defined by SEQ ID NO: 2. A further preferred is accessory enzyme is an LPMO9B as defined herein. The multi-enzyme composition may further comprise at least one hemicellulase. A hemicellulase may be selected from the group consisting of a xylanase, an arabinofuranosidase, an acetyl xylan esterase, a glucuronidase, an endo-galactanase, a mannanase, an endo-arabinase, an exo-arabinase, an exo-galactanase, a ferulic acid esterase, a galactomannanase, a xyloglucanase, and mixtures thereof. A xylanase may be selected from the group consisting of endoxylanases, exoxylanases, and β-xylosidases in the absence of enzymes which lead to the formation of cellobionolactone/cellobionic acid and/or gluconolactone/gluconic acid. The multi-enzyme composition may further comprise at least one cellulase. The multi-enzyme composition may also include oxidases, oxygenases, monoxygenases, Baeyer-Villiger monooxygenases, dioxygenases, peroxidases, dehydrogenases, reductases that catalyze an oxidationreduction reaction. The multi-enzyme composition may further comprise at least one protein for degrading an arabinoxylan-containing material or a fragment thereof that has biological activity. The multi-enzyme composition may further comprise at least one endoxylanase, at least one β-xylosidase, and at least one arabinofuranosidase. An arabinofuranosidase may comprise an arabinofuranosidase with specificity towards single substituted xylose residues, an arabinofuranosidase with specificity towards double substituted xylose residues, or a combination thereof. The multi-enzyme compositions of a method can also include ligninases and lipases. While the multi-enzyme composition may contain many types of enzymes, mixtures comprising enzymes that increase or enhance sugar release from biomass, or biomass saccharification mixtures, are contemplated. Also preferred are mixtures that comprise enzymes that are capable of degrading cell walls and releasing cellular contents. Synergistic enzyme combinations and related methods are contemplated. The disclosure includes methods to identify the optimum ratios and compositions of enzymes. These methods entail tests to identify the optimum enzyme composition and ratios for efficient conversion of any biomass substrate to its constituent sugars. The Examples below include assays that may be used to identify optimum ratios and compositions of enzymes with which to degrade (ligno)cellulosic materials. Suitable enzymes may include, for example, enzymes disclosed in PCT Application Publication Nos. WO03/027306, WO200352118_A2, WO200352054_A2, WO200352057_A2, WO200352055_A2, WO200352056_A2, WO200416760_A2, WO9210581_A1, WO200448592_A2, WO200443980_A2, WO200528636_A2, WO200501065_A2, WO2005/001036, WO2005/093050, WO200593073_A1, WO200674005_A2, WO2009/149202, WO2011/038019, WO2010/141779, WO2011/063308, WO2012/125951, WO2012/125925, WO2012125937, WO/2011/153276, WO2014/093275, WO2014/070837, WO2014/070841, WO2014/070844, WO2014/09328 1, WO2014/093282, WO2014/093287, WO2014/093294, WO2015/084596, WO201517254_A1, WO201517255_A1, WO201517256_A1, or WO2016/069541.

The disclosure also provides for a multi-enzyme composition, comprising at least the enzyme of the disclosure, that is for use in a method for clarification and/or increasing the rate of filtration and/or decreasing haze formation of juices and/or beverages such as wine and beer. Such multi-enzyme composition preferably further comprises an LPMO (preferably the AA9 LPMO as defined herein above), pectinase, carboxymethylcellulase and/or amylase.

The disclosure also provides for a multi-enzyme composition, comprising at least the enzyme of the disclosure, that is for use in a method for macerating vegetables and/or fruit. Such multi-enzyme composition preferably further comprises an LPMO (preferably the AA9 LPMO or other LPMO as defined herein above), pectinase, carboxymethylcellulase and/or amylase.

The disclosure also provides for a multi-enzyme composition, comprising at least the enzyme of the disclosure, that is for use in a method for increasing digestibility and/or nutritional properties of animal feedstocks or animal food, such as, but not limited to, agricultural silage and grain feed. Such multi-enzyme composition preferably further comprises at least an LPMO (preferably the AA9 LPMO as defined herein above).

The disclosure also provides for a multi-enzyme composition, comprising at least the enzyme of the disclosure, that is for use in a method for increasing the quality of bakery products and or improve textual characteristics (break and shred quality and crumb quality) of the bakery products such as, but not limited to bread, e.g. preferably for increasing the volume of the bakery product, *e.g.* a high-rising and/or light loaf of whole meal bread. Such multi-enzyme composition preferably further comprises an LPMO (preferably the AA9 LPMO as defined herein above), a cellulase, such as beta-glucanase, cellulase, cellobiohydrolase and/or beta-glucosidase. Preferably, the cellulase preparation is a complex of cellulases and/or hemicellulases, preferably obtained from *Trichoderma sp.,* more preferably from *T. reesei.* Also preferred are cellulase preparations obtained from Aspergillus, preferably obtained from *A. niger. A* suitable cellulase composition may be CELLUCLAST™ (available from Novozymes) or Bakezyme® XU, Bakezyme® XE, BakeZyme® X-cell or Bakezyme® W (all available from DSM). The one or more additional enzymes for use in a method of the disclosure may further comprise additional enzymes, e.g., glucoamylase, α-amylase, xylanase, protease, lipase, phospholipase may be used together with the LPMO in the dough or the composition. The additional enzyme may be of any origin, including mammalian and plant, and preferably of microbial (bacterial, yeast or fungal) origin. The glucoamylase in this multi-enzyme composition may be a glucoamylase having a sequence identity of at least 50%, at least 60%, at least 65%, at least 70%, at least 75%, at least 80%, at least 85%, at least 90%, at least 95%, at least 96%, at least 97%, at least 98%, or at least 99% to the amino acid sequence of the *A. niger* G1 or G2 glucoamylase (Boel et al.(1984), EMBO J. 3(5), p. 1097-1102), the *A. awamori* glucoamylase disclosed in WO84/02921,or the *A. oryzae* glucoamylase (Agric. Biol. Chem. (1991), 55 (4), p. 941-949). The amylase may be fungal or bacterial, e.g. a maltogenic α-amylase from *B. stearothermophilus* or an α-amylase from *Bacillus,* e.g. *B. licheniformis* or *B. amyloliquefaciens,* a beta-amylase, e.g. from plant (e.g. soy bean) or from microbial sources (e.g. *Bacillus),* a glucoamylase, e.g. from *A. niger,* or a fungal α-amylase, e.g. from *A.oryzae.* Suitable commercial maltogenic α-amylases include NOVAMYLO (Novozymes A/S) and OPTICAKE® (Novozymes A/S). Suitable commercial fungal a-amylase compositions include, e.g., BAKEZYME P 300 (available from DSM) and FUNGAMYL 2500 SG, FUNGAMYL 4000 BG, FUNGAMYL 800 L, FUNGAMYL ULTRA BG and FUNGAMYL ULTRA SG (available from Novozymes A/S). The hemicellulase may be a pentosanase, e.g. a xylanase which may be of microbial origin, e.g. derived from a bacterium or fungus, such as a strain of *Aspergillus,* in particular of *A. aculeatus, A. niger, A. awamori,* or *A. tubigensis,* from a strain of *Trichoderma, e.g. T. reesei,* or from a strain of *Humicola,* e.g. *H. insolens.* Suitable commercially available xylanase preparations for use in the present invention include PENTOPAN MONO BG and PENTOPAN 500 BG (available from Novozymes), GRINDAMYL POWERBAKE (available from Danisco), and BAKEZYME BXP 5000 and BAKEZYME BXP 5001 (available from DSM). The protease may be from *Bacillus,* e.g. *B. amyloliquefaciens.* The lipase may be derived from a strain of Thermomyces (Humicola), Rhizomucor, Candida, Aspergillus, Rhizopus, or Pseudomonas, in particular from *T. lanuginosus (H. lanuginosa), Rhizomucor miehei,* C. *antarctica, A. niger, R. delemar, R. arrhizus* or *P. cepacia.* The phospholipase may have phospholipase A1, A2, B, C, D or lysophospholipase activity; it may or may not have lipase activity. It may be of animal origin, e.g. from pancreas, snake venom or bee venom, or it may be of microbial origin, e.g. from filamentous fungi, yeast or bacteria, such as Aspergillus or Fusarium, *e.g. A. niger, A. oryzae* or *F. oxysporum.* A preferred lipase/phospholipase from *F. oxysporum* is disclosed in WO 98/26057. Also, the variants described in WO 00/32758 may be used. The additional enzyme may be of any origin, including mammalian and plant, and preferably of microbial (bacterial, yeast or fungal) origin and may be obtained by techniques conventionally used in the art. Suitable phospholipase compositions are LIPOPAN F and LIPOPAN XTRA (available from Novozymes) or PANAMORE GOLDEN and PANAMORE SPRING (available from DSM).

The disclosure also provides for a multi-enzyme composition, comprising at least the enzyme of the disclosure, that is for use in the production of xylo-oligomers, (XOs), or oxidised xylo-oligomers, which may be used in pharmaceutical, agriculture and feed products. XOs are known to have pre-biotic effects, as they are neither hydrolyzed nor absorbed in the upper gastrointestinal tract, and they affect the host by selectively stimulating the growth or activity of one or a number of bacteria in the colon, thus improving health. Furthermore, XOs are believed to reduce cholesterol levels, maintenance of gastrointestinal health, and improvement of the biological availability of calcium. Furthermore, XOs inhibit starch retrogradation, improving the nutritional and sensory properties of food. The LPMO as defined herein is highly suitable for XOs production because of the low exoxylanase and/or β-xylosidase activity. This is beneficial as exoxylanase and/or β-xylosidase activity results in production of xylose, which has inhibitory effects on XO production. Such multi-enzyme composition preferably further comprises at least an LPMO (preferably the AA9 LPMO as defined herein above).

A preferred multi-enzyme composition of the disclosure is any multi-enzyme composition as defined above that comprises the enzyme of the disclosure and an LPMO (preferably the AA9 LPMO as defined herein above). The inventors have found that the enzyme of the disclosure has a surprising effect on the LPMO enzymatic activity resulting in hemi-cellulose degradation/modification, on substrates that comprise (hemi-)cellulose and R-substituted mono- or di-methoxyphenols. Without being bound by any theory, this surprising effect may be due to the production of reducing agents due to the newly discovered demethylating activity of the enzyme of the disclosure.

Also preferred is a multi-enzyme composition of the disclosure that is any multi-enzyme composition as defined above that comprises the enzyme of the disclosure and an LPMO (preferably the AA9 LPMO as defined herein above), and optionally further comprising an endoglucanase, preferably an endoglucanase that has the amino acid sequence of SEQ ID NO: 3 (encoding *Tv*EG I), SEQ ID NO: 10 (encoding *Mt*EG VIII), SEQ ID NO: 14 (encoding *Mt*EG II), SEQ ID NO: 18 (encoding *Mt*EG I), SEQ ID NO: 22 (encoding *Mt*EG III), SEQ ID NO: 26 (encoding *Mt*EG V) or SEQ ID NO: 30 (encoding *Mt*EG VI), and/or an endoglucanase as defined herein that has an amino acid sequence that is at least 50%, 60%, 70%, 80%, more preferably at least 90%, 91%, 92%, 93%, 94%, even more preferably at least 95%, 96%, 97%, 98%, or at least 99% identical to SEQ ID NO: 3, SEQ ID NO: 10, SEQ ID NO: 14, SEQ ID NO: 18, SEQ ID NO: 22, SEQ ID NO: 26 or SEQ ID NO: 30.

The enzymes of the multi-enzyme composition can be provided by a variety of sources. In one disclosure, the enzymes can be produced by growing organisms such as bacteria, algae, fungi, and plants which produce the enzymes naturally or by virtue of being genetically modified to express the enzyme or enzymes. In another disclosure, at least one enzyme of the multi-enzyme composition is a commercially available enzyme. Multi-enzyme compositions may be composed of enzymes from (1) commercial suppliers; (2) cloned genes expressing enzymes; (3) complex broth (such as that resulting from growth of a microbial strain in media, wherein the strains secrete proteins and enzymes into the media); (4) cell lysates of strains grown as in (3); and, (5) plant material expressing enzymes capable of degrading lignocellulose. In some disclosures, the accessory enzyme is a glucoamylase, a pectinase, or a ligninase.

The multi-enzyme compositions, in some disclosures, comprise microorganisms or a crude fermentation product of microorganisms. A crude fermentation product refers to the fermentation broth, which has been separated from the microorganism biomass (by filtration, for example). In general, the microorganisms are grown in fermenters, optionally centrifuged or filtered to remove biomass, and optionally concentrated, formulated, and dried to produce an enzyme(s) or a multi-enzyme composition that is a crude fermentation product. In other disclosures, enzyme(s) or multi-enzyme compositions produced by the microorganism (including a genetically modified microorganism as described herein) are subjected to one or more purification steps, such as ammonium sulfate precipitation, chromatography, and/or ultrafiltration, which result in a partially purified or purified enzyme(s). If the microorganism has been genetically modified to express the enzyme(s), the enzyme(s) will include recombinant enzymes. If the genetically modified microorganism also naturally expresses the enzyme(s) or other enzymes useful in a method of the disclosure, such as for lignocellulosic saccharification or any other useful application mentioned herein, the enzyme(s) may include both naturally occurring and recombinant enzymes.

Preferably, the multi-enzyme composition for use in a method of the disclosure comprising at least about 500 ng, and preferably at least about 1 µg, and more preferably at least about 5 µg, and more preferably at least about 10 µg, and more preferably at least about 25 µg, and more preferably at least about 50 µg, and more preferably at least about 75 µg, and more preferably at least about 100 µg, and more preferably at least about 250 µg, and more preferably at least about 500 µg, and more preferably at least about 750 µg, and more preferably at least about 1 mg, and more preferably at least about 5 mg, of an enzyme, an (isolated) protein comprising any of the proteins or homologues, variants, or fragments thereof discussed herein. Such a multi-enzyme composition may include any carrier with which the protein is associated by virtue of the protein preparation method, a protein purification method, or a preparation of the protein for use in any method according to the present disclosure. For example, such a carrier can include any suitable buffer, extract, or medium that is suitable for combining with the protein of the present disclosure so that the protein can be used in any method described herein according to the present disclosure.

In one embodiment of the disclosure, one or more enzymes in the multi-enzyme composition of the disclosure are bound to a solid support, *i.e*., an immobilized enzyme. As used herein, an immobilized enzyme includes immobilized isolated enzymes, immobilized microbial cells which contain one or more enzymes of the disclosure, other stabilized intact cells that produce one or more enzymes of the disclosure, and stabilized cell/membrane homogenates. Stabilized intact cells and stabilized cell/membrane homogenates include cells and homogenates from naturally occurring microorganisms expressing the enzymes of the disclosure and preferably, from genetically modified microorganisms as disclosed elsewhere herein. Thus, although methods for immobilizing enzymes are discussed below, it will be appreciated that such methods are equally applicable to immobilizing microbial cells and in such a disclosure, the cells can be lysed, if desired. A variety of methods for immobilizing an enzyme are disclosed in Industrial Enzymology 2nd Ed., Godfrey, T. and West, S. Eds., Stockton Press, New York, N.Y., 1996, pp. 267-272; Immobilized Enzymes, Chibata, I. Ed., Halsted Press, New York, N.Y., 1978; Enzymes and Immobilized Cells in Biotechnology, Laskin, A. Ed., Benjamin/Cummings Publishing Co., Inc., Menlo Park, California, 1985; and Applied Biochemistry and Bioengineering, Vol. 4, Chibata, I. and Wingard, Jr., L. Eds, Academic Press, New York, N.Y., 1983.

The PPO enzyme of the disclosure in the formulation may be an enzyme or protein as defined herein, *e.g.* a crude fermentation product, or a protein preparation that has been purified or partially purified (e.g., from a microorganism) using protein purification procedures known in the art, or an enzyme, protein or peptide that can be produced synthetically (e.g., chemically, such as by peptide synthesis) or recombinantly. The PPO in the formulation may also be a nucleic acid molecule as defined herein, *e.g.* a recombinant nucleic acid molecule and/or vector and/or host cell encoding said PPO enzyme as defined herein. The PPO comprising formulation may be a cell-free composition or a composition comprising a host cell which comprises a nucleic acid molecule encoding the PPO as defined herein, which is preferably capable of expressing the PPO as defined herein. The same description applies to reference to other enzymes, proteins or peptides described herein and to other microbial sources for such proteins or peptides.

The enzyme or multi-enzyme compositions, in some disclosures, comprise microorganisms or a crude fermentation product of microorganisms. A crude fermentation product refers to the fermentation broth, which has been separated from the microorganism biomass (by filtration, for example). In general, the microorganisms are grown in fermenters, optionally centrifuged or filtered to remove biomass, and optionally concentrated, formulated, and dried to produce an enzyme(s) or a multi-enzyme composition that is a crude fermentation product. In other disclosures, enzyme(s) or multi-enzyme compositions produced by the microorganism (including a genetically modified microorganism as described below) are subjected to one or more purification steps, such as ammonium sulfate precipitation, chromatography, and/or ultrafiltration, which result in a partially purified or purified enzyme(s). If the microorganism has been genetically modified to express the enzyme(s), the enzyme(s) will include recombinant enzymes. If the genetically modified microorganism also naturally expresses the enzyme(s) or other enzymes useful for lignocellulosic saccharification or any other useful application mentioned herein, the enzyme(s) may include both naturally occurring and recombinant enzymes.

Preferably, the enzyme or multi-enzyme composition for use in a method of the disclosure comprising at least about 500 ng, and preferably at least about 1 µg, and more preferably at least about 5 µg, and more preferably at least about 10 µg, and more preferably at least about 25 µg, and more preferably at least about 50 µg, and more preferably at least about 75 µg, and more preferably at least about 100 µg, and more preferably at least about 250 µg, and more preferably at least about 500 µg, and more preferably at least about 750 µg, and more preferably at least about 1 mg, and more preferably at least about 5 mg, of an enzyme, an (isolated) protein comprising any of the proteins or homologues, variants, or fragments thereof discussed herein. Such a multi-enzyme composition may include any carrier with which the protein is associated by virtue of the protein preparation method, a protein purification method, or a preparation of the protein for use in any method according to the present invention. For example, such a carrier can include any suitable buffer, extract, or medium that is suitable for combining with the protein of the present disclosure so that the protein can be used in any method described herein, according to the present disclosure.

In one disclosure, one or more enzymes for use in a method of the disclosure are bound to a solid support, *i.e*., an immobilized enzyme. As used herein, an immobilized enzyme includes immobilized isolated enzymes, immobilized microbial cells which contain one or more enzymes of the disclosure, other stabilized intact cells that produce one or more enzymes of the disclosure, and stabilized cell/membrane homogenates. Stabilized intact cells and stabilized cell/membrane homogenates include cells and homogenates from naturally occurring microorganisms expressing the enzymes of the disclosure and preferably, from genetically modified microorganisms as disclosed elsewhere herein. Thus, although methods for immobilizing enzymes are discussed below, it will be appreciated that such methods are equally applicable to immobilizing microbial cells and the cells can be lysed, if desired. A variety of methods for immobilizing an enzyme are disclosed in Industrial Enzymology 2nd Ed., Godfrey, T. and West, S. Eds., Stockton Press, New York, N.Y., 1996, pp. 267-272; Immobilized Enzymes, Chibata, I. Ed., Halsted Press, New York, N.Y., 1978; Enzymes and Immobilized Cells in Biotechnology, Laskin, A. Ed., Benjamin/Cummings Publishing Co., Inc., Menlo Park, California, 1985; and Applied Biochemistry and Bioengineering, Vol. 4, Chibata, I. and Wingard, Jr., L. Eds, Academic Press, New York, N.Y., 1983.

Disclosed herein is a method/process to produce an enzyme of the disclosure and/or multi-enzyme composition of the disclosure. Preferably, said method or process encompasses the step of transfectng a host cell with one or more nucleic acid molecules, for example expression vectors, which may be expression vectors of the disclosure, for example in a host cell which may be as disclosed herein. One or more protein(s) encoded by said nucleic acid molecule or expression vector may be produced by culturing the transfected host cell under conditions effective to produce the protein. Encompassed herein is a host cell produces at least two enzymes of a multi-enzyme complex as disclosed herein, preferably said at least two enzymes or the PPO of the disclosure and the AA9 LPMO as disclosed herein. In some instances, the protein may be recovered, and in others, the cell may be harvested in whole, either of which can be used in a composition. Microorganisms for use as host cells are cultured in an appropriate fermentation medium. An appropriate, or effective, fermentation medium refers to any medium in which the hosed cell, when cultured, is capable of expressing the enzyme of the disclosure. The microorganisms can be cultured by any fermentation process which includes, but is not limited to, batch, fedbatch, cell recycle, and continuous fermentation. In general, fungal strains are grown in fermenters, optionally centrifuged or filtered to remove biomass, and optionally concentrated, formulated, and dried to produce an enzyme(s) or a multi-enzyme composition that is a crude fermentation product. Particularly suitable conditions for culturing filamentous fungi are described, for example, in U.S. Patent No. 6,015,707 and U.S. Patent No. 6,573,086, *supra.*

Optionally, the method or process further comprises recovering the enzyme and/or multi-enzyme composition of the disclosure. Depending on the vector and host system used for production, resultant proteins may either remain within the recombinant cell; be secreted into the culture medium; be secreted into a space between two cellular membranes; or be retained on the outer surface of a cell membrane. The phrase "recovering the enzyme" refers to collecting the whole culture medium containing the protein and optionally implies additional steps of separation or purification. The enzyme and/or multi-enzyme composition produced can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential precipitation or solubilisation, resulting in substantially pure enzyme. As used herein, "substantially pure" refers to a purity that allows for the effective use of the enzyme in any method according to the present disclosure, *i*.*e*. substantially free of contaminants, other proteins and/or chemicals that might interfere or that would interfere with its use in a method of the present disclosure *(e.g*., that might interfere with enzyme activity), or that at least would be undesirable for inclusion with an enzyme of the disclosure when it is used in a method of the present disclosure (described in detail below). Preferably, a "substantially pure" enzyme, as referenced herein, is an enzyme that can be produced by any method *(i.e.,* by direct purification from a natural source, recombinantly, or synthetically), and that has been purified from other protein components such that the enzyme makes up at least about 80% weight/weight of the total protein in a given composition (e.g., the enzyme of interest is about 80% of the protein in a solution/composition/buffer), and more preferably, at least about 85%, and more preferably at least about 90%, and more preferably at least about 91%, and more preferably at least about 92%, and more preferably at least about 93%, and more preferably at least about 94%, and more preferably at least about 95%, and more preferably at least about 96%, and more preferably at least about 97%, and more preferably at least about 98%, and more preferably at least about 99%, weight/weight of the total protein in a given composition.

Disclosed herein is a method of demethylation (or demethylation method) of an R-substituted mono- or di-methoxyphenol in a R-substituted mono- or di-methoxyphenol comprising substrate, wherein said method comprises the step of contacting said substrate with an enzyme of the disclosure and/or a multi-enzyme composition of the disclosure. Preferably, the R-substituted mono-or di-methoxyphenol comprising substrate is lignin or lignin biomass or lignin biomiass saccharification mixture or a lignin derived compound, more preferably lignin or lignin biomass or lignin biomass saccharification mixture rich in S-groups and/or G-groups, most preferably rich in S-groups. Lignin or lignin biomass rich in S-groups and/or G groups is understood herein as being lignin or lignin biomass wherein the total percentage of S and G units together are at least 80% of the total amount of S, G and H. Examples of such biomass resources being rich in S and G units are: wheat straw, rice straw, rey straw, hemp, tall fescue, jute, sisal, banana plant leaf, loblolly pine, spurce (Picea Abies), Eucalyptus globus, Eucalyptus grandis, Birch pendula, Beech, Acacia, Carpinus betulus MWL (Milled-Wood Lignin), Eucryphia codrifolia MWL, Bambusa sp. MWL and Fagurs sylfatica. Examples thereof that are specifically rich in S units (*i.e.* at least 60% of the total of S, G and H): jute, sisal, Eucalyptus globus, Eucalyptus grandis, Birch pendula, Carpinus betulus MWL, Fagus sylvatica. Preferably, said lignin is low molecular lignin suchs as Biolignin and/or organosolv lignin. The R-substituted mono- or di-methoxyphenol comprising substrate may also be any R-substituted mono-or di-methoxyphenol comprising substrate that is not lignin-derived. Preferred R-substituted mono- or di-methoxyphenols comprised within said substrate are selected from, but not limited to, the group consisting of: syringic acid, vanillic acid, 3,4-dihydroxy-5-methoxybenzoic acid, sinapic acid, ferulic acid, 3,4-dihydroxy-5-methoxycinnamic acid, syringol, 2-methoxyphenol (guaiacol), 3-methoxycatechol and any combination thereof.

Preferably, R-substituted mono- or di-methoxyphenol comprising substrate further comprises between about 1 and 1000µM of a copper salt, preferably between 20 and about 1000µM a copper salt, wherein said copper salt is preferably selected from the group consisting of CuS04, CuCl₂, CuBr₂, CuF₂, Cu(OH)₂ and Cu(NO₃)₂. Preferably the pH between of the R-substituted mono- or di-methoxyphenol comprising substrate is between about 5.0 and about 8.0. Preferably, the method step is performed at a temperature between about 20 and about 60°C. Even more preferably, the R-substituted mono- or di-methoxyphenol comprising substrate further comprises between about 1 and about 1000µM a copper salt, preferably between 20 and about 1000µM a copper salt, wherein said copper salt is preferably selected from the group consisting of CuSO4, CuCl₂, CuBr₂, CuF₂, Cu(OH)₂ and Cu(NO₃)₂, has a pH between about 5.0 and about 8.0 and the method step is performed at a temperature between about 20 and about 60°C.

Preferably, the demethylation method is part of a larger process (*i.e*. comprising additional steps) as detailed below. Preferably, said process further comprises the step of contacting the substrate with any further enzyme of the specific multi-enzyme composition as indicated to be useful for said process as detailed in the fourth aspect herein, either at once (for instance via the addition of a multi-enzyme composition) or sequentially.

Prerably, the demethylation method is part of a process to convert lignin into a value-added product, *e*.*g*. the production of biofuel, macromolecules and/or aromatic chemicals.

In a disclosure, the demethylation method of is part of the process to produce of macromolecules such as dispersants, emulsifiers, binders, sequestrants, carbon fibres, polymer modifiers, resins and adhesives, wherein the production process takes advantage of lignin's polymer and polyelectrolyte properties. Such process preferably further comprises the step of sulfonation, introduction of ethyoxy, epoxy,vinyl and/or carbonyl moieties, depolymerization, intermolecular crosslinking, increasing phenolic functionality (such as carbonylation, carboxylation, amination, epoxidation and de-etherification) and/or melt-spinning.

In a further disclosure, the demethylation method is part of the process to produce aromatic monomers such as benzene, toluene, xylene, phenol, terephthalic acid monomeric lignin molecules. Preferably, such process further comprises the step of catalysis, such as dehyroxylation, dealkylation, hydrogenolysis, depolymerization, polymerization, sulfonation. A depolymerization step is preferably a selective depolymerziation step. A selective depolymerization step may give rise to complex aromatics that are difficult to make via conventional petrochemical routes. In turn, these aromatic monomers may serve as building blocks for further conversion such as polymerization.

In a further disclosure, the demethylation method may be part of a process to obtain fermentable products, such as sugars (saccarification process), from degrading biomass and/or lignocellulosic material, preferably biomass rich in (hemi-)cellubse. Preferably, said lignocellulosic material is agro-waste or a residue produced by agriculture and forestry. The lignocellulosic material may be partially or completely degraded to fermentable sugars. Economical levels of degradation at commercially viable costs are contemplated. Preferably, said method further comprises the step of contacting the substrate with at least one cellobiohydrolase, at least one xylanase, at least one endoglucanase, at least one β-glucosidase, at least one β-xylosidase, and at least one accessory enzyme, wherein these enzymes are composed in a multi enzyme composition as defined herein and/or are provided as individual compounds. Preferred endoglucanases, β-g|ucosidases, β-xylosidases, accessory enzymes and/or optional further enzymes useful in a saccarification process are disclosed herein.

Preferably, the demethylation method is part of a process to dissolve pulp for the production of chemicals such as, but not limited to, rayon, cellophane and several chemicals such as cellulose esters (acetates, nitrates, propionates and butyrates) and cellulose ethers (carboxymethyl cellulose and methyl and ethyl cellulose) and ethanol (for instance as bioethanol biofuel). Also preferred is the method for paper and pulp bleaching. The substrate comprising R-substituted mono- or di-methoxyphenol (either present already in the substrate or added as part of the method) in this disclosure comprises or consists of paper or pulp, preferably soft and/or hardwood pulp. The method may be used to improve bleachability of pulp in the pulp and paper industry.

In a further disclosure, the demethylation method is part of a process to degrade Distillers Dried Grain (DDG), wherein the substrate comprises preferably DDG derived from corn, to sugars. In certain disclosures, at least 10% of fermentable sugars are liberated. Preferably, at least 15% of the sugars are liberated, or at least 20% of the sugars are liberated, or at least 23% of the sugars are liberated, or at least 24% of the sugars are liberated, or at least 25% of the sugars are liberated, or at least 26% of the sugars are liberated, or at least 27% of the sugars are liberated, or at least 28% of the sugars are liberated.

Preferably, the demethylation process comprises a pretreatment process. In general, a pretreatment process will result in components of the lignocellulose being more accessible for downstream applications such as digestion by enzymes. The pretreatment can be a chemical, physical or biological pretreatment. The lignocellulose may have been previously treated to release some or all of the sugars, as in the case of DDG. Physical treatments, such as grinding, boiling, freezing, milling, vacuum infiltration, and the like may also be used with the methods of the invention. In one embodiment, the heat treatment comprises heating the lignocellulosic material to 121°C for 15 minutes. A physical treatment such as milling can allow a higher concentration of lignocellulose to be used in the methods of the invention. A higher concentration refers to about 20%, up to about 25%, up to about 30%, up to about 35%, up to about 40%, up to about 45%, or up to about 50% lignocellulose. The lignocellulose may also be contacted with a metal ion, ultraviolet light, ozone, and the like. Additional pretreatment processes are known to those skilled in the art, and can include, for example, organosoly treatment, steam explosion treatment, lime impregnation with steam explosion treatment, hydrogen peroxide treatment, hydrogen peroxide/ozone (peroxone) treatment, acid treatment, dilute acid treatment, and base treatment, including ammonia fiber explosion (AFEX) technology. Details on pretreatment technologies and processes can be found in Wyman et al., Bioresource Tech. 96:1959 (2005); Wyman et al., Bioresource Tech. 96:2026(2005); Hsu, "Pretreatment of biomass" In Handbook on Bioethanol: Production and Utilization, Wyman, Taylor and Francis Eds., p. 179-212 (1996); and Mosier et al., Bioresource Tech. 96:673 (2005).

In some disclosures, the method/process steps may be performed one or more times in whole or in part. That is, one may perform one or more pretreatments, followed by one or more reactions with a composition of a method of the disclosure. The enzymes may be added in a single dose, or may be added in a series of small doses. Further, the entire process may be repeated one or more times as necessary. Therefore, one or more additional treatments with heat and enzymes are contemplated.

The methods described above result in the production of fermentable sugars. During, or subsequent to the method, the fermentable sugars may be recovered and/or purified by any method known in the art. The sugars can be subjected to further processing; e.g., they can also be sterilized, for example, by filtration.

In an additional embodiment, provided is a method/process, comprising the demethylation method, that is for producing an organic substance, comprising saccharifying a lignocellulosic material with an effective amount of the enzyme of the disclosure and LPMO as defined herein fermenting the saccharified lignocellulosic material obtained with one or more microorganisms, and recovering the organic substance from the fermentation. Sugars released from biomass can be converted to useful fermentation products including but not limited to amino acids, vitamins, pharmaceuticals, animal feed supplements, specialty chemicals, chemical feedstocks, plastics, solvents, fuels, or other organic polymers, lactic acid, and ethanol, including fuel ethanol. Specific products that may be produced by the methods described herein include, but not limited to, biofuels (including ethanol); lactic acid; plastics; specialty chemicals; organic acids, including citric acid, succinic acid, itaconic and maleic acid; solvents; animal feed supplements; pharmaceuticals; vitamins; amino acids, such as lysine, methionine, tryptophan, threonine, and aspartic acid; industrial enzymes, such as proteases, cellulases, amylases, glucanases, lactases, lipases, lyases, oxidoreductases, and transferases; and chemical feedstocks. The methods described herein are also useful to generate feedstocks for fermentation by fermenting microorganisms. In one embodiment, the method further comprises the addition of at least one fermenting organism. As used herein, "fermenting organism" refers to an organism capable of fermentation, such as bacteria and fungi, including yeast. Such feedstocks have additional nutritive value above the nutritive value provided by the liberated sugars.

The one or more additional enzymes for use in a method/process of the present invention preferably also comprise enzyme combinations that break down or modify lignin or lignin-containing material, reducing or preventing unwanted adsorption of other components of multi-enzyme compositions applied. Such enzyme combinations or mixtures can include a multi-enzyme composition that contains at least one protein of the host organism or one or more enzymes or other proteins from other microorganisms, plants, or similar organisms. Synergistic enzyme combinations and related methods are contemplated. The invention includes methods to identify the optimum ratios and compositions of enzymes with which to degrade each lignin and lignocellulosic material. These methods entail tests to identify the optimum enzyme composition and ratios for efficient conversion of any biomass substrate to its constituent sugars. The Examples below include assays that may be used to identify optimum ratios and compositions of enzymes with which to degrade lignocellulosic materials.

The demethylation method may also be part of a process that is for use is a method for clarification and/or increasing the rate of filtration and/or decreasing haze formation of juices and/or beverages such as wine and beer. In this embodiment, the R-substituted mono- or di-methoxyphenol-comprising substrate preferably comprises or consists of juice from fruit or vegetable such as, but not limited to, apple-, pineapple- , orange- and tomotato-, prune-, cranberry- juice, and/or cereals, such as, but not limited to barley, corn, wheat, rye, oats and maize. In this disclosure, the one or more additional enzymes for use in a method of the invention further preferably comprise an LPMO (preferably the AA9 LPMO as defined herein above), pectinase, carboxymethylcellulase and/or amylase.

In a further disclosure, the method of the demethylation method is part of a process for macerating vegetables and/or fruit. In this disclosure, the R-substituted mono- or di-methoxyphenol-comprising substrate is a fruit and/or a vegetable. In this disclosure, the one or more additional enzymes for use in a method further preferably comprise an LPMO (preferably the AA9 LPMO as defined herein above), pectinase, carboxymethylcellulase and/or amylase.

In another disclosure, the demethylation method is part of a process for extraction of coffee, plant oils, and starch, wherein the method comprises at contacting the substrate with the enzyme of the disclosure and an LPMO (preferably the AA9 LPMO as defined herein above).

In another disclosure, the demethylation method is part of a process the production of xylitol, a valuable sweetener that has applications in both the pharmaceutical and food industries, wherein the method comprises contacting the substrate with the enzyme of the disclosure and an LPMO (preferably the AA9 LPMO as defined herein above).

In another disclosure the demethylation method of the invention is part of a process to increase digestibility and/or nutritional properties of animal feedstocks or animal food, such as, but not limited to, agricultural silage and grain feed, wherein the method comprises contacting the substrate with the enzyme of the disclosure and an LPMO (preferably the AA9 LPMO as defined herein above). Said animal feedstock preferably contains cereals (e.g. barley, wheat, maize, rye or oats) or cereal byproducts. Preferably, the substrate in this embodiment comprises or consists of animal feedstock such as, but not limited to, agricultural silage and grain feed, preferably comprising cereals, cereal and/or cereal solution.

In another disclosure, the demethylation method is part of a process to increase the quality of bakery products and/or improve textual characteristics (break and shred quality and crumb quality) of the bakery products such as, but not limited to bread. Preferably, the method is applied in order to increase the volume of the bakery product, e.g. a high-rising and/or light loaf of whole meal bread. In this disclosure, the one or more additional enzymes for use in a method of the disclosure further preferably comprise an LPMO (preferably the AA9 LPMO as defined herein above), a cellulase, such as beta-glucanase, cellulase, cellobiohydrolase and/or beta-glucosidase. Preferably, the cellulase preparation is a complex of cellulases and/or hemicellulases, preferably obtained from *Trichoderma sp.*, more preferably from *T. reesei.* Also preferred are cellulase preparations obtained from Aspergillus, preferably obtained from *A. niger.* A suitable cellulase composition may be CELLUCLAST™ (available from Novozymes) or Bakezyme® XU, Bakezyme® XE, BakeZyme® X-cell or Bakezyme® W (all available from DSM). The one or more additional enzymes for use in a method of the invention may further comprise additional enzymes, e.g., glucoamylase, α-amylase, xylanase, protease, lipase, phospholipase may be used together with the LPMO in the dough or the composition. The additional enzyme may be of any origin, including mammalian and plant, and preferably of microbial (bacterial, yeast or fungal) origin. The glucoamylase for use in the present disclosure also include glucoamylases having a sequence identity of at least 50%, at least 60%, at least 65%, at least 70%, at least 75%, at least 80%, at least 85%, at least 90%, at least 95%, at least 96%, at least 97%, at least 98%, or at least 99% to the amino acid sequence of the *A*. *niger* G1 or G2 glucoamylase (Boel et al. (1984), EMBO J. 3(5), p. 1097-1102), the *A. awamori* glucoamylase disclosed in WO84/02921, or the *A. oryzae* glucoamylase (Agric. Biol. Chem. (1991), 55 (4), p. 941-949). The amylase may be fungal or bacterial, e.g. a maltogenic α-amylase from *B*. *stearothermophilus* or an α-amylase from *Bacillus, e.g. B. licheniformis* or *B*. *amyhliquefaciens,* a beta-amylase, e.g. from plant (e.g. soy bean) or from microbial sources (e.g. *Bacillus),* a glucoamylase, e.g. from *A. niger,* or a fungal α-amylwse, e.g. from *A.oryzae.* Suitable commercial maltogenic α-amylases include NOVAMYLO (Novozymes A/S) and OPTICAKE® (Novozymes A/S). Suitable commercial fungal α-amylase compositions include, e.g., BAKEZYME P 300 (available from DSM) and FUNGAMYL 2500 SG, FUNGAMYL 4000 BG, FUNGAMYL 800 L, FUNGAMYL ULTRA BG and FUNGAMYL ULTRA SG (available from Novozymes A/S). The hemicellulase may be a pentosanase, e.g. a xylanase which may be of microbial origin, e.g. derived from a bacterium or fungus, such as a strain of *Aspergillus*, in particular of *A. aculeatus, A. niger, A. awamori,* or *A. tubigensis,* from a strain of *Trichoderma,* e.g. *T. reesei,* or from a strain of *Humicola,* e.g. *H. insolens.* Suitable commercially available xylanase preparations include PENTOPAN MONO BG and PENTOPAN 500 BG (available from Novozymes), GRINDAMYL POWERBAKE (available from Danisco), and BAKEZYME BXP 5000 and BAKEZYME BXP 5001 (available from DSM). The protease may be from *Bacillus,* e.g. *B*. *amyloliquefaciens.* The lipase may be derived from a strain of Thermomyces (Humicola), Rhizomucor, Candida, Aspergillus, Rhizopus, or Pseudomonas, in particular from *T. lanuginosus (H. lanuginosa), Rhizomucor miehei, C. antarctica, A. niger; R. delemar, R. arrhizus* or *P. cepacia.* The phospholipase may have phospholipase A1, A2, B, C, D or lysophospholipase activity; it may or may not have lipase activity. It may be of animal origin, e.g. from pancreas, snake venom or bee venom, or it may be of microbial origin, e.g. from filamentous fungi, yeast or bacteria, such as Aspergillus or Fusarium, *e.g. A*. *niger, A. oryzae* or *F. oxysporum.* A preferred lipase/phospholipase from *F. oxysporum* is disclosed in WO 98/26057. Also, the variants described in WO 00/32758 may be used. The additional enzyme may be of any origin, including mammalian and plant, and preferably of microbial (bacterial, yeast or fungal) origin and may be obtained by techniques conventionally used in the art. Suitable phospholipase compositions are LIPOPAN F and LIPOPAN XTRA (available from Novozymes) or PANAMORE GOLDEN and PANAMORE SPRING (available from DSM). In this disclosure, the R-substituted mono- or di-methoxyphenol-comprising substrate preferably comprises or consists of a baking composition or a dough, preferably comprising a flour such as whole meal flour. Whole meal flour is a flour which may be derived by grinding or mashing the whole cereal grain. When used in baking it is typically added to other more refined white flours to provide nutrients (especially fiber and protein), texture, and body to the finished product. The word "whole" refers to the fact that not only the starchy endosperm but also the bran and germ are used in making the flour.

In another disclosure, the demethylation method is part of a process to produce xylo-oligomers (XOs), or oxidised xylo-oligomers, which may be used in pharmaceutical, agriculture and feed products. XOs are known to have pre-biotic effects, as they are neither hydrolyzed nor absorbed in the upper gastrointestinal tract, and they affect the host by selectively stimulating the growth or activity of one or a number of bacteria in the colon, thus improving health. Furthermore, XOs are believed to reduce cholesterol levels, maintenance of gastrointestinal health, and improvement of the biological availability of calcium. Furthermore, XOs inhibit starch retrogradation, improving the nutritional and sensory properties of food.

The demethylation method may be part of a process wherein ferulic acid is present. The demethylation method may be part of a process wherein a substrate is contacted with ferulic acid esterase (FAE) under conditions whereby R-substituted mono-methoxyphenol is produced. The substrate may be a pretreated lignocellulosic biomass substrate. The substrate may be contacted with ferulic acid esterase, LPMO, and PPO. The ferulic acid esterase may be at least about 70% identical, at least about 80% identical, at least about 90% identical, at least about 95% identical, or at least about 98% to the amino acid sequence of XynZ from *Clostridium thermocellum* (SEQ ID NO:73), Type D FAE from *Neurospora crassa* (SEQ ID NO: 74), Type C FAE from *Talaromyces stipitatus* (SEQ ID NO: 75), FAE from *Thielavia terrestris* (SEQ ID NO: 76), from *Humicola insolens* (SEQ ID NO: 77), from *Ruminococcus sp.* (SEQ ID NO: 78), from PC-2 from *Orpinomyces sp*. (SEQ ID NO: 79), from *Schizophyllum commune* (SEQ ID NO: 80, SEQ ID NO: 81), from *Aspergillus oryzae* (SEQ ID NO: 82), type A FAE from *Aspergillus niger* (SEQ ID NO: 83), type B FAE from *Aspergillus niger* (SEQ ID NO: 84), type C FAE from *Emericella nidulans* (SEQ ID NO: 85), from *Fusarium oxysporum* (SEQ ID NO: 86), FaeA1 from *Myceliophthora thermophila* C1 (SEQ ID NO: 87), FaeA2 from *Myceliophthora thermophila* C1 (SEQ ID NO: 88), FaeB1 from *Myceliophthora thermophila* C1 (SEQ ID NO: 89), or FaeB2 from *Myceliophthora thermophila* C1 (SEQ ID NO: 90). Where the sequence given comprises a signal sequence, one of skill in the art will appreciate that the ferulic acid esterase may be at least about 70% identical, at least about 80% identical, at least about 90% identical, at least about 95% identical, or at least about 98% to the amino acid sequence of the mature sequence (which does not comprise the signal sequence). The LPMO may be at least about 70% identical, at least about 80% identical, at least about 90% identical, at least about 95% identical, or at least about 98% to the amino acid sequence of SEQ ID NO: 1 or to SEQ ID NO: 93. The PPO may be at least about 70% identical, at least about 80% identical, at least about 90% identical, at least about 95% identical, or at least about 98% identical to the amino acid sequence of SEQ ID NO: 37, 41, or45. The LPMO may have the amino acid sequence of SEQ ID NO: 1 or SEQ ID NO: 93. The PPO may have the amino acid sequence of SEQ ID NO: 41. The PPO may have the amino acid sequence of SEQ ID NO: 45.

Typically, the amount of PPO in a composition for use in a method/process of the invention will depend on the amount of R-substituted mono- or di-methoxyphenols present in the substrate to be degraded and/or modified. The amount of enzyme (herein to be understood as PPO and/or further enzyme) may be from about 0.1 to about 200 mg enzyme or enzyme composition per gram of R-substituted mono- or di-methoxyphenols; or from about 3 to about 20 mg enzyme or enzyme composition per gram of R-substituted mono- or di-methoxyphenols. The invention encompasses the use of any suitable or sufficient amount of PPO between about 0.1 mg and about 200 mg PPO per gram R-substituted mono- or di-methoxyphenols, in increments of 0.05 mg (*i.e.*, 0.1 mg, 0.15 mg, 0.2 mg... 199.9 mg, 199.95 mg, 200 mg).

Furthermore, the amount of PPO in a composition for use in a method/process of disclosure the disclosure may be between about 0.5 to about 1000 mg enzyme per kg dry matter of the R-substituted mono- or di-methoxyphenol comprising substrate, or 0.5-100 mg/kg, or 0.5-100 mg/kg or 0.5-50 mg/kg, or 1-25 mg/kg or 1-15 mg/kg, or 2-10 mg/kg enzyme/kg dry matter of the R-substituted mono- or di-rnethoxyphenol-comprising substrate.

The disclosure also relates to a commercial process comprising the demethylation method, such as washing or treating of clothing or fabrics, detergent processes, animal feed, food, baking, beverage, biofuel, starch preparation, liquefaction, biorefining, de-inking and biobleaching of paper and pulp, oil and waste dispersing, and treatment of waste streams.

The PPO for use in a method may be an enzyme or protein as defined herein. The PPO for use in a method may also be a nucleic acid molecule, preferably a recombinant nucleic acid molecule and/or vector, encoding said PPO as defined herein. In a disclosure, the PPO for use in a method is a part of a cell-free composition. In another disclosure, wherein the PPO for use in a method is a nucleic acid molecule, said nucleic acid molecule is part of a host cell as defined herein, preferably a genetically modified organism as defined herein, which is preferably modified to recombinantly express the PPO for use in a method.

In a disclosure, the demethylation method of the disclosure wherein a genetically modified microorganism is cultivated in a nutrient medium comprising the R-substituted mono- or di-methoxyphenol to be demethylated.

When further enzymes are added following the demethylation method, the conditions (such as temperature and pH) may be altered to those optimal for the further enzyme before, during, or after addition of the further enzyme. Multiple rounds of enzyme addition are also encompassed. The further enzyme may also be present in the R-substituted mono- or di-methoxyphenol-comprising substrate itself as a result of genetically modifying the plant. The nutrient medium used in a fermentation reaction can also comprise one or more accessory enzymes.

In one aspect, the invention provides a method or process for degrading and/or modifying (hemi-)cellulose, for example in a (hemi-)celhilosc-comprising substrate, wherein said method comprises the step of contacting the (hemi-)cellulose-comprising substrate composition with an LPMO (preferably the AA9 or LPMO9B LPMO as defined herein above), and a PPO. Preferably said PPO is a PPO as defined herein. The method may be a method for degrading or modifying xylan, for example in a xylan-comprising substrate.

An alternative PPO that can be used in the methods disclosed herein, other than disclosed the PPO disclosed, preferably is a PPO that comprises or consists of a common central tyrosinase domain, preferably of the family with NCBI accession number pfam00264 (Volbeda and Hol, J. Mol. Biol. Volume 209(2): pages 249-279,1989) and preferably comprises or consists of an amino acid sequence that is at least 50%, 60%, 70%, 80%, more preferably at least 90%, 91%, 92%, 93%, 94%, even more preferably at least 95%, 96%, 97%, 98%, 99% or at least 100% identical to any one of SEQ ID NO: 49 (PPO1), SEQ ID NO: 53 (PPO3), SEQ ID NO: 57 (PPO8), SEQ ID NO: 61 (PPO5), SEQ ID NO: 65 (PPO 10) or SEQ ID NO: 69 (PPO9). SEQ ID NO: 49, 53, 57, 61, 65 and 69 represent the mature form of PPO1, PPO3, PPO8, PPO5, PPO10 and PPO9, respectively. SEQ ID NO: 50, 54, 58, 62, 66 and 70 represent PPO1, PPO3, PPO8, PPO5, PPO10 and PPO9 including the signal sequence, respectively. Preferably said PPO is obtainable from a fungus, preferably of the genus *Myceliophthora.* Being "obtainable from" is to be understood herein as that the enzyme originates from the indicated organism, i.e. is naturally produced by the indicated organism as a native enzyme. More preferably, the PPO originates from *M. thermophila* C1 fungus or derivatives thereof, such as a *M. thermophila* C1 fungus selected from Garg 27K, (Accession No. VKM F- 3500D), UV13-6 (Accession No. VKM F-3632 D); NG7C-19 (Accession No. VKM F-3633 D); UV18-25 (Accession No. VKM F-3631 D); strain W1L (Accession No. CBS122189) or W1L#100L (Accession No. CBS122190), preferably a *M. thermophila* C1 (Accession No. VKM F- 3500D).

Preferably, the alternative PPO is encoded by a nucleic acid sequence has at least 50%, 60%, 70%, 80%, or more preferably at least 90%, 91%, 92%, 93%, 94%, 95%, 96%, 97%, 98%, 99% or 100% identity with SEQ ID NO: 51 (cDNA encoding PPO1), SEQ ID NO: 52 (gDNA encoding PPO1), SEQ ID NO: 55 (cDNA encoding PPO3), SEQ ID NO: 56 (gDNA encoding PPO3), SEQ ID NO: 59 (cDNA encoding PPO8), SEQ ID NO: 60 (gDNA encoding PPO8), SEQ ID NO: 63 (cDNA encoding PPO5), SEQ ID NO: 64 (gDNA encoding PPO5), SEQ ID NO: 67 (cDNA encoding PPO10), SEQ ID NO: 68 (gDNA encoding PPO10), SEQ ID NO: 71 (cDNA encoding PP09) or SEQ ID NO: 72 (gDNA encoding PPO9). Preferably, said encoding nucleic acid sequence has at least 50%, 60%, 70%, 80%, or more preferably at least 90%, 91%, 92%, 93%, 94%, 95%, 96%, 97%, 98%, 99% or 100% identity with SEQ ID NO: 52 (gDNA encoding PPO1), SEQ ID NO: 56 (gDNA encoding PPO3), SEQ ID NO: 60 (gDNA encoding PPO8), SEQ ID NO: 64 (gDNA encoding PPO5), SEQ ID NO: 68 (gDNA encoding PPO10), or SEQ ID NO: 72 (gDNA encoding PPO9).

Depending on the amount of R-substituted mono- or di-mcthoxyphcnol present in a hemicellulose-comprising substrate, the method further comprises the step of adding and/or admixing R-substituted mono- or di-methoxyphenol, preferably a substantial amount of R-substituted mono- or di-methoxyphenol.

In a disclosure of the invention, the (hemi-)cellulose-comprising substrate is substantially free of R-substituted mono- or di-methoxyphenol. A method or process of this disclosure comprises the step of adding a substantial amount of R-substituted mono- or di-methoxyphenol.

In a further embodiment, the (hemi-)cellulose-comprising substrate does comprise R-substituted mono- or di-methoxyphenol, however, in a sub-optimal amount or concentration or in a form that is less accessible for demethylation by the PPO enzyme. A method or process of this embodiment also comprises the step of adding a substantial amount of R-substituted mono- or di-methoxyphenol.

In yet a further embodiment, the (hemi-)cellulose-comprising substrate does comprise R-substituted mono- or di-methoxyphenol in optimal amount or concentration and in a form that accessible for demethylation by the PPO enzyme. A method or process of this embodiment no further step of adding a R-substituted mono- or di-methoxyphenol is required.

A method of this aspect and of each embodiment herein encompasses a method wherein the LPMO, the PPO and/or R-substituted mono- or di-methoxyphenol are contacted, added and/or admixed with the (hemi-)cellulose-comprising substrate simultaneously, and sequentially.

In disclosure the PPO is PPO7 and the R-substituted mono- or di-methoxyphenol is syringic acid, synapic acid, 3-hydroxy-4-methoxycinnamic acid, 4-hydroxy-3-methoxyphenylactone, coniferyl aldehyde, ferulic acid, guaiacol, hesperidin, homovanillic acid, vanillic acid, 3,4-dihydroxybenzoic acid, caffeic acid, 3,4-dihydroxy-5-methoxycinnamic acid, or combinations thereof.

A substrate being substantially free of R-substituted mono- or di-methoxyphenol is understood herein as to comprise no appreciable amounts of R-substituted mono- or di-methoxyphenol, *i.e.* less than about 0.1 %, more preferably less than about 0.01%, more preferably less than about 0.01% or most preferably less than about 0.0001% of the total dry weight of the substrate.

Adding or admixing a substantial amount of R-substituted mono- or di-methoxyphenol is understood herein as adding or admixing an amount of R-substituted mono- or di-methoxyphenol that results in an in increase in (hemi-)cellulose degradation and/or modification, optionally in an increase in XO production.

In embodiments, said (hemi-)cellulose-comprising substrate comprises xylan that consists of large unsubstituted, linear xylan chains, preferably such as is present in oat spelt xylan and birchwood xylan. Preferably, said xylan-comprising substrate does not consist only of, or preferably does not comprise mainly of, or even more preferably does not comprise, xylan that has arabinosyl-substituents present on the β-1,4-xylan backbone such as the branched xylan present in wheat arabinoxylan. Preferably, the xylan to be degraded and/or modified in the xylan-comprising substrate is xylan that consists of large unsubstituted, linear xylan chains, preferably such as is present in oat spelt xylan and birchwood xylan. Preferably, the xylan to be degraded an dor modified in the xylan-comprising substrate is not xylan that has arabinosyl-substituents present on the β-1,4-xylan backbone such as the branched xylan present in wheat arabinoxylan.

Preferably said (hemi-)cellulose-comprising substrate comprises cellulose, more preferably hemicelluloses-associated cellulose and/or amorphous cellulose. Optionally, said (hemi-)celtulose-comprising substrate of this method is substantially free of R-substituted mono- or di-methoxyphenol compounds as defined herein. Optionally, R-substituted mono- or di-methoxyphenol compounds as defined herein are added as part of the method, preferably before contacting the (hemi-)cellulose-comprising substrate with either of the accessory enzyme and the PPO.

Preferably, the R-substituted mono- or di-methoxyphenol to be added can be any R-substituted mono- or di-methoxyphenol comprising compound as defined herein. Preferred within this method are low molecular weight R-substituted mono- or di-methoxyphenol such as present in biolignin and/or organosolv lignin. Even more preferred for use in method of the present aspect is the use of monomers of syringic acid, vanillic acid, 3,4-dihydroxy-5-methoxybenzoic acid, sinapic acid, ferulic acid, 3,4-dihydroxy-5-methoxycinnamic acid, syringol, 2-methoxyphenol (guaiacol), 3-methoxycatechol or any combination thereof.

Preferably, the substrate further comprises between about 1 and 1000µM of a copper salt, preferably between 20 and about 1000µM a copper salt, wherein said copper salt is preferably selected from the group consisting of CuSO4, CuCl₂, CuBr₂, CuF₂, Cu(OH)₂ and Cu(NO₃)₂. Preferably the pH of the substrate is between about 5.0 and about 8.0. Preferably, the method is performed at a temperature between about 20 and about 60°C. Even more preferably, the substrate further comprises between about 1 and about 1000µM a copper salt, preferably between 20 and about 1000µM a copper salt, wherein said copper salt is preferably selected from the group consisting of CuSO4, CuCl₂, CuBr₂, CuF₂, Cu(OH)₂ and Cu(NO₃)₂, has a pH between about 5.0 and about 8.0 and the method is performed at a temperature between about 20 and about 60°C.

Preferably, the method of this aspect is part of a method or process for saccharification, for dissolving pulp for the production of chemicals, for paper and pulp bleaching, for DDG degradation, for producing an organic substance, for clarification and/or increasing the rate of filtration and/or decreasing haze formation of juices and/or beverages such as wine and beer, for extraction of coffee, plant oils, and starch, for production of xylitol, for increasing digestibility and/or nutritional properties of animal feedstocks or animal food, and for increasing the quality of bakery products and/or improve textual characteristics (break and shred quality and crumb quality) of the bakery products such as, but not limited to bread, as further disclosed herein, and may comprise further steps as further disclosed herein, such as contacting the substrate with further enzymes that are indicated to be particular suitable for said process disclosed herein.

Disclosed herein is an R-substituted mono- or di-methoxyphenol-comprising substrate and/or a substrate as defined in any one of the preceding aspects, further comprising the PPO enzyme of the disclosure, the nucleic acid molecule of the disclosure, the host cell of the enzyme composition or formulation of the disclosure. Preferably, said R-substituted mono- or di-methoxyphenol-comprising substrate and/or a xylan-comprising substrate is for or suitable for use in a method or process of the disclosure.

### Example 1: Cloning of Myceliophthora thermophila C1 PPO polypeptide coding sequences having demethylating activity

Genomic DNA encoding PPO2 (SEQ ID NO: 36), PPO4 (SEQ ID NO: 40), PPO6 (SEQ ID NO: 44) and PPO7 (SEQ ID NO: 48) were synthesized (GeneArt) and cloned in pChi vector as previously described (WO 2010/107303 A2). Transformation of W1L#100.1Δpyr5ΔAlp1 strain using the pyr5 marker as previously described.

### Example 2: Preparation of PPO polypeptides having demethylating activity

PPO4, PPO6 and PPO7 having demethylating activity and PP02 were prepared as described previously. In short, fermentation of W1L#100.1Δpyr5ΔAlp1 strain overexpressing the PPO were performed as described in WO 2010/107303 A2 broth was harvested, filtrated to remove fungal biomass, concentrated and dialyzed against 20 mM KPi pH 7.0 plus 150 mM KC1 using a 5 kDa PES membrane (Vivacell® 70, Sartorius). Samples were subsequently freeze-dried for storage.

### Example 3: Catechol oxidase activity assay

PPO activity was determined by measuring the initial rate of 3-methoxycatechol oxidation at 400 nm (extinction coefficient used: ε 1200 M⁻¹cm⁻¹). One unit of activity is defined as the amount of PPO activity that oxidizes 1 µmοl of 3-methoxycatechol to quinone per minute. Substrate was used at a concentration of 4 mM.

### Example 4: Characterization of PPO polypeptides having demethylating activity

The pH optima were determined by measuring the initial rate of 3-methoxycatechol oxidation with varying pHs, at room temperature and using a CuSO₄ concentration of 10 µM. The pH was varied in the range of pH3-9 using Carmody buffers. Enzyme loading was applied resulting in A₄₀₀ absorbance values in the range of 0.1-1. Protein concentration was determined using the BCA method.

Optimal copper concentrations for the PPOs were determined at the optimal pH by varying the concentration of CuSO₄ in the range of 1-1000 µM. In these experiments the pH was controlled using 50 mM Bis-Tris buffer pH 7.0.

Temperature optima were determined by measuring A₄₀₀ absorbance after 1h incubation at temperatures in the range of 20 to 70 °C. In these experiments the pH was controlled using 50 mM Bis-Tris buffer pH 7.0. Copper concentrations of (20 or 50 µM) were used to prevent chemical oxidation as background activity at higher copper concentrations.

**Table 1. Optima determined for PPO 4, 6 and 7**

| Enzyme | pHₒₚₜ | pH range** | [CuSO₄]ₒₚₜ (µM) | [CuSO₄] range *** (µM) | Tₒₚₜ (°C) | T range** (°C) |
|---|---|---|---|---|---|---|
| PPO4 | 7.0 | 5.0 - 7.5 | 200 | 20 - 1000 | 55 | 30 - 60 |
| PPO6 | 7.0 | 6.0 - 8.5 | 200 | 50 - 1000 | 30 | 20 - 50 |
| PPO7 | 7.0 | 6.5 - 8.0 | 400 | 100 - 1000 | 40 | 20 - 55 |

| | | | | | | |
|---|---|---|---|---|---|---|
| *PPO activity was determined by measuring the rate of 3-methoxycatechol oxidation under optimal conditions. 3-methoxy-catechol oxidation was followed at 400 nm (extinction coefficient used: ε 1200 M-1cm-1). ** pH and T range: >50% of maximum activity *** Copper concentration range: >90% of maximum activity | | | | | | |

### Example 5: Purification of PPO7 having demethylating activity and purification of PPO2

Freeze-dried material of PPO7 was dissolved in MilliQ water, 0.45 µm filtered, applied on a 50 mL Source™ 30Q column (XK16/20, GE Healthcare) equilibrated with 20 mM KPi pH 7.0 and eluted using a gradient of 0 to 1 M KCl (stepwise) in this buffer at a flow rate of 3.5 mL per minute. As a second purification step, a 50 mL Phenyl Sepharose™ 6 Fast Flow (high sub) column (XK16/20, GE Healthcare) equilibrated with 10 mM KPi pH 7.0 plus 1 M ammonium sulphate was used. A stepwise elution to buffer without ammonium sulphate was performed at a flow rate of 3.5 mL per minute. Pools were concentrated and dialyzed against 20 mM KPi pH 7.0 plus 150 mM KCI using a 5 kDa PES membrane (Vivacell® 70, Sartorius). The final purification step was performed on a 120 mL Superdex® 200 PG column (HiLoad 16/600) equilibrated with 20 mM KPi pH 7.0 plus 150 mM KCl at a flow rate of 1 mL per minute. Pure fractions were pooled and concentrated using a 5 kDa PES membrane (Vivacell® 70, Sartorius).

Freeze-dried material of PPO2 was dissolved in MilliQ water, 0.45 µm filtered, applied on a 50 mL Source™ 30Q column (XK16/20, GE Healthcare) equilibrated with 10 mM KPi pH 7.0 and eluted using a gradient of 0 to 1 M KCl (stepwise) in this buffer at a flow rate of 3.5 mL per minute. As a second purification step, a 20 mL Butyl Sepharose™ Fast Flow column (HiPrep 16/10, GE Healthcare) equilibrated with 10 mM KPi pH 7.0 plus 1.5 M ammonium sulphate was used. A stepwise elution to buffer without ammonium sulphate was performed at a flow rate of 4 mL per minute. Pools were concentrated using a 5 kDa PES membrane (Vivacell® 70, Sartorius). Finally, size exclusion chromatography was performed on a 120 mL Superdex® 200 PG column (HiLoad 16/600) equilibrated with 20 mM KPi pH 7.0 plus 150 mM KCl at a flow rate of 1 mL per minute. Pure fractions were pooled and concentrated using a 5 kDa PES membrane (Vivacell® 70, Sartorius).

### Example 6: Conversion of syringic acid by PPO polypeptides having demethylating activity

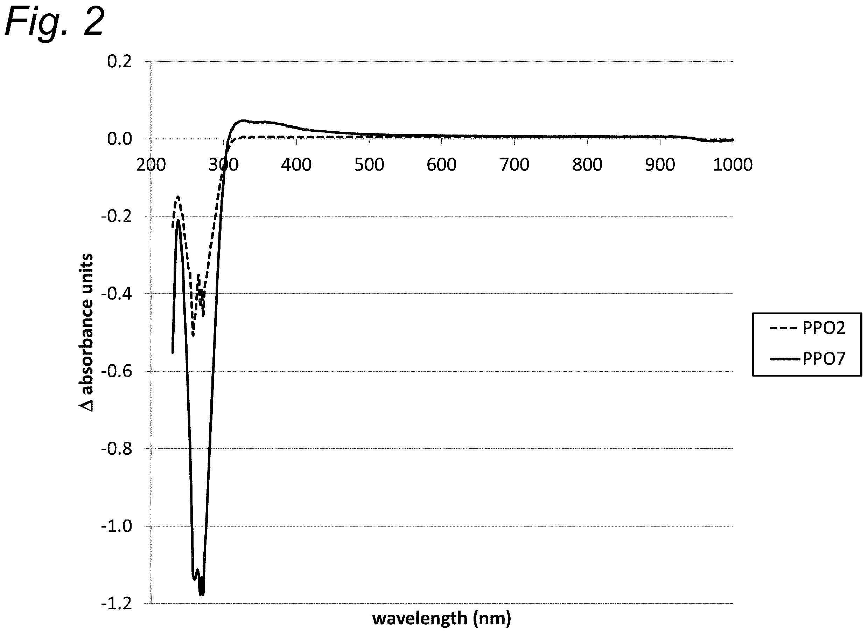

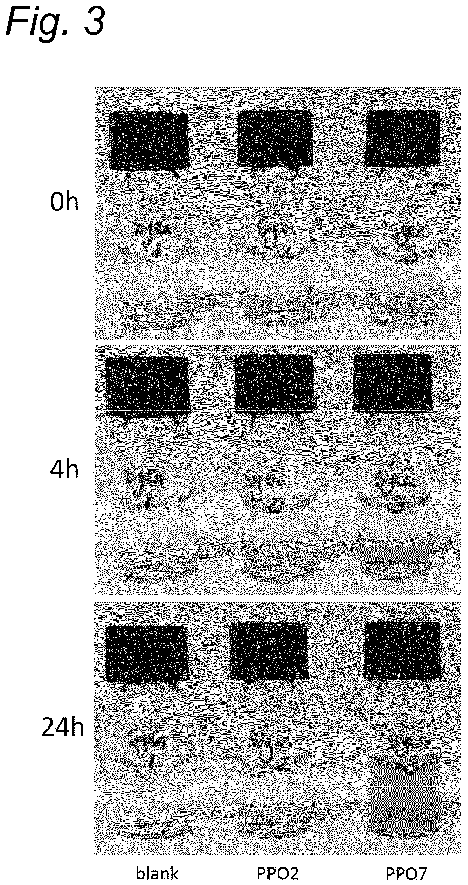

Syringic acid (12 mM, 2.38 g/L) was incubated with PPO7 and PPO2 from Example 5 (0.048 g/L, 2% loading (w/w)) in 50 mM BisTris buffer pH 6.5 with 10 µM CuSO₄. Blank prepared without the addition of enzyme was included. Incubations were performed in HPLC vials with a total of 750 µL reaction volume at room temperature for 24h. The reaction was stopped by adding 21 µL 1M HCl to lower the pH to -3. Samples were filtrated and absorbance spectra were measured (230-1000 nm, 2 nm interval). Figure 2 shows the difference absorbance spectra (syringic acid blank subtracted) after 24h incubation. Syringic acid conversion induced the negative peak in the difference absorbance spectra between 230 and300 nm, which was more significant for PPO7 than for PPO2. Moreover, PPO7 reaction products resulted in a spectral increase between 300 and 500 nm (which was confirmed by the visual colour change of the reaction vial; see Figure 3). It is to be noted that any change in absorbance in time indicates syringic acid conversion.

### Example 7: Conversion of syringic acid, sinapic acid and syringol by PPO polypeptides having demethylating activity

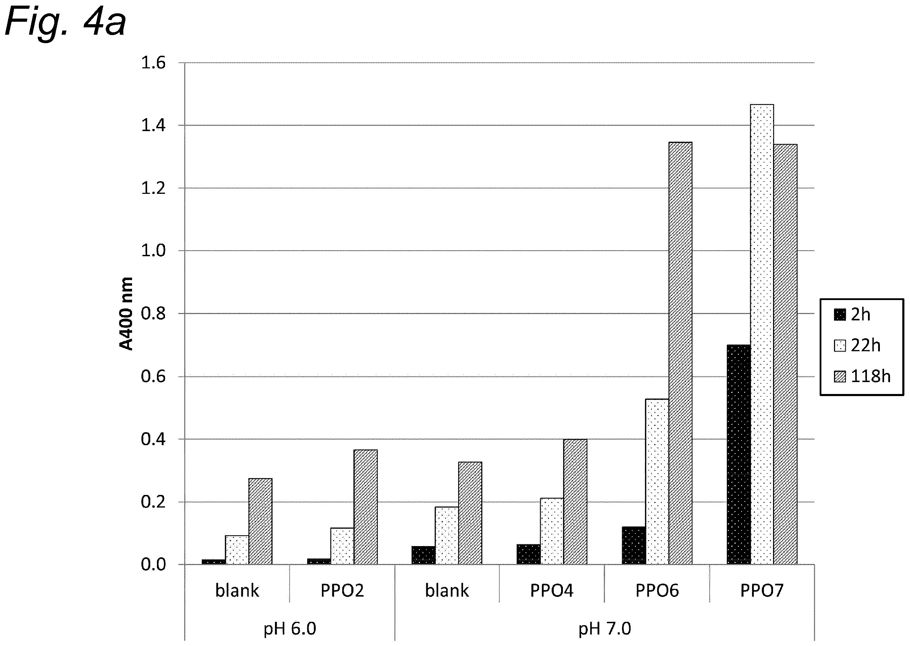

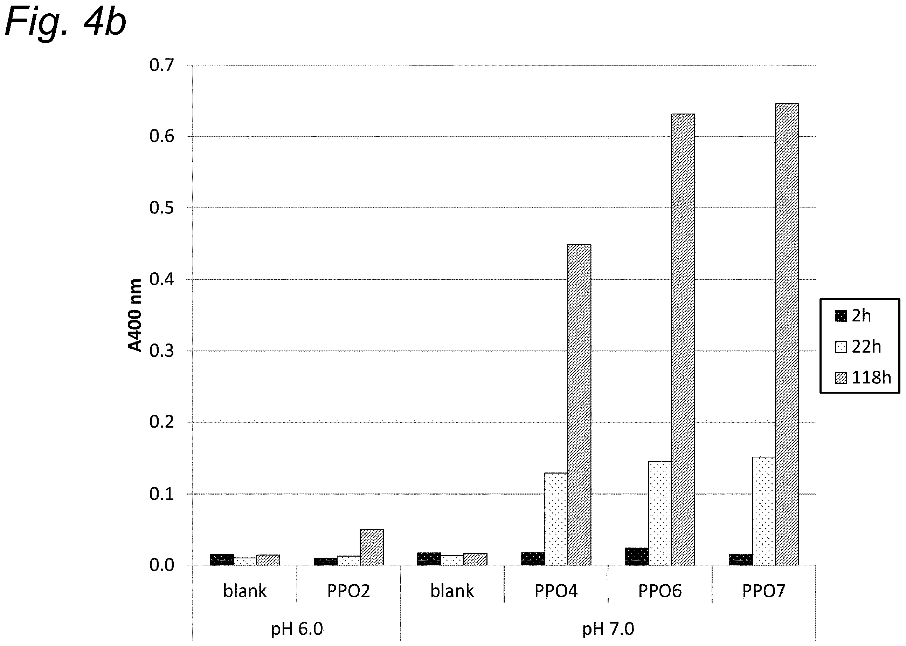

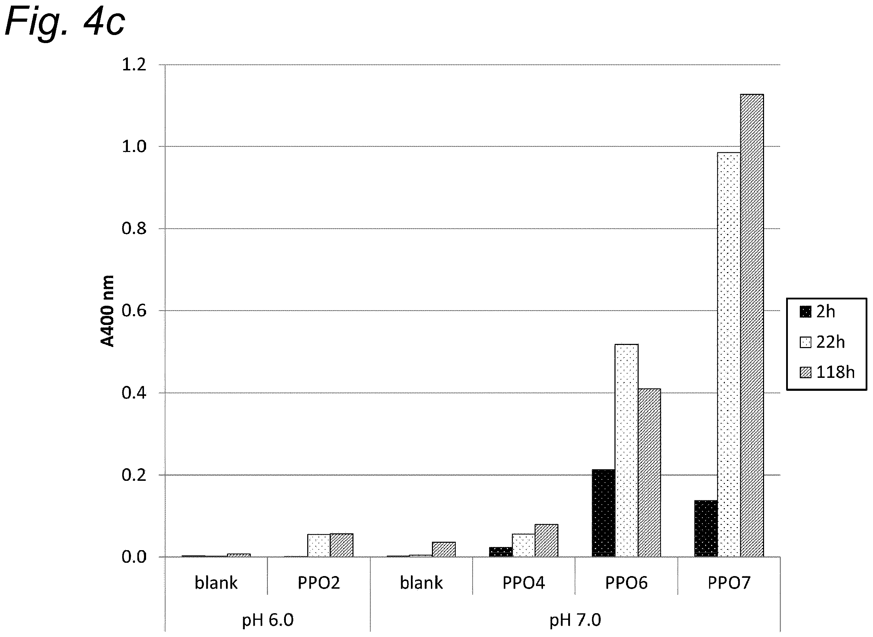

Syringic acid, sinapic acid and syringol (4 mM) were incubated with crude PPO2, 4, 6 and 7 from Example 5 in 100 mM BisTris buffer pH 6.0 or 7.0 with 50 µM CuSO₄. Crude PPOs were dosed on target protein with a final concentration of 8.2 µg/mL. Substrate blanks prepared without the addition of enzyme and enzyme blanks without the addition of substrate were included. Incubations were performed in microtiterplates with a total reaction volume of 150 µL at room temperature for approximately 120h. After 2, 22 and 118h absorbance at 400 nm was measured. Figure 4 shows UV-VIS data at 400 nm; enzyme blanks were subtracted.

PPO7 and PPO6 were able to convert syringic acid, sinapic acid and syringol, whereas PPO4 was only able to use syringic acid as a substrate. PPO2 showed no significant activity on those substrates compared to the substrate blanks.

### Example 8: Demethylation of syringic acid

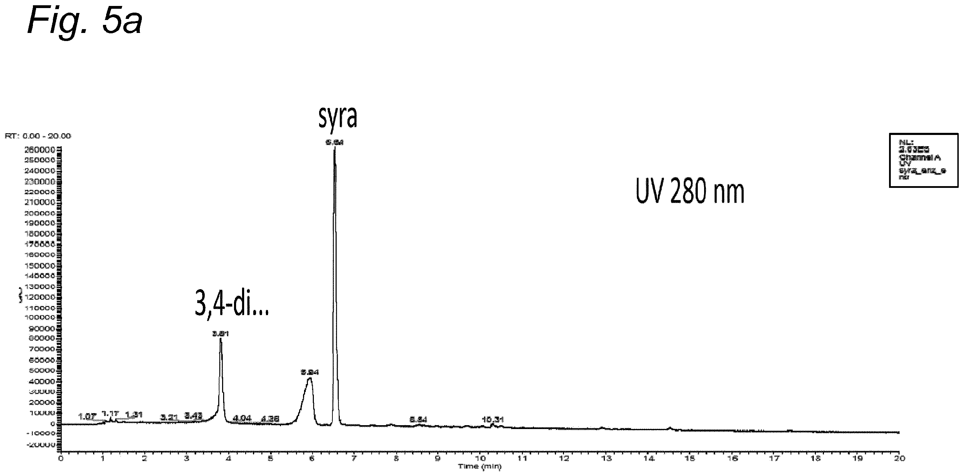

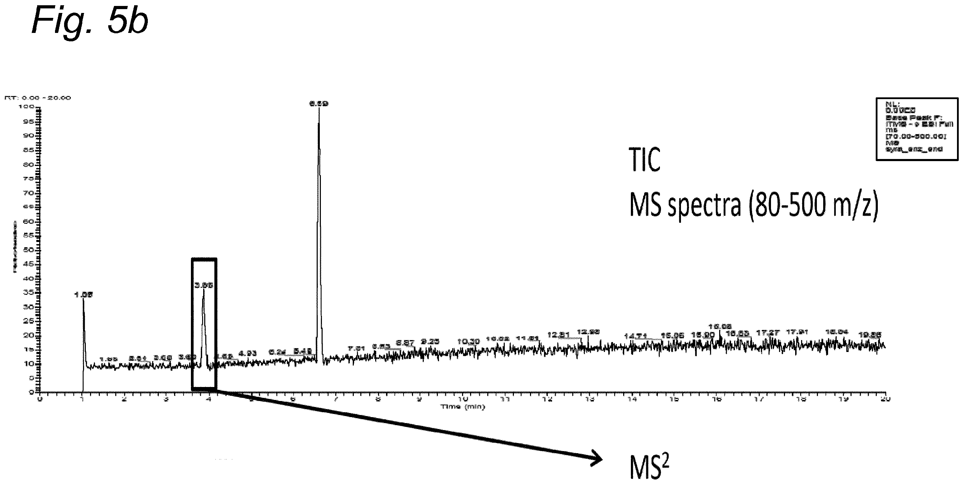

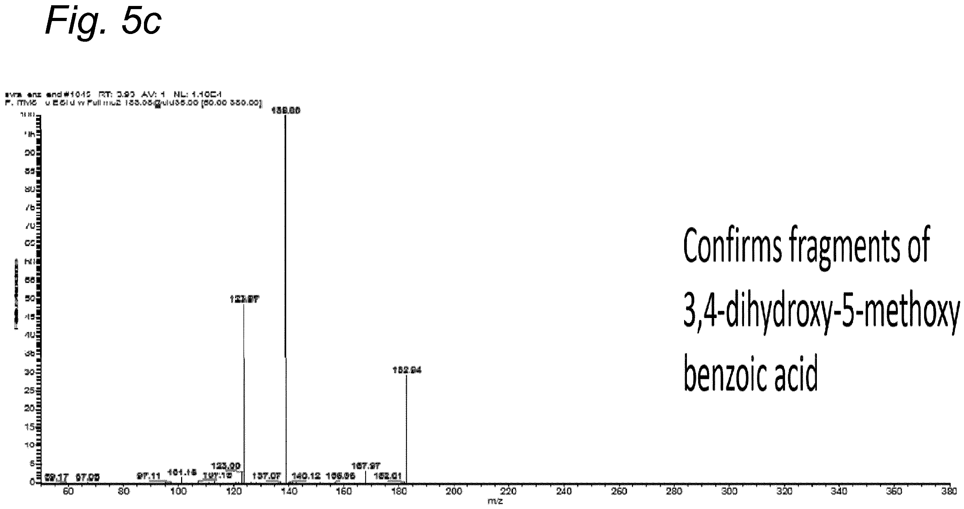

Syringic acid (12 mM, 2.38 g/L) was incubated with PPO7 from Example 2 (1.2 µM, 0.048 g/L, 2% loading (w/w)) in 50 mM BisTris buffer pH 6.5 with 10 µM CuSO₄. Blank prepared without the addition of enzyme was included. Incubations were performed in GC headspace vials with a total of 1 mL reaction volume in duplicate at room temperature for approximately 90h. The reaction was stopped by adding 28 µL 1M HCl to lower the pH to -3. One of the replicates was analyzed by GC for the methanol formation. A calibration range of 0.23 to 11.6 mM methanol in buffer with HCl was included. The second replicate was analyzed using UHPLC and the formation 3,4-dihydroxy-5-methoxybenzoic acid was confirmed using MSMS (see Figure 5). Table 1 shows the concentrations of syringic acid, methanol and 3,4-dihydroxy-5-methoxybenzoic acid. PPO7 catalyzed the demethylation of syringic acid, resulting in the formation of 3,4-dihydroxy-5-methoxybenzoic acid and methanol.

**Table 1. Concentrations (mM) of syringic acid (UHPLC), methanol (GC) and 3,4-dihydroxy-5-methoxybenzoic acid (UHPLC)**

| | syringic acid (mM) | methanol (mM) | 3,4-dihydroxy-5-methoxybenzoic acid (mM) |
|---|---|---|---|
| Blank | 10.86 | 0 | 0 |
| PPO7 from Example 2 | 3.02 | 4.84 | 1.61 |

### Example 9 Demethylation of lignin-like model compounds, lignin and lignin-derived compounds by PPO7 polypeptide having demethylating activity.

Model compounds syringic acid, syringol, 1,3-dimethoxybenzene and 3,5-dimethoxybenzoic acid (12 mM) were incubated with PPO7 from Example 2 (2% loading (w/w)) in 50 mM BisTris buffer pH 6.5 with 10 µM CuSO₄. For syringic acid and syringol PPO2 from Example 2 was included as a control. Lignin and lignin-derived compounds (10 g/L) were incubated with PPO7 from Example 2 (0.5 g/L, 5% loading (w/w)). PPO2 from Example 2 was included as a control and incubated using the same conditions. Blanks prepared without the addition of enzyme were included. Incubations were performed in GC headspace vials with a total of 1 mL reaction volume at 25°C for approximately 48h while shaking. The reaction was stopped by adding 28 µL 1M HCl to lower the pH to ∼3. Samples were analyzed by GC for methanol formation. A calibration range of 0.23 to 11.5 mM methanol in buffer with HCl was included. Table 2 shows the concentrations of methanol as determined by GC.

PPO7 catalyzed the demethylation of syringic acid and syringol, whereas PPO2 did not. Incubations on 1,3-dimethoxybenzene and 3,5-dimethoxybenzoic acid showed no methanol release for PPO7.

On lignin and lignin-derived compounds PPO7 showed an increased methanol concentration compared to the blank.

**Table 2. Methanol concentration (mM) as determined bv GC**

| | methanol (mM) | | |
|---|---|---|---|
| | PPO7 | PPO2 | Blank |
| *Model compounds* | | | |
| Syringic acid | 4.58 | 0 | 0 |
| Syringol | 5.73 | 0.07 | 0.06 |
| 1,3-dimethoxybenzene | 0 | n.a. | 0 |
| 3,5-dimethoxybenzoic acid | 0 | n.a. | 0 |
| | | | |

| *Lignin* | | | |
|---|---|---|---|
| Wheat straw BioLignin | 0.28 | 0.09 | 0.10 |
| Corn stover BioLignin | 0.27 | 0.05 | 0 |
| | | | |
| | | | |
| | | | |

| *Lignin-derived compounds* | | | |
|---|---|---|---|
| Fractionated BioLignin | 0.46 | 0.36 | 0.28 |
| Purified wheat straw BioLignin | 0.21 | 0.15 | 0.05 |
| Lowest MW from lignin fractionation | 1.43 | 0.36 | 0.04 |
| Carboxymodified lignin (wheat)1 | 0.31 | 0 | 0 |
| Carboxymodified lignin (wheat)2 | 0.33 | 0 | 0 |
| Carboxymodified lignin (corn) | 0.35 | 0 | 0 |

### Example 10 Cloning, preparation and purification of Myceliophthora thermophila LPMO9B

DNA encoding LPMO9B (SEQ ID NO: 91; amino acid sequence: SEQ ID NO: 92; "mature" amino acid sequence without the signal sequence: SEQ ID NO: 93) was amplified from genomic DNA and cloned in pChi vector as previously described (WO 2010/107303 A2). Transformation of W1L#100.lΔpyr5ΔAlp1 strain was performed using the pyr5 marker and LPMO9B was prepared by fermentation of the W1L#100.lΔpyr5ΔAlp1 strain overexpressing the LPMO as described in WO 2010/107303 A2. Broth was harvested, filtrated to remove fungal biomass, concentrated and dialyzed against 10 mM potassium phosphate pH 7.0 plus 150 mM KCl using a 5 kDa PES membrane (Vivacell® 70, Sartorius). MtLPMO9B was purified in three subsequent chromatographic steps from the corresponding dialyzed enzyme preparations using an ÄKTA-Explorer preparative chromatography system (GE Healthcare, Uppsala, Sweden). The LPMO9B-containing enzyme preparation was loaded on a Source 30Q column (50ml, GE Healthcare). A 20 mM potassium phosphate buffer (pH 7.8) was used to pre-equilibrate the column. Elution was performed using a linear gradient from 0 to 1 M NaCl in 20 mM potassium phosphate buffer (pH 7.8) at a flow rate of 10 mL min-1 and monitored at 220 and 280 nm. Fractions containing LPMO9B were pooled concentrated by ultrafiltration (Amicon Ultra, molecular mass cut-off of 3 kDa, Merck Millipore, Cork, Ireland) and subsequently loaded on a Source 30S column (50 mL, GE Healthcare). A 20 mM sodium acetate buffer (pH 5.0) was used to pre-equilibrate the column and elution was performed using a linear gradient from 0 to 1 M NaCl in 20 mM sodium acetate (pH 5.0) at a flow rate of 5 mL min-1. Peak fractions were pooled, concentrated and loaded on a Source 30S column (50 mL, GE Healthcare pre-equilibrated using a 10 mM sodium acetate buffer (pH 5.0)). The column was washed with 20 column volumes of starting buffer and subsequently eluted using a linear gradient from 0 to 1 M KCl in 20 mM sodium acetate (pH 5.0) at a flow rate of 5 mL min-1. Fractions containing the purified LPMO9B were pooled, concentrated as described above.

### Example 11 Regenerated Amorphous Cellulose (RAC hydrolysis assay with LPMO9B and PPO2, PPO7 or AbTyr in the presence of dimethoxyphenol reducing agent sinapic acid or syringic acid

*Purification of mushroom tyrosinase AbTyr*: Crude mushroom tyrosinase was obtained from Sigma-Aldrich (St. Louis, MO, USA) and purified as described previously (Kuijpers TFM, et al. (2014) Potato and Mushroom Polyphenol Oxidase Activities Are Differently Modulated by Natural Plant Extracts. Journal of Agricultural and Food Chemistry 62(1):214-221 and Kuijpers TFM, Gruppen H, Sforza S, van Berkel WJH, & Vincken J-P (2013) The antibrowning agent sulfite inactivates Agaricus bisporus tyrosinase through covalent modification of the copper-B site. FEBS Journal 280(23):6184-6195). The activity of the purified tyrosinase fraction was defined as the increase of A280 by 0.001 U per min by using L-tyrosine as a reference substrate. The determined activity of the fraction used in this work was 3000 U mL⁻¹. The protein concentration of this fraction was determined to be 270 µg/ml. Protein determination was determined using the BCA method.

Reducing agents sinapic acid and syringic acid were obtained from Sigma-Aldrich (St. Louis, MO, USA). RAC was prepared from Avicel by swelling it in phosphoric acid, (Zhang YHP, Cui J, Lynd LR, & Kuang LR (2006) A transition from cellulose swelling to cellulose dissolution by o-phosphoric acid: Evidence from enzymatic hydrolysis and supramolecular structure. Biomacromolecules 7(2):644-648.). RAC was dissolved in 50 mM phospate buffer (pH 5.0) to a final concentration of 1.5 mg/ml. Copper chloride was added to a final concentration of 2.5 µM and dimethoxy containg reducing agent sinapic acid or syringic acid to a final concentration of 2 mM. LPMO9B (5 µg/ml) with or without PPO2 (5 µg/ml), PPO7 (5 µg/ml) or *Ab*Tyr (7.5 U/ml) were added and incubated for 24 h at 50 °C in a head-over-tail rotator in portions of 1 mL total volume (Stuart® rotator, Bibby Scientific LTD, Stone, UK) at 20 rpm. Products were analysed by HPAEC and MALDI-TOF MS as described previously. (Frommhagen M, Sforza S, Westphal AH, Visser J, Hinz SW, Koetsier MJ et al. Discovery of the combined oxidative cleavage of plant xylan and cellulose by a new fungal polysaccharide monooxygenase. Biotechnology for Biofuels. 2015;8:101. doi:10.1186/s13068-015-0284-1). Results are shown in Table 3 (below). The Activity denoted as "Activity*" represents total release of non-oxidized and C1-oxidized gluco-oligosaccharides from RAC incubated with LPMO9B in the presence of sinapic or syringic acid as a reducing agent and with addition of either AbTyr, PPO7 or PPO2. Activity of LPMO9B equates to 100%, no activity was detected for LPMO9B in the absence of a reducing agent, all incubations were performed in duplicates. Standard deviations were less than 2%.

**Table 3. RAC incubated with LPMO9B in the presence of dimethoxy phenol reducing agent sinapic acid or syringic acid with or without AbTyr, PPO7 or PPO2 addition**

| **Reducing Agent** | **Activity* LPMO9B** | **Activity *Ab*Tyr** | **Activity PPO7** | **Activity PPO2** |
|---|---|---|---|---|
| sinapic acid | 100 | 110 | 195 | 100 |
| syringic acid | 100 | 107 | 142 | 104 |

### Example 12 RAC hydrolysis assay with LPM09B and PPO7 or AbTyr in the presence of phenolic reducing agents

Phenolic reducing agents were obtained from Sigma-Aldrich (St. Louis, MO, USA). RAC was prepared as described above and dissolved in 50 mM phospate buffer (pH 5.0) to a final concentration of 1.5 mg/ml. Copper chloride was added to a final concentration of 2.5 µM and a phenolic reducing agent to a final concentration of 2 mM. LPMO9B (5 µg/ml) with or without PPO7 (5 µg/ml) or *Ab*Tyr (7.5 U/ml) were added and incubated for 24 h at 50 °C in a head-over-tail rotator in portions of 1 mL total volume (Stuart® rotator, Bibby Scientific LTD, Stone, UK) at 20 rpm. Products were analysed by HPAEC and MALDI-TOF MS as described previously. (Frommhagen M, Sforza S, Westphal AH, Visser J, Hinz SW, Koetsier MJ et al. Discovery of the combined oxidative cleavage of plant xylan and cellulose by a new fungal polysaccharide monooxygenase. Biotechnology for Biofuels. 2015;8:101. doi:10.1186/s13068-015-0284-1). Results are shown in Table 4 (below). The Activity denoted as Activity * represents total release of non-oxidized and C1-oxidized gluco-oligosaccharides from RAC incubated with LPMO9B in the presence of a phenolic reducing agent and with addition of either AbTyr or PPO7. Activity of LPMO9B equates to 100%, no activity was detected for LPMO9B in the absence of a reducing agent, all incubations were performed in duplicates. Standard deviations were less than 2%.

**Table 4. RAC incubated with LPMO9B in the presence of phenolic reducing agents with or without AbTyr or PPO7 addition**

| **Reducing agent** | **Activity* *Mt*LPMO9B** | **Activity *Mt*LPMO9B *Ab*Tyr** | **Activity *Mt*LPMO9B *Mt*PPO7** |
|---|---|---|---|
| 3-hydroxy-4-methoxycinnamic acid | 100 | 151 | 7558 |
| 4-hydroxy-3-methoxyphenylactone | 100 | 117 | 131 |
| coniferyl aldehyde | 100 | 128 | 562 |
| ferulic acid | 100 | 107 | 231 |
| guaiacol | 100 | 461 | 5143 |
| hesperidin | 100 | 56 | 771 |
| homovanillic acid | 100 | 156 | 580 |
| vanillic acid | 100 | 196 | 495 |
| 4-allyl-2,4-methoxyphenol | 100 | 89 | 108 |
| 3,4-dihydroxybenzoic acid | 100 | 99 | 139 |
| 3-methylcatechol | 100 | 58 | 104 |
| caffeic acid | 100 | 55 | 122 |
| 3,4-dihydroxy-5-methoxybenzoic acid | 100 | 99 | 22 |
| 3,4-dihydroxy-5-methoxycinnamic acid | 100 | 99 | 116 |
| gallic acid | 100 | 105 | 124 |

## Claims

1. A process for degrading and/or modifying cellulose in lignocellulosic biomass substrate, wherein said process comprises the step of contacting said substrate with a lytic polysaccharide monooxygenase (LPMO) and a polyphenoloxidase, wherein the polyphenoloxidase comprises or consists of an amino acid sequence that is at least 70% identical to SEQ ID NO:45 and wherein the polyphenoloxidase comprises or consists of a central tyrosinase domain, and wherein the polyphenoloxidase is capable of demethylating an R-substituted mono- or di-methoxyphenol represented by formula [1], [2] or [3]: 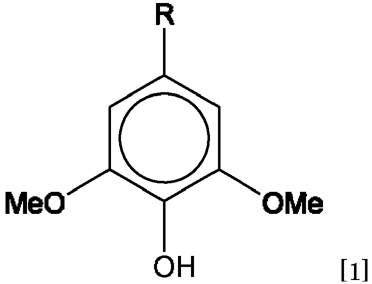 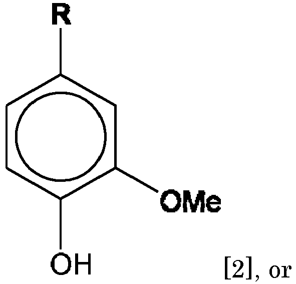 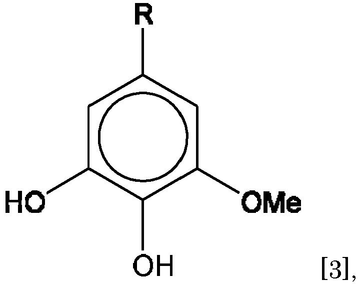 wherein R can be any single atom or chemical moiety.

2. The process of claim 1, further comprising the step of admixing an R-substituted mono- or di-methoxyphenol represented by formula [1], [2], or [3].

3. The process of claim 2, wherein R is an organic moiety.

4. The process of claim 2 or 3, wherein the R-substituted mono- or di-methoxyphenol is represented by formula [1].

5. The process of claim 4, wherein the R-substituted mono- or di-methoxyphenol is syringic acid or sinapic acid.

6. The process of any one of claims 1-5, wherein said substrate comprises 1-1000 µM of a copper salt.

7. The process of claim 6, wherein the copper salt is selected from the group consisting of CuSO₄, CuCl₂, CuBr₂, CuF₂, Cu(OH)₂ and Cu(NO₃)₂.

8. The process of any one of claims 1-7, wherein the process is performed at a pH between 5.0 and 8.0.

9. The process of any one of claims 1-8, wherein the process is performed at a temperature between 20-60 °C.

10. The process of any one of claims 1-9, wherein the polyphenoloxidase is obtainable from a fungus.

11. The process of claim 10, wherein the fungus is *Myceliophthora thermophila.*

12. The process of any one of claims 1-11, wherein the polyphenoloxidase comprises or consists of an amino acid sequence that is at least 90% identical to SEQ ID NO:45.

13. The process of any one of claims 1-12, wherein said LPMO comprises or consists of an amino acid sequence that is at least 70% identical to SEQ ID NO:1 or SEQ ID NO:93.

14. The process of claim 13, wherein the LPMO comprises or consists of an amino acid sequence that is at least 90% identical to SEQ ID NO:1 or SEQ ID NO:93.

## Patentansprüche

1. Verfahren zum Abbauen und/oder Modifizieren von Cellulose in Lignocellulose enthaltendem Substrat, wobei das Verfahren den Schritt des Inkontaktbringens des Substrats mit einer lytischen Polysaccharidmonooxygenase (LPMO) und einer Polyphenoloxidase umfasst, wobei die Polyphenoloxidase eine Aminosäuresequenz umfasst oder aus dieser besteht, die zu mindestens 70% zu SEQ ID NO: 45 identisch ist, und wobei die Polyphenoloxidase eine zentrale Tyrosinase-Domäne umfasst oder aus dieser besteht und wobei die Polyphenoloxidase zum Demethylieren eines R-substituierten Mono- oder Di-Methoxyphenols in der Lage ist, das dargestellt wird durch Formel [1], [2] oder [3]: 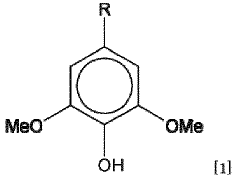 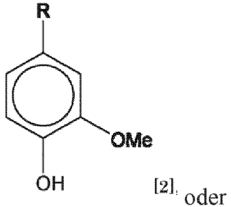 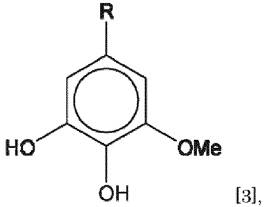 worin R ein beliebiges einzelnes Atom oder eine beliebige chemische Einheit sein kann.

2. Verfahren nach Anspruch 1, ferner umfassend den Schritt des Zumischens eines R-substituierten Mono- oder Di-Methoxyphenols, das dargestellt wird durch Formel [1], [2] oder [3].

3. Verfahren nach Anspruch 2, wobei R eine organische Einheit ist.

4. Verfahren nach Anspruch 2 oder 3, wobei das R-substituierte Mono- oder Di-Methoxyphenol dargestellt wird durch Formel [1].

5. Verfahren nach Anspruch 4, wobei das R-substituierte Mono- oder Di-Methoxyphenol Syringasäure oder Sinapinsäure ist.

6. Verfahren nach einem der Ansprüche 1 bis 5, wobei das Substrat 1 bis 1.000 µM eines Kupfersalzes umfasst.

7. Verfahren nach Anspruch 6, wobei das Kupfersalz ausgewählt wird aus der Gruppe bestehend aus CuSO₄, CuCl₂, CuBr₂, CuF₂, Cu(OH)₂ und Cu(NO₃)₂.

8. Verfahren nach einem der Ansprüche 1 bis 7, wobei das Verfahren ausgeführt wird bei einem pH-Wert zwischen 5,0 und 8,0.

9. Verfahren nach einem der Ansprüche 1 bis 8, wobei das Verfahren ausgeführt wird bei einer Temperatur zwischen 20° und 60 °C.

10. Verfahren nach einem der Ansprüche 1 bis 9, wobei die Polyphenoloxidase aus einem Fungus erhalten werden kann.

11. Verfahren nach Anspruch 10, wobei der Fungus *Myceliophthora thermophila* ist.

12. Verfahren nach einem der Ansprüche 1 bis 11, wobei die Polyphenoloxidase eine Aminosäuresequenz umfasst oder aus dieser besteht, die zu mindestens 90% zu SEQ ID NO: 45 identisch ist.

13. Verfahren nach einem der Ansprüche 1 bis 12, wobei die LPMO eine Aminosäuresequenz umfasst oder aus dieser besteht, die zu mindestens 70% zu SEQ ID NO: 1 oder SEQ ID NO: 93 identisch ist.

14. Verfahren nach Anspruch 13, wobei die LPMO eine Aminosäuresequenz umfasst oder aus dieser besteht, die zu mindestens 90% zu SEQ ID NO: 1 oder SEQ ID NO: 93 identisch ist.

## Revendications

1. Procédé pour la dégradation et/ou la modification de cellulose dans un substrat de biomasse lignocellulosique, où ledit procédé comprend l'étape de mise en contact dudit substrat avec une monooxygénase polysaccharidique lytique (LPMO) et une polyphénoloxydase, dans lequel la polyphénoloxydase comprend ou est constituée d'une séquence d'acides aminés qui est au moins 70% identique à la SEQ ID NO: 45 et dans lequel la polyphénoloxydase comprend ou est constituée d'un domaine de tyrosinase central et dans lequel la polyphénoloxydase est capable de déméthyler un mono- ou di-méthoxyphénol substitué par R représenté par la formule [1], [2] ou [3]: 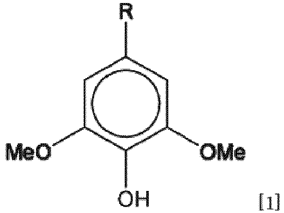 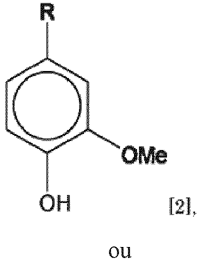 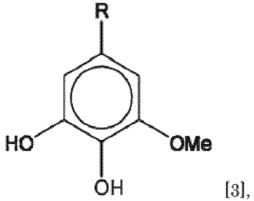 où R peut être un quelconque atome seul ou une quelconque fraction chimique.

2. Procédé selon la revendication 1, comprenant en outre l'étape de mélange d'un mono- ou di-méthoxyphénol substitué par R représenté par la formule [1], [2] ou [3].

3. Procédé selon la revendication 2, dans lequel R est une fraction organique.

4. Procédé selon la revendication 2 ou 3, dans lequel le mono- ou di-méthoxyphénol substitué par R est représenté par la formule [1].

5. Procédé selon la revendication 4, dans lequel le mono- ou di-méthoxyphénol substitué par R est l'acide syringique ou l'acide sinapique.

6. Procédé selon l'une quelconque des revendications 1-5, dans lequel ledit substrat comprend 1-1000 µM de sel de cuivre.

7. Procédé selon la revendication 6, dans lequel le sel de cuivre est choisi dans le groupe constitué de CuSO₄, CuCl₂, CuBr₂, CuF₂, Cu(OH)₂ et Cu(NO₃)₂.

8. Procédé selon l'une quelconque des revendications 1-7, où le procédé est effectué à un pH entre 5,0 et 8,0.

9. Procédé selon l'une quelconque des revendications 1-8, où le procédé est effectué à une température entre 20 et 60°C.

10. Procédé selon l'une quelconque des revendications 1-9, dans lequel la polyphénoloxydase peut être obtenue à partir d'un champignon.

11. Procédé selon la revendication 10, dans lequel le champignon est *Myceliophthora thermophila.*

12. Procédé selon l'une quelconque des revendications 1-11, dans lequel la polyphénoloxydase comprend ou est constituée d'une séquence d'acides aminés qui est au moins 90% identique à la SEQ ID NO: 45.

13. Procédé selon l'une quelconque des revendications 1-12, dans lequel ladite LPMO comprend ou est constituée d'une séquence d'acides aminés qui est au moins 70% identique à la SEQ ID NO: 1 ou la SEQ ID NO: 93.

14. Procédé selon la revendication 13, dans lequel la LPMO comprend ou est constituée d'une séquence d'acides aminés qui est au moins 90% identique à la SEQ ID NO: 1 ou la SEQ ID NO: 93.


### SEQUENCE LISTING

<110> Genencor International B.V.
<120> Polypeptides having demethylating activity
<130> P6056764PCT
<150> NL2015020
   <151> 2015-06-24
<160> 93
<170> PatentIn version 3.3
<210> 1
   <211> 208
   <212> PRT
   <213> Myceliophthora thermophila
<400> 1
   His Tyr Thr Leu Pro Arg Val Gly Thr Gly Ser Asp Trp Gln His Val
   1               5                   10                  15
   Arg Arg Ala Asp Asn Trp Gln Asn Asn Gly Phe Val Gly Asp Val Asn
               20                  25                  30
   Ser Glu Gln Ile Arg Cys Phe Gln Ala Thr Pro Ala Gly Ala Gln Asp
           35                  40                  45
   Val Tyr Thr Val Gln Ala Gly Ser Thr Val Thr Tyr His Ala Asn Pro
       50                  55                  60
   Ser Ile Tyr His Pro Gly Pro Met Gln Phe Tyr Leu Ala Arg Val Pro
   65                  70                  75                  80
   Asp Gly Gln Asp Val Lys Ser Trp Thr Gly Glu Gly Ala Val Trp Phe
                   85                  90                  95
   Lys Val Tyr Glu Glu Gln Pro Gln Phe Gly Ala Gln Leu Thr Trp Pro
               100                 105                 110
   Ser Asn Gly Lys Ser Ser Phe Glu Val Pro Ile Pro Ser Cys Ile Arg
           115                 120                 125
   Ala Gly Asn Tyr Leu Leu Arg Ala Glu His Ile Ala Leu His Val Ala
       130                 135                 140
   Gln Ser Gln Gly Gly Ala Gln Phe Tyr Ile Ser Cys Ala Gln Leu Gln
   145                 150                 155                 160
   Val Thr Gly Gly Gly Ser Thr Glu Pro Ser Gln Lys Val Ser Phe Pro
                   165                 170                 175
   Gly Ala Tyr Lys Ser Thr Asp Pro Gly Ile Leu Ile Asn Ile Asn Tyr
               180                 185                 190
   Pro Val Pro Thr Ser Tyr Gln Asn Pro Gly Pro Ala Val Phe Arg Cys
           195                 200                 205
<210> 2
   <211> 225
   <212> PRT
   <213> Myceliophthora thermophila
<400> 2
   Met Leu Thr Thr Thr Phe Ala Leu Leu Thr Ala Ala Leu Gly Val Ser
   1               5                   10                  15
   Ala His Tyr Thr Leu Pro Arg Val Gly Thr Gly Ser Asp Trp Gln His
               20                  25                  30
   Val Arg Arg Ala Asp Asn Trp Gln Asn Asn Gly Phe Val Gly Asp Val
           35                  40                  45
   Asn Ser Glu Gln Ile Arg Cys Phe Gln Ala Thr Pro Ala Gly Ala Gln
       50                  55                  60
   Asp Val Tyr Thr Val Gln Ala Gly Ser Thr Val Thr Tyr His Ala Asn
   65                  70                  75                  80
   Pro Ser Ile Tyr His Pro Gly Pro Met Gln Phe Tyr Leu Ala Arg Val
                   85                  90                  95
   Pro Asp Gly Gln Asp Val Lys Ser Trp Thr Gly Glu Gly Ala Val Trp
               100                 105                 110
   Phe Lys Val Tyr Glu Glu Gln Pro Gln Phe Gly Ala Gln Leu Thr Trp
           115                 120                 125
   Pro Ser Asn Gly Lys Ser Ser Phe Glu Val Pro Ile Pro Ser Cys Ile
       130                 135                 140
   Arg Ala Gly Asn Tyr Leu Leu Arg Ala Glu His Ile Ala Leu His Val
   145                 150                 155                 160
   Ala Gln Ser Gln Gly Gly Ala Gln Phe Tyr Ile Ser Cys Ala Gln Leu
                   165                 170                 175
   Gln Val Thr Gly Gly Gly Ser Thr Glu Pro Ser Gln Lys Val Ser Phe
               180                 185                 190
   Pro Gly Ala Tyr Lys Ser Thr Asp Pro Gly Ile Leu Ile Asn Ile Asn
           195                 200                 205
   Tyr Pro Val Pro Thr Ser Tyr Gln Asn Pro Gly Pro Ala Val Phe Arg
       210                 215                 220
   Cys
   225
<210> 3
   <211> 459
   <212> PRT
   <213> Thielavia terrestris
<400> 3
   Met Ala Pro Ser Val Thr Leu Pro Leu Thr Thr Ala Ile Leu Ala Ile
   1               5                   10                  15
   Ala Arg Leu Val Ala Ala Gln Gln Pro Gly Thr Ser Thr Pro Glu Val
               20                  25                  30
   His Pro Lys Leu Thr Thr Tyr Lys Cys Thr Lys Ser Gly Gly Cys Val
           35                  40                  45
   Ala Gln Asp Thr Ser Val Val Leu Asp Trp Asn Tyr Arg Trp Met His
       50                  55                  60
   Asp Ala Asn Tyr Asn Ser Cys Thr Val Asn Gly Gly Val Asn Thr Thr
   65                  70                  75                  80
   Leu Cys Pro Asp Glu Ala Thr Cys Gly Lys Asn Cys Phe Ile Glu Gly
                   85                  90                  95
   Val Asp Tyr Ala Ala Ser Gly Val Thr Thr Ser Gly Ser Ser Leu Thr
               100                 105                 110
   Met Asn Gln Tyr Met Pro Ser Ser Ser Gly Gly Tyr Ile Ser Val Ser
           115                 120                 125
   Pro Arg Leu Tyr Leu Leu Asp Ser Asp Gly Glu Tyr Val Met Leu Lys
       130                 135                 140
   Leu Asn Gly Gln Glu Leu Ser Phe Asp Val Asp Leu Ser Ala Leu Pro
   145                 150                 155                 160
   Cys Gly Glu Asn Gly Ser Leu Tyr Leu Ser Gln Met Asp Glu Asn Gly
                   165                 170                 175
   Gly Ala Asn Gln Tyr Asn Thr Ala Gly Ala Asn Tyr Gly Ser Gly Tyr
               180                 185                 190
   Cys Asp Ala Gln Cys Pro Val Gln Thr Trp Arg Asn Gly Thr Leu Asn
           195                 200                 205
   Thr Ser His Gln Gly Phe Cys Cys Asn Glu Ile Asp Ile Leu Glu Gly
       210                 215                 220
   Asn Ser Arg Ala Asn Ala Leu Thr Pro His Ser Cys Thr Gly Thr Ala
   225                 230                 235                 240
   Cys Asp Ser Ala Gly Cys Ser Phe Asn Pro Tyr Gly Ser Gly Tyr Lys
                   245                 250                 255
   Ser Tyr Tyr Gly Pro Gly Asp Thr Val Asp Thr Ser Lys Thr Phe Thr
               260                 265                 270
   Ile Ile Thr Gln Phe Asn Thr Asp Asn Gly Ser Pro Ser Arg Asn Phe
           275                 280                 285
   Val Gly Ile Thr Arg Lys Tyr Gln Gln Asn Gly Val Asp Ile Pro Ser
       290                 295                 300
   Ala Gln Pro Gly Gly Asp Thr Ile Ser Ser Cys Pro Ser Ala Ser Ala
   305                 310                 315                 320
   Tyr Gly Gly Leu Ala Thr Met Gly Lys Ala Leu Ser Ser Gly Met Val
                   325                 330                 335
   Leu Val Phe Ser Ile Arg Asn Asp Asn Ser Gln Tyr Met Asn Trp Leu
               340                 345                 350
   Asp Ser Gly Asn Ala Gly Arg Cys Ser Ser Thr Glu Gly Asn Pro Ser
           355                 360                 365
   Asn Ile Leu Pro Asn Asn Pro Asn Thr His Phe Val Phe Ser Asn Ile
       370                 375                 380
   Cys Trp Gly Asp Ile Gly Ser Thr Thr Asn Ser Thr Ala Pro Pro Pro
   385                 390                 395                 400
   Pro Pro Ala Ser Ser Thr Thr Phe Ser Thr Thr Arg Arg Ser Ser Thr
                   405                 410                 415
   Thr Ser Ser Ser Pro Ser Cys Thr Gln Thr His Trp Gly Gln Cys Gly
   420 425 430
   Ala Ile Gly Cys Ser Gly Cys Lys Thr Cys Thr Ser Gly Thr Thr Cys
   435 440 445
   Gln Tyr Ser Asn Asp Tyr Tyr Ser Gln Cys Leu
   450 455
<210> 4
   <211> 828
   <212> PRT
   <213> Myceliophthora thermophila
<400> 4
   Met Arg Thr Ser Ser Arg Leu Ile Gly Ala Leu Ala Ala Ala Leu Leu
   1 5 10 15
   Pro Ser Ala Leu Ala Gln Asn Asn Ala Pro Val Thr Phe Thr Asp Pro
   20 25 30
   Asp Ser Gly Ile Thr Phe Asn Thr Trp Gly Leu Ala Glu Asp Ser Pro
   35 40 45
   Gln Thr Lys Gly Gly Phe Thr Phe Gly Val Ala Leu Pro Ser Asp Ala
   50 55 60
   Leu Thr Thr Asp Ala Lys Glu Phe Ile Gly Tyr Leu Lys Cys Ala Arg
   65 70 75 80
   Asn Asp Glu Ser Gly Trp Cys Gly Val Ser Leu Gly Gly Pro Met Thr
   85 90 95
   Asn Ser Leu Leu Ile Ala Ala Trp Pro His Glu Asp Thr Val Tyr Thr
   100 105 110
   Ser Leu Arg Phe Ala Thr Gly Tyr Ala Met Pro Asp Val Tyr Gln Gly
   115 120 125
   Asp Ala Glu Ile Thr Gln Val Ser Ser Ser Val Asn Ser Thr His Phe
   130 135 140
   Ser Leu Ile Phe Arg Cys Glu Asn Cys Leu Gln Trp Ser Gln Ser Gly
   145 150 155 160
   Ala Thr Gly Gly Ala Ser Thr Ser Asn Gly Val Leu Val Leu Gly Trp
   165 170 175
   Val Gln Ala Phe Ala Asp Pro Gly Asn Pro Thr Cys Pro Asp Gln Ile
               180                 185                 190
   Thr Leu Glu Gln His Asp Asn Gly Met Gly Ile Trp Gly Ala Gln Leu
           195                 200                 205
   Asn Ser Asp Ala Ala Ser Pro Ser Tyr Thr Glu Trp Ala Ala Gln Ala
       210                 215                 220
   Thr Lys Thr Val Thr Gly Asp Cys Gly Gly Pro Thr Glu Thr Ser Val
   225                 230                 235                 240
   Val Gly Val Pro Val Pro Thr Gly Val Ser Phe Asp Tyr Ile Val Val
                   245                 250                 255
   Gly Gly Gly Ala Gly Gly Ile Pro Ala Ala Asp Lys Leu Ser Glu Ala
               260                 265                 270
   Gly Lys Ser Val Leu Leu Ile Glu Lys Gly Phe Ala Ser Thr Ala Asn
           275                 280                 285
   Thr Gly Gly Thr Leu Gly Pro Glu Trp Leu Glu Gly His Asp Leu Thr
       290                 295                 300
   Arg Phe Asp Val Pro Gly Leu Cys Asn Gln Ile Trp Val Asp Ser Lys
   305                 310                 315                 320
   Gly Ile Ala Cys Glu Asp Thr Asp Gln Met Ala Gly Cys Val Leu Gly
                   325                 330                 335
   Gly Gly Thr Ala Val Asn Ala Gly Leu Trp Phe Lys Pro Tyr Ser Leu
               340                 345                 350
   Asp Trp Asp Tyr Leu Phe Pro Ser Gly Trp Lys Tyr Lys Asp Val Gln
           355                 360                 365
   Pro Ala Ile Asn Arg Ala Leu Ser Arg Ile Pro Gly Thr Asp Ala Pro
       370                 375                 380
   Ser Thr Asp Gly Lys Arg Tyr Tyr Gln Gln Gly Phe Asp Val Leu Ser
   385                 390                 395                 400
   Lys Gly Leu Ala Gly Gly Gly Trp Thr Ser Val Thr Ala Asn Asn Ala
                   405                 410                 415
   Pro Asp Lys Lys Asn Arg Thr Phe Ser His Ala Pro Phe Met Phe Ala
               420                 425                 430
   Gly Gly Glu Arg Asn Gly Pro Leu Gly Thr Tyr Phe Gln Thr Ala Lys
           435                 440                 445
   Lys Arg Ser Asn Phe Lys Leu Trp Leu Asn Thr Ser Val Lys Arg Val
       450                 455                 460
   Ile Arg Gln Gly Gly His Ile Thr Gly Val Glu Val Glu Pro Phe Arg
   465                 470                 475                 480
   Asp Gly Gly Tyr Gln Gly Ile Val Pro Val Thr Lys Val Thr Gly Arg
                   485                 490                 495
   Val Ile Leu Ser Ala Gly Thr Phe Gly Ser Ala Lys Ile Leu Leu Arg
               500                 505                 510
   Ser Gly Ile Gly Pro Asn Asp Gln Leu Gln Val Val Ala Ala Ser Glu
           515                 520                 525
   Lys Asp Gly Pro Thr Met Ile Ser Asn Ser Ser Trp Ile Asn Leu Pro
       530                 535                 540
   Val Gly Tyr Asn Leu Asp Asp His Leu Asn Thr Asp Thr Val Ile Ser
   545                 550                 555                 560
   His Pro Asp Val Val Phe Tyr Asp Phe Tyr Glu Ala Trp Asp Asn Pro
                   565                 570                 575
   Ile Gln Ser Asp Lys Asp Ser Tyr Leu Asn Ser Arg Thr Gly Ile Leu
               580                 585                 590
   Ala Gln Ala Ala Pro Asn Ile Gly Pro Met Phe Trp Glu Glu Ile Lys
           595                 600                 605
   Gly Ala Asp Gly Ile Val Arg Gln Leu Gln Trp Thr Ala Arg Val Glu
       610                 615                 620
   Gly Ser Leu Gly Ala Pro Asn Gly Lys Thr Met Thr Met Ser Gln Tyr
   625                 630                 635                 640
   Leu Gly Arg Gly Ala Thr Ser Arg Gly Arg Met Thr Ile Thr Pro Ser
                   645                 650                 655
   Leu Thr Thr Val Val Ser Asp Val Pro Tyr Leu Lys Asp Pro Asn Asp
               660                 665                 670
   Lys Glu Ala Val Ile Gln Gly Ile Ile Asn Leu Gln Asn Ala Leu Lys
           675                 680                 685
   Asn Val Ala Asn Leu Thr Trp Leu Phe Pro Asn Ser Thr Ile Thr Pro
   690 695 700
   Arg Gln Tyr Val Asp Ser Met Val Val Ser Pro Ser Asn Arg Arg Ser
   705 710 715 720
   Asn His Trp Met Gly Thr Asn Lys Ile Gly Thr Asp Asp Gly Arg Lys
   725 730 735
   Gly Gly Ser Ala Val Val Asp Leu Asn Thr Lys Val Tyr Gly Thr Asp
   740 745 750
   Asn Leu Phe Val Ile Asp Ala Ser Ile Phe Pro Gly Val Pro Thr Thr
   755 760 765
   Asn Pro Thr Ser Tyr Ile Val Thr Ala Ser Glu His Ala Ser Ala Arg
   770 775 780
   Ile Leu Ala Leu Pro Asp Leu Thr Pro Val Pro Lys Tyr Gly Gln Cys
   785 790 795 800
   Gly Gly Arg Glu Trp Ser Gly Ser Phe Val Cys Ala Asp Gly Ser Thr
   805 810 815
   Cys Gln Met Gln Asn Glu Trp Tyr Ser Gln Cys Leu
   820 825
<210> 5
   <211> 787
   <212> PRT
   <213> Myceliophthora thermophila
<400> 5
   Met Lys Leu Leu Ser Arg Val Gly Ala Thr Ala Leu Ala Ala Thr Leu
   1 5 10 15
   Ser Leu Gln Gln Cys Ala Ala Gln Met Thr Glu Gly Thr Tyr Thr Asp
   20 25 30
   Glu Ala Thr Gly Ile Gln Phe Lys Thr Trp Thr Ala Ser Glu Gly Ala
   35 40 45
   Pro Phe Thr Phe Gly Leu Thr Leu Pro Ala Asp Ala Leu Glu Lys Asp
   50 55 60
   Ala Thr Glu Tyr Ile Gly Leu Leu Arg Cys Gln Ile Thr Asp Pro Ala
   65 70 75 80
   Ser Pro Ser Trp Cys Gly Ile Ser His Gly Gln Ser Gly Gln Met Thr
                   85                  90                  95
   Gln Ala Leu Leu Leu Val Ala Trp Ala Ser Glu Asp Thr Val Tyr Thr
               100                 105                 110
   Ser Phe Arg Tyr Ala Thr Gly Tyr Thr Leu Pro Gly Leu Tyr Thr Gly
           115                 120                 125
   Asp Ala Lys Leu Thr Gln Ile Ser Ser Ser Val Ser Glu Asp Ser Phe
       130                 135                 140
   Glu Val Leu Phe Arg Cys Glu Asn Cys Phe Ser Trp Asp Gln Asp Gly
   145                 150                 155                 160
   Thr Lys Gly Asn Val Ser Thr Ser Asn Gly Asn Leu Val Leu Gly Arg
                   165                 170                 175
   Ala Ala Ala Lys Asp Gly Val Thr Gly Pro Thr Cys Pro Asp Thr Ala
               180                 185                 190
   Glu Phe Gly Phe His Asp Asn Gly Phe Gly Gln Trp Gly Ala Val Leu
           195                 200                 205
   Glu Gly Ala Thr Ser Asp Ser Tyr Glu Glu Trp Ala Lys Leu Ala Thr
       210                 215                 220
   Thr Thr Pro Glu Thr Thr Cys Asp Gly Thr Gly Pro Gly Asp Lys Glu
   225                 230                 235                 240
   Cys Val Pro Ala Pro Glu Asp Thr Tyr Asp Tyr Ile Val Val Gly Ala
                   245                 250                 255
   Gly Ala Gly Gly Ile Thr Val Ala Asp Lys Leu Ser Glu Ala Gly His
               260                 265                 270
   Lys Val Leu Leu Ile Glu Lys Gly Pro Pro Ser Thr Gly Leu Trp Asn
           275                 280                 285
   Gly Thr Met Lys Pro Glu Trp Leu Glu Ser Thr Asp Leu Thr Arg Phe
       290                 295                 300
   Asp Val Pro Gly Leu Cys Asn Gln Ile Trp Val Asp Ser Ala Gly Ile
   305                 310                 315                 320
   Ala Cys Thr Asp Thr Asp Gln Met Ala Gly Cys Val Leu Gly Gly Gly
                   325                 330                 335
   Thr Ala Val Asn Ala Gly Leu Trp Trp Lys Pro His Pro Ala Asp Trp
               340                 345                 350
   Asp Glu Asn Phe Pro Glu Gly Trp Lys Ser Ser Asp Leu Ala Asp Ala
           355                 360                 365
   Thr Glu Arg Val Phe Lys Arg Ile Pro Gly Thr Ser His Pro Ser Gln
       370                 375                 380
   Asp Gly Lys Leu Tyr Arg Gln Glu Gly Phe Glu Val Ile Ser Lys Gly
   385                 390                 395                 400
   Leu Ala Asn Ala Gly Trp Lys Glu Ile Ser Ala Asn Glu Ala Pro Ser
                   405                 410                 415
   Glu Lys Asn His Thr Tyr Ala His Thr Glu Phe Met Phe Ser Gly Gly
               420                 425                 430
   Glu Arg Gly Gly Pro Leu Ala Thr Tyr Leu Ala Ser Ala Ala Glu Arg
           435                 440                 445
   Ser Asn Phe Asn Leu Trp Leu Asn Thr Ala Val Arg Arg Ala Val Arg
       450                 455                 460
   Ser Gly Ser Lys Val Thr Gly Val Glu Leu Glu Cys Leu Thr Asp Gly
   465                 470                 475                 480
   Gly Phe Ser Gly Thr Val Asn Leu Asn Glu Gly Gly Gly Val Ile Phe
                   485                 490                 495
   Ser Ala Gly Ala Phe Gly Ser Ala Lys Leu Leu Leu Arg Ser Gly Ile
               500                 505                 510
   Gly Pro Glu Asp Gln Leu Glu Ile Val Ala Ser Ser Lys Asp Gly Glu
           515                 520                 525
   Thr Phe Thr Pro Lys Asp Glu Trp Ile Asn Leu Pro Val Gly His Asn
       530                 535                 540
   Leu Ile Asp His Leu Asn Thr Asp Leu Ile Ile Thr His Pro Asp Val
   545                 550                 555                 560
   Val Phe Tyr Asp Phe Tyr Ala Ala Trp Asp Glu Pro Ile Thr Glu Asp
                   565                 570                 575
   Lys Glu Ala Tyr Leu Asn Ser Arg Ser Gly Ile Leu Ala Gln Ala Ala
               580                 585                 590
   Pro Asn Ile Gly Pro Met Met Trp Asp Gln Val Thr Pro Ser Asp Gly
           595                 600                 605
   Ile Thr Arg Gln Phe Gln Trp Thr Cys Arg Val Glu Gly Asp Ser Ser
       610                 615                 620
   Lys Thr Asn Ser Thr His Ala Met Thr Leu Ser Gln Tyr Leu Gly Arg
   625                 630                 635                 640
   Gly Val Val Ser Arg Gly Arg Met Gly Ile Thr Ser Gly Leu Ser Thr
                   645                 650                 655
   Thr Val Ala Glu His Pro Tyr Leu His Asn Asn Gly Asp Leu Glu Ala
               660                 665                 670
   Val Ile Gln Gly Ile Gln Asn Val Val Asp Ala Leu Ser Gln Val Ala
           675                 680                 685
   Asp Leu Glu Trp Val Leu Pro Pro Pro Asp Gly Thr Val Ala Asp Tyr
       690                 695                 700
   Val Asn Ser Leu Ile Val Ser Pro Ala Asn Arg Arg Ala Asn His Trp
   705                 710                 715                 720
   Met Gly Thr Ala Lys Leu Gly Thr Asp Asp Gly Arg Ser Gly Gly Thr
                   725                 730                 735
   Ser Val Val Asp Leu Asp Thr Lys Val Tyr Gly Thr Asp Asn Leu Phe
               740                 745                 750
   Val Val Asp Ala Ser Val Phe Pro Gly Met Ser Thr Gly Asn Pro Ser
           755                 760                 765
   Ala Met Ile Val Ile Val Ala Glu Gln Ala Ala Gln Arg Ile Leu Ala
       770                 775                 780
   Leu Arg Ser
   785
<210> 6
   <211> 577
   <212> PRT
   <213> Myceliophthora thermophila
<400> 6
   Met Gln Val Ala Ser Lys Leu Val Ala Val Thr Gly Gly Ala Leu Ala
   1               5                   10                  15
   Leu Trp Leu His Pro Val Ala Ala Gln Glu Gly Cys Thr Asn Ile Ser
               20                  25                  30
   Ser Thr Glu Thr Tyr Asp Tyr Ile Val Val Gly Ser Gly Ala Gly Gly
           35                  40                  45
   Ile Pro Val Ala Asp Arg Leu Ser Glu Ala Gly His Lys Val Leu Leu
       50                  55                  60
   Ile Glu Lys Gly Pro Pro Ser Thr Gly Arg Trp Gly Gly Ile Met Lys
   65                  70                  75                  80
   Pro Glu Trp Leu Ile Gly Thr Asn Leu Thr Arg Phe Asp Val Pro Gly
                   85                  90                  95
   Leu Cys Asn Gln Ile Trp Ala Asp Pro Thr Gly Ala Ile Cys Thr Asp
               100                 105                 110
   Val Asp Gln Met Ala Gly Cys Met Leu Gly Gly Gly Thr Ala Val Asn
           115                 120                 125
   Ala Gly Leu Trp Trp Lys Pro His Pro Ala Asp Trp Asp Val Asn Phe
       130                 135                 140
   Pro Glu Gly Trp His Ser Glu Asp Met Ala Glu Ala Thr Glu Arg Val
   145                 150                 155                 160
   Phe Glu Arg Ile Pro Gly Thr Ile Thr Pro Ser Met Asp Gly Lys Arg
                   165                 170                 175
   Tyr Leu Ser Gln Gly Phe Asp Met Leu Gly Gly Ser Leu Glu Ala Ala
               180                 185                 190
   Gly Trp Glu Tyr Leu Val Pro Asn Glu His Pro Asp Arg Lys Asn Arg
           195                 200                 205
   Thr Tyr Gly His Ser Thr Phe Met Tyr Ser Gly Gly Glu Arg Gly Gly
       210                 215                 220
   Pro Leu Ala Thr Tyr Leu Val Ser Ala Val Gln Arg Glu Gly Phe Thr
   225                 230                 235                 240
   Leu Trp Met Asn Thr Thr Val Thr Arg Ile Ile Arg Glu Gly Gly His
                   245                 250                 255
   Ala Thr Gly Val Glu Val Gln Cys Ser Asn Ser Glu Ala Gly Gln Ala
               260                 265                 270
   Gly Ile Val Pro Leu Thr Pro Lys Thr Gly Arg Val Ile Val Ser Ala
           275                 280                 285
   Gly Ala Phe Gly Ser Ala Lys Leu Leu Phe Arg Ser Gly Ile Gly Pro
       290                 295                 300
   Lys Asp Gln Leu Asn Ile Val Lys Asn Ser Thr Asp Gly Pro Ser Met
   305                 310                 315                 320
   Ile Ser Glu Asp Gln Trp Ile Glu Leu Pro Val Gly Tyr Asn Leu Asn
                   325                 330                 335
   Asp His Val Gly Thr Asp Ile Glu Ile Ala His Pro Asp Val Val Phe
               340                 345                 350
   Tyr Asp Tyr Tyr Gly Ala Trp Asp Glu Pro Ile Val Glu Asp Thr Glu
           355                 360                 365
   Arg Tyr Val Ala Asn Arg Thr Gly Pro Leu Ala Gln Ala Ala Pro Asn
       370                 375                 380
   Ile Gly Pro Ile Phe Trp Glu Thr Ile Lys Gly Ser Asp Gly Val Ser
   385                 390                 395                 400
   Arg His Leu Gln Trp Gln Ala Arg Val Glu Gly Lys Leu Asn Thr Ser
                   405                 410                 415
   Met Thr Ile Thr Gln Tyr Leu Gly Thr Gly Ser Arg Ser Arg Gly Arg
               420                 425                 430
   Met Thr Ile Thr Arg Arg Leu Asn Thr Val Val Ser Thr Pro Pro Tyr
           435                 440                 445
   Leu Arg Asp Glu Tyr Asp Arg Glu Ala Val Ile Gln Gly Ile Ala Asn
       450                 455                 460
   Leu Arg Glu Ser Leu Lys Gly Val Ala Asn Leu Thr Trp Ile Thr Pro
   465                 470                 475                 480
   Pro Ser Asn Val Thr Val Glu Asp Phe Val Asp Ser Ile Pro Ala Thr
                   485                 490                 495
   Pro Ala Arg Arg Cys Ser Asn His Trp Ile Gly Thr Ala Lys Ile Gly
               500                 505                 510
   Leu Asp Asp Gly Arg Glu Gly Gly Thr Ser Val Val Asp Leu Asn Thr
   515 520 525
   Lys Val Tyr Gly Thr Asp Asn Ile Phe Val Val Asp Ala Ser Ile Phe
   530 535 540
   Pro Gly His Ile Thr Gly Asn Pro Ser Ala Ala Ile Val Ile Ala Ala
   545 550 555 560
   Glu Tyr Ala Ala Ala Lys Ile Leu Ala Leu Pro Ala Pro Glu Asp Ala
   565 570 575
   Ala
<210> 7
   <211> 675
   <212> DNA
   <213> Artificial
<220>
   <223> cDNA encoding MtLPMO9A
<400> 7
   atgctgacaa caaccttcgc cctcctgacg gccgctctcg gcgtcagcgc ccattatacc 60
   ctccccaggg tcgggaccgg ttccgactgg cagcacgtgc ggcgggctga caactggcaa 120
   aacaacggct tcgtcggcga cgtcaactcg gagcagatca ggtgcttcca ggcgacccct 180
   gccggcgccc aagacgtcta cactgttcag gcgggatcga ccgtgaccta ccacgccaac 240
   cccagtatct accaccccgg ccccatgcag ttctacctgg cccgcgttcc ggacggacag 300
   gacgtcaagt cgtggaccgg cgagggtgcc gtgtggttca aggtgtacga ggagcagcct 360
   caatttggcg cccagctgac ctggcctagc aacggcaaga gctcgttcga ggttcctatc 420
   cccagctgca ttcgggcggg caactacctc ctccgcgctg agcacatcgc cctgcacgtt 480
   gcccaaagcc agggcggcgc ccagttctac atctcgtgcg cccagctcca ggtcactggt 540
   ggcggcagca ccgagccttc tcagaaggtt tccttcccgg gtgcctacaa gtccaccgac 600
   cccggcattc ttatcaacat caactacccc gtccctacct cgtaccagaa tccgggtccg 660
   gctgtcttcc gttgc 675
<210> 8
   <211> 963
   <212> DNA
   <213> Myceliophthora thermophila
<400> 8
   agcggcgttc tgaaaaggag gaacagaact aacgaagacg acccaagcat accaaggtcg      60
   tgttgattag cacccccgat tcttccagtt ctttccgcca tctgcaatgc tgacaacaac     120
   cttcgccctc ctgacggccg ctctcggcgt cagcgcccat tataccctcc ccagggtcgg     180
   gaccggttcc gactggcagc acgtgcggcg ggctgacaac tggcaaaaca acggcttcgt     240
   cggcgacgtc aactcggagc agatcaggtg cttccaggcg acccctgccg gcgcccaaga     300
   cgtctacact gttcaggcgg gatcgaccgt gacctaccac gccaacccca gtatctacca     360
   ccccggcccc atgcagttct acctggcccg cgttccggac ggacaggacg tcaagtcgtg     420
   gaccggcgag ggtgccgtgt ggttcaaggt gtacgaggag cagcctcaat ttggcgccca     480
   gctgacctgg cctagcaacg gtgcgttgat cattttcctt cttcttcctt ctttcttccg     540
   ttgcatatgc taactgttct cttgcttgca ggcaagagct cgttcgaggt tcctatcccc     600
   agctgcattc gggcgggcaa ctacctcctc cgcgctgagc acatcgccct gcacgttgcc     660
   caaagccagg gcggcgccca gttctacatc tcgtgcgccc agctccaggt cactggtggc     720
   ggcagcaccg agccttctca gaaggtttcc ttcccgggtg cctacaagtc caccgacccc     780
   ggcattctta tcaacatcaa ctaccccgtc cctacctcgt accagaatcc gggtccggct     840
   gtcttccgtt gctaagcggg cacaacaagg gaattggaac ggggcaatgg gggcgggctg     900
   gagccgcctg cgtccttgtt ttcttaccct cgccgttcgt acgaatcagg gccaagcaaa     960
   aca                                                                   963
<210> 9
   <211> 1380
   <212> DNA
   <213> Artificial
<220>
   <223> cDNA encoding TvEG I
<400> 9
   atggcgccct cagttacact gccgttgacc acggccatcc tggccattgc ccggctcgtc      60
   gccgcccagc aaccgggtac cagcaccccc gaggtccatc ccaagttgac aacctacaag     120
   tgtacaaagt ccggggggtg cgtggcccag gacacctcgg tggtccttga ctggaactac     180
   cgctggatgc acgacgcaaa ctacaactcg tgcaccgtca acggcggcgt caacaccacg     240
   ctctgccccg acgaggcgac ctgtggcaag aactgcttca tcgagggcgt cgactacgcc     300
   gcctcgggcg tcacgacctc gggcagcagc ctcaccatga accagtacat gcccagcagc     360
   tctggcggct acatcagcgt ctctcctcgg ctgtatctcc tggactctga cggtgagtac     420
   gtgatgctga agctcaacgg ccaggagctg agcttcgacg tcgacctctc tgctctgccg     480
   tgtggagaga acggctcgct ctacctgtct cagatggacg agaacggggg cgccaaccag     540
   tataacacgg ccggtgccaa ctacgggagc ggctactgcg atgctcagtg ccccgtccag     600
   acatggagga acggcaccct caacactagc caccagggct tctgctgcaa cgagattgat     660
   atcctggagg gcaactcgag ggcgaatgcc ttgacccctc actcttgcac gggcacggcc     720
   tgcgactctg ccggttgcag cttcaacccc tatggcagcg gctacaaaag ctactacggc     780
   cccggagata ccgttgacac ctccaagacc ttcaccatca tcacccagtt caacacggac     840
   aacggctcgc cctcgcgcaa ctttgtgggc atcacccgca agtaccagca aaacggcgtc     900
   gacatcccca gcgcccagcc cggcggtgac accatctcgt cctgcccgtc cgcctcagcc     960
   tacggcggcc tcgccaccat gggcaaggcc ctgagcagcg gcatggtgct cgtgttcagc    1020
   attcggaacg acaacagcca gtacatgaac tggctcgaca gcggcaacgc cggccgctgc   1080
   agcagcaccg agggcaaccc atccaacatc ctgcccaaca accccaacac gcacttcgtc    1140
   ttctccaaca tctgctgggg agacattggg tctactacga actcgactgc gcccccgccc    1200
   ccgcctgcgt ctagcacgac gttctcgact acacggagga gctcgacgac ttcgagcagc    1260
   ccgagctgca cgcagactca ctgggggcag tgcggtgcca ttgggtgcag cgggtgcaag    1320
   acgtgcacgt cgggcactac gtgccagtat agcaacgact actactcgca atgcctttag    1380
<210> 10
   <211> 337
   <212> PRT
   <213> Myceliophthora thermophila
<400> 10
   Met Lys Phe Leu Asp Val Leu Leu Gly Ala Ala Ala Ala Ser Ser Ala
   1               5                   10                  15
   Leu Ala Ala Pro Thr Cys Thr Thr Lys Thr Lys Arg Ala Gly Lys Phe
               20                  25                  30
   Lys Phe Val Gly Val Asn Gln Ser Cys Ala Glu Phe Gly Gln Asp Thr
           35                  40                  45
   Leu Pro Gly Gln Leu Asn Lys His Tyr Thr Trp Pro Ala Lys Ser Ser
       50                  55                  60
   Ile Asp Thr Leu Leu Ala Thr Gly Met Asn Thr Ile Arg Ile Pro Phe
   65                  70                  75                  80
   Met Met Glu Arg Leu Ile Pro Asn Gln Leu Thr Gly Thr Val Asn Glu
                   85                  90                  95
   Thr Tyr Ser Ala Gly Leu Ile Asp Thr Val Ser Tyr Val Thr Ser Lys
               100                 105                 110
   Gly Ala Tyr Ala Val Ile Asp Pro His Asn Phe Gly Arg Tyr Tyr Thr
               115                 120                 125
   Gln Val Ile Thr Asp Val Glu Gly Phe Lys Ala Trp Trp Thr Thr Thr
       130                 135                 140
   Ala Gly Leu Phe Ala Asp Asn Asp Lys Val Ile Phe Asp Thr Asn Asn
   145                 150                 155                 160
   Glu Tyr His Asp Met Asp Gln Ser Leu Val Val Asn Leu Asn Gln Ala
                   165                 170                 175
   Ala Ile Asp Gly Ile Arg Ala Ala Gly Ala Thr Ser Gln Tyr Ile Phe
               180                 185                 190
   Ile Glu Gly Asn Ser Trp Thr Gly Ala Trp Thr Trp Val Ser Ser Gly
           195                 200                 205
   Asn Gly Glu Ser Leu Leu Asn Leu Ser Asp Pro Glu Gly Asp Asp Lys
       210                 215                 220
   Leu Ile Tyr Glu Met His Gln Tyr Leu Asp Glu Asp Gly Ser Gly Thr
   225                 230                 235                 240
   His Glu Gln Cys Val Ser Gly Thr Ile Gly Arg Glu Arg Leu Gln Ala
                   245                 250                 255
   Ala Thr Glu Trp Leu Lys Ala Asn Gly Lys Lys Ala Ile Leu Gly Glu
               260                 265                 270
   Thr Ala Gly Gly Ala Asn Asp Gln Cys Ile Ser Ala Leu Thr Gly Met
           275                 280                 285
   Leu Ser Phe Met Glu Glu Asn Ser Asp Val Trp Gln Gly Trp Leu Trp
       290                 295                 300
   Trp Ala Ala Gly Pro Trp Trp Ala Asp Tyr Met Tyr Ser Ile Glu Pro
   305                 310                 315                 320
   Pro Ser Gly Thr Ala Tyr Thr Lys Val Leu Pro Ser Leu Gln Pro Tyr
                   325                 330                 335
   Ile
<210> 11
   <211> 1014
   <212> DNA
   <213> Artificial
<220>
   <223> cDNA encoding MtEG VIII
<400> 11
   atgaagttcc tcgacgtcct tctcggtgcc gcagctgcca gctcggccct ggcagcgccg      60
   acttgcacaa ccaagacaaa gcgcgcgggc aagttcaagt ttgtcggcgt caaccagtcc     120
   tgcgccgagt ttggccagga cacgctcccg ggccagctca acaagcacta cacctggccg     180
   gccaagtcga gcattgatac actcctcgcc accggaatga acaccattcg catcccgttt     240
   atgatggagc gtcttatccc caaccaactg acgggaaccg tcaacgaaac gtactccgcg     300
   gggcttatcg ataccgtttc ctacgtcacg agcaagggag cctatgctgt cattgacccc     360
   cataacttcg gccggtacta cacgcaagtc atcaccgacg tcgaaggctt caaggcctgg     420
   tggacgacca cggccgggct gtttgccgac aacgacaagg tcatcttcga caccaacaac     480
   gagtaccacg acatggacca atcgctcgtt gtcaatctga accaggccgc catcgacggc     540
   atccgggccg ccggcgccac ctcgcagtac atctttatcg agggcaactc gtggacgggc     600
   gcatggacct gggtctcctc gggcaacggc gagtccctgc tgaacctgtc ggacccggag     660
   ggcgacgaca agctcatcta cgagatgcac cagtacctgg acgaggacgg gtcgggcacg     720
   cacgagcagt gcgtctcggg caccatcggc cgggagcgcc tccaggcggc gaccgagtgg     780
   ctcaaggcca acggcaagaa ggccatcctg ggcgagacgg ccggcggcgc caacgaccag     840
   tgcatctcgg ccctgaccgg catgctctcc ttcatggagg agaactcgga cgtctggcag     900
   ggctggctct ggtgggctgc cggtccctgg tgggccgact acatgtactc gattgagccg     960
   ccgagcggta ccgcgtacac caaggttttg cccagcctcc agccttatat ctga          1014
<210> 12
   <211> 2000
   <212> DNA
   <213> Myceliophthora thermophila
<400> 12
   gccccggggc gcgttccgag ttgaatgaat gagcgggtta atatagtaaa gatctgcgct     60
   ataacctggt ctcgagcgcg gagccaacct ttccgagcac gcatcttgat aatgagtaaa    120
   cccccttcaa caaacttgtg aaagagctac ctatagctgg acatataaat ctgcaatggc    180
   catctcagaa agggacccca tgcggaaccg ttggtgcagc aggaagcggt ttactttctg    240
   tcgacaacca actcaaccat gaagttcctc gacgtccttc tcggtgccgc agctgccagc    300
   tcggccctgg cagcgccgac ttgcacaacc aagacaaagc gcgcgggcaa gttcaagttt    360
   gtcggcgtca accagtcctg cgccgagttt ggccaggaca cgctcccggg ccagctcaac    420
   aagcactaca cctggccggc caagtcgagc attgatgtaa gtgtctttga acttcgagct    480
   accatgacga aaaaggaaca atggaagtaa ccaggctttc gaaaatcaga cactcctcgc    540
   caccggaatg aacaccattc gcatcccgtt tatgatgtag gactgagtgc cctttccgct 600
   tcgctttaca agtaaagagg gcacacattg ctgacaacgg gttgcaggga gcgtcttatc 660
   cccaaccaac tgacgggaac cgtcaacgaa acgtactccg cggggcttat cgatgtatga 720
   aacagacata gagcttctca agaacggggg gccggaattt gctgaccgga atttcagacc 780
   gtttcctacg tcacgagcaa gggagcctat gctgtcattg acccccataa cttcggtacg 840
   accagcttac aatgaccatg tcttgggacc gtggcagaca agctaaccaa gacttgctcc 900
   accaaatttc acaggccggt actacacgca agtcatcacc gacgtcgaag gcttcaaggc 960
   ctggtggacg accacggccg ggctgtttgc cgacaacgac aaggtcatct tcgacaccaa 1020
   caacgagtac cacgacatgg accaatcgct cgttgtcaat ctgaaccagg ccgccatcga 1080
   cggcatccgg gccgccggcg ccacctcgca gtacatcttt atcgagggca actcgtggac 1140
   gggcgcatgg acctgggtac gaacacggat ttctgatccg gatatttata ttgattttat 1200
   gattttgttt ttttggaccg gtgtaatagc agctgacaat catgtcattt ccaggtctcc 1260
   tcgggcaacg gcgagtccct gctgaacctg tcggacccgg agggcgacga caagctcatc 1320
   tacgagatgc accagtacct ggacgaggac gggtcgggca cgcacgagca gtgcgtctcg 1380
   ggcaccatcg gccgggagcg cctccaggcg gcgaccgagt ggctcaaggc caacggcaag 1440
   aaggccatcc tgggcgagac ggccggcggc gccaacgacc agtgcatctc ggccctgacc 1500
   ggcatgctct ccttcatgga ggagaactcg gacgtctggc agggctggct ctggtgggct 1560
   gccggtccct ggtgggccga ctacatgtac tcgatgtgag tgttttctta tttttcttct 1620
   ctcatttcat tcctttctga agatcttgtt tttttttccc tccctgtcta acctcgctta 1680
   gtgagccgcc gagcggtacc gcgtacacca aggttttgcc cagcctccag ccttatatct 1740
   gagctcggtc ggttctgcaa acgttgcgga gcggaagaag attccactgt tggggttgat 1800
   cacaaagata ctgagcctta agtgtacttt acttcatcag ggtagatgaa atcgagcaag 1860
   atacatacag ttccctgaga gctatggtcc ttacgtcacg tcactcaact gaggacccaa 1920
   attgcccgag tcttaatcca aagagtactc cgtaccgcta ccagggcgat gactcctgta 1980
   aattacactg ataatacaaa 2000
<210> 13
   <211> 389
   <212> PRT
   <213> Myceliophthora thermophila
<400> 13
   Met Lys Ser Ser Ile Leu Ala Ser Val Phe Ala Thr Gly Ala Val Ala
   1 5 10 15
   Gln Ser Gly Pro Trp Gln Gln Cys Gly Gly Ile Gly Trp Gln Gly Ser
               20                  25                  30
   Thr Asp Cys Val Ser Gly Tyr His Cys Val Tyr Gln Asn Asp Trp Tyr
           35                  40                  45
   Ser Gln Cys Val Pro Gly Ala Ala Ser Thr Thr Leu Gln Thr Ser Thr
       50                  55                  60
   Thr Ser Arg Pro Thr Ala Thr Ser Thr Ala Pro Pro Ser Ser Thr Thr
   65                  70                  75                  80
   Ser Pro Ser Lys Gly Lys Leu Lys Trp Leu Gly Ser Asn Glu Ser Gly
                   85                  90                  95
   Ala Glu Phe Gly Glu Gly Asn Tyr Pro Gly Leu Trp Gly Lys His Phe
               100                 105                 110
   Ile Phe Pro Ser Thr Ser Ala Ile Gln Thr Leu Ile Asn Asp Gly Tyr
           115                 120                 125
   Asn Ile Phe Arg Ile Asp Phe Ser Met Glu Arg Leu Val Pro Asn Gln
       130                 135                 140
   Leu Thr Ser Ser Phe Asp Glu Gly Tyr Leu Arg Asn Leu Thr Glu Val
   145                 150                 155                 160
   Val Asn Phe Val Thr Asn Ala Gly Lys Tyr Ala Val Leu Asp Pro His
                   165                 170                 175
   Asn Tyr Gly Arg Tyr Tyr Gly Asn Val Ile Thr Asp Thr Asn Ala Phe
               180                 185                 190
   Arg Thr Phe Trp Thr Asn Leu Ala Lys Gln Phe Ala Ser Asn Ser Leu
           195                 200                 205
   Val Ile Phe Asp Thr Asn Asn Glu Tyr Asn Thr Met Asp Gln Thr Leu
       210                 215                 220
   Val Leu Asn Leu Asn Gln Ala Ala Ile Asp Gly Ile Arg Ala Ala Gly
   225                 230                 235                 240
   Ala Thr Ser Gln Tyr Ile Phe Val Glu Gly Asn Ala Trp Ser Gly Ala
                   245                 250                 255
   Trp Ser Trp Asn Thr Thr Asn Thr Asn Met Ala Ala Leu Thr Asp Pro
               260                 265                 270
   Gln Asn Lys Ile Val Tyr Glu Met His Gln Tyr Leu Asp Ser Asp Ser
   275 280 285
   Ser Gly Thr His Ala Glu Cys Val Ser Ser Asn Ile Gly Ala Gln Arg
   290 295 300
   Val Val Gly Ala Thr Gln Trp Leu Arg Ala Asn Gly Lys Leu Gly Val
   305 310 315 320
   Leu Gly Glu Phe Ala Gly Gly Ala Asn Ala Val Cys Gln Gln Ala Val
   325 330 335
   Thr Gly Leu Leu Asp His Leu Gln Asp Asn Ser Asp Val Trp Leu Gly
   340 345 350
   Ala Leu Trp Trp Ala Ala Gly Pro Trp Trp Gly Asp Tyr Met Tyr Ser
   355 360 365
   Phe Glu Pro Pro Ser Gly Thr Gly Tyr Val Asn Tyr Asn Ser Ile Leu
   370 375 380
   Lys Lys Tyr Leu Pro
   385
<210> 14
   <211> 373
   <212> PRT
   <213> Myceliophthora thermophila
<400> 14
   Gln Ser Gly Pro Trp Gln Gln Cys Gly Gly Ile Gly Trp Gln Gly Ser
   1 5 10 15
   Thr Asp Cys Val Ser Gly Tyr His Cys Val Tyr Gln Asn Asp Trp Tyr
   20 25 30
   Ser Gln Cys Val Pro Gly Ala Ala Ser Thr Thr Leu Gln Thr Ser Thr
   35 40 45
   Thr Ser Arg Pro Thr Ala Thr Ser Thr Ala Pro Pro Ser Ser Thr Thr
   50 55 60
   Ser Pro Ser Lys Gly Lys Leu Lys Trp Leu Gly Ser Asn Glu Ser Gly
   65 70 75 80
   Ala Glu Phe Gly Glu Gly Asn Tyr Pro Gly Leu Trp Gly Lys His Phe
   85 90 95
   Ile Phe Pro Ser Thr Ser Ala Ile Gln Thr Leu Ile Asn Asp Gly Tyr
               100                 105                 110
   Asn Ile Phe Arg Ile Asp Phe Ser Met Glu Arg Leu Val Pro Asn Gln
           115                 120                 125
   Leu Thr Ser Ser Phe Asp Glu Gly Tyr Leu Arg Asn Leu Thr Glu Val
       130                 135                 140
   Val Asn Phe Val Thr Asn Ala Gly Lys Tyr Ala Val Leu Asp Pro His
   145                 150                 155                 160
   Asn Tyr Gly Arg Tyr Tyr Gly Asn Val Ile Thr Asp Thr Asn Ala Phe
                   165                 170                 175
   Arg Thr Phe Trp Thr Asn Leu Ala Lys Gln Phe Ala Ser Asn Ser Leu
               180                 185                 190
   Val Ile Phe Asp Thr Asn Asn Glu Tyr Asn Thr Met Asp Gln Thr Leu
           195                 200                 205
   Val Leu Asn Leu Asn Gln Ala Ala Ile Asp Gly Ile Arg Ala Ala Gly
       210                 215                 220
   Ala Thr Ser Gln Tyr Ile Phe Val Glu Gly Asn Ala Trp Ser Gly Ala
   225                 230                 235                 240
   Trp Ser Trp Asn Thr Thr Asn Thr Asn Met Ala Ala Leu Thr Asp Pro
                   245                 250                 255
   Gln Asn Lys Ile Val Tyr Glu Met His Gln Tyr Leu Asp Ser Asp Ser
               260                 265                 270
   Ser Gly Thr His Ala Glu Cys Val Ser Ser Asn Ile Gly Ala Gln Arg
           275                 280                 285
   Val Val Gly Ala Thr Gln Trp Leu Arg Ala Asn Gly Lys Leu Gly Val
       290                 295                 300
   Leu Gly Glu Phe Ala Gly Gly Ala Asn Ala Val Cys Gln Gln Ala Val
   305                 310                 315                 320
   Thr Gly Leu Leu Asp His Leu Gln Asp Asn Ser Asp Val Trp Leu Gly
                   325                 330                 335
   Ala Leu Trp Trp Ala Ala Gly Pro Trp Trp Gly Asp Tyr Met Tyr Ser
               340                 345                 350
   Phe Glu Pro Pro Ser Gly Thr Gly Tyr Val Asn Tyr Asn Ser Ile Leu
           355                 360                 365
   Lys Lys Tyr Leu Pro
       370
<210> 15
   <211> 1170
   <212> DNA
   <213> Artificial
<220>
   <223> cDNA encoding MtEG II
<400> 15
   atgaagtcct ccatcctcgc cagcgtcttc gccacgggcg ccgtggctca aagtggtccg     60
   tggcagcaat gtggtggcat cggatggcaa ggatcgaccg actgtgtgtc gggttaccac    120
   tgcgtctacc agaacgattg gtacagccag tgcgtgcctg gcgcggcgtc gacaacgctc    180
   cagacatcta ccacgtccag gcccaccgcc accagcaccg cccctccgtc gtccaccacc    240
   tcgcctagca agggcaagct caagtggctc ggcagcaacg agtcgggcgc cgagttcggg    300
   gagggcaact accccggcct ctggggcaag cacttcatct tcccgtcgac ttcggcgatt    360
   cagacgctca tcaatgatgg atacaacatc ttccggatcg acttctcgat ggagcgtctg    420
   gtgcccaacc agttgacgtc gtccttcgac gagggctacc tccgcaacct gaccgaggtg    480
   gtcaacttcg tgacgaacgc gggcaagtac gccgtcctgg acccgcacaa ctacggccgg    540
   tactacggca acgtcatcac ggacacgaac gcgttccgga ccttctggac caacctggcc    600
   aagcagttcg cctccaactc gctcgtcatc ttcgacacca acaacgagta caacacgatg    660
   gaccagaccc tggtgctcaa cctcaaccag gccgccatcg acggcatccg ggccgccggc    720
   gcgacctcgc agtacatctt cgtcgagggc aacgcgtgga gcggggcctg gagctggaac    780
   acgaccaaca ccaacatggc cgccctgacg gacccgcaga acaagatcgt gtacgagatg    840
   caccagtacc tcgactcgga cagctcgggc acccacgccg agtgcgtcag cagcaacatc    900
   ggcgcccagc gcgtcgtcgg agccacccag tggctccgcg ccaacggcaa gctcggcgtc    960
   ctcggcgagt tcgccggcgg cgccaacgcc gtctgccagc aggccgtcac cggcctcctc   1020
   gaccacctcc aggacaacag cgacgtctgg ctgggtgccc tctggtgggc cgccggtccc   1080
   tggtggggcg actacatgta ctcgttcgag cctccttcgg gcaccggcta tgtcaactac   1140
   aactcgatcc taaagaagta cttgccgtaa                                    1170
<210> 16
   <211> 1616
   <212> DNA
   <213> Myceliophthora thermophila
<400> 16
   atgaagtcct ccatcctcgc cagcgtcttc gccacgggcg ccgtggctca aagtggtccg      60
   tggcagcaat gtggtggcat cggatggcaa ggatcgaccg actgtgtgtc gggttaccac     120
   tgcgtctacc agaacgattg gtacagccag tgcgtgcctg gcgcggcgtc gacaacgctc     180
   cagacatcta ccacgtccag gcccaccgcc accagcaccg cccctccgtc gtccaccacc     240
   tcgcctagca agggcaagct caagtggctc ggcagcaacg agtcgggcgc cgagttcggg     300
   gagggcaact accccggcct ctggggcaag cacttcatct tcccgtcgac ttcggcgatt     360
   caggtacggc caataataat atattattat agcaggcagg agggagcagg agaagaaggg     420
   aggggcaggt ggccaacaat cggaagaaga ccgggaggca ctgaccgttg attcctttgt     480
   gtaatagacg ctcatcaatg atggatacaa catcttccgg atcgacttct cgatggagcg     540
   tctggtgccc aaccagttga cgtcgtcctt cgacgagggc tacctccgca acctgaccga     600
   ggtggtcaac ttcgtgacga acgcgggcaa gtacgccgtc ctggacccgc acaactacgg     660
   ccggtactac ggcaacgtca tcacggacac gaacgcgttc cggaccttct ggaccaacct     720
   ggccaagcag ttcgcctcca actcgctcgt catcttcgac accaacaacg agtacaacac     780
   gatggaccag accctggtgc tcaacctcaa ccaggccgcc atcgacggca tccgggccgc     840
   cggcgcgacc tcgcagtaca tcttcgtcga gggcaacgcg tggagcgggg cctggagctg     900
   gaacacgacc aacaccaaca tggccgccct gacggacccg cagaacaaga tcgtgtacga     960
   gatgcaccag tacctcgact cggacagctc gggcacccac gccgagtgcg tcagcagcaa    1020
   catcggcgcc cagcgcgtcg tcggagccac ccagtggctc cgcgccaacg gcaagctcgg    1080
   cgtcctcggc gagttcgccg gcggcgccaa cgccgtctgc cagcaggccg tcaccggcct    1140
   cctcgaccac ctccaggaca acagcgacgt ctggctgggt gccctctggt gggccgccgg    1200
   tccctggtgg ggcgactaca tgtactcgtt cggtaagttt ctcccttgtt cttggctttc    1260
   cccccagtaa gggagtcagg caacatgccc aagaccggct cggcttcgct tcaaggcgtt    1320
   cgttgtacac actgaagagt tccaacttcc aaccctgttc gtgtcctccg atcagcttcg    1380
   acggggtgaa gggggaaggg atttgggagt gaggtggagg tcaaaaggag ggatatcccc    1440
   agatctccac aaacggccct gagccaacaa cagcctctgg ggtcaaaatg ggcgccaacc    1500
   atacggtcat tcactcagga cacctgctaa cgcgtctctt ttttttgttt ccagagcctc    1560
   cttcgggcac cggctatgtc aactacaact cgatcctaaa gaagtacttg ccgtaa        1616
<210> 17
   <211> 456
   <212> PRT
   <213> Myceliophthora thermophila
<400> 17
   Met Gly Arg Gly Ala Ala Phe Leu Gly Leu Ala Ser Leu Leu Val Gly
   1               5                   10                  15
   Ala Ala Lys Ala Gln Thr Pro Gly Glu Gly Glu Glu Val His Pro Gln
               20                  25                  30
   Ile Thr Thr Tyr Arg Cys Thr Lys Ala Asp Gly Cys Glu Glu Lys Thr
           35                  40                  45
   Asn Tyr Ile Val Leu Asp Ala Leu Ser His Pro Val His Gln Val Asp
       50                  55                  60
   Asn Pro Tyr Asn Cys Gly Asp Trp Gly Gln Lys Pro Asn Glu Thr Ala
   65                  70                  75                  80
   Cys Pro Asp Leu Glu Ser Cys Ala Arg Asn Cys Ile Met Asp Pro Val
                   85                  90                  95
   Ser Asp Tyr Gly Arg His Gly Val Ser Thr Asp Gly Thr Ser Leu Arg
               100                 105                 110
   Leu Lys Gln Leu Val Gly Gly Asn Val Val Ser Pro Arg Val Tyr Leu
           115                 120                 125
   Leu Asp Glu Thr Lys Glu Arg Tyr Glu Met Leu Lys Leu Thr Gly Asn
       130                 135                 140
   Glu Phe Thr Phe Asp Val Asp Ala Thr Lys Leu Pro Cys Gly Met Asn
   145                 150                 155                 160
   Ser Ala Leu Tyr Leu Ser Glu Met Asp Ala Thr Gly Ala Arg Ser Glu
                   165                 170                 175
   Leu Asn Pro Gly Gly Ala Thr Phe Gly Thr Gly Tyr Cys Asp Ala Gln
               180                 185                 190
   Cys Tyr Val Thr Pro Phe Ile Asn Gly Leu Gly Asn Ile Glu Gly Lys
           195                 200                 205
   Gly Ala Cys Cys Asn Glu Met Asp Ile Trp Glu Ala Asn Ala Arg Ala
       210                 215                 220
   Gln His Ile Ala Pro His Pro Cys Ser Lys Ala Gly Pro Tyr Leu Cys
   225                 230                 235                 240
   Glu Gly Ala Glu Cys Glu Phe Asp Gly Val Cys Asp Lys Asn Gly Cys
   245 250 255
   Ala Trp Asn Pro Tyr Arg Val Asn Val Thr Asp Tyr Tyr Gly Glu Gly
   260 265 270
   Ala Glu Phe Arg Val Asp Thr Thr Arg Pro Phe Ser Val Val Thr Gln
   275 280 285
   Phe Arg Ala Gly Gly Asp Ala Gly Gly Gly Lys Leu Glu Ser Ile Tyr
   290 295 300
   Arg Leu Phe Val Gln Asp Gly Arg Val Ile Glu Ser Tyr Val Val Asp
   305 310 315 320
   Lys Pro Gly Leu Pro Pro Thr Asp Arg Met Thr Asp Glu Phe Cys Ala
   325 330 335
   Ala Thr Gly Ala Ala Arg Phe Thr Glu Leu Gly Ala Met Glu Ala Met
   340 345 350
   Gly Asp Ala Leu Thr Arg Gly Met Val Leu Ala Leu Ser Ile Trp Trp
   355 360 365
   Ser Glu Gly Asp Asn Met Asn Trp Leu Asp Ser Gly Glu Ala Gly Pro
   370 375 380
   Cys Asp Pro Asp Glu Gly Asn Pro Ser Asn Ile Ile Arg Val Gln Pro
   385 390 395 400
   Asp Pro Glu Val Val Phe Ser Asn Leu Arg Trp Gly Glu Ile Gly Ser
   405 410 415
   Thr Tyr Glu Ser Ala Val Asp Gly Pro Val Gly Lys Gly Lys Gly Lys
   420 425 430
   Gly Lys Gly Lys Ala Pro Ala Gly Asp Gly Asn Gly Lys Glu Lys Ser
   435 440 445
   Asn Gly Lys Arg Phe Arg Arg Phe
   450 455
<210> 18
   <211> 436
   <212> PRT
   <213> Myceliophthora thermophila
<400> 18
   Gln Thr Pro Gly Glu Gly Glu Glu Val His Pro Gln Ile Thr Thr Tyr
   1               5                   10                  15
   Arg Cys Thr Lys Ala Asp Gly Cys Glu Glu Lys Thr Asn Tyr Ile Val
               20                  25                  30
   Leu Asp Ala Leu Ser His Pro Val His Gln Val Asp Asn Pro Tyr Asn
           35                  40                  45
   Cys Gly Asp Trp Gly Gln Lys Pro Asn Glu Thr Ala Cys Pro Asp Leu
       50                  55                  60
   Glu Ser Cys Ala Arg Asn Cys Ile Met Asp Pro Val Ser Asp Tyr Gly
   65                  70                  75                  80
   Arg His Gly Val Ser Thr Asp Gly Thr Ser Leu Arg Leu Lys Gln Leu
                   85                  90                  95
   Val Gly Gly Asn Val Val Ser Pro Arg Val Tyr Leu Leu Asp Glu Thr
               100                 105                 110
   Lys Glu Arg Tyr Glu Met Leu Lys Leu Thr Gly Asn Glu Phe Thr Phe
           115                 120                 125
   Asp Val Asp Ala Thr Lys Leu Pro Cys Gly Met Asn Ser Ala Leu Tyr
       130                 135                 140
   Leu Ser Glu Met Asp Ala Thr Gly Ala Arg Ser Glu Leu Asn Pro Gly
   145                 150                 155                 160
   Gly Ala Thr Phe Gly Thr Gly Tyr Cys Asp Ala Gln Cys Tyr Val Thr
                   165                 170                 175
   Pro Phe Ile Asn Gly Leu Gly Asn Ile Glu Gly Lys Gly Ala Cys Cys
               180                 185                 190
   Asn Glu Met Asp Ile Trp Glu Ala Asn Ala Arg Ala Gln His Ile Ala
           195                 200                 205
   Pro His Pro Cys Ser Lys Ala Gly Pro Tyr Leu Cys Glu Gly Ala Glu
       210                 215                 220
   Cys Glu Phe Asp Gly Val Cys Asp Lys Asn Gly Cys Ala Trp Asn Pro
   225                 230                 235                 240
   Tyr Arg Val Asn Val Thr Asp Tyr Tyr Gly Glu Gly Ala Glu Phe Arg
                   245                 250                 255
   Val Asp Thr Thr Arg Pro Phe Ser Val Val Thr Gln Phe Arg Ala Gly
               260                 265                 270
   Gly Asp Ala Gly Gly Gly Lys Leu Glu Ser Ile Tyr Arg Leu Phe Val
           275                 280                 285
   Gln Asp Gly Arg Val Ile Glu Ser Tyr Val Val Asp Lys Pro Gly Leu
       290                 295                 300
   Pro Pro Thr Asp Arg Met Thr Asp Glu Phe Cys Ala Ala Thr Gly Ala
   305                 310                 315                 320
   Ala Arg Phe Thr Glu Leu Gly Ala Met Glu Ala Met Gly Asp Ala Leu
                   325                 330                 335
   Thr Arg Gly Met Val Leu Ala Leu Ser Ile Trp Trp Ser Glu Gly Asp
               340                 345                 350
   Asn Met Asn Trp Leu Asp Ser Gly Glu Ala Gly Pro Cys Asp Pro Asp
           355                 360                 365
   Glu Gly Asn Pro Ser Asn Ile Ile Arg Val Gln Pro Asp Pro Glu Val
       370                 375                 380
   Val Phe Ser Asn Leu Arg Trp Gly Glu Ile Gly Ser Thr Tyr Glu Ser
   385                 390                 395                 400
   Ala Val Asp Gly Pro Val Gly Lys Gly Lys Gly Lys Gly Lys Gly Lys
                   405                 410                 415
   Ala Pro Ala Gly Asp Gly Asn Gly Lys Glu Lys Ser Asn Gly Lys Arg
               420                 425                 430
   Phe Arg Arg Phe
           435
<210> 19
   <211> 1368
   <212> DNA
   <213> Artificial
<220>
   <223> cDNA encoding MtEG I
<400> 19
   atgggtcgcg gcgctgcttt cctaggcctc gcctcgctcc tcgtgggcgc ggccaaggcc      60
   cagacgcccg gcgagggcga ggaggtgcac ccgcagatca cgacgtaccg ctgcaccaag     120
   gcggacgggt gcgaggagaa gaccaactac atcgtgctgg acgccctatc gcacccggtc     180
   caccaggtcg acaacccgta caactgcggc gactggggcc agaagcccaa cgagacggcc     240
   tgcccggacc tcgagtcgtg cgccaggaac tgcatcatgg acccggtctc ggactacggc     300
   cggcacgggg tctcgaccga cggcacctcg ctgcgcctca agcagctagt cggcggcaac     360
   gtcgtcagcc cgcgcgtcta cctgctcgac gagaccaagg agcgctacga gatgctcaag     420
   ctgaccggca acgagttcac ctttgacgtc gacgccacca agctgccctg cggcatgaac     480
   agcgccctct acctctccga gatggacgcc accggcgccc ggagcgagct caacccgggc     540
   ggcgccacct ttggcaccgg ctactgcgac gcccagtgct acgtcacccc cttcatcaac     600
   ggcctcggca acatcgaggg caagggcgcg tgctgcaacg agatggatat ctgggaggcc     660
   aacgcgcggg cgcagcacat cgcgccgcac ccgtgcagca aggcggggcc gtacctgtgc     720
   gagggcgccg agtgcgagtt cgacggcgtg tgcgacaaga acggctgcgc ctggaacccg     780
   taccgggtca acgtgacgga ctactacggc gagggcgccg agttcagggt ggacacgacc     840
   cggcccttct cggtcgtcac gcagttccgc gccggcggcg acgcgggggg cggcaagctc     900
   gagagcatct accggctctt cgtccaggac ggcagggtga tcgagtcgta cgtcgtcgac     960
   aagcccggcc tgcccccgac ggaccgcatg acggacgagt tctgcgccgc caccggcgcc    1020
   gcccgcttca cggagctcgg cgccatggag gccatgggcg acgccctgac gcgcggcatg    1080
   gtcctcgccc tcagcatctg gtggagcgag ggcgacaaca tgaactggct cgactcgggc    1140
   gaggccggcc cctgcgaccc ggacgagggc aacccgtcca acatcatccg cgtccagccc    1200
   gacccggagg tcgtcttcag caacctgcgc tggggcgaga tcggctcaac ctacgagtcc    1260
   gccgtcgacg ggcccgtcgg caagggcaag ggcaagggca agggcaaggc tcccgccggc    1320
   gacggcaacg ggaaggagaa gagcaatggc aagcgcttca ggaggttc                 1368
<210> 20
   <211> 2507
   <212> DNA
   <213> Myceliophthora thermophila
<400> 20
   ctatataaag gtacttacag ataaaatccc caggttgaca gccctttcga gcataagaat     60
   ccaggtccta tagtggccgg ctcgaagccc agatttgagc cctattgaga ataactatat    120
   ggcactggat gaaggattgg cttgaacatc gctacgatgt ttaatcgctg agtcttacag    180
   ggcttcgact ggctgttctg gaggcgtggg aggcagtccc ccccgagttt cttcagtgcc    240
   tggcacactc aatgcctcga cggctagcaa aaaccagggg ggagagactg ggtattgata    300
   cttctgtatt atagaaattt gtattgggtt ggttgtacct agacttcttg gctacttttc    360
   tgatttgcgt tagttgtggg gcatgtatcg cactaggatt aataatgctg gaagaggtgt    420
   agagatgttg cgtcgcatgg ccaccgagat ccggcctggc gtcgtgcctt gcagctcgca    480
   cgatgcgcgc gacgatgcgg gacacccctc ccccctcccc cctcccccct acattaggag 540
   gggatccggg gccgctgccc gttttccgtc ttggtattcc cggctgacac aacggttctc 600
   cagagtgata accgaggcac accagtagca gtccacgatg gcacggcagg ggatacattc 660
   cgcccggaac aacccaagat ctgggaatcc cagtgacgac cccggggtcc tccggggtcc 720
   cccgatttcg tctgaaccga cgaactggaa ggaggcgagc cttggaacga tgggggatat 780
   gatatatatt aagatgcaac attctctccc tccctcctct ctctctccct ccccccctct 840
   ctcctcctct tcccctcctc cccgccgttc tctctccatg agcctcaatt cttgctccga 900
   gcgcggtgta tttcccccac gaggaattga caaaagaaaa agaaaaagac aagactctcg 960
   agaacgatgg gtcgcggcgc tgctttccta ggcctcgcct cgctcctcgt gggcgcggcc 1020
   aaggcccaga cgcccggcga gggcgaggag gtgcacccgc agatcacgac gtaccgctgc 1080
   accaaggcgg acgggtgcga ggagaagacc aactacatcg tgctggacgc cctatcgcac 1140
   ccggtccacc aggtcgacaa cccgtacaac tgcggcgact ggggccagaa gcccaacgag 1200
   acggcctgcc cggacctcga gtcgtgcgcc aggaactgca tcatggaccc ggtctcggac 1260
   tacggccggc acggggtctc gaccgacggc acctcgctgc gcctcaagca gctagtcggc 1320
   ggcaacgtcg tcagcccgcg cgtctacctg ctcgacgaga ccaaggagcg ctacgagatg 1380
   ctcaagctga ccggcaacga gttcaccttt gacgtcgacg ccaccaagct gccctgcggc 1440
   atgaacagcg ccctctacct ctccgagatg gacgccaccg gcgcccggag cgagctcaac 1500
   ccgggcggcg ccacctttgg caccggctac tgcgacgccc agtgctacgt cacccccttc 1560
   atcaacggcc tcgtacgtat tctcctatta atcttgtttc ttttttcctt ttcttttttt 1620
   gatataatag aattaagaag aactcggtgg gctgacatga cacagggcaa catcgagggc 1680
   aagggcgcgt gctgcaacga gatggatatc tgggaggcca acgcgcgggc gcagcacatc 1740
   gcgccgcacc cgtgcagcaa ggcggggccg tacctgtgcg agggcgccga gtgcgagttc 1800
   gacggcgtgt gcgacaagaa cggctgcgcc tggaacccgt accgggtcaa cgtgacggac 1860
   tactacggcg agggcgccga gttcagggtg gacacgaccc ggcccttctc ggtcgtcacg 1920
   cagttccgcg ccggcggcga cgcggggggc ggcaagctcg agagcatcta ccggctcttc 1980
   gtccaggacg gcagggtgat cgagtcgtac gtcgtcgaca agcccggcct gcccccgacg 2040
   gaccgcatga cggacgagtt ctgcgccgcc accggcgccg cccgcttcac ggagctcggc 2100
   gccatggagg ccatgggcga cgccctgacg cgcggcatgg tcctcgccct cagcatctgg 2160
   tggagcgagg gcgacaacat gaactggctc gactcgggcg aggccggccc ctgcgacccg 2220
   gacgagggca acccgtccaa catcatccgc gtccagcccg acccggaggt cgtcttcagc 2280
   aacctgcgct ggggcgagat cggctcaacc tacgagtccg ccgtcgacgg gcccgtcggc 2340
   aagggcaagg gcaagggcaa gggcaaggct cccgccggcg acggcaacgg gaaggagaag 2400
   agcaatggca agcgcttcag gaggttctga gcaaccttga tattattttt ttctttcttt 2460
   ccttcaccag ttaattagtt gcctttgatt agaaagagag agagaaa 2507
<210> 21
   <211> 247
   <212> PRT
   <213> Myceliophthora thermophila
<400> 21
   Met Gln Pro Phe Leu Leu Leu Phe Leu Ser Ser Val Thr Ala Ala Ser
   1               5                   10                  15
   Pro Leu Thr Ala Leu Asp Lys Arg Gln Gln Ala Thr Leu Cys Glu Gln
               20                  25                  30
   Tyr Gly Tyr Trp Ser Gly Asn Gly Tyr Glu Val Asn Asn Asn Asn Trp
           35                  40                  45
   Gly Lys Asp Ser Ala Ser Gly Gly His Gln Cys Thr Tyr Val Asp Ser
       50                  55                  60
   Ser Ser Ser Ser Gly Val Ala Trp His Thr Thr Trp Gln Trp Glu Gly
   65                  70                  75                  80
   Gly Gln Asn Gln Val Lys Ser Phe Ala Asn Cys Gly Leu Gln Val Pro
                   85                  90                  95
   Lys Gly Arg Thr Ile Ser Ser Ile Ser Asn Leu Gln Thr Ser Ile Ser
               100                 105                 110
   Trp Ser Tyr Ser Asn Thr Asn Ile Arg Ala Asn Val Ala Tyr Asp Leu
           115                 120                 125
   Phe Thr Ala Ala Asp Pro Asn His Ala Thr Ser Ser Gly Asp Tyr Glu
       130                 135                 140
   Leu Met Ile Trp Leu Ala Arg Phe Gly Asp Val Tyr Pro Ile Gly Ser
   145                 150                 155                 160
   Ser Gln Gly His Val Asn Val Ala Gly Gln Asp Trp Glu Leu Trp Thr
                   165                 170                 175
   Gly Phe Asn Gly Asn Met Arg Val Tyr Ser Phe Val Ala Pro Ser Pro
               180                 185                 190
   Arg Asn Ser Phe Ser Ala Asn Val Lys Asp Phe Phe Asn Tyr Leu Gln
           195                 200                 205
   Ser Asn Gln Gly Phe Pro Ala Ser Ser Gln Tyr Leu Leu Ile Phe Gln
       210                 215                 220
   Ala Gly Thr Glu Pro Phe Thr Gly Gly Glu Thr Thr Leu Thr Val Asn
   225                 230                 235                 240
   Asn Tyr Ser Ala Arg Val Ala
                   245
<210> 22
   <211> 232
   <212> PRT
   <213> Myceliophthora thermophila
<400> 22
   Ser Pro Leu Thr Ala Leu Asp Lys Arg Gln Gln Ala Thr Leu Cys Glu
   1               5                   10                  15
   Gln Tyr Gly Tyr Trp Ser Gly Asn Gly Tyr Glu Val Asn Asn Asn Asn
               20                  25                  30
   Trp Gly Lys Asp Ser Ala Ser Gly Gly His Gln Cys Thr Tyr Val Asp
           35                  40                  45
   Ser Ser Ser Ser Ser Gly Val Ala Trp His Thr Thr Trp Gln Trp Glu
       50                  55                  60
   Gly Gly Gln Asn Gln Val Lys Ser Phe Ala Asn Cys Gly Leu Gln Val
   65                  70                  75                  80
   Pro Lys Gly Arg Thr Ile Ser Ser Ile Ser Asn Leu Gln Thr Ser Ile
                   85                  90                  95
   Ser Trp Ser Tyr Ser Asn Thr Asn Ile Arg Ala Asn Val Ala Tyr Asp
               100                 105                 110
   Leu Phe Thr Ala Ala Asp Pro Asn His Ala Thr Ser Ser Gly Asp Tyr
           115                 120                 125
   Glu Leu Met Ile Trp Leu Ala Arg Phe Gly Asp Val Tyr Pro Ile Gly
       130                 135                 140
   Ser Ser Gln Gly His Val Asn Val Ala Gly Gln Asp Trp Glu Leu Trp
   145                 150                 155                 160
   Thr Gly Phe Asn Gly Asn Met Arg Val Tyr Ser Phe Val Ala Pro Ser
                   165                 170                 175
   Pro Arg Asn Ser Phe Ser Ala Asn Val Lys Asp Phe Phe Asn Tyr Leu
               180                 185                 190
   Gln Ser Asn Gln Gly Phe Pro Ala Ser Ser Gln Tyr Leu Leu Ile Phe
           195                 200                 205
   Gln Ala Gly Thr Glu Pro Phe Thr Gly Gly Glu Thr Thr Leu Thr Val
       210                 215                 220
   Asn Asn Tyr Ser Ala Arg Val Ala
   225                 230
<210> 23
   <211> 741
   <212> DNA
   <213> Artificial
<220>
   <223> cDNA encoding MtEG III
<400> 23
   atgcagccgt ttctgctctt gttcctctcg tcggtcacgg cggcgagccc cctgacggcg     60
   ctcgacaagc ggcagcaggc gacgttgtgc gagcagtacg gctactggtc gggcaacggt    120
   tacgaggtca acaacaacaa ctggggcaag gattcggcct cgggcggcca tcagtgcacc    180
   tacgtcgaca gcagcagctc cagcggcgtc gcctggcaca cgacctggca gtgggaagga    240
   ggccagaacc aggtcaagag cttcgccaac tgcggcctgc aggtgcccaa gggcaggacc    300
   atctcgtcca tcagcaacct gcagacctcc atctcgtggt cctacagcaa caccaacatc    360
   cgcgccaacg tggcctacga cctcttcacc gcggcagacc cgaaccacgc gaccagcagc    420
   ggcgactacg agctcatgat ctggctggcg agattcggcg acgtctaccc catcggctcg    480
   tcccagggcc acgtcaacgt ggccggccag gactgggagc tgtggacggg cttcaacggc    540
   aacatgcggg tctacagctt cgtagcgccc agcccccgca acagcttcag cgccaacgtc    600
   aaggacttct tcaactatct ccagtccaac cagggcttcc cggccagcag ccaatacctt    660
   ctcatcttcc aggcgggcac cgagcccttc accggcggcg agaccaccct taccgtcaac    720
   aactactctg caagggttgc t                                              741
<210> 24
   <211> 1305
   <212> DNA
   <213> Myceliophthora thermophila
<400> 24
   cgacaaagac ccgtcagtga ttaataataa ttagtagcag tttctttctt tcaagactca      60
   agaatactcc tttccgccat cgtggcagcg tttagattca tcatgcagcc gtttctgctc     120
   ttgttcctct cgtcggtcac ggcggcgagc cccctgacgg cgctcgacaa gcggcagcag     180
   gcgacgttgt gcgagcagta cggctactgg tcgggcaacg gttacgaggt caacaacaac     240
   aactggggca aggattcggc ctcgggcggc catcagtgca cctacgtcga cagcagcagc     300
   tccagcggcg tcgcctggca cacgacctgg cagtgggaag gaggccagaa ccaggtcaag     360
   agcttcgcca actgcggcct gcaggtgccc aagggcagga ccatctcgtc catcagcaac     420
   ctgcagacct ccatctcgtg gtcctacagc aacaccaaca tccgcgccaa cgtggcctac     480
   gacctcttca ccgcggcaga cccgaaccac gcgaccagca gcggcgacta cgagctcatg     540
   atctggtcag tttttttttt cttttttctt ttcttctctt ttcttttctt ttcctttctc     600
   ctgttttatt ttcttatcca ttgcttcgcc ctctttcctt aaccctgctg actctctctt     660
   cttgtcaatg atactgtaat aggctggcga gattcggcga cgtctacccc atcggctcgt     720
   cccagggcca cgtcaacgtg gccggccagg actgggagct gtggacgggc ttcaacggca     780
   acatgcgggt ctacagcttc gtagcgccca gcccccgcaa cagcttcagc gccaacgtca     840
   aggacttctt caactatctc cagtccaacc agggcttccc ggccagcagc caataccttc     900
   tcagtaagga gacgagatct cgaacagcat accatatatg cgtgcggtac aagtgcacta     960
   accccctttt ttttcccgtt cgcagtcttc caggcgggca ccgagccctt caccggcggc    1020
   gagaccaccc ttaccgtcaa caactactct gcaagggttg cttaaacagg aaggccgagg    1080
   atggccccca aggccgttgc gggttcacga gctctcttct tttcaagtgc tgtacataca    1140
   taattagcgt accaagtcat agctgtttgt cagcttcaaa ctaagtgctc gcccacaaaa    1200
   gaggggggag gggaaaataa caaattgccg aacgcagtga taagcttctg ggagcgttga    1260
   aagcagtcta cagtaggtgg ctgtacgaag gaaaagagtg cctta                    1305
<210> 25
   <211> 225
   <212> PRT
   <213> Myceliophthora thermophila
<400> 25
   Met His Leu Ser Ala Thr Thr Gly Phe Leu Ala Leu Pro Ala Leu Ala
   1               5                   10                  15
   Leu Ala Gln Leu Ser Gly Ser Gly Gln Thr Thr Arg Tyr Trp Asp Cys
               20                  25                  30
   Cys Lys Pro Ser Cys Ala Trp Pro Gly Lys Gly Pro Ser Ser Pro Val
           35                  40                  45
   Gln Ala Cys Asp Lys Asn Asp Asn Pro Leu Asn Asp Gly Gly Ser Thr
       50                  55                  60
   Arg Ser Gly Cys Asp Ala Gly Gly Ser Ala Tyr Met Cys Ser Ser Gln
   65                  70                  75                  80
   Ser Pro Trp Ala Val Ser Asp Glu Leu Ser Tyr Gly Trp Ala Ala Val
                   85                  90                  95
   Lys Leu Ala Gly Ser Ser Glu Ser Gln Trp Cys Cys Ala Cys Tyr Glu
               100                 105                 110
   Leu Thr Phe Thr Ser Gly Pro Val Ala Gly Lys Lys Met Ile Val Gln
           115                 120                 125
   Ala Thr Asn Thr Gly Gly Asp Leu Gly Asp Asn His Phe Asp Leu Ala
       130                 135                 140
   Ile Pro Gly Gly Gly Val Gly Ile Phe Asn Ala Cys Thr Asp Gln Tyr
   145                 150                 155                 160
   Gly Ala Pro Pro Asn Gly Trp Gly Asp Arg Tyr Gly Gly Ile His Ser
                   165                 170                 175
   Lys Glu Glu Cys Glu Ser Phe Pro Glu Ala Leu Lys Pro Gly Cys Asn
               180                 185                 190
   Trp Arg Phe Asp Trp Phe Gln Asn Ala Asp Asn Pro Ser Val Thr Phe
           195                 200                 205
   Gln Glu Val Ala Cys Pro Ser Glu Leu Thr Ser Lys Ser Gly Cys Ser
       210                 215                 220
   Arg
   225
<210> 26
   <211> 207
   <212> PRT
   <213> Myceliophthora thermophila
<400> 26
   Gln Leu Ser Gly Ser Gly Gln Thr Thr Arg Tyr Trp Asp Cys Cys Lys
   1               5                   10                  15
   Pro Ser Cys Ala Trp Pro Gly Lys Gly Pro Ser Ser Pro Val Gln Ala
               20                  25                  30
   Cys Asp Lys Asn Asp Asn Pro Leu Asn Asp Gly Gly Ser Thr Arg Ser
           35                  40                  45
   Gly Cys Asp Ala Gly Gly Ser Ala Tyr Met Cys Ser Ser Gln Ser Pro
       50                  55                  60
   Trp Ala Val Ser Asp Glu Leu Ser Tyr Gly Trp Ala Ala Val Lys Leu
   65                  70                  75                  80
   Ala Gly Ser Ser Glu Ser Gln Trp Cys Cys Ala Cys Tyr Glu Leu Thr
                   85                  90                  95
   Phe Thr Ser Gly Pro Val Ala Gly Lys Lys Met Ile Val Gln Ala Thr
               100                 105                 110
   Asn Thr Gly Gly Asp Leu Gly Asp Asn His Phe Asp Leu Ala Ile Pro
           115                 120                 125
   Gly Gly Gly Val Gly Ile Phe Asn Ala Cys Thr Asp Gln Tyr Gly Ala
       130                 135                 140
   Pro Pro Asn Gly Trp Gly Asp Arg Tyr Gly Gly Ile His Ser Lys Glu
   145                 150                 155                 160
   Glu Cys Glu Ser Phe Pro Glu Ala Leu Lys Pro Gly Cys Asn Trp Arg
                   165                 170                 175
   Phe Asp Trp Phe Gln Asn Ala Asp Asn Pro Ser Val Thr Phe Gln Glu
               180                 185                 190
   Val Ala Cys Pro Ser Glu Leu Thr Ser Lys Ser Gly Cys Ser Arg
           195                 200                 205
<210> 27
   <211> 675
   <212> DNA
   <213> Artificial
<220>
   <223> cDNA encoding MtEG V
<400> 27
   atgcatctct ccgccaccac cgggttcctc gccctcccgg ccctggccct ggcccagctc      60
   tcgggcagcg gccagacgac ccggtactgg gactgctgca agccgagctg cgcctggccc     120
   ggcaagggcc cctcgtctcc ggtgcaggcc tgcgacaaga acgacaaccc gctcaacgac     180
   ggcggctcca cccggtccgg ctgcgacgcg ggcggcagcg cctacatgtg ctcctcccag     240
   agcccctggg ccgtcagcga cgagctgtcg tacggctggg cggccgtcaa gctcgccggc     300
   agctccgagt cgcagtggtg ctgcgcctgc tacgagctga ccttcaccag cgggccggtc     360
   gcgggcaaga agatgattgt gcaggcgacc aacaccggtg gcgacctggg cgacaaccac     420
   tttgacctgg ccatccccgg tggcggtgtc ggtattttca acgcctgcac cgaccagtac     480
   ggcgctcccc cgaacggctg gggcgaccgc tacggcggca tccattccaa ggaagagtgc     540
   gaatccttcc cggaggccct caagcccggc tgcaactggc gcttcgactg gttccaaaac     600
   gccgacaacc cgtcggtcac cttccaggag gtggcctgcc cgtcggagct cacgtccaag     660
   agcggctgct cccgt                                                      675
<210> 28
   <211> 1448
   <212> DNA
   <213> Myceliophthora thermophila
<400> 28
   atatataagt gagagtgttt tttgactgcc ccgggttctg gtagttgaag ggaagttcga 60
   tgctctctgc tgtcgtcgct ctcgtcgctc tcgtcggcat cctccatccg tccgcctttg 120
   ataacccgct ccccgactca gtcaagacga cgcatacttg gcaccatgca tctctccgcc 180
   accaccgggt tcctcgccct cccggccctg gccctggccc agctctcggg cagcggccag 240
   acgacccggt actgggactg ctgcaagccg agctgcgcct ggcccggcaa gggcccctcg 300
   tctccggtgc aggcctgcga caagaacgac aacccgctca acgacggcgg ctccacccgg 360
   tccggctgcg acgcgggcgg cagcgcctac atgtgctcct cccagagccc ctgggccgtc 420
   agcgacgagc tgtcgtacgg ctgggcggcc gtcaagctcg ccggcagctc cgagtcgcag 480
   tggtgctgcg cctgctacga gctgaccttc accagcgggc cggtcgcggg caagaagatg 540
   attgtgcagg cgaccaacac cggtggcgac ctgggcgaca accactttga cctggccgtg 600
   agttgcctcc ccttctcccc ggaccgctca gattagatga gattagactt tgctcgtaaa 660
   tcggtccaag attcccgttg actgaccaac aaacatcata cgggcagatc cccggtggcg 720
   gtgtcggtat tttcaacggt aagctggtgc ccccggaccc ctccccggac ccctccccct 780
   tttcctccag cgagccgagt tgggatcgcc gagatcgaga actcacacaa cttctctctc 840
   gacagcctgc accgaccagt acggcgctcc cccgaacggc tggggcgacc gctacggcgg 900
   catccattcc aaggaagagt gcgaatcctt cccggaggcc ctcaagcccg gctgcaactg 960
   gcgcttcgac tggtacgttg ctttgacata ccggaaccca attcctccaa ccccccccct 1020
   tttctccccc aactccgggg gtagtcggaa tgtcgcgact gaccctattt caggttccaa 1080
   aacgccgaca acccgtcggt caccttccag gaggtggcct gcccgtcgga gctcacgtcc 1140
   aagagcggct gctcccgtta agagggaaga gagggggctg gaaggaccga aagattcaac 1200
   ctctgctcct gctggggaag ctcgggcgcg agtgtgaaac tggtgtaaat attgtggcac 1260
   acacaagcta ctacagtccg tctcgccgtc cggctaacta gccttgctgc ggatctgtcc 1320
   atcttcggtc cgaactgtcc gttgctgttt tggctcggtg cctcatcttc tcccaaccta 1380
   gtcaagaatg aatcgtgaga gaggctgaga gagataagat cgacttcaga aatccagggt 1440
   tgaaagca 1448
<210> 29
   <211> 381
   <212> PRT
   <213> Myceliophthora thermophila
<400> 29
   Met Arg Val Ser Ser Leu Val Ala Ala Leu Ala Thr Gly Gly Leu Val
   1               5                   10                  15
   Ala Ala Thr Pro Lys Pro Lys Gly Ser Ser Pro Pro Gly Ala Val Asp
               20                  25                  30
   Ala Asn Pro Phe Lys Gly Lys Thr Gln Phe Val Asn Pro Ala Trp Ala
           35                  40                  45
   Ala Lys Leu Glu Gln Thr Lys Lys Ala Phe Leu Ala Arg Asn Asp Thr
       50                  55                  60
   Val Asn Ala Ala Lys Thr Glu Lys Val Gln Gln Thr Ser Ser Phe Val
   65                  70                  75                  80
   Trp Val Ser Arg Ile Ala Glu Leu Ser Asn Ile Asp Asp Ala Ile Ala
                   85                  90                  95
   Ala Ala Arg Lys Ala Gln Lys Lys Thr Gly Arg Arg Gln Ile Val Gly
               100                 105                 110
   Leu Val Leu Tyr Asn Leu Pro Asp Arg Asp Cys Ser Ala Gly Glu Ser
           115                 120                 125
   Ala Gly Glu Leu Ser Ser Asp Lys Asn Gly Leu Glu Ile Tyr Lys Thr
       130                 135                 140
   Glu Phe Val Lys Pro Phe Ala Asp Lys Val Ala Ala Ala Lys Asp Leu
   145                 150                 155                 160
   Asp Phe Ala Ile Val Leu Glu Pro Asp Ser Leu Ala Asn Leu Val Thr
                   165                 170                 175
   Asn Leu Gly Ile Glu Phe Cys Ala Asn Ala Ala Pro Val Tyr Arg Glu
               180                 185                 190
   Gly Ile Ala Tyr Ala Ile Ser Ser Leu Gln Gln Pro Asn Val His Leu
               195                 200                 205
   Tyr Ile Asp Ala Ala His Gly Gly Trp Leu Gly Trp Asp Asp Asn Leu
       210                 215                 220
   Pro Leu Ala Ala Lys Glu Phe Ala Glu Val Val Lys Leu Ala Gly Glu
   225                 230                 235                 240
   Gly Lys Lys Ile Arg Gly Phe Val Thr Asn Val Ser Asn Tyr Asn Pro
                   245                 250                 255
   Phe His Ala Val Val Arg Glu Asn Phe Thr Glu Trp Ser Asn Ser Trp
               260                 265                 270
   Asp Glu Ser His Tyr Ala Ser Ser Leu Thr Pro Phe Leu Glu Lys Glu
           275                 280                 285
   Gly Leu Pro Ala Arg Phe Ile Val Asp Gln Gly Arg Val Ala Leu Pro
       290                 295                 300
   Gly Ala Arg Lys Glu Trp Gly Glu Trp Cys Asn Val Ala Pro Ala Gly
   305                 310                 315                 320
   Phe Gly Pro Ala Pro Thr Thr Arg Val Asn Asn Thr Val Val Asp Ala
                   325                 330                 335
   Leu Val Trp Val Lys Pro Gly Gly Glu Ser Asp Gly Glu Cys Gly Leu
               340                 345                 350
   Ala Gly Ala Pro Lys Ala Gly Gln Trp Phe Asp Glu Tyr Ala Gln Met
           355                 360                 365
   Leu Val Glu Asn Ala His Pro Ser Val Val His Lys Trp
       370                 375                 380
<210> 30
   <211> 363
   <212> PRT
   <213> Myceliophthora thermophila
<400> 30
   Thr Pro Lys Pro Lys Gly Ser Ser Pro Pro Gly Ala Val Asp Ala Asn
   1               5                   10                  15
   Pro Phe Lys Gly Lys Thr Gln Phe Val Asn Pro Ala Trp Ala Ala Lys
               20                  25                  30
   Leu Glu Gln Thr Lys Lys Ala Phe Leu Ala Arg Asn Asp Thr Val Asn
   35 40 45
   Ala Ala Lys Thr Glu Lys Val Gln Gln Thr Ser Ser Phe Val Trp Val
   50 55 60
   Ser Arg Ile Ala Glu Leu Ser Asn Ile Asp Asp Ala Ile Ala Ala Ala
   65 70 75 80
   Arg Lys Ala Gln Lys Lys Thr Gly Arg Arg Gln Ile Val Gly Leu Val
   85 90 95
   Leu Tyr Asn Leu Pro Asp Arg Asp Cys Ser Ala Gly Glu Ser Ala Gly
   100 105 110
   Glu Leu Ser Ser Asp Lys Asn Gly Leu Glu Ile Tyr Lys Thr Glu Phe
   115 120 125
   Val Lys Pro Phe Ala Asp Lys Val Ala Ala Ala Lys Asp Leu Asp Phe
   130 135 140
   Ala Ile Val Leu Glu Pro Asp Ser Leu Ala Asn Leu Val Thr Asn Leu
   145 150 155 160
   Gly Ile Glu Phe Cys Ala Asn Ala Ala Pro Val Tyr Arg Glu Gly Ile
   165 170 175
   Ala Tyr Ala Ile Ser Ser Leu Gln Gln Pro Asn Val His Leu Tyr Ile
   180 185 190
   Asp Ala Ala His Gly Gly Trp Leu Gly Trp Asp Asp Asn Leu Pro Leu
   195 200 205
   Ala Ala Lys Glu Phe Ala Glu Val Val Lys Leu Ala Gly Glu Gly Lys
   210 215 220
   Lys Ile Arg Gly Phe Val Thr Asn Val Ser Asn Tyr Asn Pro Phe His
   225 230 235 240
   Ala Val Val Arg Glu Asn Phe Thr Glu Trp Ser Asn Ser Trp Asp Glu
   245 250 255
   Ser His Tyr Ala Ser Ser Leu Thr Pro Phe Leu Glu Lys Glu Gly Leu
   260 265 270
   Pro Ala Arg Phe Ile Val Asp Gln Gly Arg Val Ala Leu Pro Gly Ala
   275 280 285
   Arg Lys Glu Trp Gly Glu Trp Cys Asn Val Ala Pro Ala Gly Phe Gly
       290                 295                 300
   Pro Ala Pro Thr Thr Arg Val Asn Asn Thr Val Val Asp Ala Leu Val
   305                 310                 315                 320
   Trp Val Lys Pro Gly Gly Glu Ser Asp Gly Glu Cys Gly Leu Ala Gly
                   325                 330                 335
   Ala Pro Lys Ala Gly Gln Trp Phe Asp Glu Tyr Ala Gln Met Leu Val
               340                 345                 350
   Glu Asn Ala His Pro Ser Val Val His Lys Trp
           355                 360
<210> 31
   <211> 1143
   <212> DNA
   <213> Artificial
<220>
   <223> cDNA encoding MtEG VI
<400> 31
<210> 32
   <211> 1658
   <212> DNA
   <213> Myceliophthora thermophila
<400> 32
   aggaacgggg aggaggaggg cttggttagg gtcgcgttcg tttggagatt gctgagctct      60
   gagccttcgg tccttggatc cctgcggtcc ccggtctcct ctctctctct ctctctctct     120
   ctctctctct cttcttccca cgctcgttcg acagacgcct ccccttcttc gctctccttt     180
   ccctcgcacg tagcacacta atagtgcacc atgcgcgtct ctagtttggt cgcggccctt     240
   gctaccggtg gtcttgtcgc cgccacgcct aagcccaagg ggtcgtcgcc ccctggggcc     300
   gtggacgcga accctttcaa gggcaagacg cagttcgtca acccggcatg ggcggccaag     360
   ctggaacaga ccaaaaaggc gttcctggcc aggaacgaca ccgtcaatgc cgccaagacg     420
   gagaaggtcc agcagaccag ctcgttcgtc tgggtctcga ggatcgccga gctctccaac     480
   atcgacgacg ccatcgcggc tgcccgcaag gcgcagaaga agacgggcag gaggcagatc     540
   gtcggcctgg tgctctacaa ccttccggac cgcgactgca gcgcgggcga gagcgcgggc     600
   gagctcagca gcgacaagaa cgggctcgag atctacaaga ctgagttcgt caagcccttc     660
   gccgacaagg tggcggccgc aaaggacctc gacttcgcca tcgtcctgga gcccgactcg     720
   ctggccaacc tggtcaccaa cctgggcatc gagttctgcg ccaacgccgc ccccgtctac     780
   cgcgagggca tcgcctatgc catctccagc cttcagcagc caaacgtgca cttgtacatc     840
   gatgctgccc acggcggctg gctcggctgg gacgacaacc tgccgctggc cgccaaggag     900
   tttgccgagg tggtcaagct tgccggcgag ggcaagaaga tccgcggctt cgtcaccaac     960
   gtgtccaact acaacccctt ccacgccgtc gtgcgcgaga actttaccga gtggagcaac    1020
   tcgtgggacg agtctcacta cgcctcctcg ctcacaccgt tcctcgagaa agaggggctg    1080
   ccggcacgct tcatcgtcga ccagggtcgc gttgccctcc cgggagcccg caaggagtgg    1140
   tgagtttcga ccagattgac cctcgaccca tgcgaccgag attgctgacg attgaattgc    1200
   gtgtcccgtc ccccaggggt gaatggtgca acgtggcacc cgccggattt ggccccgcgc    1260
   ccacgaccag ggtcaacaac accgtcgtcg atgctctcgt ctgggtcaag cctggcggcg    1320
   agagcgacgg cgagtgtggc ttggctggcg cccccaaggc cggccagtgg ttcgacgagt    1380
   acgcccagat gctggtcgag aatgcccacc cgtctgtcgt ccacaagtgg tagataaatt    1440
   ttggagtccg agaagggtcc cagatagact tttgttttaa aacaaaatgc aaggtgtcga    1500
   cagatactgg cttaacatta accaagcacc atgaacatga cttgtcaaca tattgataca    1560
   ttccgctgct ttcccatacg tgctctcagg tctcagggat caaatggata ggtcggtaat    1620
   gcaaaacgat ccattggata tccagaagag agaaaaaa                            1658
<210> 33
   <211> 402
   <212> PRT
   <213> Myceliophthora thermophila
<400> 33
   Arg Cys Ser Ser Asp Ala Pro Pro Pro Ala Pro Val Gly Asp Asp Leu
   1               5                   10                  15
   Thr Glu Pro Lys Glu Leu Thr Asp Leu Phe Glu Lys Ala Lys Lys Ala
               20                  25                  30
   Val Ile Asp Arg Leu His Glu Asp Glu Lys Ala Leu Arg Ala Arg Gly
           35                  40                  45
   Glu Ala Pro Arg Cys Thr Ala Asp Lys Leu Ile Phe Arg Arg Glu Tyr
       50                  55                  60
   Gly Ser Leu Ser Lys Asp Glu Arg Leu Ala Tyr Val Asn Ala Val Lys
   65                  70                  75                  80
   Cys Leu Gln Ser Lys Pro Pro Arg Thr Pro Ala Ser Val Ala Pro Gly
                   85                  90                  95
   Ala Arg Ser Arg Phe Asp Asp Phe Val Val Val His Ile Gln Gln Thr
               100                 105                 110
   Leu Asp Ile His Tyr Ser Gly Ile Phe Gln Ala Trp His Arg Trp Phe
           115                 120                 125
   Val Tyr Gln Tyr Glu Lys Ala Leu Arg Asp Glu Cys Gly Tyr Thr Gly
       130                 135                 140
   Tyr Gln Pro Tyr Trp Asp Trp Pro Lys Tyr Ala Ser Ala Pro Gln Asp
   145                 150                 155                 160
   Ser Pro Leu Phe Asn Gly Asp Pro Tyr Ser Leu Gly Gly Asn Gly Glu
                   165                 170                 175
   Tyr Val Pro His Asp Gly Pro Val Ile Val Pro Pro Glu Gly Val Ser
               180                 185                 190
   Gly Gly Asn Ile Ser Leu Pro Ala Gly Val Gly Gly Gly Phe Val Arg
   195 200 205
   Thr Gly Pro Phe Ala Asn Met Thr Val Asn Leu Gly Pro Val Gly Gly
   210 215 220
   Leu Ala Asp Thr Ala Pro Gly Pro Gln Gly Gly Leu Gly Tyr Asn Pro
   225 230 235 240
   Arg Gly Leu Lys Arg Asp Leu Gly Gly Ala Met Asn Thr Arg Tyr Ala
   245 250 255
   Asn Tyr Thr Thr Val Leu Arg Leu Leu Thr Gln Pro Asp Val Asp Ala
   260 265 270
   Phe Arg Thr Val Ser Glu Gly Val Pro Tyr Thr Val Glu Ile Gly Pro
   275 280 285
   His Gly Gly Ile His Tyr Thr Ile Gly Gly Asp Pro Gly Gly Asp Leu
   290 295 300
   Phe Thr Ser Pro Gly Asp Pro Ala Phe Trp Val His His Ala Gln Met
   305 310 315 320
   Asp Arg Val Trp Ala Thr Trp Gln Ala Leu Gly Leu Leu Pro Pro Ala
   325 330 335
   Asp Gly Gly Asp Pro Asp Pro Ala Arg Arg Tyr Thr Asp Leu Gly Lys
   340 345 350
   Gly Asp Tyr Ala His Arg Thr Trp Gln Asn Ser Pro Pro Ser Pro Phe
   355 360 365
   Ala Glu Leu Ser Asp Val Ile Asp Met Gly Tyr Ala Ala Pro Ser Thr
   370 375 380
   Thr Ile Gly Ala Val Met Ser Thr Thr Glu Gly Glu Leu Cys Tyr Phe
   385 390 395 400
   Tyr Leu
<210> 34
   <211> 424
   <212> PRT
   <213> Myceliophthora thermophila
<400> 34
   Met Lys Pro Ala Ala Leu Leu Gly Ala Ala Leu Ala Ala Val Ala Phe
   1               5                   10                  15
   Pro Ala Gly Ala His Ala Arg Cys Ser Ser Asp Ala Pro Pro Pro Ala
               20                  25                  30
   Pro Val Gly Asp Asp Leu Thr Glu Pro Lys Glu Leu Thr Asp Leu Phe
           35                  40                  45
   Glu Lys Ala Lys Lys Ala Val Ile Asp Arg Leu His Glu Asp Glu Lys
       50                  55                  60
   Ala Leu Arg Ala Arg Gly Glu Ala Pro Arg Cys Thr Ala Asp Lys Leu
   65                  70                  75                  80
   Ile Phe Arg Arg Glu Tyr Gly Ser Leu Ser Lys Asp Glu Arg Leu Ala
                   85                  90                  95
   Tyr Val Asn Ala Val Lys Cys Leu Gln Ser Lys Pro Pro Arg Thr Pro
               100                 105                 110
   Ala Ser Val Ala Pro Gly Ala Arg Ser Arg Phe Asp Asp Phe Val Val
           115                 120                 125
   Val His Ile Gln Gln Thr Leu Asp Ile His Tyr Ser Gly Ile Phe Gln
       130                 135                 140
   Ala Trp His Arg Trp Phe Val Tyr Gln Tyr Glu Lys Ala Leu Arg Asp
   145                 150                 155                 160
   Glu Cys Gly Tyr Thr Gly Tyr Gln Pro Tyr Trp Asp Trp Pro Lys Tyr
                   165                 170                 175
   Ala Ser Ala Pro Gln Asp Ser Pro Leu Phe Asn Gly Asp Pro Tyr Ser
               180                 185                 190
   Leu Gly Gly Asn Gly Glu Tyr Val Pro His Asp Gly Pro Val Ile Val
           195                 200                 205
   Pro Pro Glu Gly Val Ser Gly Gly Asn Ile Ser Leu Pro Ala Gly Val
       210                 215                 220
   Gly Gly Gly Phe Val Arg Thr Gly Pro Phe Ala Asn Met Thr Val Asn
   225                 230                 235                 240
   Leu Gly Pro Val Gly Gly Leu Ala Asp Thr Ala Pro Gly Pro Gln Gly
                   245                 250                 255
   Gly Leu Gly Tyr Asn Pro Arg Gly Leu Lys Arg Asp Leu Gly Gly Ala
   260 265 270
   Met Asn Thr Arg Tyr Ala Asn Tyr Thr Thr Val Leu Arg Leu Leu Thr
   275 280 285
   Gln Pro Asp Val Asp Ala Phe Arg Thr Val Ser Glu Gly Val Pro Tyr
   290 295 300
   Thr Val Glu Ile Gly Pro His Gly Gly Ile His Tyr Thr Ile Gly Gly
   305 310 315 320
   Asp Pro Gly Gly Asp Leu Phe Thr Ser Pro Gly Asp Pro Ala Phe Trp
   325 330 335
   Val His His Ala Gln Met Asp Arg Val Trp Ala Thr Trp Gln Ala Leu
   340 345 350
   Gly Leu Leu Pro Pro Ala Asp Gly Gly Asp Pro Asp Pro Ala Arg Arg
   355 360 365
   Tyr Thr Asp Leu Gly Lys Gly Asp Tyr Ala His Arg Thr Trp Gln Asn
   370 375 380
   Ser Pro Pro Ser Pro Phe Ala Glu Leu Ser Asp Val Ile Asp Met Gly
   385 390 395 400
   Tyr Ala Ala Pro Ser Thr Thr Ile Gly Ala Val Met Ser Thr Thr Glu
   405 410 415
   Gly Glu Leu Cys Tyr Phe Tyr Leu
   420
<210> 35
   <211> 1275
   <212> DNA
   <213> Artificial
<220>
   <223> cDNA encoding PPO2
<400> 35
   atgaaacctg ccgcgctgct cggagcggcc cttgcggccg tggccttccc cgcgggcgcc 60
   catgcccgct gttcttccga tgcgccgcct cccgcccccg ttggcgatga cctcaccgag 120
   cccaaggagc tgacggacct cttcgagaag gccaagaagg ccgtcatcga tcgcctccac 180
   gaggacgaga aggccctgcg cgcccgcggc gaggcgcctc gctgcaccgc cgataagctc 240
   atctttcgcc gcgaatacgg gtctctgtcc aaggatgagc gtctcgcgta cgtcaacgcg 300
   gtcaagtgcc tgcagagcaa gccgccgcgg acgccggcga gcgtggcgcc cggggcgcgg 360
   tcgcggttcg acgactttgt ggtggtgcac atccagcaga cgctcgacat ccactactcg 420
   ggcatcttcc aggcgtggca ccgctggttc gtgtaccagt acgagaaggc gctgcgggac 480
   gagtgcggct acacgggcta ccagccgtat tgggactggc ccaagtacgc gagcgcgccc 540
   caggactcgc ccctgttcaa cggcgacccg tacagcctgg gcggcaacgg cgagtacgtg 600
   ccccacgacg ggcccgtcat cgtgccgccc gagggcgtca gcggcggcaa catctcgctc 660
   ccggccggcg tcggcggcgg cttcgtcagg acggggccct tcgccaacat gacggtcaac 720
   ctgggccccg tcggcggcct ggccgacacc gccccgggcc cccagggcgg cctcggctac 780
   aacccgcgcg ggctcaagcg cgacctgggc ggggccatga acacgcgcta cgccaactac 840
   accaccgtcc tgcgcctgct gacccagccc gacgtcgacg ccttccgcac cgtctccgag 900
   ggcgtcccct acacggtcga gatcggcccc cacggcggca tccactacac catcggcggc 960
   gacccgggcg gcgacctctt cacctccccc ggcgacccgg ccttctgggt ccaccacgcc 1020
   cagatggacc gcgtctgggc cacctggcag gccctcggcc tcctcccccc cgccgacggc 1080
   ggcgaccccg acccggcccg ccgctacacc gacctcggca agggcgacta cgcccaccgc 1140
   acctggcaga actccccgcc ctcccccttt gccgagctgt ccgacgtcat cgatatgggc 1200
   tacgccgccc cctccaccac catcggcgcc gtcatgtcca ccaccgaagg cgagctgtgc 1260
   tacttttatc tctga 1275
<210> 36
   <211> 1490
   <212> DNA
   <213> Myceliophthora thermophila
<400> 36
   atgaaacctg ccgcgctgct cggagcggcc cttgcggccg tggccttccc cgcgggcgcc      60
   catgcccgct gttcttccga tgcgccgcct cccgcccccg ttggcgatga cctcaccgag     120
   cccaaggagc tgacggacct cttcgagaag gccaagaagg ccgtcatcga tcgcctccac     180
   gaggacgaga aggccctgcg cgcccgcggc gaggcgcctc gctgcaccgc cgataagctc     240
   atctttcgcc gcgaatagta cgttcatgat gcctcttctt agtcccttta taatacacaa     300
   acaccgaata ttcccacgct ttaattcgtg tttttttttt tggcgttctt ctcttcaatc      360
   atgcccatgt tttatacaaa ggcatcatga accgtgtccc ccccttgacg tgagagtgtt      420
   tgctgaccgg ccgactttgt tccatcgtcg ccgtcgtcgt cgtcgtccac agcgggtctc     480
   tgtccaagga tgagcgtctc gcgtacgtca acgcggtcaa gtgcctgcag agcaagccgc     540
   cgcggacgcc ggcgagcgtg gcgcccgggg cgcggtcgcg gttcgacgac tttgtggtgg     600
   tgcacatcca gcagacgctc gacatccact actcgggcat cttccaggcg tggcaccgct     660
   ggttcgtgta ccagtacgag aaggcgctgc gggacgagtg cggctacacg ggctaccagc     720
   cgtattggga ctggcccaag tacgcgagcg cgccccagga ctcgcccctg ttcaacggcg     780
   acccgtacag cctgggcggc aacggcgagt acgtgcccca cgacgggccc gtcatcgtgc     840
   cgcccgaggg cgtcagcggc ggcaacatct cgctcccggc cggcgtcggc ggcggcttcg     900
   tcaggacggg gcccttcgcc aacatgacgg tcaacctggg ccccgtcggc ggcctggccg     960
   acaccgcccc gggcccccag ggcggcctcg gctacaaccc gcgcgggctc aagcgcgacc    1020
   tgggcggggc catgaacacg cgctacgcca actacaccac cgtcctgcgc ctgctgaccc    1080
   agcccgacgt cgacgccttc cgcaccgtct ccgagggcgt cccctacacg gtcgagatcg    1140
   gcccccacgg cggcatccac tacaccatcg gcggcgaccc gggcggcgac ctcttcacct    1200
   cccccggcga cccggccttc tgggtccacc acgcccagat ggaccgcgtc tgggccacct    1260
   ggcaggccct cggcctcctc ccccccgccg acggcggcga ccccgacccg gcccgccgct     1320
   acaccgacct cggcaagggc gactacgccc accgcacctg gcagaactcc ccgccctccc    1380
   cctttgccga gctgtccgac gtcatcgata tgggctacgc cgccccctcc accaccatcg    1440
   gcgccgtcat gtccaccacc gaaggcgagc tgtgctactt ttatctctga               1490
<210> 37
   <211> 352
   <212> PRT
   <213> Myceliophthora thermophila
<400> 37
   Asp Ala Val Leu Asp Leu Gln Glu Lys Gly Arg Pro Ala Ile Asp Ala
   1               5                   10                  15
   Gln Leu Ala Lys Ser Thr Thr Cys Thr Lys Glu Lys Leu Gln Val Arg
               20                  25                  30
   Arg Glu Trp Gly Asp Ile Ser Ala Glu Glu Lys Lys Ala Tyr Ile Ala
           35                  40                  45
   Ala Met Leu Cys Ile Met Glu Lys Pro Ser Arg Leu Asp Pro Asn Gln
       50                  55                  60
   Phe Pro Gly Ala Lys Ser Arg Tyr Asp Asp Phe Val Val Val His Met
   65                  70                  75                  80
   Asn Gln Thr Leu Ser Ile His Gly Thr Gly Asn Phe Leu Ser Trp His
                   85                  90                  95
   Arg Tyr Tyr Thr Trp Val Phe Glu Arg Ala Leu Gln Glu Glu Cys Gly
               100                 105                 110
   Tyr Asn Gly Thr Gln Pro Tyr Trp Asp Trp Gly Arg Trp Ala Asp Asp
               115                 120                 125
   Pro Glu Asn Ser Pro Ile Phe Asp Gly Ser Asp Thr Ser Leu Ser Gly
       130                 135                 140
   Asn Gly Glu Tyr Ile Glu His Arg Ala Ser Gly Phe Ile Pro Ala Gly
   145                 150                 155                 160
   Asn Gly Gly Gly Cys Val Lys Ser Gly Pro Phe Lys Asp Met Val Val
                   165                 170                 175
   His Leu Gly Pro Val Ala Pro Ala Ile Asp Pro Ala Pro Pro Ala Asn
               180                 185                 190
   Pro Arg Arg Asp Gly Tyr Gly Asp Asn Pro Arg Cys Leu Arg Arg Asp
           195                 200                 205
   Ile Ser Asn Gln Leu Ser Ser Lys Tyr Ala Arg Thr Gln Asp Ile Val
       210                 215                 220
   Asp Leu Ile Thr Thr Ser Ala Asp Ile Gly Thr Phe Gln Thr Val Met
   225                 230                 235                 240
   Gln Gly Ala Gly Gly Phe Gly Val Gly Pro Gly Gly Met Gly Val His
                   245                 250                 255
   Ala Ala Gly His Phe Thr Ile Ala Gly Asp Pro Gly Gly Asp Phe Tyr
               260                 265                 270
   Thr Ser Pro Asn Asp Pro Ala Phe Trp Val His His Gly Met Ile Asp
           275                 280                 285
   Arg Thr Trp Thr Ile Trp Gln Ser Gln Asp Leu Asp Asn Arg Leu Gln
       290                 295                 300
   Val Ile Ala Gly Gly Ser Ser Met Met Gly Gly Pro Pro Gln Thr Leu
   305                 310                 315                 320
   Asp Asp Pro Val Asp Leu Gly Val Val Ala Asp Lys Val Tyr Pro Ile
                   325                 330                 335
   Arg Asp Leu Val Ser Val Val Asp Gly Pro Phe Cys Tyr Val Tyr Glu
               340                 345                 350
<210> 38
   <211> 375
   <212> PRT
   <213> Myceliophthora thermophila
<400> 38
   Met Ala Arg Leu Thr Leu Ala Gly Leu Leu Ala Val Pro Trp Leu Leu
   1 5 10 15
   Leu Pro Gly Leu Val Ser Gly Asp Ala Val Leu Asp Leu Gln Glu Lys
   20 25 30
   Gly Arg Pro Ala Ile Asp Ala Gln Leu Ala Lys Ser Thr Thr Cys Thr
   35 40 45
   Lys Glu Lys Leu Gln Val Arg Arg Glu Trp Gly Asp Ile Ser Ala Glu
   50 55 60
   Glu Lys Lys Ala Tyr Ile Ala Ala Met Leu Cys Ile Met Glu Lys Pro
   65 70 75 80
   Ser Arg Leu Asp Pro Asn Gln Phe Pro Gly Ala Lys Ser Arg Tyr Asp
   85 90 95
   Asp Phe Val Val Val His Met Asn Gln Thr Leu Ser Ile His Gly Thr
   100 105 110
   Gly Asn Phe Leu Ser Trp His Arg Tyr Tyr Thr Trp Val Phe Glu Arg
   115 120 125
   Ala Leu Gln Glu Glu Cys Gly Tyr Asn Gly Thr Gln Pro Tyr Trp Asp
   130 135 140
   Trp Gly Arg Trp Ala Asp Asp Pro Glu Asn Ser Pro Ile Phe Asp Gly
   145 150 155 160
   Ser Asp Thr Ser Leu Ser Gly Asn Gly Glu Tyr Ile Glu His Arg Ala
   165 170 175
   Ser Gly Phe Ile Pro Ala Gly Asn Gly Gly Gly Cys Val Lys Ser Gly
   180 185 190
   Pro Phe Lys Asp Met Val Val His Leu Gly Pro Val Ala Pro Ala Ile
   195 200 205
   Asp Pro Ala Pro Pro Ala Asn Pro Arg Arg Asp Gly Tyr Gly Asp Asn
   210 215 220
   Pro Arg Cys Leu Arg Arg Asp Ile Ser Asn Gln Leu Ser Ser Lys Tyr
   225 230 235 240
   Ala Arg Thr Gln Asp Ile Val Asp Leu Ile Thr Thr Ser Ala Asp Ile
                   245                 250                 255
   Gly Thr Phe Gln Thr Val Met Gln Gly Ala Gly Gly Phe Gly Val Gly
               260                 265                 270
   Pro Gly Gly Met Gly Val His Ala Ala Gly His Phe Thr Ile Ala Gly
           275                 280                 285
   Asp Pro Gly Gly Asp Phe Tyr Thr Ser Pro Asn Asp Pro Ala Phe Trp
       290                 295                 300
   Val His His Gly Met Ile Asp Arg Thr Trp Thr Ile Trp Gln Ser Gln
   305                 310                 315                 320
   Asp Leu Asp Asn Arg Leu Gln Val Ile Ala Gly Gly Ser Ser Met Met
                   325                 330                 335
   Gly Gly Pro Pro Gln Thr Leu Asp Asp Pro Val Asp Leu Gly Val Val
               340                 345                 350
   Ala Asp Lys Val Tyr Pro Ile Arg Asp Leu Val Ser Val Val Asp Gly
           355                 360                 365
   Pro Phe Cys Tyr Val Tyr Glu
       370                 375
<210> 39
   <211> 1128
   <212> DNA
   <213> Artificial
<220>
   <223> cDNA encoding PPO4
<400> 39
   atggcacgtc tcaccctcgc ggggctgctt gcagtcccct ggctcctgct cccgggcctc     60
   gtgtccggcg acgcagtctt ggatctccag gaaaagggtc gtcccgccat cgatgctcaa    120
   ctggccaagt cgacgacatg caccaaagag aaactccagg tccgccgcga gtggggcgac    180
   atctcggccg aagagaagaa ggcctacatc gccgccatgc tgtgcatcat ggagaagccg    240
   tccaggttgg acccgaacca gtttcccggc gccaagtcac ggtacgacga cttcgtcgtc    300
   gttcacatga accagactct gtccatccac ggcacgggca acttcctctc gtggcatcgc    360
   tactacacct gggtgtttga gcgcgccctc caggaggagt gcgggtataa cgggacccag    420
   ccctactggg actgggggcg ctgggccgac gacccggaga actcgcccat ctttgacggc    480
   agcgacacct ctctcagcgg caacggcgag tacattgagc acagggcctc gggcttcata 540
   cccgccggca acggaggtgg ctgcgtcaag tcgggtccct tcaaagacat ggtcgtccac 600
   ctgggccccg tcgctcccgc catcgacccg gcgccgccgg cgaacccgcg gcgcgacggg 660
   tacggcgaca acccgcggtg cctgcggcgt gacatctcga accagctgtc gagcaagtac 720
   gcgcggacgc aggacattgt cgacctgatc acgacgtcgg ccgacattgg caccttccag 780
   acggtcatgc agggcgccgg cggcttcggc gtcggccccg gcggcatggg cgtgcacgcc 840
   gccggccact tcaccatcgc cggcgacccg ggcggcgact tctacacctc ccccaacgac 900
   ccggccttct gggtccacca cggcatgatc gaccgcacct ggaccatctg gcagagccag 960
   gacctcgaca accgcctcca ggtcatcgcc ggcggttcca gcatgatggg cggccctccc 1020
   cagaccctcg acgaccccgt tgacctcggc gtcgtcgccg acaaggtcta tcccatcagg 1080
   gacctggtca gcgtcgtcga cgggcccttc tgctacgtct atgagtga 1128
<210> 40
   <211> 1468
   <212> DNA
   <213> Myceliophthora thermophila
<400> 40
   atggcacgtc tcaccctcgc ggggctgctt gcagtcccct ggctcctgct cccgggcctc      60
   gtgtccggcg acgcagtctt ggatctccag gaaaagggtc gtcccgccat cgatgctcaa     120
   ctggccaagt cgacgacatg caccaaagag aaactccagg tccgccgcga gtggtgagtc     180
   tgccgccgcc gtcgtcctcc ttaagtatcc ggttaagccg tgatgtccac gcaaactgac     240
   agagaggggg ggggggttga aaccaggggc gacatctcgg ccgaagagaa gaaggcctac     300
   atcgccgcca tgctgtgcat catggagaag ccgtccaggt tggacccgaa ccagtttccc     360
   ggcgccaagt cacggtacga cgacttcgtc gtcgttcaca tgaaccagac tctgtccatc     420
   cacggcacgg taattaattg ccttccccga gaggttgccc tcacctcagt gccatggcat     480
   cgctgactga ctcttcttct tcttcttctt cttcttcttc ttcacagggc aacttcctct     540
   cgtggcatcg ctactacacc tgggtgtttg agcgcgccct ccaggaggag tgcgggtata     600
   acgggaccca gcccgtaaga ctctcccccc cccccccggg gccccgccaa ataacccgta     660
   ttatgcccgc tgaccacttt tgcacagtac tgggactggg ggcgctgggc cgacgacccg     720
   gagaactcgc ccatctttga cggcagcgac acctctctca gcggcaacgg cgagtacatt     780
   gagcacaggg cctcgggctt catacccgcc ggcaacggag gtggctgcgt caagtcgggt     840
   cccttcaaag agtgagtctc atcatcactt attagttgtc tcttttatct ctctccaacc     900
   ctttcctggc taaccccttc gccccagcat ggtcgtccac ctgggccccg tcgctcccgc     960
   catcgacccg gcgccgccgg cgaacccgcg gcgcgacggg tacggcgaca acccgcggtg    1020
   cctgcggcgt gacatctcga accagctgtc gagcaagtac gcgcggacgc aggacattgt    1080
   cgacctgatc acgacgtcgg ccgacattgg caccttccag acggtcatgc agggcgccgg    1140
   cggcttcggc gtcggccccg gcggcatggg cgtgcacgcc gccggccact tcaccatcgc    1200
   cggcgacccg ggcggcgact tctacacctc ccccaacgac ccggccttct gggtccacca    1260
   cggcatgatc gaccgcacct ggaccatctg gcagagccag gacctcgaca accgcctcca    1320
   ggtcatcgcc ggcggttcca gcatgatggg cggccctccc cagaccctcg acgaccccgt    1380
   tgacctcggc gtcgtcgccg acaaggtcta tcccatcagg gacctggtca gcgtcgtcga    1440
   cgggcccttc tgctacgtct atgagtga                                       1468
<210> 41
   <211> 510
   <212> PRT
   <213> Myceliophthora thermophila
<400> 41
   Ala Val Lys Thr Val Val Ile Thr Ala Thr Thr Thr Val Cys Pro Leu
   1               5                   10                  15
   Ser Thr Arg Val Cys His Gly Ser Ser Met Ile Ser Gln Ser Thr Asn
               20                  25                  30
   Ala Pro Cys Ser Glu Ser Thr Gly Ile Thr Gly Ser Gln Ser Thr Ala
           35                  40                  45
   Val Thr Ile Pro Gln Ser Thr Asp Ile Ile Ser Ser Gln Phe Ser Gln
       50                  55                  60
   Phe Thr Asp Thr Thr Ser Ser Leu Ser Thr Gly Val Ala Cys Ser Ser
   65                  70                  75                  80
   Ser Pro Cys Pro Ser Asn Ser Gln Ser Thr Gly Thr Thr Ser Phe Glu
                   85                  90                  95
   Ser Thr Gly Thr Thr Ile Ser Gln Pro Thr Ala Val Thr Asn Ser Ser
               100                 105                 110
   Ser Thr Cys Leu Thr Ser Ser Gln Ser Thr Asp Ala Pro Ser Ser Glu
           115                 120                 125
   Ser Thr Gly Ile Thr Ile Ser Gln Ser Thr Gly Val Thr Ser Ser Gln
       130                 135                 140
   Ser Met Ser Thr Pro Thr Ser Lys Ser Thr Ala Val Pro Ser Ser Gln
   145                 150                 155                 160
   Ser Ala Arg Val Ser Thr Thr Ser Ser Ser Ala Lys Pro Ser Ser Thr
                   165                 170                 175
   Thr Glu Thr Arg Pro Glu Pro Thr Leu Pro Asn Arg Ala Tyr Pro Ala
               180                 185                 190
   Asp Ile Val Asp Lys Leu Arg Asp Ser Gly Val Asp Lys Leu Lys Glu
           195                 200                 205
   Tyr Leu Lys Ser Asn Pro Ser Asn Thr Gly Cys Thr Leu Glu Asn Ala
       210                 215                 220
   Ser Ile Arg Arg Glu Trp Ser Asp Leu Ser Val Ala Glu Arg Glu Glu
   225                 230                 235                 240
   Tyr Ile Ala Ala Val Lys Cys Leu Gln Ser Ala Ser Pro Lys Ser Ser
                   245                 250                 255
   Lys Gln Gln Val Pro Gly Ala Arg Ser Arg Phe Asp Asp Phe Val Ala
               260                 265                 270
   Thr His Ile Asn Gln Thr Asp Ser Ile His Tyr Thr Ala Asn Phe Leu
           275                 280                 285
   Ser Trp His Arg Tyr Tyr Val Trp Ala Tyr Glu Lys Ala Leu Arg Glu
       290                 295                 300
   Glu Cys Gly Tyr Thr Gly Phe Gln Pro Tyr Trp Asn Trp Asp Arg Tyr
   305                 310                 315                 320
   Ala Ala Asp Pro Ala Asn Ser Pro Leu Phe Ser Gly Asn Ser Ser Ser
                   325                 330                 335
   Leu Ser Gly Asn Ser Val Asn Gly Gly Cys Val Thr Thr Gly Pro Phe
               340                 345                 350
   Ala Asp Met Lys Val Asn Leu Gly Pro Gly Ala Ser Leu Ala Tyr Asn
           355                 360                 365
   Pro Arg Cys Leu Lys Arg Asn Ile Ser Lys Ser Tyr Ala Ala Met Thr
       370                 375                 380
   Thr Ala Asp Lys Thr Tyr Ala Leu Ile Thr Gly Ser Ala Asp Ile Ala
   385                 390                 395                 400
   Arg Phe Gln Asp Thr Met Gln Ala Val Pro Gly Val His Ala Gly Gly
                   405                 410                 415
   His Phe Thr Ile Gly Gly Asp Pro Gly Gly Asp Val Tyr Ser Ser Pro
   420 425 430
   Gly Asp Pro Ala Phe Trp Leu His His Ala Met Ile Asp Arg Val Trp
   435 440 445
   Trp Ile Trp Gln Thr His Gly Leu Pro Gly Arg Leu Ser Glu Val Ala
   450 455 460
   Gly Ser Val Ala Gly Ser Ser Arg Pro Gly Ser Gly Lys Asp Leu Val
   465 470 475 480
   Asn Leu Gly Val Asn Gly Pro Ala Val Ala Ile Glu Asn Leu Leu Asn
   485 490 495
   Thr Val Gly Gly Leu Asp Gly Lys Met Cys Tyr Ile Tyr Leu
   500 505 510
<210> 42
   <211> 529
   <212> PRT
   <213> Myceliophthora thermophila
<400> 42
   Met Gln Leu Ser Leu Leu Ser Thr Gly Ala Leu Leu Leu Gly Val Gly
   1 5 10 15
   Thr Ser His Ala Val Lys Thr Val Val Ile Thr Ala Thr Thr Thr Val
   20 25 30
   Cys Pro Leu Ser Thr Arg Val Cys His Gly Ser Ser Met Ile Ser Gln
   35 40 45
   Ser Thr Asn Ala Pro Cys Ser Glu Ser Thr Gly Ile Thr Gly Ser Gln
   50 55 60
   Ser Thr Ala Val Thr Ile Pro Gln Ser Thr Asp Ile Ile Ser Ser Gln
   65 70 75 80
   Phe Ser Gln Phe Thr Asp Thr Thr Ser Ser Leu Ser Thr Gly Val Ala
   85 90 95
   Cys Ser Ser Ser Pro Cys Pro Ser Asn Ser Gln Ser Thr Gly Thr Thr
   100 105 110
   Ser Phe Glu Ser Thr Gly Thr Thr Ile Ser Gln Pro Thr Ala Val Thr
   115 120 125
   Asn Ser Ser Ser Thr Cys Leu Thr Ser Ser Gln Ser Thr Asp Ala Pro
       130                 135                 140
   Ser Ser Glu Ser Thr Gly Ile Thr Ile Ser Gln Ser Thr Gly Val Thr
   145                 150                 155                 160
   Ser Ser Gln Ser Met Ser Thr Pro Thr Ser Lys Ser Thr Ala Val Pro
                   165                 170                 175
   Ser Ser Gln Ser Ala Arg Val Ser Thr Thr Ser Ser Ser Ala Lys Pro
               180                 185                 190
   Ser Ser Thr Thr Glu Thr Arg Pro Glu Pro Thr Leu Pro Asn Arg Ala
           195                 200                 205
   Tyr Pro Ala Asp Ile Val Asp Lys Leu Arg Asp Ser Gly Val Asp Lys
       210                 215                 220
   Leu Lys Glu Tyr Leu Lys Ser Asn Pro Ser Asn Thr Gly Cys Thr Leu
   225                 230                 235                 240
   Glu Asn Ala Ser Ile Arg Arg Glu Trp Ser Asp Leu Ser Val Ala Glu
                   245                 250                 255
   Arg Glu Glu Tyr Ile Ala Ala Val Lys Cys Leu Gln Ser Ala Ser Pro
               260                 265                 270
   Lys Ser Ser Lys Gln Gln Val Pro Gly Ala Arg Ser Arg Phe Asp Asp
           275                 280                 285
   Phe Val Ala Thr His Ile Asn Gln Thr Asp Ser Ile His Tyr Thr Ala
       290                 295                 300
   Asn Phe Leu Ser Trp His Arg Tyr Tyr Val Trp Ala Tyr Glu Lys Ala
   305                 310                 315                 320
   Leu Arg Glu Glu Cys Gly Tyr Thr Gly Phe Gln Pro Tyr Trp Asn Trp
                   325                 330                 335
   Asp Arg Tyr Ala Ala Asp Pro Ala Asn Ser Pro Leu Phe Ser Gly Asn
               340                 345                 350
   Ser Ser Ser Leu Ser Gly Asn Ser Val Asn Gly Gly Cys Val Thr Thr
           355                 360                 365
   Gly Pro Phe Ala Asp Met Lys Val Asn Leu Gly Pro Gly Ala Ser Leu
       370                 375                 380
   Ala Tyr Asn Pro Arg Cys Leu Lys Arg Asn Ile Ser Lys Ser Tyr Ala
   385                 390                 395                 400
   Ala Met Thr Thr Ala Asp Lys Thr Tyr Ala Leu Ile Thr Gly Ser Ala
                   405                 410                 415
   Asp Ile Ala Arg Phe Gln Asp Thr Met Gln Ala Val Pro Gly Val His
               420                 425                 430
   Ala Gly Gly His Phe Thr Ile Gly Gly Asp Pro Gly Gly Asp Val Tyr
           435                 440                 445
   Ser Ser Pro Gly Asp Pro Ala Phe Trp Leu His His Ala Met Ile Asp
       450                 455                 460
   Arg Val Trp Trp Ile Trp Gln Thr His Gly Leu Pro Gly Arg Leu Ser
   465                 470                 475                 480
   Glu Val Ala Gly Ser Val Ala Gly Ser Ser Arg Pro Gly Ser Gly Lys
                   485                 490                 495
   Asp Leu Val Asn Leu Gly Val Asn Gly Pro Ala Val Ala Ile Glu Asn
               500                 505                 510
   Leu Leu Asn Thr Val Gly Gly Leu Asp Gly Lys Met Cys Tyr Ile Tyr
           515                 520                 525
   Leu
<210> 43
   <211> 1590
   <212> DNA
   <213> Artificial
<220>
   <223> cDNA encoding PPO6
<400> 43
   atgcagcttt ctttgttgag taccggagcg ctgctcctcg gggttgggac cagtcatgcg 60
   gtaaagacgg tcgtgattac cgctaccacg accgtctgcc ccctttctac acgcgtctgt 120
   catgggtcgt caatgatctc gcagtctacg aatgcgccgt gctcggagtc tacgggtata 180
   acgggttcgc aatctacggc tgtaacgatc ccgcagtcta cggacattat tagttcgcaa 240
   ttttcgcaat tcacggacac cacgagttcg ctgtctacgg gtgtggcgtg ctcgtcgtct 300
   ccgtgtccct caaactcgca gtctacaggc acaacgagtt tcgagtctac ggggacgact 360
   atctctcagc ctacggctgt aacgaactcg tcgtctacct gtctcacaag ctcgcagtct 420
   accgatgcac cgagctcgga gtctacgggc attacaattt ctcaatctac gggtgtaacg 480
   agctcgcagt ctatgagtac accgacttcc aagtctacgg ctgtaccgag ctcccaatct 540
   gctcgggtct cgacgacatc atcttcggcc aagccctcgt ccactacgga aacacgtcct 600
   gaacccaccc tgcccaatcg agcttatccc gccgacattg tcgacaagct ccgagactca 660
   ggtgtggaca agctcaagga gtacctcaag agcaatccct cgaatacagg ctgcacgctc 720
   gagaacgcgt ctatccgccg cgaatggtcc gacctctccg tcgccgagcg agaggagtac 780
   attgccgccg tcaagtgcct gcagtcggcg agccccaagt cttccaaaca gcaagtcccg 840
   ggcgcgcgta gccgcttcga cgactttgtg gccacgcaca ttaaccagac cgactcgatc 900
   cattacacgg cgaacttcct ctcgtggcac cgatactacg tgtgggcata cgagaaggcc 960
   ctccgcgagg agtgcggcta caccggcttc cagccgtact ggaactggga ccggtacgca 1020
   gccgacccgg ccaactcgcc cctgttcagc ggcaactcgt ccagcctgag cggaaactcg 1080
   gtcaacggcg gttgtgtcac cacgggccct ttcgccgaca tgaaagtcaa cctcggcccc 1140
   ggggcctctc tcgcctacaa cccgcgctgc ctgaagcgca acatcagcaa gagctacgcg 1200
   gccatgacca cggccgacaa gacgtatgcg ctcatcacgg gtagcgccga cattgcccgc 1260
   ttccaggaca ccatgcaggc cgtccctggc gtgcatgccg gcggccactt taccatcggc 1320
   ggcgaccctg gcggcgacgt ctactcgtcg ccgggcgacc cggccttctg gctgcaccac 1380
   gccatgatcg accgcgtctg gtggatctgg cagacgcacg gcctgcccgg ccggctgtcc 1440
   gaggtggccg gttccgtcgc cggctccagc cgcccgggct ccggcaagga cctcgtcaac 1500
   ctgggcgtca acggccccgc cgtcgccatc gagaacctgc tcaacaccgt gggcggcctg 1560
   gacggcaaga tgtgctacat ctacctctga 1590
<210> 44
   <211> 2051
   <212> DNA
   <213> Myceliophthora thermophila
<400> 44
   atgcagcttt ctttgttgag taccggagcg ctgctcctcg gggttgggac cagtcatgcg     60
   gtaaagacgg tcgtgattac cgctaccacg accgtctgcc ccctttctac acgcgtctgt    120
   catgggtcgt caatgatctc gcagtctacg aatgcgccgt gctcggagtc tacgggtata    180
   acgggttcgc aatctacggc tgtaacgatc ccgcagtcta cggacattat tagttcgcaa    240
   ttttcgcaat tcacggacac cacgagttcg ctgtctacgg gtgtggcgtg ctcgtcgtct    300
   ccgtgtccct caaactcgca gtctacaggc acaacgagtt tcgagtctac ggggacgact    360
   atctctcagc ctacggctgt aacgaactcg tcgtctacct gtctcacaag ctcgcagtct    420
   accgatgcac cgagctcgga gtctacgggc attacaattt ctcaatctac gggtgtaacg     480
   agctcgcagt ctatgagtac accgacttcc aagtctacgg ctgtaccgag ctcccaatct     540
   gctcgcgtct cgacgacatc atcttcggcc aagccctcgt ccactacgga aacacgtcct     600
   gaacccaccc tgcccaatcg agcttatccc gccgacattg tcgacaagct ccgagactca     660
   ggtgtggaca agctcaagga gtacctcaag agcaatccct cgaatacagg ctgcacgctc     720
   gagaacgcgt ctatccgccg cgaatggtaa gccacccacc ccccctgctt ctcgaacagt     780
   aattacgttc cgtcgagacg agagaaacca aggctaacaa gaggcacctc cggagcaaac     840
   aggtccgacc tctccgtcgc cgagcgagag gagtacattg ccgccgtcaa gtgcctgcag     900
   tcggcgagcc ccaagtcttc caaacagcaa gtcccgggcg cgcgtagccg cttcgacgac     960
   tttgtggcca cgcacattaa ccagaccgac tcgatccatt acacggtacg tattcggcct    1020
   tgcgtacttc ccatcctttc tctacgtatc tatctccctt gtccagttag cgagcccgtc    1080
   atatgtttga gactgacttc gcatgtccct cgattcaagg cgaacttcct ctcgtggcac    1140
   cgatactacg tgtgggcata cgagaaggcc ctccgcgagg agtgcggcta caccggcttc    1200
   cagccgtact ggaactggga ccggtacgca gccgacccgg ccaactcgcc cctgttcagc    1260
   ggcaactcgt ccagcctgag cggaaactcg gtcaacggcg gttgtgtcac cacgggccct    1320
   ttcgccgagt aagttctttg ctcctcttcc ccccataagg ggtggccaga taattattca    1380
   gacattgcta accgggcctc tcccttcgct tcagcatgaa agtcaacctc ggccccgggg    1440
   cctctctcgc ctacaacccg cgctgcctga agcgcaacat cagcaagagc tacgcggcca    1500
   tgaccacggc cgacaagacg tatgcgctca tcacgggtag cgccgacatt gcccgcttcc    1560
   aggacaccat gcaggccgtc cctggcgtgc atgccggcgg ccactttacc atcggcggcg    1620
   accctggcgg cgtaagttgg atttttgatt ctccgaccct tccccccccc cccccccaaa    1680
   aaaaaaaaaa aaaaccaacc ctcctttctt ttctcagaaa gagaagagcc cttttatttt    1740
   tttccctctg tcgcctggcc cagctgacac gagttctctc ttgggaacaa cgacaggacg    1800
   tctactcgtc gccgggcgac ccggccttct ggctgcacca cgccatgatc gaccgcgtct    1860
   ggtggatctg gcagacgcac ggcctgcccg gccggctgtc cgaggtggcc ggttccgtcg    1920
   ccggctccag ccgcccgggc tccggcaagg acctcgtcaa cctgggcgtc aacggccccg    1980
   ccgtcgccat cgagaacctg ctcaacaccg tgggcggcct ggacggcaag atgtgctaca    2040
   tctacctctg a                                                         2051
<210> 45
   <211> 402
   <212> PRT
   <213> Myceliophthora thermophila
<400> 45
   Asn Pro Met His Lys Arg Trp Lys Pro Asn Ala Pro Arg Asn Pro Asp
   1               5                   10                  15
   Phe Pro Leu Asp Glu Val Asp Lys Leu Glu Glu Ser Ile Met Pro Asn
               20                  25                  30
   Val Glu Ala Trp Met Ser Lys Arg Pro Ala Lys Tyr Ser Asn Cys Thr
           35                  40                  45
   Leu Glu Asn Ala Gly Ile Arg Arg Glu Trp Ser Asp Leu Ser Val Asp
       50                  55                  60
   Glu Arg Lys Glu Tyr Ile Ser Ala Val Leu Cys Leu Gln Ser Lys Pro
   65                  70                  75                  80
   Ser Lys Ile Pro Glu Gly Glu Val Pro Gly Ala Leu Ser Arg Phe Asp
                   85                  90                  95
   Asp Phe Val Ala Thr His Met Thr Met Ala Gly Gln Leu His Ser Pro
               100                 105                 110
   Thr Asn Leu Phe Ala Ala His Arg Tyr Phe Ile His Ala Tyr Glu Lys
           115                 120                 125
   Ala Leu Arg Glu Glu Cys Gly Tyr Lys Gly Tyr Gln Pro Tyr Met Asn
       130                 135                 140
   Tyr Asp Arg Tyr Ala Asp Asp Pro Leu Asn Thr Pro Leu Phe Asp Gly
   145                 150                 155                 160
   Ser Asp Ser Ser Met Ser Gly Asn Gly Gly Pro Tyr Asn Tyr Ser Gly
                   165                 170                 175
   Ile Pro Gln Pro Phe Pro Lys Pro Tyr Asn Leu Ile Pro Pro Ala Gly
               180                 185                 190
   Gly Gly Gly Cys Val Thr Glu Gly Pro Phe Arg Asp Met Val Val Ser
           195                 200                 205
   Leu Gly Pro Lys Gly Gly Leu Phe His Asp Ile Pro Arg Asn Pro Arg
       210                 215                 220
   Ala Asp Gly Leu Gly Ser Asn Pro Arg Cys Leu Arg Arg Asp Val Asn
   225                 230                 235                 240
   Lys Tyr Ser Ala Arg Gly Ala Arg Ala Asn Tyr Thr Tyr Ser Ala Ile
   245 250 255
   Met Asp Asn Pro Asp Ile Asp Ser Phe Tyr Asn Arg Tyr Met Gly Met
   260 265 270
   Pro Gln Leu Lys Gly Asp Pro Tyr Pro Trp Gly Leu His Ser Ser Gly
   275 280 285
   His Tyr Leu Ile Gly Gly Asp Pro Gly Gly Asp Phe Tyr Cys Ser Pro
   290 295 300
   Gly Asp Pro Leu Phe Tyr Phe His His Gly Met Leu Asp Arg Val Trp
   305 310 315 320
   Trp Ile Trp Gln Met Gln Asp Pro Glu Asn Arg Val Gln Ala Val Pro
   325 330 335
   Gly Thr Asn Thr Met Pro Met Pro Pro Leu Met Ala Ala Asn Leu Glu
   340 345 350
   Ser Glu Ala Ala Ala Thr Thr Thr His Lys Arg Ala Val Asn Val Ser
   355 360 365
   Asp Leu Val Val Asp Leu Gly Trp Thr Ala Pro Pro Val Lys Leu Val
   370 375 380
   Asp Leu Asn Glu Gln Leu Gly Gly Leu Gly Gly Glu Leu Cys Tyr Ile
   385 390 395 400
   Tyr Val
<210> 46
   <211> 420
   <212> PRT
   <213> Myceliophthora thermophila
<400> 46
   Met Arg Gly Ile Gln Ser Leu Val Ser Leu Ser Leu Leu Ala Gly Ala
   1               5                   10                  15
   Trp Ala Asn Pro Met His Lys Arg Trp Lys Pro Asn Ala Pro Arg Asn
               20                  25                  30
   Pro Asp Phe Pro Leu Asp Glu Val Asp Lys Leu Glu Glu Ser Ile Met
           35                  40                  45
   Pro Asn Val Glu Ala Trp Met Ser Lys Arg Pro Ala Lys Tyr Ser Asn
   50 55 60
   Cys Thr Leu Glu Asn Ala Gly Ile Arg Arg Glu Trp Ser Asp Leu Ser
   65 70 75 80
   Val Asp Glu Arg Lys Glu Tyr Ile Ser Ala Val Leu Cys Leu Gln Ser
   85 90 95
   Lys Pro Ser Lys Ile Pro Glu Gly Glu Val Pro Gly Ala Leu Ser Arg
   100 105 110
   Phe Asp Asp Phe Val Ala Thr His Met Thr Met Ala Gly Gln Leu His
   115 120 125
   Ser Pro Thr Asn Leu Phe Ala Ala His Arg Tyr Phe Ile His Ala Tyr
   130 135 140
   Glu Lys Ala Leu Arg Glu Glu Cys Gly Tyr Lys Gly Tyr Gln Pro Tyr
   145 150 155 160
   Met Asn Tyr Asp Arg Tyr Ala Asp Asp Pro Leu Asn Thr Pro Leu Phe
   165 170 175
   Asp Gly Ser Asp Ser Ser Met Ser Gly Asn Gly Gly Pro Tyr Asn Tyr
   180 185 190
   Ser Gly Ile Pro Gln Pro Phe Pro Lys Pro Tyr Asn Leu Ile Pro Pro
   195 200 205
   Ala Gly Gly Gly Gly Cys Val Thr Glu Gly Pro Phe Arg Asp Met Val
   210 215 220
   Val Ser Leu Gly Pro Lys Gly Gly Leu Phe His Asp Ile Pro Arg Asn
   225 230 235 240
   Pro Arg Ala Asp Gly Leu Gly Ser Asn Pro Arg Cys Leu Arg Arg Asp
   245 250 255
   Val Asn Lys Tyr Ser Ala Arg Gly Ala Arg Ala Asn Tyr Thr Tyr Ser
   260 265 270
   Ala Ile Met Asp Asn Pro Asp Ile Asp Ser Phe Tyr Asn Arg Tyr Met
   275 280 285
   Gly Met Pro Gln Leu Lys Gly Asp Pro Tyr Pro Trp Gly Leu His Ser
   290 295 300
   Ser Gly His Tyr Leu Ile Gly Gly Asp Pro Gly Gly Asp Phe Tyr Cys
   305                 310                 315                 320
   Ser Pro Gly Asp Pro Leu Phe Tyr Phe His His Gly Met Leu Asp Arg
                   325                 330                 335
   Val Trp Trp Ile Trp Gln Met Gln Asp Pro Glu Asn Arg Val Gln Ala
               340                 345                 350
   Val Pro Gly Thr Asn Thr Met Pro Met Pro Pro Leu Met Ala Ala Asn
           355                 360                 365
   Leu Glu Ser Glu Ala Ala Ala Thr Thr Thr His Lys Arg Ala Val Asn
       370                 375                 380
   Val Ser Asp Leu Val Val Asp Leu Gly Trp Thr Ala Pro Pro Val Lys
   385                 390                 395                 400
   Leu Val Asp Leu Asn Glu Gln Leu Gly Gly Leu Gly Gly Glu Leu Cys
                   405                 410                 415
   Tyr Ile Tyr Val
               420
<210> 47
   <211> 1263
   <212> DNA
   <213> Artificial
<220>
   <223> cDNA encoding PPO7
<400> 47
   atgcgaggga ttcagagtct cgtgagcctg tccttgctcg ccggagcttg ggcgaacccg 60
   atgcacaaga gatggaagcc caacgcgccc cggaacccgg acttccccct tgacgaagtc 120
   gacaagctcg aggagtccat catgcccaat gttgaggcat ggatgtccaa gcgcccggcc 180
   aaatacagca actgcactct cgagaatgcg ggcatccgga gggaatggag cgacctctcg 240
   gtggacgagc gcaaggagta catcagcgcc gtcctctgcc tccagtccaa gccttccaag 300
   attcccgagg gcgaggtccc tggggccctg agccgcttcg acgactttgt ggcgacgcac 360
   atgaccatgg caggccagct gcacagccca accaacctat ttgccgccca ccggtacttc 420
   atccacgcct acgagaaggc actgcgtgag gagtgcggct acaaggggta tcagccgtac 480
   atgaactacg accgctatgc tgacgacccc ctcaacacac ccttgtttga cggcagtgac 540
   agcagcatga gtggtaacgg cggtccgtac aactactccg gcatccccca gcccttccca 600
   aagccgtata acttgattcc tccggctggt ggtggcggtt gtgtcaccga gggccccttc 660
   agagacatgg tagtctcgct gggccccaag ggcggcctct tccacgacat accgcgcaac 720
   ccgcgggcgg acggactagg ctccaacccg cggtgcctcc gccgcgacgt caacaagtat 780
   tcggcccgag gcgcccgcgc caactacacc tacagcgcca tcatggacaa cccggatatc 840
   gacagcttct acaaccgcta catgggcatg ccgcagctca agggggaccc ttacccgtgg 900
   ggtctccata gctccggcca ctacctcatc ggtggcgacc cgggcggcga cttctactgc 960
   tctcccggcg acccgctctt ctacttccac cacggcatgc tcgaccgggt ctggtggatc 1020
   tggcagatgc aggacccgga gaaccgcgtc caagccgtcc cggggaccaa cacgatgccg 1080
   atgccgccct tgatggccgc caacttggag agcgaggcgg ccgcgacgac gacgcacaag 1140
   cgggcggtca acgtcagcga cctggttgtg gacctgggct ggacggcgcc cccggtcaag 1200
   ttggtcgacc tgaacgagca gctgggcggt ctgggcggcg agctgtgcta catctacgtg 1260
   tga 1263
<210> 48
   <211> 1979
   <212> DNA
   <213> Myceliophthora thermophila
<400> 48
   atgcgaggga ttcagagtct cgtgagcctg tccttgctcg ccggagcttg ggcgaacccg     60
   atgcacaaga gatggaagcc caacgcgtag gcaatattgt cacgcccaaa gaccgtctcg    120
   tctctgttcc caactgacat gccgccaggc cccggaaccc ggacttcccc cttgacgaag    180
   tcgacaagct cgaggagtcc atcatgccca atgttgaggc atggatgtcc aagcgcccgg    240
   ccaaatacag caactgcact ctcgagaatg cgggcatccg gagggaatgg tacgatcaga    300
   ggccatgcca taccggctga ctgactgact gactgactgc ctgactgatt ggcctgactg    360
   actaataaac gcccaacagg agcgacctct cggtggacga gcgcaaggag tacatcagcg    420
   ccgtcctctg cctccagtcc aagccttcca agattcccga gggcgaggtc cctggggccc    480
   tgagccgctt cgacgacttt gtggcgacgc acatgaccat ggcaggccag ctgcacagcc    540
   caaccaacct atttgccgcc caccggtact tcatccacgc ctacgagaag gcactgcgtg    600
   aggagtgcgg ctacaagggg tatcagccgg taagagtgac atgacccttt gtttccccta    660
   ccttggaaaa taagaaacaa aaagttttaa aaggttgtta tgttctgttt tgtcccactg    720
   gctgaccctc tttctctcgt ttccttttaa agtacatgaa ctacgaccgc tatgctgacg    780
   accccctcaa cacacccttg tttgacggca gtgacagcag catgagtggt aacggcggtc    840
   cgtacaacta ctccggcatc ccccagccct tcccaaagcc gtataacttg attcctccgg    900
   ctggtggtgg cggttgtgtc accgagggcc ccttcagaga gtgagttcaa gctgacaatc    960
   tcctcctcct cttgctcctc ccttttctcc cctccctttt ctcccctccc ttttctcccc   1020
   tcccttttct cccctccctt ttcttccccc cctcccccct tcttctctct taattatccc   1080
   cctggcctct tccgcccggg tttgtttcct cggttacatc gttacatcgt tacataaaac   1140
   tgacagagag gatgcgcaca acaacagcat ggtagtctcg ctgggcccca agggcggcct   1200
   cttccacgac ataccgcgca acccgcgggc ggacggacta ggctccaacc cgcggtgcct   1260
   ccgccgcgac gtcaacaagt attcggcccg aggcgcccgc gccaactaca cctacagcgc   1320
   catcatggac aacccggata tcgacagctt ctacaaccgc tacatgggca tgccgcagct   1380
   caagggggac ccttacccgt ggggtctcca tagctccggc cactacctca tcggtggcga   1440
   cccgggcggc gtaagtagcc ttttcttgat tttttttttg ttcttgattt ttgttattta   1500
   ttcccccccc cttttttttt tgtcctgtga ttgttttgaa gtgtccatta attacctagt   1560
   caactgttca aattaattat tatctgtccc tcacgcagca ctcccagatt gccaaccaat   1620
   tctgctaacc atggttctca cctcccctcc ccccttgggt tcaggacttc tactgctctc   1680
   ccggcgaccc gctcttctac ttccaccacg gcatgctcga ccgggtctgg tggatctggc   1740
   agatgcagga cccggagaac cgcgtccaag ccgtcccggg gaccaacacg atgccgatgc   1800
   cgcccttgat ggccgccaac ttggagagcg aggcggccgc gacgacgacg cacaagcggg   1860
   cggtcaacgt cagcgacctg gttgtggacc tgggctggac ggcgcccccg gtcaagttgg   1920
   tcgacctgaa cgagcagctg ggcggtctgg gcggcgagct gtgctacatc tacgtgtga    1979
<210> 49
   <211> 629
   <212> PRT
   <213> Myceliophthora thermophila
<400> 49
   Gln Tyr Ser Gly Tyr Asp Tyr Gly Phe Asp Val Lys Lys Arg Val Lys
   1               5                   10                  15
   Arg Gln Leu Gly Gln Arg Ser Ala Met Val Val Gln Asp Lys Thr Gly
               20                  25                  30
   Ser Glu Ile Gln Val Arg Gln Glu Ile Arg Gln Leu Glu Gln Asp His
           35                  40                  45
   Asp Leu Trp Thr Leu Tyr Ile Leu Gly Leu Ser Met Leu Gln Tyr Thr
       50                  55                  60
   Asp Gln Glu Ser Pro Val Ser Tyr Tyr Gly Leu Ala Gly Ile His Gly
   65                  70                  75                  80
   Met Pro His Gln Thr Trp Gly Gly Met Gly Pro Val Thr Gly Asn Glu
                   85                  90                  95
   Asn Thr Gly Tyr Cys Thr His Ser Ser Val Leu Phe Pro Thr Trp His
               100                 105                 110
   Arg Ala Tyr Met Ala Leu Tyr Glu Met Ile Ala Thr Phe Trp Pro Asp
           115                 120                 125
   Ser Glu Arg Gln Arg Tyr Glu Ser Ala Ala Arg Arg Phe Arg Leu Pro
       130                 135                 140
   Tyr Trp Asp Trp Ala Ala Ser Pro Pro Pro Gly Gln Ser Val Leu Pro
   145                 150                 155                 160
   Glu Ser Ile Gly Gly Ser Pro Phe Ile Asp Val Asn Gly Pro Asn Gly
                   165                 170                 175
   Leu Gln Arg Ile Ala Asn Pro Leu Phe Ser Tyr Gln Phe Asn Pro Leu
               180                 185                 190
   Asp Gln Lys Ala Phe Glu Phe Pro Pro Ser Gln Trp Asn Ile Trp Thr
           195                 200                 205
   Arg Thr Leu Arg Ser Pro Ser Ser Gly Gly Pro Asp Ala Gln Ser Asn
       210                 215                 220
   Asn Thr Leu Val Ala Leu Asn Leu Asp Arg Ser Arg Ala Ser Ile Ala
   225                 230                 235                 240
   Gln Arg Leu Tyr Asp Leu Phe Ser His Asn Asp Asn Tyr Thr Leu Phe
                   245                 250                 255
   Ser Asn Asn Ala Val Gly Gly Gln Ala Glu Ser Val Glu Ser Leu His
               260                 265                 270
   Asp Thr Ile His Ser Leu Val Gly Gly Val Gly Pro Ser Gln Ser Val
           275                 280                 285
   Pro Gln Pro Gly His Met Thr Tyr Ile Gln Trp Ser Ala Phe Asp Pro
       290                 295                 300
   Val Phe Phe Leu His His Cys Met Val Asp Arg Ile Phe Ala Leu Trp
   305                 310                 315                 320
   Gln Ala Ile His Pro Asn Thr Trp Val Pro Ser Ser Gln Ala Leu Leu
                   325                 330                 335
   Asp Ser Tyr Thr Ile Arg Arg Gly Gln Ser Ile Asp Ser Gly Thr Ala
               340                 345                 350
   Leu Thr Pro Phe Phe Ser Asn Asp Asn Gly Thr Phe Trp Thr Ser Asp
           355                 360                 365
   Gly Val Arg Asp His Thr Arg Phe Gly Tyr Thr Tyr Ala Glu Leu Leu
       370                 375                 380
   Arg Gly Pro Val Thr Gly Ser Ser Asn Asn Thr Leu Leu Ala Ala Ser
   385                 390                 395                 400
   Gln Ile Arg Ile Val Lys Gln Ala Val Asn Arg Met Tyr Gly Ser Phe
                   405                 410                 415
   Ser Pro Ala Phe Phe Phe Leu Glu Glu Leu Arg Ile Gln Gly Gly Ala
               420                 425                 430
   Ala Val Val Gly Ala Gly Phe Arg Asn Asn His Arg Lys Thr Ala Pro
           435                 440                 445
   Leu His Gly Leu Leu Glu Ser Lys Ile Phe Val Ala Gly Ser Asn Gly
       450                 455                 460
   Asn Arg Tyr Tyr Glu Trp Lys Val Asp Val Cys Val Gly Arg Asn Gly
   465                 470                 475                 480
   Ser Gln Gly Ile Leu Gly Gly Asp Gly Ile Asp Gly Ser Ser Ile Ser
                   485                 490                 495
   Phe Phe Leu Gly Asp Leu Ser Ser Leu Asn Asn Leu Leu Asn Asp Thr
               500                 505                 510
   His Val Gly Thr Met Gly Val Phe Thr Ser Ala Arg Leu Ser Gln His
           515                 520                 525
   Glu Pro Ala Gly Gly His Asp Val Pro Ile Ser Gly Ser Val Pro Leu
       530                 535                 540
   Thr Ala Ala Leu Val Lys Lys Ile Cys Glu Gly Glu Leu Ala Gly Leu
   545                 550                 555                 560
   Ala Ser Gly His Val Val Pro Tyr Leu Lys Arg Asn Leu Lys Met Val
                   565                 570                 575
   Met Arg Gly Thr Gln Arg Glu Val Val Ala Gly Thr Asp Asp Lys Lys
               580                 585                 590
   Ile Cys Glu Thr Leu Leu Ser Leu Arg Ile Val Ser Ser Val Val Glu
   595 600 605
   Ala Pro Trp Ser Glu Asp Glu Leu Pro Lys Trp Gly Glu Glu Arg Val
   610 615 620
   Glu Phe Asp Val Cys
   625
<210> 50
   <211> 651
   <212> PRT
   <213> Myceliophthora thermophila
<400> 50
   Met Ala Arg Cys Gln Met Thr Leu Leu Phe Pro Ile Val Cys Leu Val
   1               5                   10                  15
   Ser Ala Leu Val Ser Ala Gln Tyr Ser Gly Tyr Asp Tyr Gly Phe Asp
               20                  25                  30
   Val Lys Lys Arg Val Lys Arg Gln Leu Gly Gln Arg Ser Ala Met Val
           35                  40                  45
   Val Gln Asp Lys Thr Gly Ser Glu Ile Gln Val Arg Gln Glu Ile Arg
       50                  55                  60
   Gln Leu Glu Gln Asp His Asp Leu Trp Thr Leu Tyr Ile Leu Gly Leu
   65                  70                  75                  80
   Ser Met Leu Gln Tyr Thr Asp Gln Glu Ser Pro Val Ser Tyr Tyr Gly
                   85                  90                  95
   Leu Ala Gly Ile His Gly Met Pro His Gln Thr Trp Gly Gly Met Gly
               100                 105                 110
   Pro Val Thr Gly Asn Glu Asn Thr Gly Tyr Cys Thr His Ser Ser Val
           115                 120                 125
   Leu Phe Pro Thr Trp His Arg Ala Tyr Met Ala Leu Tyr Glu Met Ile
       130                 135                 140
   Ala Thr Phe Trp Pro Asp Ser Glu Arg Gln Arg Tyr Glu Ser Ala Ala
   145                 150                 155                 160
   Arg Arg Phe Arg Leu Pro Tyr Trp Asp Trp Ala Ala Ser Pro Pro Pro
                   165                 170                 175
   Gly Gln Ser Val Leu Pro Glu Ser Ile Gly Gly Ser Pro Phe Ile Asp
               180                 185                 190
   Val Asn Gly Pro Asn Gly Leu Gln Arg Ile Ala Asn Pro Leu Phe Ser
           195                 200                 205
   Tyr Gln Phe Asn Pro Leu Asp Gln Lys Ala Phe Glu Phe Pro Pro Ser
       210                 215                 220
   Gln Trp Asn Ile Trp Thr Arg Thr Leu Arg Ser Pro Ser Ser Gly Gly
   225                 230                 235                 240
   Pro Asp Ala Gln Ser Asn Asn Thr Leu Val Ala Leu Asn Leu Asp Arg
                   245                 250                 255
   Ser Arg Ala Ser Ile Ala Gln Arg Leu Tyr Asp Leu Phe Ser His Asn
               260                 265                 270
   Asp Asn Tyr Thr Leu Phe Ser Asn Asn Ala Val Gly Gly Gln Ala Glu
           275                 280                 285
   Ser Val Glu Ser Leu His Asp Thr Ile His Ser Leu Val Gly Gly Val
       290                 295                 300
   Gly Pro Ser Gln Ser Val Pro Gln Pro Gly His Met Thr Tyr Ile Gln
   305                 310                 315                 320
   Trp Ser Ala Phe Asp Pro Val Phe Phe Leu His His Cys Met Val Asp
                   325                 330                 335
   Arg Ile Phe Ala Leu Trp Gln Ala Ile His Pro Asn Thr Trp Val Pro
               340                 345                 350
   Ser Ser Gln Ala Leu Leu Asp Ser Tyr Thr Ile Arg Arg Gly Gln Ser
           355                 360                 365
   Ile Asp Ser Gly Thr Ala Leu Thr Pro Phe Phe Ser Asn Asp Asn Gly
       370                 375                 380
   Thr Phe Trp Thr Ser Asp Gly Val Arg Asp His Thr Arg Phe Gly Tyr
   385                 390                 395                 400
   Thr Tyr Ala Glu Leu Leu Arg Gly Pro Val Thr Gly Ser Ser Asn Asn
                   405                 410                 415
   Thr Leu Leu Ala Ala Ser Gln Ile Arg Ile Val Lys Gln Ala Val Asn
               420                 425                 430
   Arg Met Tyr Gly Ser Phe Ser Pro Ala Phe Phe Phe Leu Glu Glu Leu
           435                 440                 445
   Arg Ile Gln Gly Gly Ala Ala Val Val Gly Ala Gly Phe Arg Asn Asn
       450                 455                 460
   His Arg Lys Thr Ala Pro Leu His Gly Leu Leu Glu Ser Lys Ile Phe
   465                 470                 475                 480
   Val Ala Gly Ser Asn Gly Asn Arg Tyr Tyr Glu Trp Lys Val Asp Val
                   485                 490                 495
   Cys Val Gly Arg Asn Gly Ser Gln Gly Ile Leu Gly Gly Asp Gly Ile
               500                 505                 510
   Asp Gly Ser Ser Ile Ser Phe Phe Leu Gly Asp Leu Ser Ser Leu Asn
           515                 520                 525
   Asn Leu Leu Asn Asp Thr His Val Gly Thr Met Gly Val Phe Thr Ser
       530                 535                 540
   Ala Arg Leu Ser Gln His Glu Pro Ala Gly Gly His Asp Val Pro Ile
   545                 550                 555                 560
   Ser Gly Ser Val Pro Leu Thr Ala Ala Leu Val Lys Lys Ile Cys Glu
                   565                 570                 575
   Gly Glu Leu Ala Gly Leu Ala Ser Gly His Val Val Pro Tyr Leu Lys
               580                 585                 590
   Arg Asn Leu Lys Met Val Met Arg Gly Thr Gln Arg Glu Val Val Ala
           595                 600                 605
   Gly Thr Asp Asp Lys Lys Ile Cys Glu Thr Leu Leu Ser Leu Arg Ile
       610                 615                 620
   Val Ser Ser Val Val Glu Ala Pro Trp Ser Glu Asp Glu Leu Pro Lys
   625                 630                 635                 640
   Trp Gly Glu Glu Arg Val Glu Phe Asp Val Cys
                   645                 650
<210> 51
   <211> 1956
   <212> DNA
   <213> Artificial
<220>
   <223> cDNA encoding PPO1
<400> 51
   atggcgcgct gtcaaatgac tctcctcttc ccgatcgtct gcctcgtcag cgctctcgtt 60
   tcagcccaat attcaggtta tgactatgga tttgacgtca agaagcgggt caagcgccag 120
   ctcggccaac gttcggccat ggtcgtgcag gacaagaccg gcagcgagat ccaggtgcgc 180
   caggaaattc gtcaacttga gcaggaccat gacctctgga ccctgtacat cctaggcttg 240
   agcatgctgc agtataccga ccaggaatct cccgtgtctt attatggact cgcggggata 300
   cacggaatgc ctcaccagac ttggggcggc atgggaccag tcaccggaaa cgagaatacc 360
   gggtattgta cccattcgtc cgtcctgttc ccgacatggc accgggcata catggctcta 420
   tacgagatga tcgccacgtt ctggcccgac agcgagagac agcgctacga gagtgccgcc 480
   cgcaggttcc gcctccctta ctgggactgg gcggcaagcc ctcctccggg acaaagcgtt 540
   ctccctgaga gcatcggagg aagtccattt attgacgtca acggaccgaa tggcttgcag 600
   cggatcgcga accccttatt cagctaccag ttcaaccctc tcgatcagaa ggcttttgag 660
   ttccccccct cacagtggaa catctggact cgaactctta gaagtccatc aagtggcggc 720
   cctgacgcgc agtccaacaa cacgctcgtc gccctgaacc tcgatcggag ccgcgcctcc 780
   atcgctcagc gcctctatga tttattttcg cacaacgata actacactct gttcagcaac 840
   aatgccgtgg ggggacaggc tgaatcggta gaatcgctgc acgacaccat ccatagctta 900
   gttggcggcg tcgggcctag tcagtcagta cctcagcctg ggcacatgac gtacatccag 960
   tggtcggcct tcgatcccgt cttcttcctg catcactgca tggttgaccg gattttcgcc 1020
   ttgtggcagg cgattcaccc caacacttgg gttccctcgt ctcaagctct gctggactcg 1080
   tacaccattc ggaggggcca gtccatagac tctggtacag cactcactcc cttcttttcc 1140
   aacgacaacg gcacattctg gacttctgac ggcgtgcgcg accacacaag gttcggctat 1200
   acgtacgccg agttgctgcg cggtcccgtc accggctctt caaacaatac cctcttagcc 1260
   gcatctcaaa tacgcatagt aaaacaggcc gtaaaccgca tgtatggctc tttcagcccg 1320
   gccttcttct ttctcgaaga gcttcgcatt caaggcggcg ctgccgtggt aggcgccggc 1380
   ttccgaaaca accacaggaa aacggcgccg ctccatggtc tactggaatc caaaatcttt 1440
   gttgcgggga gcaacggcaa ccgctactac gagtggaagg tagatgtgtg cgtagggcgc 1500
   aacggcagcc aaggtattct cggcggcgac ggaattgacg ggtcttcaat cagtttcttc 1560
   cttggcgacc tttcttctct taacaatttg ttgaatgaca cccatgttgg gaccatgggc 1620
   gtgtttacct ccgctcgctt gtcgcaacat gaaccggctg gtgggcatga tgtgcctatc 1680
   tcagggagcg tgcctctgac ggccgcgctg gtgaagaaga tctgcgaagg cgagctcgcg 1740
   gggttggctt caggacatgt ggtgccgtac ttgaagagaa acctgaagat ggtgatgcga 1800
   gggacccaga gagaggtggt ggcgggcaca gacgataaga agatttgtga aacactcttg 1860
   agtcttcgga tcgttagctc ggtggttgag gcgccgtgga gcgaggatga attgccgaaa 1920
   tggggagagg aaagagtaga gtttgatgtg tgctga 1956
<210> 52
   <211> 2160
   <212> DNA
   <213> Myceliophthora thermophila
<400> 52
   atggcgcgct gtcaaatgac tctcctcttc ccgatcgtct gcctcgtcag cgctctcgtt     60
   tcagcccaat attcaggtta tgactatgga tttgacgtca agaagcgggt caagcgccag    120
   ctcggccaac gttcggccat ggtcgtgcag gacaagaccg gcagcgagat ccaggtgcgc    180
   caggaaattc gtcaacttga gcaggaccat gacctctgga ccctgtacat cctaggcttg    240
   agcatgctgc agtataccga ccaggaatct cccgtgtctt attatggact cgcgggtttg    300
   tgttgccttg ttcgaatgtg tttcggccag tgatgctaac gagatgacgg tctaacccag    360
   ggatacacgg aatgcctcac cagacttggg gcggcatggg accagtcacc ggaaacgaga    420
   ataccgggta ttgtacccat tcgtccgtcc tgttcccgac atggcaccgg gcatacatgg    480
   ctctatacga ggtatgacag ccttcttgtc atagggtgag aaacgcggtt attaggttcc    540
   cgtatcgtat ctgacttcag ctcagcaagt tttgcacgat ctaatccaga tgatcgccac    600
   gttctggccc gacagcgaga gacagcgcta cgagagtgcc gcccgcaggt tccgcctccc    660
   ttactgggac tgggcggcaa gccctcctcc gggacaaagc gttctccctg agagcatcgg    720
   aggaagtcca tttattgacg tcaacggacc gaatggcttg cagcggatcg cgaacccctt    780
   attcagctac cagttcaacc ctctcgatca gaaggctttt gagttccccc cctgtaagtt    840
   tgggtacatt caaacttgac ttgatctgac gaagcacagt ggaacatctg gactcgaact    900
   cttagaagtc catcaagtgg cggccctgac gcgcagtcca acaacacgct cgtcgccctg    960
   aacctcgatc ggagccgcgc ctccatcgct cagcgcctct atgatttatt ttcgcacaac   1020
   gataactaca ctctgttcag caacaatgcc gtggggggac aggctgaatc ggtagaatcg   1080
   ctgcacgaca ccatccatag cttagttggc ggcgtcgggc ctagtcagtc agtacctcag   1140
   cctgggcaca tgacgtacat ccagtggtcg gccttcgatc ccgtcttctt cctgcatcac   1200
   tgcatggttg accggatttt cgccttgtgg caggcgattc accccaacac ttgggttccc   1260
   tcgtctcaag ctctgctgga ctcgtacacc attcggaggg gccagtccat agactctggt   1320
   acagcactca ctcccttctt ttccaacgac aacggcacat tctggacttc tgacggcgtg   1380
   cgcgaccaca caaggttcgg ctatacgtac gccgagttgc tgcgcggtcc cgtcaccggc   1440
   tcttcaaaca ataccctctt agccgcatct caaatacgca tagtaaaaca ggccgtaaac   1500
   cgcatgtatg gctctttcag cccggccttc ttctttctcg aagagcttcg cattcaaggc   1560
   ggcgctgccg tggtaggcgc cggcttccga aacaaccaca ggaaaacggc gccgctccat   1620
   ggtctactgg aatccaaaat ctttgttgcg gggagcaacg gcaaccgcta ctacgagtgg   1680
   aaggtagatg tgtgcgtagg gcgcaacggc agccaaggta ttctcggcgg cgacggaatt   1740
   gacgggtctt caatcagttt cttccttggc gacctttctt ctcttaacaa tttgttgaat   1800
   gacacccatg ttgggaccat gggcgtgttt acctccgctc gcttgtcgca acatgaaccg   1860
   gctggtgggc atgatgtgcc tatctcaggg agcgtgcctc tgacggccgc gctggtgaag   1920
   aagatctgcg aaggcgagct cgcggggttg gcttcaggac atgtggtgcc gtacttgaag   1980
   agaaacctga agatggtgat gcgagggacc cagagagagg tggtggcggg cacagacgat   2040
   aagaagattt gtgaaacact cttgagtctt cggatcgtta gctcggtggt tgaggcgccg   2100
   tggagcgagg atgaattgcc gaaatgggga gaggaaagag tagagtttga tgtgtgctga   2160
<210> 53
   <211> 368
   <212> PRT
   <213> Myceliophthora thermophila
<400> 53
   Ala Pro Leu Gly Asn Val Asp Ala Glu Ala Ala Ala Ser Gln Arg Ile
   1               5                   10                  15
   Leu Glu Leu Gln Ala Gln Tyr Gln Arg Asn Val Leu Asp Ala Ile Ser
               20                  25                  30
   Asn Arg Thr Ser Gly Cys Thr Ala Gln Asn Ile Gln Arg Arg Gln Glu
           35                  40                  45
   Trp Ser Thr Leu Ser Leu Ala Asp Arg Ala Ala Phe Ile Ser Ala Ile
       50                  55                  60
   His Cys Leu Asn Ala Leu Pro Ala Arg Thr Pro Gln Ser Thr Ala Pro
   65                  70                  75                  80
   Gly Ala Arg Ala Leu Tyr Asp Asp Phe Ile Val Ala His Ile Leu Gln
                   85                  90                  95
   Thr Pro Phe Val His Ala Ser Gly Leu Phe Leu Pro Phe His Arg His
               100                 105                 110
   Leu Leu His Leu Phe Ala Gly Ala Leu Arg Asp Arg Cys Asp Tyr Ala
           115                 120                 125
   Gly Pro Leu Pro Tyr Trp Asp Trp Thr Ala Ser Tyr Ala Asp Pro Arg
       130                 135                 140
   Ala Ala Ala Val Phe Asp Gly Gly Pro His Ser Leu Gly Gly Asn Gly
   145                 150                 155                 160
   Ala Phe Val Pro Gly Arg Asn Gly Thr Val Ile Ser Val Pro Gly Gly
                   165                 170                 175
   Ala His Val Val Ile Pro Pro Ala Thr Gly Gly Gly Cys Val Thr Thr
               180                 185                 190
   Gly Pro Phe Arg Gln Gly Arg Phe Glu Val Arg Leu Gly Pro Val Ala
           195                 200                 205
   Phe Glu Pro Arg Gly Pro His Gly Gly Leu Gly Tyr Asn Pro Arg Cys
       210                 215                 220
   Leu Arg Arg Asp Leu Ser Pro His Phe Ser Leu Gly Thr Arg Pro Gly
   225                 230                 235                 240
   Ala Val Val Ala Leu Leu Asp Gly Cys Ala Gly Asp Leu Gly Cys Leu
                   245                 250                 255
   Val Arg Asp Met Asp Ala Pro Gly Gly Val Pro Gly Gly Val His Ala
               260                 265                 270
   Ser Gly His Trp Gln Val Gly Pro Asp Ala Leu Asp Val Phe Ala Ser
           275                 280                 285
   Pro Ser Asp Pro Val Phe Trp Leu His His Ala Gln Val Asp Arg Val
       290                 295                 300
   Trp Thr Ile Trp Gln Gly Leu Asp Leu Glu Gly Arg Thr Tyr Gln Val
   305                 310                 315                 320
   Trp Gly Thr Ser Thr Ala Ala Asn Asp Pro Pro Ser Asp Asp Val Thr
                   325                 330                 335
   Leu Glu Thr Ala Met Asp Phe Gly Ile Leu Gly Glu Ala Lys Thr Val
               340                 345                 350
   Gly Glu Val Ala Ser Thr Val Asp Gly Glu Tyr Cys Tyr Met Tyr Glu
           355                 360                 365
<210> 54
   <211> 390
   <212> PRT
   <213> Myceliophthora thermophila
<400> 54
   Met Lys Leu Ser Thr Leu Pro Ala Ala Leu Leu Ser Leu Ile Leu Leu
   1 5 10 15
   Ala His Gln Ala Ala Ala Ala Pro Leu Gly Asn Val Asp Ala Glu Ala
   20 25 30
   Ala Ala Ser Gln Arg Ile Leu Glu Leu Gln Ala Gln Tyr Gln Arg Asn
   35 40 45
   Val Leu Asp Ala Ile Ser Asn Arg Thr Ser Gly Cys Thr Ala Gln Asn
   50 55 60
   Ile Gln Arg Arg Gln Glu Trp Ser Thr Leu Ser Leu Ala Asp Arg Ala
   65 70 75 80
   Ala Phe Ile Ser Ala Ile His Cys Leu Asn Ala Leu Pro Ala Arg Thr
   85 90 95
   Pro Gln Ser Thr Ala Pro Gly Ala Arg Ala Leu Tyr Asp Asp Phe Ile
   100 105 110
   Val Ala His Ile Leu Gln Thr Pro Phe Val His Ala Ser Gly Leu Phe
   115 120 125
   Leu Pro Phe His Arg His Leu Leu His Leu Phe Ala Gly Ala Leu Arg
   130 135 140
   Asp Arg Cys Asp Tyr Ala Gly Pro Leu Pro Tyr Trp Asp Trp Thr Ala
   145 150 155 160
   Ser Tyr Ala Asp Pro Arg Ala Ala Ala Val Phe Asp Gly Gly Pro His
   165 170 175
   Ser Leu Gly Gly Asn Gly Ala Phe Val Pro Gly Arg Asn Gly Thr Val
   180 185 190
   Ile Ser Val Pro Gly Gly Ala His Val Val Ile Pro Pro Ala Thr Gly
   195 200 205
   Gly Gly Cys Val Thr Thr Gly Pro Phe Arg Gln Gly Arg Phe Glu Val
   210 215 220
   Arg Leu Gly Pro Val Ala Phe Glu Pro Arg Gly Pro His Gly Gly Leu
   225 230 235 240
   Gly Tyr Asn Pro Arg Cys Leu Arg Arg Asp Leu Ser Pro His Phe Ser
                   245                 250                 255
   Leu Gly Thr Arg Pro Gly Ala Val Val Ala Leu Leu Asp Gly Cys Ala
               260                 265                 270
   Gly Asp Leu Gly Cys Leu Val Arg Asp Met Asp Ala Pro Gly Gly Val
           275                 280                 285
   Pro Gly Gly Val His Ala Ser Gly His Trp Gln Val Gly Pro Asp Ala
       290                 295                 300
   Leu Asp Val Phe Ala Ser Pro Ser Asp Pro Val Phe Trp Leu His His
   305                 310                 315                 320
   Ala Gln Val Asp Arg Val Trp Thr Ile Trp Gln Gly Leu Asp Leu Glu
                   325                 330                 335
   Gly Arg Thr Tyr Gln Val Trp Gly Thr Ser Thr Ala Ala Asn Asp Pro
               340                 345                 350
   Pro Ser Asp Asp Val Thr Leu Glu Thr Ala Met Asp Phe Gly Ile Leu
           355                 360                 365
   Gly Glu Ala Lys Thr Val Gly Glu Val Ala Ser Thr Val Asp Gly Glu
       370                 375                 380
   Tyr Cys Tyr Met Tyr Glu
   385                 390
<210> 55
   <211> 1173
   <212> DNA
   <213> Artificial
<220>
   <223> cDNA encoding PPO3
<400> 55
   atgaagctct caacactacc agcagcactc ctgtccttga ttctcctggc tcaccaggcc     60
   gccgccgccc cgctcggcaa cgtcgacgct gaagcggccg catcccagag aatcctcgag    120
   ctgcaagctc agtaccagcg aaacgttcta gacgccatca gcaaccgcac ctcaggctgc    180
   acagcccaaa atatccagcg ccgccaggaa tggagcaccc tctccctggc tgaccgcgcc    240
   gctttcatct cggcgatcca ctgcctcaac gcgctgccgg cgcggacgcc gcagtccacc    300
   gccccgggcg cccgggcgct ctacgacgac ttcatcgtcg cccacatcct acagactccc    360
   ttcgtccacg cgtcgggcct cttcctgccc ttccaccgcc accttctgca cctgttcgca    420
   ggcgcgctgc gcgaccgctg cgactacgcc ggcccgctgc cctactggga ctggacggcg    480
   agctacgccg acccgcgcgc ggcggccgtc ttcgacggcg ggccgcactc gctcggcggc    540
   aacggcgcct tcgtgcccgg ccgcaacggc accgtcatct ccgtccccgg cggcgcgcac    600
   gtggtcatcc cgcccgcgac cggcggcggg tgcgtcacga cggggccgtt ccgccagggc    660
   cgcttcgagg tgcgcctcgg ccccgtcgcg ttcgagcccc gcggcccgca cggagggctg    720
   ggctacaacc cgcggtgcct ccggcgggac ctgagcccgc acttcagcct cggcacgcgc    780
   cccggcgcgg tcgtggcctt gctggacggc tgcgccggcg acctcggctg cctcgtgcgc    840
   gacatggacg cccccggggg cgtgcccggg ggcgtgcacg cgtccgggca ctggcaggtc    900
   gggccggacg cgctcgacgt cttcgccagc ccgagcgacc ccgtcttttg gctgcaccac    960
   gcccaggtcg accgcgtctg gaccatctgg cagggactgg acctcgaggg gaggacgtat   1020
   caggtctggg gcacgagcac ggcggcgaac gatccgccaa gtgacgacgt aacgctcgag   1080
   acggcaatgg actttggcat tcttggtgag gcaaagacgg tcggtgaggt tgcctcgacg   1140
   gtggacggag aatattgcta catgtatgag tga                                1173
<210> 56
   <211> 1348
   <212> DNA
   <213> Myceliophthora thermophila
<400> 56
   atgaagctct caacactacc agcagcactc ctgtccttga ttctcctggc tcaccaggcc      60
   gccgccgccc cgctcggcaa cgtcgacgct gaagcggccg catcccagag aatcctcgag     120
   ctgcaagctc agtaccagcg aaacgttcta gacgccatca gcaaccgcac ctcaggctgc     180
   acagcccaaa atatccagcg ccgccaggaa tggtacgttc tccctttccc tcattcttcc     240
   cttcccttcg actccggaca aaacgccaga gccccagtgt aagtacctaa acagatcctt     300
   caccgcgcag gagcaccctc tccctggctg accgcgccgc tttcatctcg gcgatccact     360
   gcctcaacgc gctgccggcg cggacgccgc agtccaccgc cccgggcgcc cgggcgctct     420
   acgacgactt catcgtcgcc cacatcctac agactccctt cgtccacgcg tcgggcctct     480
   tcctgccctt ccaccgccac cttctgcacc tgttcgcagg cgcgctgcgc gaccgctgcg     540
   actacgccgg cccgctgccc tactgggact ggacggcgag ctacgccgac ccgcgcgcgg     600
   cggccgtctt cgacggcggg ccgcactcgc tcggcggcaa cggcgccttc gtgcccggcc     660
   gcaacggcac cgtcatctcc gtccccggcg gcgcgcacgt ggtcatcccg cccgcgaccg     720
   gcggcgggtg cgtcacgacg gggccgttcc gccagggccg cttcgaggtg cgcctcggcc     780
   ccgtcgcgtt cgagccccgc ggcccgcacg gagggctggg ctacaacccg cggtgcctcc     840
   ggcgggacct gagcccgcac ttcagcctcg gcacgcgccc cggcgcggtc gtggccttgc     900
   tggacggctg cgccggcgac ctcggctgcc tcgtgcgcga catggacgcc cccgggggcg     960
   tgcccggggg cgtgcacgcg tccgggcact ggcaggtcgg gccggacgcg ctcgacgtct    1020
   tcgccagccc gagcgacccc gtcttttggc tgcaccacgc ccaggtcgac cgcgtctgga    1080
   ccatctggca gggactggac ctcgagggga ggacgtatca ggtctggggc acgagcacgg    1140
   cggcgaacgg tgagttccct tcccttccct tttttttttt tttttttttt cgttcccttt    1200
   gaacggatga tgctgacctg cttcagatcc gccaagtgac gacgtaacgc tcgagacggc    1260
   aatggacttt ggcattcttg gtgaggcaaa gacggtcggt gaggttgcct cgacggtgga    1320
   cggagaatat tgctacatgt atgagtga                                       1348
<210> 57
   <211> 365
   <212> PRT
   <213> Myceliophthora thermophila
<400> 57
   Thr Phe Asn Ile Pro Ala Phe Glu Gln Ser Ala Ile Asp Ser Gly Leu
   1               5                   10                  15
   Ala Leu Ala Gly Leu Asn Gly Ile Ala Leu Leu Gln Ser Ala Thr Arg
               20                  25                  30
   Tyr Gly Gly Ser Cys Asn Leu Gly Asn Val Lys Ile Arg Arg Glu Trp
           35                  40                  45
   Arg Thr Leu Ser Lys Ala Gln Arg Lys Ser Tyr Ile Lys Ala Val Gln
       50                  55                  60
   Cys Val Gln Ala Lys Pro Ser Arg Leu Ala Glu Gly Ile Ala Ala Gly
   65                  70                  75                  80
   Ser Lys Thr Leu Phe Asp Asp Phe Val Tyr Val His Met Asn Ala Thr
                   85                  90                  95
   Leu Phe Ile His Tyr Thr Gly Asn Phe Leu Ile Trp His Arg Asn Phe
               100                 105                 110
   Ile His Val Tyr Glu Lys Glu Leu Asn Ala Cys Gly Tyr Lys Asp Ala
           115                 120                 125
   Leu Pro Tyr Trp Glu Trp Gly Phe Asp Val Glu Ala Pro His Leu Ser
       130                 135                 140
   Pro Val Phe Asp Gly Ser Asp Thr Ser Met Ser Gly Asp Gly Glu Phe
   145                 150                 155                 160
   Val Pro Gly Pro Pro Leu Glu Leu Leu Val Pro Gly Asn Ala Glu Pro
   165 170 175
   Val Val Leu Ala Lys Gly Thr Gly Gly Gly Cys Val Lys Thr Gly Pro
   180 185 190
   Phe Ala Asn Met Thr Val Arg Leu Gly Pro Ile Thr Gln Pro Asp Pro
   195 200 205
   Thr Ala Asp Asn Pro Arg Cys Leu Lys Arg Asp Leu Asn Ala Asp Ala
   210 215 220
   Gly Lys Arg Phe Ser Ser Phe Arg Asn Thr Thr Asn Leu Ile Ile Asn
   225 230 235 240
   Ser Pro Thr Ile Glu Tyr Phe Arg Thr Thr Met Glu Gly His Pro Asn
   245 250 255
   Tyr Val Pro Asn Ser Leu Gly Val His Gly Gly Gly His Phe Met Ile
   260 265 270
   Ser Gly Asp Pro Gly Ala Asp Ala Phe Ile Ser Pro Gly Asp Pro Ala
   275 280 285
   Phe Tyr Leu His His Ala Gln Ile Asp Arg Val Tyr Trp Ile Trp Gln
   290 295 300
   Met Leu Asp Phe Glu Asn Arg Gln Gly Val Phe Gly Thr Asn Thr Met
   305 310 315 320
   Leu Asn Tyr Pro Pro Ser Glu Asn Thr Thr Val Glu Asp Thr Val Asp
   325 330 335
   Val Gly His Leu Gly Gly Pro Ile Lys Ile Lys Asn Leu Met Ser Thr
   340 345 350
   Thr Gly Gln Asn Gly Ser Pro Leu Cys Tyr Ile Tyr Leu
   355 360 365
<210> 58
   <211> 382
   <212> PRT
   <213> Myceliophthora thermophila
<400> 58
   Met Lys Leu Ser Ala Ser Leu Ala Leu Ala Ala Ala Met Gly Ala Ser
   1 5 10 15
   Ala Thr Phe Asn Ile Pro Ala Phe Glu Gln Ser Ala Ile Asp Ser Gly
               20                  25                  30
   Leu Ala Leu Ala Gly Leu Asn Gly Ile Ala Leu Leu Gln Ser Ala Thr
           35                  40                  45
   Arg Tyr Gly Gly Ser Cys Asn Leu Gly Asn Val Lys Ile Arg Arg Glu
       50                  55                  60
   Trp Arg Thr Leu Ser Lys Ala Gln Arg Lys Ser Tyr Ile Lys Ala Val
   65                  70                  75                  80
   Gln Cys Val Gln Ala Lys Pro Ser Arg Leu Ala Glu Gly Ile Ala Ala
                   85                  90                  95
   Gly Ser Lys Thr Leu Phe Asp Asp Phe Val Tyr Val His Met Asn Ala
               100                 105                 110
   Thr Leu Phe Ile His Tyr Thr Gly Asn Phe Leu Ile Trp His Arg Asn
           115                 120                 125
   Phe Ile His Val Tyr Glu Lys Glu Leu Asn Ala Cys Gly Tyr Lys Asp
       130                 135                 140
   Ala Leu Pro Tyr Trp Glu Trp Gly Phe Asp Val Glu Ala Pro His Leu
   145                 150                 155                 160
   Ser Pro Val Phe Asp Gly Ser Asp Thr Ser Met Ser Gly Asp Gly Glu
                   165                 170                 175
   Phe Val Pro Gly Pro Pro Leu Glu Leu Leu Val Pro Gly Asn Ala Glu
               180                 185                 190
   Pro Val Val Leu Ala Lys Gly Thr Gly Gly Gly Cys Val Lys Thr Gly
           195                 200                 205
   Pro Phe Ala Asn Met Thr Val Arg Leu Gly Pro Ile Thr Gln Pro Asp
       210                 215                 220
   Pro Thr Ala Asp Asn Pro Arg Cys Leu Lys Arg Asp Leu Asn Ala Asp
   225                 230                 235                 240
   Ala Gly Lys Arg Phe Ser Ser Phe Arg Asn Thr Thr Asn Leu Ile Ile
                   245                 250                 255
   Asn Ser Pro Thr Ile Glu Tyr Phe Arg Thr Thr Met Glu Gly His Pro
               260                 265                 270
   Asn Tyr Val Pro Asn Ser Leu Gly Val His Gly Gly Gly His Phe Met
           275                 280                 285
   Ile Ser Gly Asp Pro Gly Ala Asp Ala Phe Ile Ser Pro Gly Asp Pro
       290                 295                 300
   Ala Phe Tyr Leu His His Ala Gln Ile Asp Arg Val Tyr Trp Ile Trp
   305                 310                 315                 320
   Gln Met Leu Asp Phe Glu Asn Arg Gln Gly Val Phe Gly Thr Asn Thr
                   325                 330                 335
   Met Leu Asn Tyr Pro Pro Ser Glu Asn Thr Thr Val Glu Asp Thr Val
               340                 345                 350
   Asp Val Gly His Leu Gly Gly Pro Ile Lys Ile Lys Asn Leu Met Ser
           355                 360                 365
   Thr Thr Gly Gln Asn Gly Ser Pro Leu Cys Tyr Ile Tyr Leu
       370                 375                 380
<210> 59
   <211> 1149
   <212> DNA
   <213> Artificial
<220>
   <223> cDNA encoding PPO8
<400> 59
   atgaagctct cggcgtctct ggccttggct gcggccatgg gggcctcggc caccttcaac      60
   attcccgcct ttgagcagtc cgctatcgac tccggcctgg ctcttgctgg tctgaacggc     120
   atcgccctgc tgcagtcagc caccaggtac ggcgggtcgt gcaatcttgg gaacgtcaag     180
   atccgccgag aatggaggac gctgtccaaa gcccagcgga agagttacat caaggcggtg     240
   cagtgcgtcc aagctaagcc cagcagactg gccgagggta ttgccgctgg gtccaagacg     300
   ctcttcgatg actttgttta cgtacacatg aatgcgaccc tcttcatcca ctacaccggc     360
   aacttcctca tctggcaccg caacttcatc cacgtgtacg agaaggagct taacgcctgt     420
   ggctacaaag atgccctccc ctattgggaa tggggcttcg atgtcgaggc gccacacctt     480
   tcacctgtct ttgacgggtc tgatacatcg atgagtggcg atggcgagtt cgtccctggc     540
   cccccacttg agctcctggt tcccggcaac gccgagcccg tggtactggc gaagggtact     600
   ggtggcggct gtgtcaagac gggcccgttt gccaacatga cggtccggtt gggcccaatc     660
   acgcagccgg acccgaccgc ggacaacccg cgctgcctga agcgcgacct gaacgccgat     720
   gccggcaagc gcttctctag cttccgcaac acaacgaacc tcattatcaa ctcgcccacg     780
   atcgagtatt tccggaccac catggagggc caccccaact atgtgcccaa ctcgctgggg     840
   gtgcacggcg gcggccactt catgatcagc ggcgacccgg gcgcggatgc cttcatctcg     900
   cccggcgacc ccgcgttcta ccttcatcat gcccaaatcg atcgcgtcta ctggatctgg     960
   cagatgctgg attttgagaa ccgccagggc gtcttcggca ccaacactat gctcaactac    1020
   cctcccagcg agaacaccac cgtcgaggac accgtcgatg tgggccacct cggcggccca    1080
   atcaagatca agaacctcat gagcactacc gggcaaaatg gctctcctct ttgctacatt    1140
   tatctttag                                                            1149
<210> 60
   <211> 1345
   <212> DNA
   <213> Myceliophthora thermophila
<400> 60
   atgaagctct cggcgtctct ggccttggct gcggccatgg gggcctcggc caccttcaac      60
   attcccgcct ttgagcagtc cgctatcgac tccggcctgg ctcttgctgg tctgaacggc     120
   atcgccctgc tgcagtcagc caccaggtac ggcgggtcgt gcaatcttgg gaacgtcaag     180
   atccgccgag aatggtatgt cgatcacccg tcgaccctga tctcctgcat tccgcaacag     240
   ctccgtgttg acccggggat taggaggacg ctgtccaaag cccagcggaa gagttacatc     300
   aaggcggtgc agtgcgtcca agctaagccc agcagactgg ccgagggtat tgccgctggg     360
   tccaagacgc tcttcgatga ctttgtttac gtacacatga atgcgaccct cttcatccac     420
   tacaccgtga gcatccgccg atagttcgag gagtcctatt gctaccgaga cccccggctt     480
   acccttccta cagggcaact tcctcatctg gcaccgcaac ttcatccacg tgtacgagaa     540
   ggagcttaac gcctgtggct acaaaggtat tacgtgttct tgaaggtcgc tatgtccgat     600
   gccacgtagc tgatcaagga atgcagatgc cctcccctat tgggaatggg gcttcgatgt     660
   cgaggcgcca cacctttcac ctgtctttga cgggtctgat acatcgatga gtggcgatgg     720
   cgagttcgtc cctggccccc cacttgagct cctggttccc ggcaacgccg agcccgtggt     780
   actggcgaag ggtactggtg gcggctgtgt caagacgggc ccgtttgcca acatgacggt     840
   ccggttgggc ccaatcacgc agccggaccc gaccgcggac aacccgcgct gcctgaagcg     900
   cgacctgaac gccgatgccg gcaagcgctt ctctagcttc cgcaacacaa cgaacctcat     960
   tatcaactcg cccacgatcg agtatttccg gaccaccatg gagggccacc ccaactatgt    1020
   gcccaactcg ctgggggtgc acggcggcgg ccacttcatg atcagcggcg acccgggcgc    1080
   ggatgccttc atctcgcccg gcgaccccgc gttctacctt catcatgccc aaatcgatcg    1140
   cgtctactgg atctggcaga tgctggattt tgagaaccgc cagggcgtct tcggcaccaa    1200
   cactatgctc aactaccctc ccagcgagaa caccaccgtc gaggacaccg tcgatgtggg    1260
   ccacctcggc ggcccaatca agatcaagaa cctcatgagc actaccgggc aaaatggctc    1320
   tcctctttgc tacatttatc tttag                                          1345
<210> 61
   <211> 373
   <212> PRT
   <213> Myceliophthora thermophila
<400> 61
   Ser Val Pro Ala Ala Gly Asn Leu Asp Ile Val Lys Asn Leu Ala His
   1               5                   10                  15
   Arg Met Gly Glu Glu Asp Met Asp Ala Phe Pro Val Leu Ser Trp Asp
               20                  25                  30
   Glu Ala Val Ala Ile Met Thr Gly Asn Asp Glu Gly Gly Gly Ser Ser
           35                  40                  45
   Ser Ser Ser Ser Ser Gly Ser Gly Arg Gly Pro Asp Asn Asn Arg Val
       50                  55                  60
   Ser Val Asn Asn Tyr Ser Asn Lys Thr Lys Pro Ala Tyr Pro Pro Ala
   65                  70                  75                  80
   Tyr Val Pro His Ala Ser Ser Cys Gly Lys Pro Lys Val Arg Val Glu
                   85                  90                  95
   Trp Lys Lys Leu Lys Glu Ser Glu Lys Phe Ala Tyr Leu Gly Ala Val
               100                 105                 110
   Lys Cys Leu Met Asp Thr Lys Pro Val Gly Val Trp Ser Lys Ala Thr
           115                 120                 125
   Ser Ile Trp Asp Glu Met Ala Trp Ala His Asn Glu Ala Lys Trp Asn
       130                 135                 140
   Ile His Glu Thr Asp Asn Phe Leu Pro Trp His Arg Tyr Tyr Ile Tyr
   145                 150                 155                 160
   Met Leu Glu Thr Leu Leu Ser Lys His Cys Ser Tyr Arg Gly Pro Ile
                   165                 170                 175
   Pro Trp Trp Arg Glu Thr Asp Asp Thr Gly Asn Leu Ala Gly Ala Asn
               180                 185                 190
   Leu Phe Ser Pro Asn Tyr Phe Gly Ser Leu Pro Pro Arg Thr Glu Asp
           195                 200                 205
   Gly Lys Thr Thr Cys Ile Thr Asp Gly Trp Phe Ala Asn Thr Thr Val
       210                 215                 220
   Leu Leu Gly Pro Gly Ala Pro Thr Cys Leu Ala Arg Gly Glu Asp Lys
   225                 230                 235                 240
   Arg Ile Ala Ala Glu Val Thr Thr Ala Ala Leu Asp Leu Cys Gln Gly
                   245                 250                 255
   Asp Arg Asp Thr Lys Tyr Glu Arg His Arg Arg Cys Val Glu Ala Thr
               260                 265                 270
   Ile His Ser Ser Thr His Arg Ala Val Gly Gly Ala Met Glu Ser Ile
           275                 280                 285
   Ser Ala Ser Pro Ser Asp Pro Val Phe Tyr Leu His His Gly Phe Val
       290                 295                 300
   Asp Trp Gln Trp Ala Arg Trp Gln Asn Val Glu Ser Ser Arg Lys Thr
   305                 310                 315                 320
   Thr Ile Ser Gly Cys Ser Glu Pro Ser Pro Val Pro Gly Lys Cys Val
                   325                 330                 335
   Glu Leu Thr Leu Asp Thr Val Leu Lys Gly Tyr Gly Leu Ile Pro Asp
               340                 345                 350
   Met Lys Val Arg Asp Val Leu Asp Ile Glu Asn Gly Ile Leu Cys Tyr
           355                 360                 365
   Thr Tyr Asp Glu Phe
       370
<210> 62
   <211> 392
   <212> PRT
   <213> Myceliophthora thermophila
<400> 62
   Met Arg Leu Thr Arg Tyr Ala Pro Val Leu Ser Ala Ala Val Leu Ala
   1               5                   10                  15
   Ala Ala Ala Ser Val Pro Ala Ala Gly Asn Leu Asp Ile Val Lys Asn
               20                  25                  30
   Leu Ala His Arg Met Gly Glu Glu Asp Met Asp Ala Phe Pro Val Leu
           35                  40                  45
   Ser Trp Asp Glu Ala Val Ala Ile Met Thr Gly Asn Asp Glu Gly Gly
       50                  55                  60
   Gly Ser Ser Ser Ser Ser Ser Ser Gly Ser Gly Arg Gly Pro Asp Asn
   65                  70                  75                  80
   Asn Arg Val Ser Val Asn Asn Tyr Ser Asn Lys Thr Lys Pro Ala Tyr
                   85                  90                  95
   Pro Pro Ala Tyr Val Pro His Ala Ser Ser Cys Gly Lys Pro Lys Val
               100                 105                 110
   Arg Val Glu Trp Lys Lys Leu Lys Glu Ser Glu Lys Phe Ala Tyr Leu
           115                 120                 125
   Gly Ala Val Lys Cys Leu Met Asp Thr Lys Pro Val Gly Val Trp Ser
       130                 135                 140
   Lys Ala Thr Ser Ile Trp Asp Glu Met Ala Trp Ala His Asn Glu Ala
   145                 150                 155                 160
   Lys Trp Asn Ile His Glu Thr Asp Asn Phe Leu Pro Trp His Arg Tyr
                   165                 170                 175
   Tyr Ile Tyr Met Leu Glu Thr Leu Leu Ser Lys His Cys Ser Tyr Arg
               180                 185                 190
   Gly Pro Ile Pro Trp Trp Arg Glu Thr Asp Asp Thr Gly Asn Leu Ala
           195                 200                 205
   Gly Ala Asn Leu Phe Ser Pro Asn Tyr Phe Gly Ser Leu Pro Pro Arg
       210                 215                 220
   Thr Glu Asp Gly Lys Thr Thr Cys Ile Thr Asp Gly Trp Phe Ala Asn
   225                 230                 235                 240
   Thr Thr Val Leu Leu Gly Pro Gly Ala Pro Thr Cys Leu Ala Arg Gly
                   245                 250                 255
   Glu Asp Lys Arg Ile Ala Ala Glu Val Thr Thr Ala Ala Leu Asp Leu
               260                 265                 270
   Cys Gln Gly Asp Arg Asp Thr Lys Tyr Glu Arg His Arg Arg Cys Val
           275                 280                 285
   Glu Ala Thr Ile His Ser Ser Thr His Arg Ala Val Gly Gly Ala Met
       290                 295                 300
   Glu Ser Ile Ser Ala Ser Pro Ser Asp Pro Val Phe Tyr Leu His His
   305                 310                 315                 320
   Gly Phe Val Asp Trp Gln Trp Ala Arg Trp Gln Asn Val Glu Ser Ser
                   325                 330                 335
   Arg Lys Thr Thr Ile Ser Gly Cys Ser Glu Pro Ser Pro Val Pro Gly
               340                 345                 350
   Lys Cys Val Glu Leu Thr Leu Asp Thr Val Leu Lys Gly Tyr Gly Leu
           355                 360                 365
   Ile Pro Asp Met Lys Val Arg Asp Val Leu Asp Ile Glu Asn Gly Ile
       370                 375                 380
   Leu Cys Tyr Thr Tyr Asp Glu Phe
   385                 390
<210> 63
   <211> 1179
   <212> DNA
   <213> Artificial
<220>
   <223> cDNA encoding PPO5
<400> 63
   atgaggctca ccaggtatgc cccggtgctg tcggcagccg tgttggcagc agcggcctca 60
   gtcccggcag ccgggaatct cgacattgtc aagaatctgg ctcatcgcat gggtgaagag 120
   gacatggacg cgttcccagt gttatcgtgg gacgaggctg tggccatcat gacggggaat 180
   gacgagggcg gcggcagcag cagcagcagc agcagcggta gcggtcgcgg tcccgacaac 240
   aacagggtct cggtcaacaa ctacagcaac aagaccaagc ccgcctaccc ccccgcctac 300
   gtcccccacg cgtcctcctg cgggaagccc aaggtccggg tcgagtggaa gaagctgaaa 360
   gagtcggaaa agtttgcgta cctgggggcg gtcaagtgcc tgatggacac gaagccggtc 420
   ggcgtctggt ccaaggcgac gagcatctgg gacgagatgg catgggcgca caacgaggcc 480
   aaatggaaca tccacgagac cgacaacttc ctgccctggc accgctacta catctacatg 540
   ctcgagacgc tcctcagcaa gcactgctcc taccgggggc ccatcccctg gtggcgcgag 600
   accgacgaca ccggcaactt ggccggggcc aatctgtttt cgcccaacta cttcggctcg 660
   ctaccccccc ggacggaaga cggtaagacc acatgcatta ccgacggctg gtttgccaac 720
   accacggtcc ttctcgggcc cggcgctccg acttgcctcg cccggggcga ggataagaga 780
   atcgccgccg aggtgacgac ggccgccctg gacctgtgcc aaggcgacag agacaccaag 840
   tacgagaggc accgtcgctg cgttgaggcc accatccatt cctccacgca ccgggccgtc 900
   ggcggcgcaa tggaaagcat cagcgcctcc ccgagcgacc ccgtcttcta cctccaccac 960
   ggtttcgtcg actggcagtg ggcgcgctgg cagaacgtcg aatccagccg caagaccaca 1020
   atttccggct gctccgagcc ttccccggtc cctggcaaat gtgtggagct caccctagac 1080
   acggtactga agggatatgg cttgataccc gacatgaagg tccgcgacgt tctcgacatc 1140
   gaaaacggaa tcctgtgcta cacctatgac gagttttga 1179
<210> 64
   <211> 1434
   <212> DNA
   <213> Myceliophthora thermophila
<400> 64
   atgaggctca ccaggtatgc cccggtgctg tcggcagccg tgttggcagc agcggcctca      60
   gtcccggcag ccgggaatct cgacattgtc aagaatctgg ctcatcgcat gggtgaagag     120
   gacatggacg cgttcccagt gttatcgtgg gacgaggctg tggccatcat gacggggaat     180
   gacgagggcg gcggcagcag cagcagcagc agcagcggta gcggtcgcgg tcccgacaac     240
   aacagggtct cggtcaacaa ctacagcaac aagaccaagc ccgcctaccc ccccgcctac      300
   gtcccccacg cgtcctcctg cgggaagccc aaggtccggg tcgagtggaa gaagctgaaa     360
   gagtcggaaa agtttgcgta cctgggggcg gtcaagtgcc tgatggacac gaagccggtc     420
   ggcgtctggt ccaaggcgac gagcatctgg gacgagatgg catgggcgca caacgaggcc     480
   aaatggaaca tccacgagac cgacaacttc ctgccctggc accgctacta catctacatg     540
   ctcgagacgc tcctcagcaa gcactgctcc taccgggggc ccatcccctg gtggcgcgag     600
   accgacgaca ccggcaactt ggccggggcc aatctgtttt cgcccaacta cttcggctcg     660
   ctaccccccc ggacggaaga cggtaagacc acatgcatta ccgacggcgt aagtataaga     720
   tatcctcttt gattatactt gtttctttct cttttcttcc ctctcttctt ctctggtact     780
   ttcaagcata accactcggc atccgctaat taattaatcg atccccggta acaaccttga     840
   aagtggtttg ccaacaccac ggtccttctc gggcccggcg ctccgacttg cctcgcccgg     900
   ggcgaggata agagaatcgc cgccgaggtg acgacggccg ccctggacct gtgccaaggc     960
   gacagagaca ccaagtacga gaggcaccgt cgctgcgttg aggccaccat ccattcctcc    1020
   acgcaccggg ccgtcggcgg cgcaatggaa agcatcagcg cctccccgag cgaccccgtc    1080
   ttctacctcc accacggttt cgtcgactgg cagtgggcgc gctggcagaa cgtcgaatcc    1140
   agccgcaaga ccacaagtaa gtctgatttc ctgtttccat gcgggaaatc tttaaaattt    1200
   agtgaaatta agggggggaa aaaggaagac ctatgtgtga gagaacatcc tgactaatac     1260
   ctttcgtgtc acacagtttc cggctgctcc gagccttccc cggtccctgg caaatgtgtg    1320
   gagctcaccc tagacacggt actgaaggga tatggcttga tacccgacat gaaggtccgc    1380
   gacgttctcg acatcgaaaa cggaatcctg tgctacacct atgacgagtt ttga          1434
<210> 65
   <211> 271
   <212> PRT
   <213> Myceliophthora thermophila
<400> 65
   Thr Asn Pro Ala Lys Arg Arg Ala Trp His Thr Leu Ser Asn Asp Glu
   1               5                   10                  15
   Lys Gln Val Tyr Ile Gln Ala Glu Leu Cys Leu Met Glu Leu Pro Ala
               20                  25                  30
   Lys Leu Ser Phe Pro Ala Ala Arg Thr Arg Phe Asp Glu Leu Gln Val
           35                  40                  45
   Ile His Gln Leu Gln Ala Tyr Ala Thr His Phe Val Val Ser Glu Asp
       50                  55                  60
   Glu Ala Ile Ser Gly Thr Thr Lys Leu Ser Cys Pro Leu Thr Cys Phe
   65                  70                  75                  80
   Leu Cys Val Arg Gly Arg Pro Arg Arg Arg Gly Trp Ala Gly Glu Ser
                   85                  90                  95
   Lys Phe Met Asp Leu Leu Ala Phe Phe Val Pro Phe Thr Phe Thr Phe
               100                 105                 110
   Ser Ser Gly Phe Leu His Leu Phe Gly Asn Pro Leu Leu Ala Ile Arg
           115                 120                 125
   Ser Pro Arg Ala Met Val Val Leu Ala Ser Arg Ser Phe Gly Ser Leu
       130                 135                 140
   Glu Gly Asp Arg Ser Lys Gly Tyr Leu Leu Thr Leu Ser Met Leu Asn
   145                 150                 155                 160
   Gly Val Ser Ser Pro Gly Asp Pro Leu Phe Tyr Leu His Asn Thr Trp
                   165                 170                 175
   Leu Asp Lys Val Phe Trp Asp Trp Gln Ala Arg Asp Arg Ser Thr Arg
               180                 185                 190
   Thr Thr Thr Ile Gly Gly Thr Asn Ile Ala Pro Asp Ala Pro Pro Gly
           195                 200                 205
   Phe Pro Thr Arg Pro Ser Asn Ile Pro Leu Pro Ala Gly Ala Asp Gly
       210                 215                 220
   Asn Pro Gly Lys Thr Thr Thr Leu Ser His Val Leu Asn Met Tyr Gly
   225                 230                 235                 240
   Asn Met Pro Asn Ala Thr Val Gly Asp Val Leu Asp Ile Gln Gly Asp
                   245                 250                 255
   Phe Val Cys Tyr Glu Tyr Val Glu Pro Glu Glu Glu Val Thr Val
               260                 265                 270
<210> 66
   <211> 292
   <212> PRT
   <213> Myceliophthora thermophila
<400> 66
   Met Ala Arg Phe Ser Lys Leu Ala Val Gly Leu Leu Met Val Ser Leu
   1               5                   10                  15
   Ala Ser Val Ala Cys Thr Asn Pro Ala Lys Arg Arg Ala Trp His Thr
               20                  25                  30
   Leu Ser Asn Asp Glu Lys Gln Val Tyr Ile Gln Ala Glu Leu Cys Leu
           35                  40                  45
   Met Glu Leu Pro Ala Lys Leu Ser Phe Pro Ala Ala Arg Thr Arg Phe
       50                  55                  60
   Asp Glu Leu Gln Val Ile His Gln Leu Gln Ala Tyr Ala Thr His Phe
   65                  70                  75                  80
   Val Val Ser Glu Asp Glu Ala Ile Ser Gly Thr Thr Lys Leu Ser Cys
                   85                  90                  95
   Pro Leu Thr Cys Phe Leu Cys Val Arg Gly Arg Pro Arg Arg Arg Gly
               100                 105                 110
   Trp Ala Gly Glu Ser Lys Phe Met Asp Leu Leu Ala Phe Phe Val Pro
           115                 120                 125
   Phe Thr Phe Thr Phe Ser Ser Gly Phe Leu His Leu Phe Gly Asn Pro
       130                 135                 140
   Leu Leu Ala Ile Arg Ser Pro Arg Ala Met Val Val Leu Ala Ser Arg
   145                 150                 155                 160
   Ser Phe Gly Ser Leu Glu Gly Asp Arg Ser Lys Gly Tyr Leu Leu Thr
                   165                 170                 175
   Leu Ser Met Leu Asn Gly Val Ser Ser Pro Gly Asp Pro Leu Phe Tyr
               180                 185                 190
   Leu His Asn Thr Trp Leu Asp Lys Val Phe Trp Asp Trp Gln Ala Arg
           195                 200                 205
   Asp Arg Ser Thr Arg Thr Thr Thr Ile Gly Gly Thr Asn Ile Ala Pro
       210                 215                 220
   Asp Ala Pro Pro Gly Phe Pro Thr Arg Pro Ser Asn Ile Pro Leu Pro
   225                 230                 235                 240
   Ala Gly Ala Asp Gly Asn Pro Gly Lys Thr Thr Thr Leu Ser His Val
                   245                 250                 255
   Leu Asn Met Tyr Gly Asn Met Pro Asn Ala Thr Val Gly Asp Val Leu
               260                 265                 270
   Asp Ile Gln Gly Asp Phe Val Cys Tyr Glu Tyr Val Glu Pro Glu Glu
           275                 280                 285
   Glu Val Thr Val
       290
<210> 67
   <211> 879
   <212> DNA
   <213> Artificial
<220>
   <223> cDNA encoding PPO10
<400> 67
   atggctcgat tctctaagct tgccgtcggc ctcctcatgg tgtctttggc ttctgtggcc      60
   tgcaccaacc cggccaaacg cagggcatgg cataccctga gcaacgatga gaagcaggtg     120
   tacatccagg ccgagctttg tctcatggag ctgcccgcca agctgagctt cccggcggcc     180
   cggacgaggt tcgacgagct ccaagtcatc caccagctcc aggcatatgc cacccatttc     240
   gtggtgagtg aagatgaggc tatttcaggg acgacaaagt tatcttgccc gctgacgtgc     300
   tttctctgcg tcagggggcg gccacggcgg cgtgggtggg caggtgagtc taagtttatg     360
   gatctgcttg cgttctttgt tccttttacc tttacttttt cgtctggttt cctacatctg     420
   ttcggaaatc cactacttgc tatcagatcc cctcgtgcta tggtggtgtt ggcaagtcgc     480
   agtttcggta gtctcgaggg cgatcgatcg aaaggatatt tgctgacctt atccatgctc     540
   aacggtgtct cgagccccgg cgaccctcta ttctacctac acaacacatg gctggacaag     600
   gtgttctggg actggcaggc cagggacagg tcgacgcgca cgacgaccat cggcgggacc     660
   aacatcgccc cggacgcgcc gccgggcttc cccacgcggc cctcgaatat cccgttacct     720
   gctggtgccg acggcaaccc tggcaaaacc accacgctaa gccatgtgct caacatgtac     780
   ggcaatatgc ccaacgcgac ggtgggtgac gtcttggaca ttcagggcga ctttgtctgc     840
   tacgagtacg tcgagccaga ggaggaggtg acggtatga                            879
<210> 68
   <211> 1072
   <212> DNA
   <213> Myceliophthora thermophila
<400> 68
   atggctcgat tctctaagct tgccgtcggc ctcctcatgg tgtctttggc ttctgtggcc      60
   tgcaccaacc cggccaaacg cagggcatgg taagcaggat atctgcccgg atccatcgtt     120
   gctcgacacg actaactgac tgactgactg attcgtcttt gggttgggag ggcaggcata     180
   ccctgagcaa cgatgagaag caggtgtaca tccaggccga gctttgtctc atggagctgc     240
   ccgccaagct gagcttcccg gcggcccgga cgaggttcga cgagctccaa gtcatccacc     300
   agctccaggc atatgccacc catttcgtgg tgagtgaaga tgaggctatt tcagggacga     360
   caaagttatc ttgcccgctg acgtgctttc tctgcgtcag gggcggccca cggcggcgtg     420
   ggtgggcagg tgagtctaag tttatggatc tgcttgcgtt ctttgttcct tttaccttta     480
   ctttttcgtc tggtttccta catctgttcg gaaatccact acttgctatc agatcccctc     540
   gtgctatggt ggtgttggca agtcgcagtt tcggtagtct cgagggcgat cgatcgaaag     600
   gatatttgct gaccttatcc gtcggggcga aatccgactc atagatgctc aacggtgtct     660
   cgagccccgg cgaccctcta ttctacctac acaacacatg gctggacaag gtgttctggg     720
   actggcaggc cagggacagg tcgacgcgca cgacgaccat cggcgggacc aacatcgccc     780
   cggacgcgcc gccgggcttc cccacgcggc cctcgaatat cccgttagtc tgcccctgac     840
   tgatatcctc ctcatgctta gggtcgacaa ttcaatggcg ttcggcgaca ctgattgtat     900
   atctagcaag cctgctggtg ccgacggcaa ccctggcaaa accaccacgc taagccatgt     960
   gctcaacatg tacggcaata tgcccaacgc gacggtgggt gacgtcttgg acattcaggg    1020
   cgactttgtc tgctacgagt acgtcgagcc agaggaggag gtgacggtat ga            1072
<210> 69
   <211> 336
   <212> PRT
   <213> Myceliophthora thermophila
<400> 69
   Gln Thr Tyr Pro Pro Asp Val Val Asp Gln Leu Ala Lys Asp Ser Leu
   1               5                   10                  15
   Pro Lys Leu Lys Glu Trp Leu Glu Arg Asn Pro Gln Glu Gly Cys Thr
               20                  25                  30
   Tyr Glu Thr Ala Val Lys Arg Arg Glu Trp Arg Asp Leu Thr Val Glu
           35                  40                  45
   Glu Arg Lys Ala Tyr Thr Asp Ala Val Leu Cys Leu Gln Ser Lys Pro
       50                  55                  60
   Ala Leu Thr Ser Asp Gln Ala Pro Gly Ala Lys Ser Arg Phe Asp Asp
   65                  70                  75                  80
   Tyr Val Val Ile His Ile Gln Gln Thr Pro Arg Asn His Gly Ser Tyr
                   85                  90                  95
   Trp Asn Trp Asp Arg Tyr Ala Lys Asp Pro Ala Asn Ser Pro Leu Phe
               100                 105                 110
   Asp Gly Ser Glu Gly Ser Met Gly Ser Asn Gly Ala Lys Glu Glu His
           115                 120                 125
   Gln Gly Ile Pro Ile Pro Gly Ala Pro Pro Pro Tyr Asn Met Ile Pro
       130                 135                 140
   Pro Gly Asp Gly Gly Gly Cys Val Thr Ser Gly Pro Phe Lys Asn Met
   145                 150                 155                 160
   Thr Val Asn Val Gly Pro Ile Ala Pro Thr Leu Asn Val Gln Arg Asn
                   165                 170                 175
   Pro Arg Ala Asp Gly Leu Gly Tyr Asn Pro Arg Cys Leu Arg Arg Asp
               180                 185                 190
   Ile Asn Lys His Ser Ala Ala Val Thr Thr Ala Asn Tyr Thr Tyr Asp
           195                 200                 205
   Leu Ile Thr Asn Asn His Glu Ile Tyr Trp Phe Gln Thr Val Met Glu
       210                 215                 220
   Gly Gln Phe Pro Gln Gly Lys Trp Gly Val His Ala Gly Gly His Tyr
   225                 230                 235                 240
   Thr Val Ser Gly Asp Pro Ala Gly Asp Phe Tyr Val Ser Pro Gly Asp
                   245                 250                 255
   Pro Val Phe Trp Leu His His Ala Met Ile Asp Arg Val Trp Trp Ile
               260                 265                 270
   Trp Gln Leu Gln Asp Leu Asp Ser Arg Leu Thr Gln Val Ser Met Thr
           275                 280                 285
   Lys Thr Met Asn Asn Phe Pro Pro Ser Glu Asn Gly Thr Leu Asp Asp
       290                 295                 300
   Leu Ser Gly Leu Gly Val Leu Ala Pro Asp Val Thr Val Arg Glu Leu
   305                 310                 315                 320
   Met Asn Thr Met Gly Gly Ile Gly Gly Lys Phe Cys Tyr Ile Tyr Glu
                   325                 330                 335
<210> 70
   <211> 354
   <212> PRT
   <213> Myceliophthora thermophila
<400> 70
   Met Val Leu Ser Lys Val Trp Gly Ala Thr Leu Leu Ala Gly Phe Ala
   1               5                   10                  15
   Val Ala Gln Thr Tyr Pro Pro Asp Val Val Asp Gln Leu Ala Lys Asp
               20                  25                  30
   Ser Leu Pro Lys Leu Lys Glu Trp Leu Glu Arg Asn Pro Gln Glu Gly
           35                  40                  45
   Cys Thr Tyr Glu Thr Ala Val Lys Arg Arg Glu Trp Arg Asp Leu Thr
       50                  55                  60
   Val Glu Glu Arg Lys Ala Tyr Thr Asp Ala Val Leu Cys Leu Gln Ser
   65                  70                  75                  80
   Lys Pro Ala Leu Thr Ser Asp Gln Ala Pro Gly Ala Lys Ser Arg Phe
                   85                  90                  95
   Asp Asp Tyr Val Val Ile His Ile Gln Gln Thr Pro Arg Asn His Gly
               100                 105                 110
   Ser Tyr Trp Asn Trp Asp Arg Tyr Ala Lys Asp Pro Ala Asn Ser Pro
           115                 120                 125
   Leu Phe Asp Gly Ser Glu Gly Ser Met Gly Ser Asn Gly Ala Lys Glu
   130 135 140
   Glu His Gln Gly Ile Pro Ile Pro Gly Ala Pro Pro Pro Tyr Asn Met
   145 150 155 160
   Ile Pro Pro Gly Asp Gly Gly Gly Cys Val Thr Ser Gly Pro Phe Lys
   165 170 175
   Asn Met Thr Val Asn Val Gly Pro Ile Ala Pro Thr Leu Asn Val Gln
   180 185 190
   Arg Asn Pro Arg Ala Asp Gly Leu Gly Tyr Asn Pro Arg Cys Leu Arg
   195 200 205
   Arg Asp Ile Asn Lys His Ser Ala Ala Val Thr Thr Ala Asn Tyr Thr
   210 215 220
   Tyr Asp Leu Ile Thr Asn Asn His Glu Ile Tyr Trp Phe Gln Thr Val
   225 230 235 240
   Met Glu Gly Gln Phe Pro Gln Gly Lys Trp Gly Val His Ala Gly Gly
   245 250 255
   His Tyr Thr Val Ser Gly Asp Pro Ala Gly Asp Phe Tyr Val Ser Pro
   260 265 270
   Gly Asp Pro Val Phe Trp Leu His His Ala Met Ile Asp Arg Val Trp
   275 280 285
   Trp Ile Trp Gln Leu Gln Asp Leu Asp Ser Arg Leu Thr Gln Val Ser
   290 295 300
   Met Thr Lys Thr Met Asn Asn Phe Pro Pro Ser Glu Asn Gly Thr Leu
   305 310 315 320
   Asp Asp Leu Ser Gly Leu Gly Val Leu Ala Pro Asp Val Thr Val Arg
   325 330 335
   Glu Leu Met Asn Thr Met Gly Gly Ile Gly Gly Lys Phe Cys Tyr Ile
   340 345 350
   Tyr Glu
<210> 71
   <211> 1065
   <212> DNA
   <213> Artificial
<220>
   <223> cDNA encoding PPO9
<400> 71
   atggttctgt caaaagtctg gggagcgacc ctcctcgctg gttttgcggt cgcccaaacg      60
   taccctccag atgtggttga tcagctggcg aaggatagtt tgcccaagct caaagaatgg     120
   ctcgagagga acccacagga gggatgtacc tacgagacgg cagtcaagcg gcgggaatgg     180
   agggacttga ccgtcgagga acgcaaggca tacacggatg ccgtcctctg cctccagtcg     240
   aagcctgcct tgacaagtga ccaagcgccg ggagccaaga gcaggtttga cgattatgtc     300
   gtcatccata tccaacaaac accgaggaac catggctcgt actggaactg ggatcgatat     360
   gctaaagacc ccgccaactc gccgctcttc gacggcagcg agggcagcat gggtagcaac     420
   ggtgccaagg aagaacacca gggcattcct atccccggtg cgccgcctcc gtacaacatg     480
   atcccgcccg gggacggcgg tggctgcgtg acctcgggcc cctttaagaa catgaccgtc     540
   aacgtcggcc caatcgcgcc gacgctgaac gtgcagcgca acccgcgggc ggacgggctg     600
   gggtacaacc cacggtgcct gcggcgcgac atcaacaagc actccgcggc ggtgacgacg     660
   gccaactaca cgtacgacct catcaccaac aaccacgaga tctactggtt ccagacggtc     720
   atggagggcc agttcccgca aggcaagtgg ggagtgcacg cgggagggca ctacaccgtc     780
   tcgggcgacc ccgcaggcga cttctacgtc agccccggcg accccgtctt ctggctgcac     840
   cacgccatga tcgaccgcgt ctggtggatc tggcagctcc aggacctcga cagccgcctg     900
   acccaggtga gcatgaccaa gaccatgaac aacttccccc ccagcgagaa cggcaccctc     960
   gacgacctca gcggcctcgg cgtgctcgcc ccggacgtca ccgtccggga gctcatgaac    1020
   accatgggag ggatcggcgg taagttttgc tacatctacg agtaa                    1065
<210> 72
   <211> 1517
   <212> DNA
   <213> Myceliophthora thermophila
<400> 72
   atggttctgt caaaagtctg gggagcgacc ctcctcgctg gttttgcggt cgcccaaacg 60
   taccctccag atgtggttga tcagctggcg aaggatagtt tgcccaagct caaagaatgg 120
   ctcgagagga acccacagga gggatgtacc tacgagacgg cagtcaagcg gcgggaatgg 180
   tttgctgcac cctcccgggt tcctcacgag ctgaactctg acatagccca ggagggactt 240
   gaccgtcgag gaacgcaagg catacacgga tgccgtcctc tgcctccagt cgaagcctgc 300
   cttgacaagt gaccaagcgc cgggagccaa gagcaggttt gacgattatg gtacacctcc 360
   cgatctcagc ccgacagata atagaataca ccctactaac ggcgttgcat caaagtcgtc 420
   atccatatcc aacaaacacc gaggaaccat ggctcggtaa gccggcaact tcgctgctcc 480
   ctcagcgccc cacgacgcca ccatctaaca tccagcagac cttcttcctc ccgtggcatc 540
   ggtactacgt ctggcactac gagaacgcgc tgagaaccga gtgcggctac aaaggatatc 600
   agccggtaag accacatcgc cttcgcatat acgacaggac agagccgccg ctgacaaggg 660
   tggggtttga agtactggaa ctgggatcga tatgctaaag accccgccaa ctcgccgctc 720
   ttcgacggca gcgagggcag catgggtagc aacggtgcca aggaagaaca ccagggcatt 780
   cctatccccg gtgcgccgcc tccgtacaac atgatcccgc ccggggacgg cggtggctgc 840
   gtgacctcgg gcccctttaa gaagtgagag cctttgacta ttaagtccca aatccctgtc 900
   aaaaaccccc atttcttgca gcacctatga agacggaagc gtattattta ctgacccccc 960
   cttttccctt cctccaaccc agcatgaccg tcaacgtcgg cccaatcgcg ccgacgctga 1020
   acgtgcagcg caacccgcgg gcggacgggc tggggtacaa cccacggtgc ctgcggcgcg 1080
   acatcaacaa gcactccgcg gcggtgacga cggccaacta cacgtacgac ctcatcacca 1140
   acaaccacga gatctactgg ttccagacgg tcatggaggg ccagttcccg caaggcaagt 1200
   ggggagtgca cgcgggaggg cactacaccg tctcgggcga ccccgcaggc gacttctacg 1260
   tcagccccgg cgaccccgtc ttctggctgc accacgccat gatcgaccgc gtctggtgga 1320
   tctggcagct ccaggacctc gacagccgcc tgacccaggt gagcatgacc aagaccatga 1380
   acaacttccc ccccagcgag aacggcaccc tcgacgacct cagcggcctc ggcgtgctcg 1440
   ccccggacgt caccgtccgg gagctcatga acaccatggg agggatcggc ggtaagtttt 1500
   gctacatcta cgagtaa 1517
<210> 73
   <211> 837
   <212> PRT
   <213> Clostridium thermocellum
<400> 73
   Met Ser Arg Lys Leu Phe Ser Val Leu Leu Val Gly Leu Met Leu Met
   1               5                   10                  15
   Thr Ser Leu Leu Val Thr Ile Ser Ser Thr Ser Ala Ala Ser Leu Pro
               20                  25                  30
   Thr Met Pro Pro Ser Gly Tyr Asp Gln Val Arg Asn Gly Val Pro Arg
           35                  40                  45
   Gly Gln Val Val Asn Ile Ser Tyr Phe Ser Thr Ala Thr Asn Ser Thr
       50                  55                  60
   Arg Pro Ala Arg Val Tyr Leu Pro Pro Gly Tyr Ser Lys Asp Lys Lys
   65                  70                  75                  80
   Tyr Ser Val Leu Tyr Leu Leu His Gly Ile Gly Gly Ser Glu Asn Asp
                   85                  90                  95
   Trp Phe Glu Gly Gly Gly Arg Ala Asn Val Ile Ala Asp Asn Leu Ile
               100                 105                 110
   Ala Glu Gly Lys Ile Lys Pro Leu Ile Ile Val Thr Pro Asn Thr Asn
           115                 120                 125
   Ala Ala Gly Pro Gly Ile Ala Asp Gly Tyr Glu Asn Phe Thr Lys Asp
       130                 135                 140
   Leu Leu Asn Ser Leu Ile Pro Tyr Ile Glu Ser Asn Tyr Ser Val Tyr
   145                 150                 155                 160
   Thr Asp Arg Glu His Arg Ala Ile Ala Gly Leu Ser Met Gly Gly Gly
                   165                 170                 175
   Gln Ser Phe Asn Ile Gly Leu Thr Asn Leu Asp Lys Phe Ala Tyr Ile
               180                 185                 190
   Gly Pro Ile Ser Ala Ala Pro Asn Thr Tyr Pro Asn Glu Arg Leu Phe
           195                 200                 205
   Pro Asp Gly Gly Lys Ala Ala Arg Glu Lys Leu Lys Leu Leu Phe Ile
       210                 215                 220
   Ala Cys Gly Thr Asn Asp Ser Leu Ile Gly Phe Gly Gln Arg Val His
   225                 230                 235                 240
   Glu Tyr Cys Val Ala Asn Asn Ile Asn His Val Tyr Trp Leu Ile Gln
                   245                 250                 255
   Gly Gly Gly His Asp Phe Asn Val Trp Lys Pro Gly Leu Trp Asn Phe
               260                 265                 270
   Leu Gln Met Ala Asp Glu Ala Gly Leu Thr Arg Asp Gly Asn Thr Pro
           275                 280                 285
   Val Pro Thr Pro Ser Pro Lys Pro Ala Asn Thr Arg Ile Glu Ala Glu
       290                 295                 300
   Asp Tyr Asp Gly Ile Asn Ser Ser Ser Ile Glu Ile Ile Gly Val Pro
   305                 310                 315                 320
   Pro Glu Gly Gly Arg Gly Ile Gly Tyr Ile Thr Ser Gly Asp Tyr Leu
                   325                 330                 335
   Val Tyr Lys Ser Ile Asp Phe Gly Asn Gly Ala Thr Ser Phe Lys Ala
               340                 345                 350
   Lys Val Ala Asn Ala Asn Thr Ser Asn Ile Glu Leu Arg Leu Asn Gly
           355                 360                 365
   Pro Asn Gly Thr Leu Ile Gly Thr Leu Ser Val Lys Ser Thr Gly Asp
       370                 375                 380
   Trp Asn Thr Tyr Glu Glu Gln Thr Cys Ser Ile Ser Lys Val Thr Gly
   385                 390                 395                 400
   Ile Asn Asp Leu Tyr Leu Val Phe Lys Gly Pro Val Asn Ile Asp Trp
                   405                 410                 415
   Phe Thr Phe Gly Val Glu Ser Ser Ser Thr Gly Leu Gly Asp Leu Asn
               420                 425                 430
   Gly Asp Gly Asn Ile Asn Ser Ser Asp Leu Gln Ala Leu Lys Arg His
           435                 440                 445
   Leu Leu Gly Ile Ser Pro Leu Thr Gly Glu Ala Leu Leu Arg Ala Asp
       450                 455                 460
   Val Asn Arg Ser Gly Lys Val Asp Ser Thr Asp Tyr Ser Val Leu Lys
   465                 470                 475                 480
   Arg Tyr Ile Leu Arg Ile Ile Thr Glu Phe Pro Gly Gln Gly Asp Val
                   485                 490                 495
   Gln Thr Pro Asn Pro Ser Val Thr Pro Thr Gln Thr Pro Ile Pro Thr
               500                 505                 510
   Ile Ser Gly Asn Ala Leu Arg Asp Tyr Ala Glu Ala Arg Gly Ile Lys
           515                 520                 525
   Ile Gly Thr Cys Val Asn Tyr Pro Phe Tyr Asn Asn Ser Asp Pro Thr
       530                 535                 540
   Tyr Asn Ser Ile Leu Gln Arg Glu Phe Ser Met Val Val Cys Glu Asn
   545                 550                 555                 560
   Glu Met Lys Phe Asp Ala Leu Gln Pro Arg Gln Asn Val Phe Asp Phe
                   565                 570                 575
   Ser Lys Gly Asp Gln Leu Leu Ala Phe Ala Glu Arg Asn Gly Met Gln
   580 585 590
   Met Arg Gly His Thr Leu Ile Trp His Asn Gln Asn Pro Ser Trp Leu
   595 600 605
   Thr Asn Gly Asn Trp Asn Arg Asp Ser Leu Leu Ala Val Met Lys Asn
   610 615 620
   His Ile Thr Thr Val Met Thr His Tyr Lys Gly Lys Ile Val Glu Trp
   625 630 635 640
   Asp Val Ala Asn Glu Cys Met Asp Asp Ser Gly Asn Gly Leu Arg Ser
   645 650 655
   Ser Ile Trp Arg Asn Val Ile Gly Gln Asp Tyr Leu Asp Tyr Ala Phe
   660 665 670
   Arg Tyr Ala Arg Glu Ala Asp Pro Asp Ala Leu Leu Phe Tyr Asn Asp
   675 680 685
   Tyr Asn Ile Glu Asp Leu Gly Pro Lys Ser Asn Ala Val Phe Asn Met
   690 695 700
   Ile Lys Ser Met Lys Glu Arg Gly Val Pro Ile Asp Gly Val Gly Phe
   705 710 715 720
   Gln Cys His Phe Ile Asn Gly Met Ser Pro Glu Tyr Leu Ala Ser Ile
   725 730 735
   Asp Gln Asn Ile Lys Arg Tyr Ala Glu Ile Gly Val Ile Val Ser Phe
   740 745 750
   Thr Glu Ile Asp Ile Arg Ile Pro Gln Ser Glu Asn Pro Ala Thr Ala
   755 760 765
   Phe Gln Val Gln Ala Asn Asn Tyr Lys Glu Leu Met Lys Ile Cys Leu
   770 775 780
   Ala Asn Pro Asn Cys Asn Thr Phe Val Met Trp Gly Phe Thr Asp Lys
   785 790 795 800
   Tyr Thr Trp Ile Pro Gly Thr Phe Pro Gly Tyr Gly Asn Pro Leu Ile
   805 810 815
   Tyr Asp Ser Asn Tyr Asn Pro Lys Pro Ala Tyr Asn Ala Ile Lys Glu
   820 825 830
   Ala Leu Met Gly Tyr
   835
<210> 74
   <211> 290
   <212> PRT
   <213> Neurospora crassa
<400> 74
   Met Ala Gly Leu His Ser Arg Leu Thr Thr Phe Leu Leu Leu Leu Leu
   1 5 10 15
   Ser Ala Leu Pro Ala Ile Ala Ala Ala Ala Pro Ser Ser Gly Cys Gly
   20 25 30
   Lys Gly Pro Thr Leu Arg Asn Gly Gln Thr Val Thr Thr Asn Ile Asn
   35 40 45
   Gly Lys Ser Arg Arg Tyr Thr Val Arg Leu Pro Asp Asn Tyr Asn Gln
   50 55 60
   Asn Asn Pro Tyr Arg Leu Ile Phe Leu Trp His Pro Leu Gly Ser Ser
   65 70 75 80
   Met Gln Lys Ile Ile Gln Gly Glu Asp Pro Asn Arg Gly Gly Val Leu
   85 90 95
   Pro Tyr Tyr Gly Leu Pro Pro Leu Asp Thr Ser Lys Ser Ala Ile Tyr
   100 105 110
   Val Val Pro Asp Gly Leu Asn Ala Gly Trp Ala Asn Gln Asn Gly Glu
   115 120 125
   Asp Val Ser Phe Phe Asp Asn Ile Leu Gln Thr Val Ser Asp Gly Leu
   130 135 140
   Cys Ile Asp Thr Asn Leu Val Phe Ser Thr Gly Phe Ser Tyr Gly Gly
   145 150 155 160
   Gly Met Ser Phe Ser Leu Ala Cys Ser Arg Ala Asn Lys Val Arg Ala
   165 170 175
   Val Ala Val Ile Ser Gly Ala Gln Leu Ser Gly Cys Ala Gly Gly Asn
   180 185 190
   Asp Pro Val Ala Tyr Tyr Ala Gln His Gly Thr Ser Asp Gly Val Leu
   195 200 205
   Asn Val Ala Met Gly Arg Gln Leu Arg Asp Arg Phe Val Arg Asn Asn
   210 215 220
   Gly Cys Gln Pro Ala Asn Gly Glu Val Gln Pro Gly Ser Gly Gly Arg
   225 230 235 240
   Ser Thr Arg Val Glu Tyr Gln Gly Cys Gln Gln Gly Lys Asp Val Val
   245 250 255
   Trp Val Val His Gly Gly Asp His Asn Pro Ser Gln Arg Asp Pro Gly
   260 265 270
   Gln Asn Asp Pro Phe Ala Pro Arg Asn Thr Trp Glu Phe Phe Ser Arg
   275 280 285
   Phe Asn
   290
<210> 75
   <211> 530
   <212> PRT
   <213> Talaromyces stipitatus
<400> 75
   Met Met Leu Thr Ser Ala Ile Leu Leu Leu Thr Leu Gly Val Gln Leu
   1 5 10 15
   Ser His Ala Asp Asp Ser Ser Arg Glu Asn Phe Ser Asn Arg Cys Asp
   20 25 30
   Gln Leu Ala Lys Glu Ile His Ile Pro Asn Val Thr Val Asn Phe Val
   35 40 45
   Glu Tyr Val Ala Asn Gly Thr Asn Val Thr Leu Ala Asp Asn Pro Pro
   50 55 60
   Ser Cys Gly Gln Ser Asn Gln Val Val Leu Ala Asp Leu Cys Arg Val
   65 70 75 80
   Ala Met Glu Val Thr Thr Ser Asn Gln Ser Gln Ile Thr Leu Glu Ala
   85 90 95
   Trp Phe Pro Glu Asn Tyr Thr Gly Arg Phe Leu Ser Thr Gly Asn Gly
   100 105 110
   Gly Leu Ala Gly Cys Ile Gln Tyr Val Asp Met Ala Tyr Ala Ser Ser
   115 120 125
   Met Gly Phe Ala Thr Val Gly Ala Asn Gly Gly His Asn Gly Thr Ser
       130                 135                 140
   Gly Glu Ser Phe Tyr His Asn Pro Asp Ile Val Glu Asp Leu Ser Trp
   145                 150                 155                 160
   Arg Ser Val His Thr Gly Val Val Val Gly Lys Glu Leu Thr Lys Lys
                   165                 170                 175
   Phe Tyr His Glu Gly Phe His Lys Ser Tyr Tyr Leu Gly Cys Ser Thr
               180                 185                 190
   Gly Gly Arg Gln Gly Phe Lys Ala Val Gln Glu Phe Val His Asp Phe
           195                 200                 205
   Asp Gly Val Val Ala Gly Cys Pro Ala Phe Asn Phe Val Asn Leu Asn
       210                 215                 220
   Ser Trp Ser Gly His Phe Tyr Pro Ile Thr Gly Asn Ser Ser Ala Asp
   225                 230                 235                 240
   Thr Phe Leu Thr Thr Ala Gln Trp Thr Leu Val Gln Gln Ser Val Met
                   245                 250                 255
   Glu Gln Cys Asp Ser Leu Asp Gly Ala Val Asp Gly Val Ile Glu Ala
               260                 265                 270
   Ile Asp Gln Cys His Pro Val Phe Glu Gln Leu Ile Cys Arg Pro Gly
           275                 280                 285
   Gln Asn Ala Ser Glu Cys Leu Thr Gly Lys Gln Val Asn Thr Ala Gln
       290                 295                 300
   Leu Val Leu Ser Pro Ile Tyr Gly Thr Lys Gly Glu Phe Leu Tyr Pro
   305                 310                 315                 320
   Arg Met Gln Pro Gly Val Glu Asn Val Asp Met Tyr Ile Thr Tyr Asn
                   325                 330                 335
   Gly Asp Pro Phe Ala Tyr Ser Thr Asp Trp Tyr Lys Tyr Val Val Phe
               340                 345                 350
   Ser Asp Pro Asn Trp Asp Pro Ala Thr Leu Asn Ala Gln Asp Tyr Glu
           355                 360                 365
   Ile Ala Leu Ala Gln Asn Pro Ser Asn Ile Gln Thr Phe Glu Gly Asp
       370                 375                 380
   Leu Ser Ala Phe Arg Asp Ala Gly Ala Lys Val Leu Thr Tyr His Gly
   385 390 395 400
   Thr Ala Asp Pro Ile Ile Thr Gly Glu Thr Ser Lys Val Tyr Tyr Arg
   405 410 415
   His Val Ala Glu Thr Met Asn Ala Ala Pro Glu Glu Leu Asp Glu Phe
   420 425 430
   Tyr Arg Tyr Phe Arg Ile Gly Gly Met Ser His Cys Gly Gly Gly Thr
   435 440 445
   Gly Ala Thr Ala Ile Gly Asn Val Leu Ser Ala Gln Trp Ser Asn Asp
   450 455 460
   Pro Asp Ala Asn Val Leu Met Ala Met Val Arg Trp Val Glu Glu Gly
   465 470 475 480
   Val Ala Pro Glu Tyr Ile Arg Gly Ala Ser Leu Gly Ser Gly Pro Gly
   485 490 495
   Ala Lys Val Glu Tyr Thr Arg Arg His Cys Lys Tyr Pro Thr Arg Asn
   500 505 510
   Val Tyr Val Gly Pro Gly Asn Trp Thr Asp Glu Asn Ala Trp Lys Cys
   515 520 525
   Ile Leu
   530
<210> 76
   <211> 585
   <212> PRT
   <213> Thielavia terrestris
<400> 76
   Met Ala Ala Phe Ala Lys Leu Thr Thr Arg Ala Leu Ser Leu Ser Asp
   1 5 10 15
   Ile Cys Thr Val Ser Asn Val Gln Ala Ala Leu Pro Ser Asn Gly Thr
   20 25 30
   Leu Leu Gly Ile Asp Leu Ile Pro Ser Ser Val Thr Ala Ser Val Thr
   35 40 45
   Asn Gly Thr Ser Gly Gly Ser Met Gly Met Gly Gly Gly Ser Ser Pro
   50 55 60
   Gly Ser Ser Ile Thr Tyr Cys Ser Val Thr Val Thr Tyr Thr His Thr
   65                  70                  75                  80
   Gly Lys Gly Asp Thr Val Asn Leu Lys Tyr Ala Phe Pro Ser Pro Ser
                   85                  90                  95
   Asp Phe Gln Asn Arg Phe Tyr Val Ala Gly Gly Gly Gly Phe Ser Leu
               100                 105                 110
   Ser Ser Asp Ala Thr Gly Gly Leu Ser Tyr Gly Ala Ala Gly Gly Ala
           115                 120                 125
   Thr Asp Ala Gly Tyr Asp Ala Phe Ser Asn Ser Tyr Asp Lys Val Val
       130                 135                 140
   Leu Tyr Gly Asn Gly Ser Ile Asn Trp Asp Ala Thr Tyr Met Phe Gly
   145                 150                 155                 160
   Tyr Gln Ala Leu Gly Glu Met Thr Lys Val Gly Lys Val Leu Thr Lys
                   165                 170                 175
   Gly Phe Tyr Gly Leu Ser Asp Asp Thr Lys Ile Tyr Thr Tyr Phe Glu
               180                 185                 190
   Gly Cys Ser Asp Gly Gly Arg Glu Gly Met Ser Gln Val Gln Arg Trp
           195                 200                 205
   Gly Asp Glu Tyr Gly Gly Val Ile Ala Gly Ala Pro Ala Phe Arg Phe
       210                 215                 220
   Ala Gln Gln Gln Val Leu His Val Leu Ser Ser Thr Val Glu Lys Met
   225                 230                 235                 240
   Leu Asp Tyr Leu Pro Pro Pro Cys Glu Leu Ala Lys Ile Val Asn Ala
                   245                 250                 255
   Thr Ile Ala Ala Cys Asp Gly Leu Asp Gly Arg Ser Asp Gly Val Val
               260                 265                 270
   Ser Arg Thr Asp Leu Cys Lys Leu Lys Phe Asn Leu Lys Ser Val Ile
           275                 280                 285
   Gly Glu Ser Tyr Tyr Cys Ala Ala Lys Asn Ser Thr Ser Leu Gly Ser
       290                 295                 300
   Gly Phe Asn Lys Ala Lys Arg Gln Ala Pro Gly Ser Gln Thr Ser Tyr
   305 310 315 320
   Gln Leu Glu Gln Ser Gly Thr Val Ser Ala Lys Gly Val Ala Val Ala
   325 330 335
   Gln Ala Ile Tyr Asp Gly Leu His Asn Ser Ala Gly Glu Arg Ala Tyr
   340 345 350
   Leu Ser Trp Gln Ile Ala Ser Glu Leu Ala Asp Ala Ser Thr Thr Tyr
   355 360 365
   Asn Ser Ala Ile Gly Ala Trp Glu Leu Asn Ile Pro Ser Ile Gly Gly
   370 375 380
   Glu Tyr Val Thr Lys Phe Val Gln Leu Leu Asp Leu Asp Asn Leu Pro
   385 390 395 400
   Ser Leu Asp Asn Val Thr Tyr Asp Thr Leu Val Asp Trp Met Thr Thr
   405 410 415
   Gly Met Tyr Arg Tyr Met Asp Ser Leu Gln Thr Thr Leu Pro Asp Leu
   420 425 430
   Thr Thr Phe Gln Ser Ser Gly Gly Lys Leu Leu His Tyr His Gly Glu
   435 440 445
   Ser Asp Ser Ser Ile Pro Ala Ala Ser Ser Val His Tyr Trp Gln Ser
   450 455 460
   Val Arg Ser Val Met Tyr Pro Ser Leu Ser Asp Glu Gln Ser Leu Ala
   465 470 475 480
   Ala Leu Ala Asp Trp Tyr Gln Leu Tyr Leu Val Pro Gly Ala Gly His
   485 490 495
   Cys Gly Ala Asn Pro Leu Gln Pro Gly Pro Phe Pro Arg Asn Asn Met
   500 505 510
   Asn Val Met Ile Asp Trp Val Glu Asn Gly Val Arg Pro Ser Arg Leu
   515 520 525
   Asn Ala Thr Val Ser Ser Gly Lys Tyr Ala Gly Glu Thr Gln Met Leu
   530 535 540
   Cys Gln Trp Pro Thr Arg Pro Leu Trp Arg Ser Asn Asp Ser Ser Thr
   545 550 555 560
   Phe Asp Cys Val Thr Asp Ala Ala Ser Tyr Glu Ser Trp Thr Tyr Thr
   565 570 575
   Phe Pro Ala Phe Lys Val Pro Val Tyr
   580 585
<210> 77
   <211> 287
   <212> PRT
   <213> Humicola insolens
<400> 77
   Met Arg Phe Ser Thr Ile Leu Ser Val Ala Ala Thr Ala Ser Thr Ala
   1 5 10 15
   Leu Gly Ala Ser Leu Gln Gln Val Trp Asn Trp Gly Ala Asn Pro Thr
   20 25 30
   Asn Ile Gln Phe His Ile Tyr Val Pro Asp Arg Arg Ala Ala Asn Pro
   35 40 45
   Ala Ile Ile Val Ala Leu His Pro Cys Gly Gly Asn Ala Gln Gln Trp
   50 55 60
   Phe Gly Gly Thr Arg Leu Pro Gln Tyr Ala Asp Gln His Gly Phe Ile
   65 70 75 80
   Leu Ile Tyr Leu Ser Thr Pro Asn Gln Ser Asn Cys Trp Asp Val His
   85 90 95
   Asn Thr Ala Ser Leu Thr His Asn Gly Gly Gly Asp Ala Leu Gly Ile
   100 105 110
   Val Ser Met Val Asn Tyr Thr Ile Asp Arg Tyr Asn Ala Asp Arg Ser
   115 120 125
   Arg Val Tyr Val Met Gly Phe Ser Ser Gly Gly Met Met Thr Asn Val
   130 135 140
   Leu Ala Gly Ser Tyr Pro Glu Val Phe Glu Ala Gly Ala Ala Tyr Ser
   145 150 155 160
   Gly Thr Ala His Ala Cys Phe Phe Gly Glu Pro Phe Ser Pro Asn Gln
   165 170 175
   Thr Cys Ala Gln Gly Leu Ser His Thr Pro Glu Gln Trp Gly Asn Phe
   180 185 190
   Val Arg Asn Ser Tyr Pro Gly Tyr Asn Gly Arg Arg Pro Arg Met Gln
   195 200 205
   Ile Val His Gly Leu Gln Asp Phe Leu Val Arg Pro Gln Cys Gly Tyr
   210 215 220
   Glu Ala Leu Lys Gln Trp Ser Asn Val Leu Gly Ile Pro Phe Thr Arg
   225 230 235 240
   Gln Val Gln Asn Ser Pro Ser Trp Gly Trp Thr Thr Glu Leu Tyr Gly
   245 250 255
   Asp Gly Thr Gln Leu Gln Gly Leu Phe Ser Gln Asn Leu Gly His Ala
   260 265 270
   Pro Ala Val Asp Glu Gln Gln Leu Leu Arg Phe Phe Arg Leu Ile
   275 280 285
<210> 78
   <211> 789
   <212> PRT
   <213> Ruminococcus sp.
<400> 78
   Val Lys Lys Thr Val Lys Gln Phe Ile Ser Ser Ala Val Thr Ala Leu
   1 5 10 15
   Met Val Ala Ala Ser Leu Pro Ala Val Pro Ser Val Asn Ala Ala Asp
   20 25 30
   Ala Gln Gln Arg Gly Asn Ile Gly Gly Phe Asp Tyr Glu Met Trp Asn
   35 40 45
   Gln Asn Gly Gln Gly Gln Val Ser Met Thr Pro Lys Ala Gly Ser Phe
   50 55 60
   Thr Cys Ser Trp Ser Asn Ile Glu Asn Phe Leu Ala Arg Met Gly Lys
   65 70 75 80
   Asn Tyr Asp Ser Gln Lys Lys Asn Tyr Lys Ala Phe Gly Asp Ile Thr
   85 90 95
   Leu Ser Tyr Asp Val Glu Tyr Thr Pro Lys Gly Asn Ser Tyr Met Cys
   100 105 110
   Val Tyr Gly Trp Thr Arg Asn Pro Leu Met Glu Tyr Tyr Ile Val Glu
   115 120 125
   Gly Trp Gly Asp Trp Arg Pro Pro Gly Asn Asp Gly Glu Asn Lys Gly
       130                 135                 140
   Thr Val Thr Leu Asn Gly Asn Thr Tyr Asp Ile Arg Lys Thr Met Arg
   145                 150                 155                 160
   Tyr Asn Gln Pro Ser Leu Asp Gly Thr Ala Thr Phe Pro Gln Tyr Trp
                   165                 170                 175
   Ser Val Arg Gln Lys Ser Gly Ser Gln Asn Asn Thr Thr Asn Tyr Met
               180                 185                 190
   Lys Gly Thr Ile Ser Val Ser Lys His Phe Asp Ala Trp Ser Lys Ala
           195                 200                 205
   Gly Leu Asp Met Ser Gly Thr Leu Tyr Glu Val Ser Leu Asn Ile Glu
       210                 215                 220
   Gly Tyr Arg Ser Ser Gly Asn Ala Asn Val Lys Ala Ile Ser Phe Asp
   225                 230                 235                 240
   Gly Ser Ile Pro Glu Pro Thr Ser Glu Pro Val Thr Gln Pro Val Val
                   245                 250                 255
   Lys Ala Glu Pro Asp Ala Asn Gly Tyr Tyr Phe Lys Glu Lys Phe Glu
               260                 265                 270
   Ser Gly Ala Gly Asp Trp Ser Ala Arg Gly Thr Gly Ala Lys Val Thr
           275                 280                 285
   Ser Ser Asp Gly Phe Asn Gly Ser Lys Gly Ile Leu Val Ser Gly Arg
       290                 295                 300
   Gly Asp Asn Trp His Gly Ala Gln Leu Thr Leu Asp Ser Ser Ala Phe
   305                 310                 315                 320
   Thr Ala Gly Glu Thr Tyr Ser Phe Gly Ala Leu Val Lys Gln Asp Gly
                   325                 330                 335
   Glu Ser Ser Thr Ala Met Lys Leu Thr Leu Gln Tyr Asn Asp Ala Ser
               340                 345                 350
   Gly Thr Ala Asn Tyr Asp Lys Val Ala Glu Phe Thr Ala Pro Lys Gly
           355                 360                 365
   Glu Trp Val Asp Leu Ser Asn Thr Ser Phe Thr Ile Pro Ser Gly Ala
       370                 375                 380
   Ser Asp Leu Ile Leu Tyr Val Glu Ala Pro Asp Ser Leu Thr Asp Phe
   385                 390                 395                 400
   Tyr Ile Asp Asn Ala Phe Gly Gly Ile Lys Asn Thr Ser Pro Leu Glu
                   405                 410                 415
   Asp Val Gly Ser His Thr Ile Ser Thr Pro Gly Ser Glu Thr Thr Thr
               420                 425                 430
   Val Thr Thr Ala Ser Asn Lys Gly Ile Arg Gly Asp Ile Asn Gly Asp
           435                 440                 445
   Gly Val Ile Asn Ser Phe Asp Leu Ala Pro Leu Arg Arg Gly Ile Leu
       450                 455                 460
   Lys Met Met Ser Gly Ser Gly Ser Thr Pro Glu Asn Ala Asp Val Asn
   465                 470                 475                 480
   Gly Asp Gly Thr Val Asn Val Ala Asp Leu Leu Leu Leu Gln Lys Phe
                   485                 490                 495
   Ile Leu Gly Met Glu Lys Ser Phe Pro Asp Pro Val Thr Thr Thr Thr
               500                 505                 510
   Thr Lys Pro Ile Thr Thr Thr Thr Glu Lys Ile Val Thr Thr Thr Thr
           515                 520                 525
   Ser Ser Ser Ser Ser Ser Ser Gly Lys Asn Leu Asn Ala Asp Ile Arg
       530                 535                 540
   Lys Asp Met Pro Thr Ser Val Pro Gly Gly Asn Glu Lys Ser Gly Gly
   545                 550                 555                 560
   Cys Lys Val Glu Lys Lys Thr Tyr Asn Cys Lys Phe Thr Gly Gly Gln
                   565                 570                 575
   Lys Ser Cys Asn Val Ile Leu Pro Pro Asn Tyr Ser Ala Ser Lys Gln
               580                 585                 590
   Tyr Pro Val Met Tyr Val Leu His Gly Ile Gly Gly Asn Glu Gly Ser
           595                 600                 605
   Met Val Ser Gly Met Gly Val Gln Glu Leu Leu Ala Gly Leu Thr Ala
       610                 615                 620
   Asn Gly Lys Ala Glu Glu Met Ile Ile Val Leu Pro Ser Gln Tyr Thr
   625                 630                 635                 640
   Ser Lys Asn Gly Asn Gln Gly Gly Gly Phe Gly Ile Asn Gln Glu Val
   645 650 655
   Cys Ala Ala Tyr Asp Asn Phe Leu Tyr Asp Ile Ser Asp Ser Leu Ile
   660 665 670
   Pro Phe Ile Glu Ala Asn Tyr Pro Val Lys Thr Gly Arg Glu Asn Arg
   675 680 685
   Ala Ile Thr Gly Phe Ser Met Gly Gly Arg Glu Ala Ile Tyr Ile Gly
   690 695 700
   Leu Met Arg Pro Asp Leu Phe Ala Tyr Val Gly Gly Ala Cys Pro Ala
   705 710 715 720
   Pro Gly Ile Thr Pro Gly Lys Asp Met Phe Met Glu His Pro Gly Cys
   725 730 735
   Met Gln Glu Ser Glu Met Lys Phe Arg Asp Val Gly Pro Glu Pro Asn
   740 745 750
   Val Phe Met Ile Thr Gly Gly Thr Asn Asp Gly Val Val Gly Thr Phe
   755 760 765
   Pro Lys Gln Tyr Ser Asp Ile Leu Thr Arg Asn Gly Val Asp Gln Arg
   770 775 780
   Leu Pro Val Tyr Pro
   785
<210> 79
   <211> 530
   <212> PRT
   <213> Orpinomyces sp.
<400> 79
   Val Val Ser Cys Glu Thr Thr Tyr Gly Ile Thr Leu Arg Asp Thr Lys
   1 5 10 15
   Glu Lys Phe Thr Val Phe Lys Asp Gly Ser Ala Ala Thr Asp Ile Val
   20 25 30
   Glu Ser Glu Asp Gly Ser Val Ser Trp Ile Ala Thr Ala Ala Gly Gly
   35 40 45
   Ala Gly Gly Gly Val Ala Phe Tyr Val Lys Ala Asn Lys Glu Glu Ile
   50 55 60
   Asn Ile Ala Asn Tyr Glu Ser Ile Asp Ile Glu Met Glu Tyr Thr Pro
   65                  70                  75                  80
   Val Glu Asn Lys Trp Asn Asp Ala Ala Lys Asn Pro Ser Phe Cys Met
                   85                  90                  95
   Arg Ile Leu Pro Trp Asp Ser Thr Gly Met Phe Gly Gly Tyr Glu Asp
               100                 105                 110
   Leu Glu Tyr Phe Asp Thr Pro Ala Lys Ser Gly Asn Phe Lys Tyr Thr
           115                 120                 125
   Ile Lys Ile Pro Ser Phe Phe Ala Asp Lys Ile Leu Ser Ser Ser Asp
       130                 135                 140
   Leu Asp Ser Ile Leu Ser Phe Ala Ile Lys Phe Asn Asp Tyr Glu Arg
   145                 150                 155                 160
   Gly Asn Thr Asp Gly Asp Gln Ile Lys Ile Gln Leu Lys Asn Val Lys
                   165                 170                 175
   Phe Asn Pro Lys Glu Asn Ala Pro Glu Asp Lys Ala Phe Asp Asp Gly
               180                 185                 190
   Leu Arg Asp Ser Gln Arg Gly Thr Val Val Glu Met Lys Tyr Ser Ser
           195                 200                 205
   Arg Asp Tyr Thr Val Lys Glu Ser Glu Ala Asp Lys Tyr Glu Lys His
       210                 215                 220
   Ala Trp Val Tyr Leu Pro Ala Gly Tyr Glu Ala Asp Asn Lys Asp Lys
   225                 230                 235                 240
   Lys Tyr Pro Leu Val Val Leu Leu His Gly Tyr Gly Gln Asn Glu Asn
                   245                 250                 255
   Thr Trp Gly Leu Ser Asn Lys Gly Arg Gly Gly Lys Ile Lys Gly Tyr
               260                 265                 270
   Met Asp Arg Gly Met Ala Ser Gly Asn Val Glu Lys Phe Val Leu Val
           275                 280                 285
   Ala Ala Thr Gly Val Ala Ser Lys Asn Trp Gly Pro Asn Gly Ser Gly
       290                 295                 300
   Val Asp Leu Asp Gly Phe Asn Ala Phe Gly Gly Glu Leu Arg Asn Asp
   305 310 315 320
   Leu Leu Pro Tyr Ile Arg Ala His Phe Asn Val Lys Val Asp Arg Asp
   325 330 335
   His Thr Ala Leu Ala Gly Leu Ser Met Gly Gly Gly Gln Thr Ile Ser
   340 345 350
   Ile Gly Ile Gly Glu Thr Leu Asp Glu Ile Ser Asn Tyr Gly Ser Phe
   355 360 365
   Ser Pro Ala Leu Phe Gln Thr Ala Glu Glu Phe Phe Gly Lys Val Lys
   370 375 380
   Gly Asn Phe Lys Glu Glu Leu Arg Ile His Asn Leu Tyr Met Thr Cys
   385 390 395 400
   Gly Asp Ala Asp Thr Leu Val Tyr Asp Thr Tyr Pro Ser Tyr Val Glu
   405 410 415
   Ala Leu Lys Asn Trp Asp Ala Val Glu Phe Met Lys Glu Tyr Thr Tyr
   420 425 430
   Pro Gly Gly Thr His Asp Phe Pro Val Trp Tyr Arg Gly Phe Asn Glu
   435 440 445
   Phe Ile Gln Ile Val Phe Lys Asn Gln Lys Val Lys Glu Glu Pro Ile
   450 455 460
   His Ala Asp Pro Val Glu Asp Pro Ser Asp Glu Pro Val Ser Val Asp
   465 470 475 480
   Pro Ser Val Ser Val Glu Glu Pro Asn Asp Ser Glu Ser Ser Ser Glu
   485 490 495
   Asp Glu Pro Val Val Lys Lys Thr Ile Lys His Thr Ile Ala Lys Lys
   500 505 510
   Lys Pro Ser Lys Thr Arg Thr Val Thr Lys Lys Val Ile Lys Lys Lys
   515 520 525
   Asn Asn
   530
<210> 80
   <211> 499
   <212> PRT
   <213> Schizophyllum commune
<400> 80
   Gln Gly Asp Phe Ser Ala Asn Cys Ser Ser Phe Val Asp Ala Ile Thr
   1               5                   10                  15
   Leu Glu Asn Val Thr Val Gln Ser Thr Glu Phe Val Ala Ala Gly Thr
               20                  25                  30
   Asn Val Ser Val Tyr Val Pro Glu Ser Cys Met Ser Ala Ser Tyr Gln
           35                  40                  45
   Val Val Ser Ala Asp Leu Cys Arg Ala Thr Met Asn Val Ser Thr Ser
       50                  55                  60
   Asp Arg Ser Gly Ile Arg Leu Glu Ala Trp Phe Pro Gln Asn Tyr Thr
   65                  70                  75                  80
   Gly Arg Phe Leu Ser Thr Gly Asn Gly Gly Ile Gly Gly Cys Ile Gln
                   85                  90                  95
   Tyr Ser Asp Leu Asp Tyr Ala Ala Ser Leu Gly Phe Ala Ala Val Gly
               100                 105                 110
   Ala Asn Asn Gly His Asp Gly Met Thr Gly Glu Pro Phe Leu Asn Asn
           115                 120                 125
   Pro Asp Val Ile Thr Asp Phe Ala Trp Arg Ser Leu His Thr Gly Val
       130                 135                 140
   Val Val Gly Lys Gln Leu Val Glu Thr Phe Tyr Gly Ala Pro His Ser
   145                 150                 155                 160
   Lys Ser Tyr Tyr Leu Gly Cys Ser Thr Gly Gly Arg Gln Gly Trp Lys
                   165                 170                 175
   Met Val Gln Asp Phe Pro Glu Asp Phe Asp Gly Val Val Ala Gly Ala
               180                 185                 190
   Pro Ala Met Ala Phe Asn Ala Leu Leu Tyr Trp Ser Gly Ser Phe Tyr
           195                 200                 205
   Thr Val Thr Gly Thr Ser Asp Asp Pro Thr Phe Val Pro Val Asp Phe
       210                 215                 220
   Trp Ala Asn Asn Ile His Gln Ala Val Leu Asp Gln Cys Asp Ala Leu
   225                 230                 235                 240
   Asp Gly Ala Thr Asp Gly Val Leu Glu Asn Pro Asp Leu Cys Asp Phe
                   245                 250                 255
   Asp Val Thr Pro Leu Asn Cys Ala Asp Gly Asn Thr Thr Asp Cys Leu
               260                 265                 270
   Thr Glu Thr Gln Ala Glu Thr Val Arg Thr Val Leu Ser Ala Ile Tyr
           275                 280                 285
   Asp Ala Asn Gly Thr Leu Val Tyr Pro Arg Leu Gln Pro Gly Ala Glu
       290                 295                 300
   Val Leu Ala Gly Gln Gln Gln Phe Asn Gly Gln Pro Phe Asp Ile Thr
   305                 310                 315                 320
   Gln Asp Trp Tyr Arg Tyr Val Ile Tyr Asn Asp Ser Thr Trp Asp Pro
                   325                 330                 335
   Ala Thr Leu Asn Leu Gly Asp Tyr Ala Val Ala Leu Ala Gln Asn Pro
               340                 345                 350
   Ala Asn Ile Glu Thr Phe Asn Gly Asp Ile Ser Ala Phe Glu Ala Ala
           355                 360                 365
   Gly Gly Lys Val Leu His Tyr His Gly Leu Met Asp Gly Leu Ile Ser
       370                 375                 380
   Ser Asp Asn Ser Lys Arg Tyr Tyr Lys Leu Val Gln Glu Thr Met Gly
   385                 390                 395                 400
   Lys Asp Ser Ser Glu Leu Asp Asp Phe Tyr Arg Phe Phe Pro Ile Ser
                   405                 410                 415
   Gly Met Ser His Cys Ser Gly Gly Asp Gly Ala Tyr Arg Ile Gly Asn
               420                 425                 430
   Val Asp Gly Gly Ala Gly Thr Ser Ala Asp Asp Asn Val Leu Met Ser
           435                 440                 445
   Met Val Arg Trp Val Glu Glu Gly Val Ala Pro Glu Val Val Arg Gly
       450                 455                 460
   Ala Asn Ala Asn Val Thr Tyr Trp Arg Ser His Cys Lys Trp Pro Leu
   465                 470                 475                 480
   Thr Asn Lys Tyr Val Gly Pro Gly Ser Tyr Glu Asp Glu Ser Ala Trp
                   485                 490                 495
   Gln Cys Ser
<210> 81
   <211> 499
   <212> PRT
   <213> Schizophyllum commune
<400> 81
   Gln Asp Asp Phe Ala Ser Asp Cys Ser Ala Phe Ile Asp Lys Ile Ile
   1               5                   10                  15
   Leu Asp Asn Val Thr Val Thr Ser Thr Glu Phe Val Ala Ala Gly Thr
               20                  25                  30
   Asn Leu Thr Val Tyr Val Pro Asn Ser Cys Gly Ser Pro Ser Tyr Gln
           35                  40                  45
   Val Val Ser Thr Asp Leu Cys Arg Ala Thr Met Asn Val Thr Thr Ser
       50                  55                  60
   Glu Arg Ser Gly Ile Arg Leu Glu Ala Trp Phe Pro Gln Asn Tyr Thr
   65                  70                  75                  80
   Gly Arg Phe Leu Ser Thr Gly Asn Gly Gly Ile Gly Gly Cys Ile Gln
                   85                  90                  95
   Tyr Ser Asp Ile Asp Tyr Ala Ala Ser Leu Gly Phe Ala Ala Val Gly
               100                 105                 110
   Ala Asn Asn Gly His Asp Gly Met Thr Gly Glu Pro Phe Leu Asn Asn
           115                 120                 125
   Pro Asp Val Val Thr Asp Phe Ala Trp Arg Ser Leu His Thr Gly Val
       130                 135                 140
   Val Val Gly Lys Gln Leu Thr Glu Thr Phe Tyr Gly Ala Pro His Asn
   145                 150                 155                 160
   Lys Ser Tyr Tyr Leu Gly Cys Ser Thr Gly Gly Arg Gln Gly Phe Lys
                   165                 170                 175
   Ala Val Gln Asp Phe Pro Asp Asp Phe Asp Gly Val Val Ala Gly Ala
               180                 185                 190
   Pro Ala Phe Ala Phe Asn Gly Leu Leu Tyr Trp Ser Gly Ser Phe Leu
           195                 200                 205
   Pro Val Thr Gly Thr Ser Glu Asp Pro Thr Phe Val Pro Thr Asp Phe
       210                 215                 220
   Trp Ala Gln Asn Ile His Ala Glu Val Leu Gln Gln Cys Asp Ala Leu
   225                 230                 235                 240
   Asp Gly Ala Thr Asp Gly Val Leu Glu Asn Pro Asp Leu Cys Asp Phe
                   245                 250                 255
   Asp Val Thr Pro Leu Ile Cys Thr Asp Gly Asn Thr Thr Gly Cys Leu
               260                 265                 270
   Thr Glu Thr Gln Ala Glu Thr Val Arg Thr Val Leu Ser Pro Ile Tyr
           275                 280                 285
   Asp Lys Asn Gly Thr Leu Val Tyr Pro Arg Leu Gln Pro Gly Ala Glu
       290                 295                 300
   Val Leu Ala Gly Phe Ala Gln Phe Gly Gly Gln Pro Phe Leu Tyr Ser
   305                 310                 315                 320
   Thr Asp Trp Tyr Arg Tyr Val Val Tyr Asn Asp Ser Asn Trp Asp Pro
                   325                 330                 335
   Met Thr Leu Ser Ile Asp Asp Tyr Ala Ala Ala Arg Ala Gln Asn Pro
               340                 345                 350
   Ala Asn Val Glu Thr Phe Glu Gly Asp Ile Ser Ala Phe Ala Ser Ser
           355                 360                 365
   Gly Gly Lys Val Leu His Tyr His Gly Leu Met Asp Gly Leu Ile Ser
       370                 375                 380
   Ser Asp Asn Ser Lys Arg Tyr Tyr Ala Leu Val Gln Gln Thr Leu Asn
   385                 390                 395                 400
   Gln Glu Pro Ala Glu Leu Asp Glu Phe Tyr Arg Phe Phe Pro Ile Ser
                   405                 410                 415
   Gly Met Ser His Cys Ser Gly Gly Asp Gly Ala Tyr Arg Ile Gly Asn
               420                 425                 430
   Val Glu Gly Gly Ala Gly Thr Ser Ala Asp Asp Asn Val Leu Met Ser
           435                 440                 445
   Met Val Arg Trp Val Glu Glu Gly Val Ala Pro Glu Val Val Arg Gly
       450                 455                 460
   Ala Asp Ala Asn Ala Thr Tyr Trp Arg Ala His Cys Lys Trp Pro Lys
   465 470 475 480
   Thr Asn Lys Tyr Val Gly Pro Gly Asp Tyr Glu Asp Glu Ser Ala Trp
   485 490 495
   Glu Cys Ser
<210> 82
   <211> 526
   <212> PRT
   <213> Aspergillus oryzae
<400> 82
   Met Pro Ser Leu Arg Arg Leu Leu Pro Phe Leu Ala Ala Gly Ser Ala
   1 5 10 15
   Ala Leu Ala Ser Gln Asp Thr Phe Gln Gly Lys Cys Thr Gly Phe Ala
   20 25 30
   Asp Lys Ile Asn Leu Pro Asn Val Arg Val Asn Phe Val Asn Tyr Val
   35 40 45
   Pro Gly Gly Thr Asn Leu Ser Leu Pro Asp Asn Pro Thr Ser Cys Gly
   50 55 60
   Thr Thr Ser Gln Val Val Ser Glu Asp Val Cys Arg Ile Ala Met Ala
   65 70 75 80
   Val Ala Thr Ser Asn Ser Ser Glu Ile Thr Leu Glu Ala Trp Leu Pro
   85 90 95
   Gln Asn Tyr Thr Gly Arg Phe Leu Ser Thr Gly Asn Gly Gly Leu Ser
   100 105 110
   Gly Cys Ile Gln Tyr Tyr Asp Leu Ala Tyr Thr Ser Gly Leu Gly Phe
   115 120 125
   Ala Thr Val Gly Ala Asn Ser Gly His Asn Gly Thr Ser Gly Glu Pro
   130 135 140
   Phe Tyr His His Pro Glu Val Leu Glu Asp Phe Val His Arg Ser Val
   145 150 155 160
   His Thr Gly Val Val Val Gly Lys Gln Leu Thr Lys Leu Phe Tyr Glu
   165 170 175
   Glu Gly Phe Lys Lys Ser Tyr Tyr Leu Gly Cys Ser Thr Gly Gly Arg
   180 185 190
   Gln Gly Phe Lys Ser Val Gln Lys Tyr Pro Asn Asp Phe Asp Gly Val
   195 200 205
   Val Ala Gly Ala Pro Ala Phe Asn Met Ile Asn Leu Met Ser Trp Ser
   210 215 220
   Ala His Phe Tyr Ser Ile Thr Gly Pro Val Gly Ser Asp Thr Tyr Leu
   225 230 235 240
   Ser Pro Asp Leu Trp Asn Ile Thr His Lys Glu Ile Leu Arg Gln Cys
   245 250 255
   Asp Gly Ile Asp Gly Ala Glu Asp Gly Ile Ile Glu Asp Pro Ser Leu
   260 265 270
   Cys Ser Pro Val Leu Glu Ala Ile Ile Cys Lys Pro Gly Gln Asn Thr
   275 280 285
   Thr Glu Cys Leu Thr Gly Lys Gln Ala His Thr Val Arg Glu Ile Phe
   290 295 300
   Ser Pro Leu Tyr Gly Val Asn Gly Thr Leu Leu Tyr Pro Arg Met Gln
   305 310 315 320
   Pro Gly Ser Glu Val Met Ala Ser Ser Ile Met Tyr Asn Gly Gln Pro
   325 330 335
   Phe Gln Tyr Ser Ala Asp Trp Tyr Arg Tyr Val Val Tyr Glu Asn Pro
   340 345 350
   Asn Trp Asp Ala Thr Lys Phe Ser Val Arg Asp Ala Ala Val Ala Leu
   355 360 365
   Lys Gln Asn Pro Phe Asn Leu Gln Thr Trp Asp Ala Asp Ile Ser Ser
   370 375 380
   Phe Arg Lys Ala Gly Gly Lys Val Leu Thr Tyr His Gly Leu Met Asp
   385 390 395 400
   Gln Leu Ile Ser Ser Glu Asn Ser Lys Leu Tyr Tyr Ala Arg Val Ala
   405 410 415
   Glu Thr Met Asn Val Pro Pro Glu Glu Leu Asp Glu Phe Tyr Arg Phe
                   420                 425                 430
   Phe Gln Ile Ser Gly Met Ala His Cys Ser Gly Gly Asp Gly Ala Tyr
           435                 440                 445
   Gly Ile Gly Asn Gln Leu Val Thr Tyr Asn Asp Ala Asn Pro Glu Asn
       450                 455                 460
   Asn Val Leu Met Ala Met Val Gln Trp Val Glu Lys Gly Ile Ala Pro
   465                 470                 475                 480
   Glu Thr Ile Arg Gly Ala Lys Phe Thr Asn Gly Thr Gly Ser Ala Val
                   485                 490                 495
   Glu Tyr Thr Arg Lys His Cys Arg Tyr Pro Arg Arg Asn Val Tyr Lys
               500                 505                 510
   Gly Pro Gly Asn Tyr Thr Asp Glu Asn Ala Trp Gln Cys Val
           515                 520                 525
<210> 83
   <211> 281
   <212> PRT
   <213> Aspergillus niger
<400> 83
   Met Lys Gln Phe Ser Ala Lys Tyr Ala Leu Ile Leu Leu Ala Thr Ala
   1               5                   10                  15
   Gly Gln Ala Leu Ala Ala Ser Thr Gln Gly Ile Ser Glu Asp Leu Tyr
               20                  25                  30
   Asn Arg Leu Val Glu Met Ala Thr Ile Ser Gln Ala Ala Tyr Ala Asp
           35                  40                  45
   Leu Cys Asn Ile Pro Ser Thr Ile Ile Lys Gly Glu Lys Ile Tyr Asn
       50                  55                  60
   Ala Gln Thr Asp Ile Asn Gly Trp Ile Leu Arg Asp Asp Thr Ser Lys
   65                  70                  75                  80
   Glu Ile Ile Thr Val Phe Arg Gly Thr Gly Ser Asp Thr Asn Leu Gln
                   85                  90                  95
   Leu Asp Thr Asn Tyr Thr Leu Thr Pro Phe Asp Thr Leu Pro Gln Cys
               100                 105                 110
   Asn Asp Cys Glu Val His Gly Gly Tyr Tyr Ile Gly Trp Ile Ser Val
   115 120 125
   Gln Asp Gln Val Glu Ser Leu Val Lys Gln Gln Ala Ser Gln Tyr Pro
   130 135 140
   Asp Tyr Ala Leu Thr Val Thr Gly His Ser Leu Gly Ala Ser Met Ala
   145 150 155 160
   Ala Leu Thr Ala Ala Gln Leu Ser Ala Thr Tyr Asp Asn Val Arg Leu
   165 170 175
   Tyr Thr Phe Gly Glu Pro Arg Ser Gly Asn Gln Ala Phe Ala Ser Tyr
   180 185 190
   Met Asn Asp Ala Phe Gln Val Ser Ser Pro Glu Thr Thr Gln Tyr Phe
   195 200 205
   Arg Val Thr His Ser Asn Asp Gly Ile Pro Asn Leu Pro Pro Ala Asp
   210 215 220
   Glu Gly Tyr Ala His Gly Gly Val Glu Tyr Trp Ser Val Asp Pro Tyr
   225 230 235 240
   Ser Ala Gln Asn Thr Phe Val Cys Thr Gly Asp Glu Val Gln Cys Cys
   245 250 255
   Glu Ala Gln Gly Gly Gln Gly Val Asn Asp Ala His Thr Thr Tyr Phe
   260 265 270
   Gly Met Thr Ser Gly Ala Cys Thr Trp
   275 280
<210> 84
   <211> 521
   <212> PRT
   <213> Aspergillus niger
<400> 84
   Met Lys Val Ala Ser Leu Leu Ser Leu Ala Leu Pro Gly Ala Ala Leu
   1               5                   10                  15
   Ala Ala Thr Asp Pro Phe Gln Ser Arg Cys Asn Glu Phe Gln Asn Lys
               20                  25                  30
   Ile Asp Ile Ala Asn Val Thr Val Arg Ser Val Ala Tyr Val Ala Ala
           35                  40                  45
   Gly Gln Asn Ile Ser Gln Ala Glu Val Ala Ser Val Cys Lys Ala Ser
       50                  55                  60
   Val Gln Ala Ser Val Asp Leu Cys Arg Val Thr Met Asn Ile Ser Thr
   65                  70                  75                  80
   Ser Asp Arg Ser His Leu Trp Ala Glu Ala Trp Leu Pro Arg Asn Tyr
                   85                  90                  95
   Thr Gly Arg Phe Val Ser Thr Gly Asn Gly Gly Leu Ala Gly Cys Val
               100                 105                 110
   Gln Glu Thr Asp Leu Asn Phe Ala Ala Asn Phe Gly Phe Ala Thr Val
           115                 120                 125
   Gly Thr Asn Gly Gly His Asp Gly Asp Thr Ala Lys Tyr Phe Leu Asn
       130                 135                 140
   Asn Ser Glu Val Leu Ala Asp Phe Ala Tyr Arg Ser Val His Glu Gly
   145                 150                 155                 160
   Thr Val Val Gly Lys Gln Leu Thr Gln Leu Phe Tyr Asp Glu Gly Tyr
                   165                 170                 175
   Asn Tyr Ser Tyr Tyr Leu Gly Cys Ser Thr Gly Gly Arg Gln Gly Tyr
               180                 185                 190
   Gln Gln Val Gln Arg Phe Pro Asp Asp Tyr Asp Gly Val Ile Ala Gly
           195                 200                 205
   Ser Ala Ala Met Asn Phe Ile Asn Leu Ile Ser Trp Gly Ala Phe Leu
       210                 215                 220
   Trp Lys Ala Thr Gly Leu Ala Asp Asp Pro Asp Phe Ile Ser Ala Asn
   225                 230                 235                 240
   Leu Trp Ser Val Ile His Gln Glu Ile Val Arg Gln Cys Asp Leu Val
                   245                 250                 255
   Asp Gly Ala Leu Asp Gly Ile Ile Glu Asp Pro Asp Phe Cys Ala Pro
               260                 265                 270
   Val Ile Glu Arg Leu Ile Cys Asp Gly Thr Thr Asn Gly Thr Ser Cys
           275                 280                 285
   Ile Thr Gly Ala Gln Ala Ala Lys Val Asn Arg Ala Leu Ser Asp Phe
       290                 295                 300
   Tyr Gly Pro Asp Gly Thr Val Tyr Tyr Pro Arg Leu Asn Tyr Gly Gly
   305 310 315 320
   Glu Ala Asp Ser Ala Ser Leu Tyr Phe Thr Gly Ser Met Tyr Ser Arg
   325 330 335
   Thr Glu Glu Trp Tyr Lys Tyr Val Val Tyr Asn Asp Thr Asn Trp Asn
   340 345 350
   Ser Ser Gln Trp Thr Leu Glu Ser Ala Lys Leu Ala Leu Glu Gln Asn
   355 360 365
   Pro Phe Asn Ile Gln Ala Phe Asp Pro Asn Ile Thr Ala Phe Arg Asp
   370 375 380
   Arg Gly Gly Lys Leu Leu Ser Tyr His Gly Thr Gln Asp Pro Ile Ile
   385 390 395 400
   Ser Ser Thr Asp Ser Lys Leu Tyr Tyr Arg Arg Val Ala Asn Ala Leu
   405 410 415
   Asn Ala Ala Pro Ser Glu Leu Asp Glu Phe Tyr Arg Phe Phe Gln Ile
   420 425 430
   Ser Gly Met Gly His Cys Gly Asp Gly Thr Gly Ala Ser Tyr Ile Gly
   435 440 445
   Gln Gly Tyr Gly Thr Tyr Thr Ser Lys Ala Pro Gln Val Asn Leu Leu
   450 455 460
   Arg Thr Met Val Asp Trp Val Glu Asn Gly Lys Ala Pro Glu Tyr Met
   465 470 475 480
   Pro Gly Asn Lys Leu Asn Ala Asn Gly Ser Ile Glu Tyr Met Arg Lys
   485 490 495
   His Cys Arg Tyr Pro Lys His Asn Ile His Thr Gly Pro Gly Asn Tyr
   500 505 510
   Thr Asp Pro Asn Ser Trp Thr Cys Val
   515 520
<210> 85
   <211> 270
   <212> PRT
   <213> Emericella nidulans
<400> 85
   Met Leu Arg Ala Val Leu Leu Pro Thr Leu Leu Ala Phe Gly Ala Phe
   1               5                   10                  15
   Thr Pro Val His Gly Ala Asn Ser Pro Gly Cys Gly Lys Gln Pro Thr
               20                  25                  30
   Leu Thr Asn Gly Val Asn Gln Ile Asn Gly Arg Glu Tyr Val Leu Lys
           35                  40                  45
   Ile Pro Asp Gly Tyr Asp Pro Ser Lys Pro His His Leu Ile Phe Gly
       50                  55                  60
   Leu His Trp Arg Gly Gly Asn Met Tyr Asn Val Val Asn Gly Asp Ser
   65                  70                  75                  80
   Ile Gln Pro Trp Tyr Gly Leu Glu Ala Arg Ala Gln Gly Ser Ala Ile
                   85                  90                  95
   Phe Val Ala Pro Asn Gly Leu Asn Ala Gly Trp Ala Asn Thr Asn Gly
               100                 105                 110
   Glu Asp Val Ala Phe Ile Asp Ala Ile Met Glu Gln Val Glu Asp Asp
           115                 120                 125
   Leu Cys Val Asp Gln Ala Ser Arg Phe Ala Thr Gly Phe Ser Trp Gly
       130                 135                 140
   Gly Gly Met Ser Tyr Ala Leu Ala Cys Ala Arg Ala Ala Glu Phe Arg
   145                 150                 155                 160
   Ala Val Ser Val Leu Ser Gly Gly Leu Ile Ser Gly Cys Asp Gly Gly
                   165                 170                 175
   Asn Asp Pro Ile Ala Tyr Leu Gly Ile His Gly Ile Asn Asp Pro Val
               180                 185                 190
   Leu Pro Leu Asp Gly Gly Val Thr Leu Ala Asn Thr Phe Val Ser Asn
           195                 200                 205
   Asn Gly Cys Gln Pro Thr Asp Ile Gly Gln Pro Ala Ser Gly Ser Gly
       210                 215                 220
   Gly Ser Val Arg Thr Asp Phe Ser Gly Cys Ser His Pro Val Ser Phe
   225                 230                 235                 240
   Ile Ala Tyr Asp Gly Gly His Asp Gly Ala Pro Leu Gly Val Gly Ser
                   245                 250                 255
   Ser Leu Ala Pro Asp Ala Thr Trp Glu Phe Phe Met Ala Ala
   260 265 270
<210> 86
   <211> 558
   <212> PRT
   <213> Fusarium oxysporum
<400> 86
   Met Leu Phe Ala Ser Leu Val Leu Val Leu Gly Phe Ile Pro Gln Val
   1 5 10 15
   Leu Ser Asp Thr Ser Thr Asp Ile Cys Leu Pro Gln Asp Asn Met Arg
   20 25 30
   Pro Thr Phe Leu Leu Phe Ser Gly Leu Gly Ala Cys Ala Ala Ala Gly
   35 40 45
   Lys Gly Asp Asp Phe Ala Ala Lys Cys Ala Gly Phe Lys Thr Ser Leu
   50 55 60
   Lys Leu Pro Asn Thr Lys Val Trp Phe Thr Glu His Val Pro Ala Gly
   65 70 75 80
   Lys Asn Ile Thr Phe Pro Asp Asn His Pro Thr Cys Thr Pro Lys Ser
   85 90 95
   Thr Ile Thr Asp Val Glu Ile Cys Arg Val Ala Met Phe Val Thr Thr
   100 105 110
   Gly Pro Lys Ser Asn Leu Thr Leu Glu Ala Trp Leu Pro Ser Asn Trp
   115 120 125
   Thr Gly Arg Phe Leu Ser Thr Gly Asn Gly Gly Met Ala Gly Cys Ile
   130 135 140
   Gln Tyr Asp Asp Val Ala Tyr Gly Ala Gly Phe Gly Phe Ala Thr Val
   145 150 155 160
   Gly Ala Asn Asn Gly His Asn Gly Thr Ser Ala Val Ser Met Tyr Lys
   165 170 175
   Asn Ser Gly Val Val Glu Asp Tyr Val Tyr Arg Ser Val His Thr Gly
   180 185 190
   Thr Val Leu Gly Lys Glu Leu Thr Lys Lys Phe Tyr Gly Lys Lys His
   195 200 205
   Thr Lys Ser Tyr Tyr Leu Gly Cys Ser Thr Gly Gly Arg Gln Gly Trp
       210                 215                 220
   Lys Glu Ala Gln Ser Phe Pro Asp Asp Phe Asp Gly Ile Val Ala Gly
   225                 230                 235                 240
   Ala Pro Ala Met Arg Phe Asn Gly Leu Gln Ser Arg Ser Gly Ser Phe
                   245                 250                 255
   Trp Gly Ile Thr Gly Pro Pro Gly Ala Pro Thr His Leu Ser Pro Glu
               260                 265                 270
   Glu Trp Ala Met Val Gln Lys Asn Val Leu Val Gln Cys Asp Glu Pro
           275                 280                 285
   Leu Asp Gly Val Ala Asp Gly Ile Leu Glu Asp Pro Asn Leu Cys Gln
       290                 295                 300
   Tyr Arg Pro Glu Ala Leu Val Cys Ser Lys Gly Gln Thr Lys Asn Cys
   305                 310                 315                 320
   Leu Thr Gly Pro Gln Ile Glu Thr Val Arg Lys Val Phe Gly Pro Leu
                   325                 330                 335
   Tyr Gly Asn Asn Gly Thr Tyr Ile Tyr Pro Arg Ile Pro Pro Gly Ala
               340                 345                 350
   Asp Gln Gly Phe Gly Phe Ala Ile Gly Glu Gln Pro Phe Pro Tyr Ser
           355                 360                 365
   Thr Glu Trp Phe Gln Tyr Val Ile Trp Asn Asp Thr Lys Trp Asp Pro
       370                 375                 380
   Asn Thr Ile Gly Pro Asn Asp Tyr Gln Lys Ala Ser Glu Val Asn Pro
   385                 390                 395                 400
   Phe Asn Val Glu Thr Trp Glu Gly Asp Leu Ser Lys Phe Arg Lys Arg
                   405                 410                 415
   Gly Ser Lys Ile Ile His Trp His Gly Leu Glu Asp Gly Leu Ile Ser
               420                 425                 430
   Ser Asp Asn Ser Met Glu Tyr Tyr Asn His Val Ser Ala Thr Met Gly
           435                 440                 445
   Leu Ser Asn Thr Glu Leu Asp Glu Phe Tyr Arg Tyr Phe Arg Val Ser
       450                 455                 460
   Gly Cys Gly His Cys Ser Gly Gly Ile Gly Ala Asn Arg Ile Gly Asn
   465 470 475 480
   Asn Arg Ala Asn Leu Gly Gly Lys Glu Ala Lys Asn Asn Val Leu Leu
   485 490 495
   Ala Leu Val Lys Trp Val Glu Glu Asp Gln Ala Pro Glu Thr Ile Thr
   500 505 510
   Gly Val Arg Tyr Val Asn Gly Ala Thr Thr Gly Lys Val Glu Val Glu
   515 520 525
   Arg Arg His Cys Arg Tyr Pro Tyr Arg Asn Val Trp Asp Arg Lys Gly
   530 535 540
   Asn Tyr Lys Asn Pro Asp Ser Trp Lys Cys Glu Leu Pro Lys
   545 550 555
<210> 87
   <211> 279
   <212> PRT
   <213> Myceliophthora thermophila
<400> 87
   Met Ile Ser Val Pro Ala Leu Ala Leu Ala Leu Leu Ala Ala Val Gln
   1 5 10 15
   Val Val Glu Ser Ala Ser Ala Gly Cys Gly Lys Ala Pro Pro Ser Ser
   20 25 30
   Gly Thr Lys Ser Met Thr Val Asn Gly Lys Gln Arg Gln Tyr Ile Leu
   35 40 45
   Gln Leu Pro Asn Asn Tyr Asp Ala Asn Lys Ala His Arg Val Val Ile
   50 55 60
   Gly Tyr His Trp Arg Asp Gly Ser Met Asn Asp Val Ala Asn Gly Gly
   65 70 75 80
   Phe Tyr Asp Leu Arg Ser Arg Ala Gly Asp Ser Thr Ile Phe Val Ala
   85 90 95
   Pro Asn Gly Leu Asn Ala Gly Trp Ala Asn Val Gly Gly Glu Asp Ile
   100 105 110
   Thr Phe Thr Asp Gln Ile Val Asp Met Leu Lys Asn Asp Leu Cys Val
   115 120 125
   Asp Glu Thr Gln Phe Phe Ala Thr Gly Trp Ser Tyr Gly Gly Ala Met
       130                 135                 140
   Ser His Ser Val Ala Cys Ser Arg Pro Asp Val Phe Lys Ala Val Ala
   145                 150                 155                 160
   Val Ile Ala Gly Ala Gln Leu Ser Gly Cys Ala Gly Gly Thr Thr Pro
                   165                 170                 175
   Val Ala Tyr Leu Gly Ile His Gly Ala Ala Asp Asn Val Leu Pro Ile
               180                 185                 190
   Asp Leu Gly Arg Gln Leu Arg Asp Lys Trp Leu Gln Thr Asn Gly Cys
           195                 200                 205
   Asn Tyr Gln Gly Ala Gln Asp Pro Ala Pro Gly Gln Gln Ala His Ile
       210                 215                 220
   Lys Thr Thr Tyr Ser Cys Ser Arg Ala Pro Val Thr Trp Ile Gly His
   225                 230                 235                 240
   Gly Gly Gly His Val Pro Asp Pro Thr Gly Asn Asn Gly Val Lys Phe
                   245                 250                 255
   Ala Pro Gln Glu Thr Trp Asp Phe Phe Asp Ala Ala Val Gly Ala Ala
               260                 265                 270
   Gly Ala Gln Ser Pro Met Thr
           275
<210> 88
   <211> 302
   <212> PRT
   <213> Myceliophthora thermophila
<400> 88
   Met Ser Arg Tyr Leu Leu Gly Arg Leu Ile Val Ala Thr Val Ala Ile
   1               5                   10                  15
   Thr Val Ala Ser Leu Arg Gly Val Thr Ala Ala Thr Pro Ser Pro Gly
               20                  25                  30
   Cys Gly Lys Thr Pro Thr Leu Ile Thr Asp Gly Ser Ala Thr Thr Pro
           35                  40                  45
   Leu Thr Leu Thr Ser Asn Gly Lys Thr Arg Arg Phe Tyr Val Lys Leu
       50                  55                  60
   Pro Asp Asp Tyr Asp Asn Ser His Pro Tyr Arg Leu Ile Phe Ala Leu
   65                  70                  75                  80
   His Ala Leu Gly Gly Thr Ala Gln Gln Val Thr Thr Gly Thr Gly Gly
                   85                  90                  95
   Tyr Leu Pro Trp Tyr Gly Ile Pro Asp Leu Ala Ala Asn Asp Thr Val
               100                 105                 110
   Gly Ala Val Tyr Val Ala Pro Asp Gly Leu Asn Asn Gly Trp Ala Asn
           115                 120                 125
   Gln Gly Gly Glu Asp Val Ala Phe Leu Glu Ala Val Met Glu Thr Val
       130                 135                 140
   Glu Gln Asp Val Cys Val Asp Arg Asp Leu Arg Phe Ser Thr Gly Phe
   145                 150                 155                 160
   Ser Tyr Gly Ala Ala Met Ser Tyr Thr Leu Ala Cys Ala Leu Gly Arg
                   165                 170                 175
   Arg Ile Arg Ala Val Ala Val Leu Ser Gly Ser Pro Val Ile Ser Gly
               180                 185                 190
   Gly Cys Ala Gly Ala Gly Ser Gly Ala Ser Glu Pro Val Ala Tyr Tyr
           195                 200                 205
   Gly Gln His Gly Met Ser Asp Pro Val Leu Pro Val Ala Gly Gly Arg
       210                 215                 220
   Glu Met Arg Asp His Phe Val Arg Thr Asn Gly Cys Asp Ala Gly Arg
   225                 230                 235                 240
   Gly Pro Pro Arg Glu Pro Ala Arg Gly Ser Gly Thr His Val Lys Thr
                   245                 250                 255
   Val Tyr Asp Gly Cys Asp Pro Asp Tyr Pro Val Val Trp Asn Ala Phe
               260                 265                 270
   Asp Gly Asp His Thr Pro Gln Pro Val Asp Arg Gly Ala Thr Thr Thr
           275                 280                 285
   Phe Ser Ala Val Glu Thr Trp Glu Phe Phe Ser Gln Phe Lys
       290                 295                 300
<210> 89
   <211> 319
   <212> PRT
   <213> Myceliophthora thermophila
<400> 89
   Met Pro Cys Lys Ser Ser Leu Leu Leu Leu Leu Leu Ser Ser Phe Phe
   1               5                   10                  15
   Ser Ser Glu Ala Asn Thr Ala His Arg Pro Pro Cys Val Ser Ala Ile
               20                  25                  30
   Ala His Gln Asp Gly Arg His Gly Arg Pro Ala Arg Gly Ala Ser Leu
           35                  40                  45
   Gln Leu Val Thr Asn Phe Gly Asp Asn Pro Thr Gly Leu Gln Lys Tyr
       50                  55                  60
   Val Tyr Val Pro Asp Lys Val Ala Val Ser Pro Ala Ile Ile Val Ala
   65                  70                  75                  80
   Leu His Pro Cys Gly Gly Ser Ala Gln Gly Trp Tyr Ser Gln Thr Arg
                   85                  90                  95
   Leu Pro Ser Tyr Ala Asp Gln Leu Gly Phe Ile Leu Ile Tyr Ala Gly
               100                 105                 110
   Thr Thr Lys Met Ser Asn Cys Trp Asp Val Gln Asn Pro Ala Ser Leu
           115                 120                 125
   Thr His Asn Gly Gly Gly Asp Ala Gly Gly Ile Val Ser Met Val Lys
       130                 135                 140
   Tyr Ala Leu Lys Gln Tyr Asn Gly Asp Ala Ser Arg Val Tyr Val Met
   145                 150                 155                 160
   Gly Gly Ser Ser Gly Ala Met Met Thr Asn Val Leu Ala Gly Ser Tyr
                   165                 170                 175
   Pro Asp Val Phe Glu Ala Gly Ala Ala Phe Ser Gly Val Ala His Ala
               180                 185                 190
   Cys Phe Leu Gly Ala Asp Ser Ala Thr Pro Phe Ser Pro Gln Thr Cys
           195                 200                 205
   Ala Gln Gly Arg Ile Gln Arg Ser Ala Arg Glu Trp Gly Asp Leu Val
       210                 215                 220
   Arg Asn Ser Phe Pro Ala Tyr Asp Gly Arg Arg Pro Arg Met Gln Ile
   225 230 235 240
   Phe His Gly Asn Ala Asp Phe Leu Val His Pro Glu Cys Ala His Gln
   245 250 255
   Ala Leu Ala Gln Trp Ala Asp Val Leu Gly Leu Gln Leu Thr Gln Thr
   260 265 270
   Asn Lys Gly Val Pro Ser Ala Glu Tyr Thr Gln Glu Val Tyr Gly Asp
   275 280 285
   Gly Thr Gln Leu Gln Gly Phe Phe Gly Asp Gly Val Gly His Ile Ala
   290 295 300
   Pro Val Asn Glu Pro Val Met Leu Arg Phe Phe Gly Leu Met Asn
   305 310 315
<210> 90
   <211> 291
   <212> PRT
   <213> Myceliophthora thermophila
<400> 90
   Met Leu Val Arg Ser Phe Leu Gly Phe Ala Val Leu Ala Ala Thr Cys
   1               5                   10                  15
   Leu Ala Ala Ser Leu Gln Glu Val Thr Glu Phe Gly Asp Asn Pro Thr
               20                  25                  30
   Asn Ile Gln Met Tyr Ile Tyr Val Pro Asp Gln Leu Asp Thr Asn Pro
           35                  40                  45
   Pro Val Ile Val Ala Leu His Pro Cys Gly Gly Ser Ala Gln Gln Trp
       50                  55                  60
   Phe Ser Gly Thr Gln Leu Pro Ser Tyr Ala Asp Asp Asn Gly Phe Ile
   65                  70                  75                  80
   Leu Ile Tyr Pro Ser Thr Pro His Met Ser Asn Cys Trp Asp Ile Gln
                   85                  90                  95
   Asn Pro Asp Thr Leu Thr His Gly Gln Gly Gly Asp Ala Leu Gly Ile
               100                 105                 110
   Val Ser Met Val Asn Tyr Thr Leu Asp Lys His Ser Gly Asp Ser Ser
           115                 120                 125
   Arg Val Tyr Ala Met Gly Phe Ser Ser Gly Gly Met Met Thr Asn Gln
   130 135 140
   Leu Ala Gly Ser Tyr Pro Asp Val Phe Glu Ala Gly Ala Val Tyr Ser
   145 150 155 160
   Gly Val Ala Phe Gly Cys Ala Ala Gly Ala Glu Ser Ala Thr Pro Phe
   165 170 175
   Ser Pro Asn Gln Thr Cys Ala Gln Gly Leu Gln Lys Thr Ala Gln Glu
   180 185 190
   Trp Gly Asp Phe Val Arg Asn Ala Tyr Ala Gly Tyr Thr Gly Arg Arg
   195 200 205
   Pro Arg Met Gln Ile Phe His Gly Leu Glu Asp Thr Leu Val Arg Pro
   210 215 220
   Gln Cys Ala Glu Glu Ala Leu Lys Gln Trp Ser Asn Val Leu Gly Val
   225 230 235 240
   Glu Leu Thr Gln Glu Val Ser Gly Val Pro Ser Pro Gly Trp Thr Gln
   245 250 255
   Lys Ile Tyr Gly Asp Gly Thr Gln Leu Gln Gly Phe Phe Gly Gln Gly
   260 265 270
   Ile Gly His Gln Ser Thr Val Asn Glu Gln Gln Leu Leu Gln Trp Phe
   275 280 285
   Gly Leu Ile
   290
<210> 91
   <211> 1316
   <212> DNA
   <213> Myceliophthora thermophila
<400> 91
   atgaagtcct tcaccctcac cactctggcc gccctggctg gcaacgccgc cgctcacgcg      60
   accttccagg ccctctgggt cgacggcgtc gactacggcg cgcagtgtgc ccgtctgccc     120
   gcgtccaact cgccggtcac cgacgtgacc tccaacgcga tccgctgcaa cgccaacccc     180
   tcgcccgctc ggggcaagtg cccggtcaag gccggctcga ccgttacggt cgagatgcat     240
   caggtacgtt ggatgaatga gagaggaaag aaagcagaag cagaagggga agggaaagaa     300
   aaagaaaaag aaaagaaaaa gagaaagaaa aagaaagtgg aaaccgtcgg actaactggg     360
   ctcttcttcc ccccctttga tatcagcaac ccggtgaccg ctcgtgcagc agcgaggcga     420
   tcggcggggc gcactacggc cccgtgatgg tgtacatgtc caaggtgtcg gacgcggcgt     480
   cggcggacgg gtcgtcgggc tggttcaagg tgttcgagga cggctgggcc aagaacccgt     540
   ccggcgggtc gggcgacgac gactactggg gcaccaagga cctgaactcg tgctgcggga     600
   agatgaacgt caagatcccc gccgacctgc cctcgggcga ctacctgctc cgggccgagg     660
   ccctcgcgct gcacacggcc ggcagcgcgg gcggcgccca gttctacatg acctgctacc     720
   agctcaccgt gaccggctcc ggcagcgcca gcccgcccac cgtctccttc ccgggcgcct     780
   acaaggccac cgacccgggc atcctcgtca acatccacgc cccgctgtcc ggctacaccg     840
   tgcccggccc ggccgtctac tcgggcggct ccaccaagaa ggccggcagc gcctgcaccg     900
   gctgcgagtc cacttgcgcc gtcggctccg gccccaccgc caccgtctcc cagtcgcccg     960
   gttccaccgc cacctcggcc cccggcggcg gcggcggctg caccgtccag aagtaccagc    1020
   agtgcggcgg ccagggctac accggctgca ccaactgcgc ggtacgtttt tttcttcagc    1080
   ccttttttcc cgtctcctct tattatttat tccctctgga tttcttcctt gccttctctg    1140
   tccatacctc tctgcataca tagggagcct agagacgacc cctaccctac atctccctat    1200
   cttatctggt tacctaatta cttacctcct caatgtttct ggctgctgac tttttgcttt    1260
   agtccggctc cacctgcagc gcggtctcgc cgccctacta ctcgcagtgc gtctaa        1316
<210> 92
   <211> 323
   <212> PRT
   <213> Myceliophthora thermophila
<400> 92
   Met Lys Ser Phe Thr Leu Thr Thr Leu Ala Ala Leu Ala Gly Asn Ala
   1               5                   10                  15
   Ala Ala His Ala Thr Phe Gln Ala Leu Trp Val Asp Gly Val Asp Tyr
               20                  25                  30
   Gly Ala Gln Cys Ala Arg Leu Pro Ala Ser Asn Ser Pro Val Thr Asp
           35                  40                  45
   Val Thr Ser Asn Ala Ile Arg Cys Asn Ala Asn Pro Ser Pro Ala Arg
       50                  55                  60
   Gly Lys Cys Pro Val Lys Ala Gly Ser Thr Val Thr Val Glu Met His
   65                  70                  75                  80
   Gln Gln Pro Gly Asp Arg Ser Cys Ser Ser Glu Ala Ile Gly Gly Ala
                   85                  90                  95
   His Tyr Gly Pro Val Met Val Tyr Met Ser Lys Val Ser Asp Ala Ala
   100 105 110
   Ser Ala Asp Gly Ser Ser Gly Trp Phe Lys Val Phe Glu Asp Gly Trp
   115 120 125
   Ala Lys Asn Pro Ser Gly Gly Ser Gly Asp Asp Asp Tyr Trp Gly Thr
   130 135 140
   Lys Asp Leu Asn Ser Cys Cys Gly Lys Met Asn Val Lys Ile Pro Ala
   145 150 155 160
   Asp Leu Pro Ser Gly Asp Tyr Leu Leu Arg Ala Glu Ala Leu Ala Leu
   165 170 175
   His Thr Ala Gly Ser Ala Gly Gly Ala Gln Phe Tyr Met Thr Cys Tyr
   180 185 190
   Gln Leu Thr Val Thr Gly Ser Gly Ser Ala Ser Pro Pro Thr Val Ser
   195 200 205
   Phe Pro Gly Ala Tyr Lys Ala Thr Asp Pro Gly Ile Leu Val Asn Ile
   210 215 220
   His Ala Pro Leu Ser Gly Tyr Thr Val Pro Gly Pro Ala Val Tyr Ser
   225 230 235 240
   Gly Gly Ser Thr Lys Lys Ala Gly Ser Ala Cys Thr Gly Cys Glu Ser
   245 250 255
   Thr Cys Ala Val Gly Ser Gly Pro Thr Ala Thr Val Ser Gln Ser Pro
   260 265 270
   Gly Ser Thr Ala Thr Ser Ala Pro Gly Gly Gly Gly Gly Cys Thr Val
   275 280 285
   Gln Lys Tyr Gln Gln Cys Gly Gly Gln Gly Tyr Thr Gly Cys Thr Asn
   290 295 300
   Cys Ala Ser Gly Ser Thr Cys Ser Ala Val Ser Pro Pro Tyr Tyr Ser
   305 310 315 320
   Gln Cys Val
<210> 93
   <211> 305
   <212> PRT
   <213> Myceliophthora thermophila
<400> 93
   His Ala Thr Phe Gln Ala Leu Trp Val Asp Gly Val Asp Tyr Gly Ala
   1               5                   10                  15
   Gln Cys Ala Arg Leu Pro Ala Ser Asn Ser Pro Val Thr Asp Val Thr
               20                  25                  30
   Ser Asn Ala Ile Arg Cys Asn Ala Asn Pro Ser Pro Ala Arg Gly Lys
           35                  40                  45
   Cys Pro Val Lys Ala Gly Ser Thr Val Thr Val Glu Met His Gln Gln
       50                  55                  60
   Pro Gly Asp Arg Ser Cys Ser Ser Glu Ala Ile Gly Gly Ala His Tyr
   65                  70                  75                  80
   Gly Pro Val Met Val Tyr Met Ser Lys Val Ser Asp Ala Ala Ser Ala
                   85                  90                  95
   Asp Gly Ser Ser Gly Trp Phe Lys Val Phe Glu Asp Gly Trp Ala Lys
               100                 105                 110
   Asn Pro Ser Gly Gly Ser Gly Asp Asp Asp Tyr Trp Gly Thr Lys Asp
           115                 120                 125
   Leu Asn Ser Cys Cys Gly Lys Met Asn Val Lys Ile Pro Ala Asp Leu
       130                 135                 140
   Pro Ser Gly Asp Tyr Leu Leu Arg Ala Glu Ala Leu Ala Leu His Thr
   145                 150                 155                 160
   Ala Gly Ser Ala Gly Gly Ala Gln Phe Tyr Met Thr Cys Tyr Gln Leu
                   165                 170                 175
   Thr Val Thr Gly Ser Gly Ser Ala Ser Pro Pro Thr Val Ser Phe Pro
               180                 185                 190
   Gly Ala Tyr Lys Ala Thr Asp Pro Gly Ile Leu Val Asn Ile His Ala
           195                 200                 205
   Pro Leu Ser Gly Tyr Thr Val Pro Gly Pro Ala Val Tyr Ser Gly Gly
       210                 215                 220
   Ser Thr Lys Lys Ala Gly Ser Ala Cys Thr Gly Cys Glu Ser Thr Cys
   225                 230                 235                 240
   Ala Val Gly Ser Gly Pro Thr Ala Thr Val Ser Gln Ser Pro Gly Ser
                   245                 250                 255
   Thr Ala Thr Ser Ala Pro Gly Gly Gly Gly Gly Cys Thr Val Gln Lys
               260                 265                 270
   Tyr Gln Gln Cys Gly Gly Gln Gly Tyr Thr Gly Cys Thr Asn Cys Ala
           275                 280                 285
   Ser Gly Ser Thr Cys Ser Ala Val Ser Pro Pro Tyr Tyr Ser Gln Cys
       290                 295                 300
   Val
   305